# EUROPEAN PATENT APPLICATION

(11) **EP 2 891 656 A1**
(43) Date of publication of application: **08.07.2015**
(21) Application number: 13834015.3
(22) Date of filing: 30.08.2013
(51) Int. Cl.: C07D 401/04, A61K 31/4709, A61K 31/497, A61K 31/506, A61P 1/04, A61P 1/16, A61P 3/04, A61P 3/10, A61P 13/02, A61P 13/10, A61P 17/04, A61P 25/00, A61P 25/06, A61P 25/14, A61P 25/16, A61P 25/22

(54) **PYRIDINE DERIVATIVE AND MEDICINE**

(30) Priority: 30.08.2012 JP 2012190548; 28.09.2012 JP 2012215947
(71) Applicant: Nippon Shinyaku Co., Ltd., Kyoto-shi Kyoto 601-8550 (JP)
(72) Inventor: TSUJI, Takashi, Otsu-shi Shiga, 520-2102 (JP); SHIRAI, Masaaki, Kusatsu-shi Shiga 525-0055 (JP)
(74) Representative: Vossius & Partner Patentanwälte Rechtsanwälte mbB
(86) International application number: PCT/JP2013/073438
(87) International publication number: WO 2014/034898

(57) **Abstract**

The main purpose of the invention is to provide a novel pyridine derivative or a pharmaceutically acceptable salt thereof. Examples of the invention include a pyridine derivative represented by general formula [1], and a pharmaceutically acceptable salt thereof. This compound or a pharmaceutically acceptable salt thereof exhibits mGluR5 inhibitory activity, and can therefore be used as an agent for the prevention or treatment of, e.g., pain (for example, acute pain, chronic pain, inflammatory pain, neuropathic pain, hyperalgesia, thermal hyperalgesia, allodynia, pain due to noxious thermal stimulation, pain due to noxious mechanical stimulation, pain in the lower urinary tract or reproductive organs, or migraine), pruritus, lower urinary tract symptoms or lower urinary tract dysfunctions, gastroesophageal reflux disease (GERD), gastroesophageal reflux associated with transient lower esophageal sphincter relaxation (TLESR), and diseases of the central nervous system.

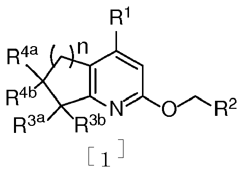

## Description

### [Field of the Invention]

The present invention relates to a novel pyridine derivative.

### [Background Art]

L-Glutamic acid is a principal excitatory neurotransmitter and plays an important role in a living body. Glutamate receptors are classified into two major groups. The first group is ionotropic glutamate receptors (iGluRs) and is composed of three subtypes, NMDA-type glutamate receptor, AMPA-type glutamate receptor, and kainate receptor. The second group is metabotropic glutamate receptors (mGluRs), which are G protein-coupled receptors (GPCR). As for mGluRs, eight subtypes are known at present (mGluR1 to mGluR8).

The eight subtypes of the metabotropic glutamate receptors are classified into three groups on the basis of homology of gene sequence, intracellular signaling pathway, and pharmacological characteristics. mGluR1 and mGluR5 belong to Group I which is coupled with Gq/11, and activation of these receptors induces intracellular Ca²⁺ mobilization. mGluR2 and mGluR3 belong to Group II, and mGluR4, mGluR6, mGlusR7, and mGluR8 belong to Group III. Each of them is coupled with Gi/o, and hence, activation of these receptors decrease intracellular cAMP.

In the nervous system, mGluR5 is expressed in a peripheral nervous system and a central nervous system (see Non-Patent Literature 1) and glial cells or the like (see Non-Patent Literature 2), and it is considered to positively regulate an excitatory synaptic transmission, e.g. by inducing a release of glutamic acid and other neurotransmitters from a presynaptic terminal (see Non-Patent Literature 3). For that reason, it is considered that an mGluR5 inhibitor will act to lower the excitation of a peripheral nerve and central nerve. In view of the fact that there are a lot of pathologies or diseases related to the excitation of a peripheral nerve or central nerve or glutamate neurotransmission, it is considered that the mGluR5 inhibitor may work as a therapeutic agent for these diseases.

### [Prior Art Literatures]

[Patent Literatures]
[Patent Literature 1] JP2012-131829

### [Non-Patent Literatures]

[Non-Patent Literature 1] Neuroscience Research vol. 28, 49-57, 1997.
[Non-Patent Literature 2] Cell vol. 146, 785-798, 2011.
[Non-Patent Literature 3] Neuroscience Letters vol. 361 220-224, 2004.
[Non-Patent Literature 4] Neuropharmacology, vol. 40, 10-19, 2001.
[Non-Patent Literature 5] Expert Opin. Ther. Targets, vol.6(3), 349-361, 2002.
[Non-Patent Literature 6] Psychopharmacology, vol. 179, 207-217, 2005.
[Non-Patent Literature 7] Current Drug Targets-CNS & Neurological Disorders, vol. 1, 283-296, 2002.
[Non-Patent Literature 8] Expert Opin Investig Drugs, vol. 19(4), 555-561, 2010.
[Non-Patent Literature 9] Molecular Pain, vol. 8, 20, 2012.
[Non-Patent Literature 10] J. Physiol., vol. 589(23), 5833-5843, 2011
[Non-Patent Literature 11] Pharmacol. Rev., vol. 63, 35-58, 2011.
[Non-Patent Literature 12] Neuroscience Letters, vol. 450, 12-17, 2009.
[Non-Patent Literature 13] J. Neural Transm., vol. 118, 1703-1716, 2011.
[Non-Patent Literature 14] Neuropharmacology, vol. 44, 562-572, 2003.
[Non-Patent Literature 15] J. Neural. Transm., vol. 115, 1609-1619, 2008.
[Non-Patent Literature 16] PLoS Biol. Vol. 5(3), e52, March 2007.
[Non-Patent Literature 17] J. Physiol., 586(6), 1503-1508, 2008.
[Non-Patent Literature 18] CNS Neurol Disord Drug Targets, vol. 8(6), 475-491, 2009.
[Non-Patent Literature 19] Brain Res., vol. 1019(1-2), 246-254, 2004.
[Non-Patent Literature 20] Neuroscience Letters, vol. 420, 155-159, 2007.
[Non-Patent Literature 21] Neuropsychopharmacology, vol. 29, 921-928, 2004.
[Non-Patent Literature 22] Psychopharmacology vol. 225, 151-159, 2013.
[Non-Patent Literature 23] J. Pharmacol. Exp. Ther., vol.313, 395-402, 2005.
[Non-Patent Literature 24] Biochimie., Vol. 94(11), 2366-2375, 2012.
[Non-Patent Literature 25] BJU INTERNATIONAL, vol. 102, 890-898, 2008
[Non-Patent Literature 26] L.W.CROCK, et al., "MGluR5 is necessary for the full expression of both inflammatory and non-inflammatory bladder pain" [online], [retrieved November 13], 2011, Neuroscience 2011, Program No. 180.08/Poster No. 0021, [retrieved August 21, 2013], searchable on the Internet <URL:http://www.abstractsonline.com/Plan/ViewAbstract.aspx? mID=2773&sKey=e4f6098c-83a5-4f51-9d69-2ec8dd0c4e29&cKey=389 c9d2c-777b-4237-a079-925c7e9ed77d&mKey=8334be29-8911-4991-8 c31-32b32dd5e6c8>

### [Summary of the Invention]

### [Problem to be solved by the invention]

A main object of the present invention is to provide a novel pyridine derivative or a pharmaceutically acceptable salt thereof. Another main object of the present invention is to provide a pharmaceutical composition comprising the pyridine derivative or a pharmaceutically acceptable salt thereof as an active ingredient.

### [Means for solving the problem]

The present inventors found that a novel pyridine derivative or a pharmaceutically acceptable salt thereof, which is described below, has an excellent mGluR5 inhibitory activity, and thus completed the present invention.

The present invention includes the following.
(A) A pyridine derivative represented by the following general formula [1] (hereinafter referred to as "the compound of the present invention") or a pharmaceutically acceptable salt thereof: 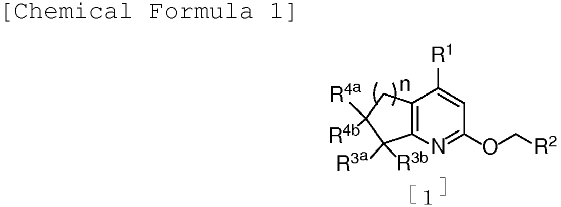 [wherein
   R¹ represents phenyl, benzo[d][1,3]dioxolyl, or heteroaryl;
   the heteroaryl relative to R¹ is bound through a carbon atom on the ring;
   the phenyl, benzo[d][1,3]dioxolyl, or heteroaryl relative to R¹ may be substituted at a substitutable arbitrary position(s) with one or three same or different groups selected from the group consisting of
      (i) halogen,
      (ii) cyano,
      (iii) hydroxy,
      (iv) nitro,
      (v) alkoxy,
      (vi) cycloalkyl,
      (vii) alkylsulfonate,
      (viii) alkyl,
      (ix) hydroxyalkyl,
      (x) alkoxyalkyl,
      (xi) monohalogenoalkyl,
      (xii) dihalogenoalkyl,
      (xiii) trihalogenoalkyl,
      (xiv) amino which may be substituted at a substitutable arbitrary position(s) with one or two same or different groups selected from the group consisting of alkyl, aminoalkyl, alkoxycarbonylaminoalkyl, monoalkylaminoalkyl, dialkylaminoalkyl, and alkylsulfonyl,
      (xv) saturated cyclic amino which may be substituted at a substitutable arbitrary position(s) with one or two oxos,
      (xvi) alkylcarbonyl,
      (xvii) alkoxycarbonyl,
      (xviii) hydroxycarbonyl,
      (xix) carbamoyl which may be substituted at a substitutable arbitrary position(s) with one or two same or different groups selected from the group consisting of alkyl, cycloalkyl, and (cycloalkyl)alkyl, and
      (xx) a group represented by the following general formula [2]: 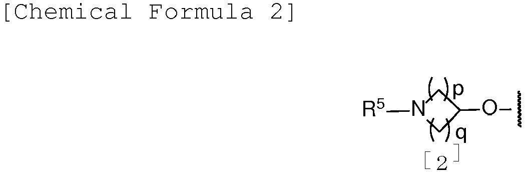 (wherein R⁵ represents hydrogen, alkyl, or alkylcarbonyl, and p and q may be the same as or different from each other and each represents 1 or 2); and
         when it contains nitrogen atom as a ring constituting atom in the heteroaryl relative to R¹, oxygen atom may coordinate to nitrogen atom;
         R² represents phenyl or heteroaryl and is bound through a carbon atom on the ring;
         the phenyl or heteroaryl relative to R² may be substituted at a substitutable arbitrary position(s) with one or two same or different groups selected from the group consisting of cyano, halogen, cycloalkyl, alkoxy, carbamoyl, alkenyl, alkyl, hydroxyalkyl, alkoxyalkyl, monohalogenoalkyl, dihalogenoalkyl, trihalogenoalkyl, amino, monoalkylamino, and dialkylamino; and
         when it contains nitrogen atom as a ring constituting atom in the heteroaryl relative to R², oxygen atom may coordinate to nitrogen atom;
         R^{3a} represents hydrogen, and R^{3b} represents hydrogen, alkyl, hydroxy, halogen, alkoxy, or alkylcarbonyloxy, or R^{3a} and R^{3b} taken together with the adjacent carbon atom represent a group represented by the following general formula [3] or [4]: 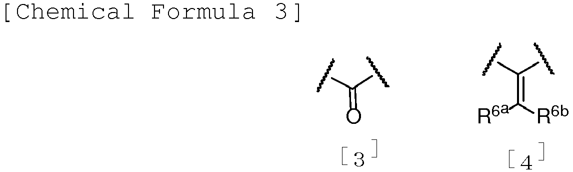 (wherein R^{6a} and R^{6b} may be the same as or different from each other and each represents hydrogen or alkyl);
         R^{4a} and R^{4b} may be the same as or different from each other and each represents hydrogen or alkyl; and
         n represents an integer of 1 to 3.]
(B) In the above general formula [I], the pyridine derivative or a pharmaceutically acceptable salt thereof according to (A), wherein R¹ is phenyl or heteroaryl.
(C) In the above general formula [I], the pyridine derivative or a pharmaceutically acceptable salt thereof according to (A), wherein R¹ is phenyl, pyridyl, pyrimidyl, pyrazinyl, or pyridazinyl.
(D) In the above general formula [I], the pyridine derivative or a pharmaceutically acceptable salt thereof according to (A), wherein R¹ is phenyl, pyridyl, pyrimidyl, pyrazinyl, or pyridazinyl, and R¹ may be substituted with one or two groups selected from the group consisting of alkyl, halogen, cyano, amino, monoalkylamino, dialkylamino, hydroxyalkyl, and alkylsulfonate.
(E) In the above general formula [I], the pyridine derivative or a pharmaceutically acceptable salt thereof according to (A), wherein R² is phenyl, pyridyl, pyrazinyl, pyrimidyl, oxazolyl, imidazolyl, or thiazolyl.
(F) In the above general formula [I], the pyridine derivative or a pharmaceutically acceptable salt thereof according to (A), wherein R² is phenyl, pyridyl, pyrazinyl, pyrimidyl, oxazolyl, imidazolyl, or thiazolyl, and R² may be substituted with one or two groups selected from the group consisting of alkyl, hydroxyalkyl, alkoxyalkyl, halogen, and cyano.
(G) In the above general formula [I], the pyridine derivative or a pharmaceutically acceptable salt thereof according to (A), wherein R^{3a} is hydrogen, and R^{3b} is hydrogen, alkyl, halogen, hydroxy, or alkoxy.
(H) In the above general formula [I], the pyridine derivative or a pharmaceutically acceptable salt thereof according to (A), wherein R^{3a}, R^{3b}, R^{4a}, and R^{4b} are each hydrogen.
(I) In the above general formula [I], the pyridine derivative or a pharmaceutically acceptable salt thereof according to (A), wherein R¹ is phenyl, pyridyl, pyrimidyl, pyrazinyl, or pyridazinyl; R² is phenyl, pyridyl, pyrazinyl, pyrimidyl, oxazolyl, imidazolyl, or thiazolyl; and R^{3a}, R^{3b}, R^{4a}, and R^{4b} are each hydrogen.
(J) In the above general formula [I], the pyridine derivative or a pharmaceutically acceptable salt thereof according to (A), wherein R¹ is phenyl, pyridyl, pyrimidyl, pyrazinyl, or pyridazinyl, and R¹ may be substituted with one or two groups selected from the group consisting of alkyl, halogen, cyano, amino, monoalkylamino, dialkylamino, hydroxyalkyl, and alkylsulfonate; R² is phenyl, pyridyl, pyrazinyl, pyrimidyl, oxazolyl, imidazolyl, or thiazolyl, and R² may be substituted with one or two gruops selected from the group consisting of alkyl, hydroxyalkyl, alkoxyalkyl, halogen, and cyano; and R^{3a}, R^{3b}, R^{4a}, and R^{4b} are each hydrogen.

Further, among the present invention, the compounds as defined by the according to any one of the following [1] to [95], or pharmaceutically acceptable salts thereof, are preferred.
(1)
   4-(pyridin-3-yl)-2-(pyridin-2-ylmethoxy)-6,7-dihydr o-5H-cyclopenta[b]pyridine,
(2)
   3-[2-(pyridin-2-ylmethoxy)-6,7-dihydro-5H-cyclopent a[b]pyridin-4-yl]pyridine-2-carbonitrile,
(3)
   5-[2-(pyridin-2-ylmethoxy)-6,7-dihydro-5H-cyclopent a[b]pyridin-4-yl]pyridin-3-yl methanesulfonate,
(4)
   5-{2-[(5-fluoropyridin-2-yl)methoxy]-6,7-dihydro-5H -cyclopenta[b]pyridin-4-yl}pyridine-2-carbonitrile,
(5)
   2-(benzyloxy)-4-(pyrimidin-5-yl)-6,7-dihydro-5H-cyc lopenta[b]pyridine,
(6)
   3-({[4-(pyrimidin-5-yl)-6,7-dihydro-5H-cyclopenta[b ]pyridin-2-yl]oxy}methyl)benzonitrile,
(7)
   2-[(3-fluoropyridin-2-yl)methoxy]-4-(pyrimidin-5-yl )-6,7-dihydro-5H-cyclopenta[b]pyridine,
(8)
   2-[(3,5-difluoropyridin-2-yl)methoxy]-4-(pyrimidin-5-yl)-6,7-dihydro-5H-cyclopenta[b]pyridine,
(9)
   2-[(6-methylpyridin-3-yl)methoxy]-4-(pyrimidin-5-yl )-6,7-dihydro-5H-cyclopenta[b]pyridine,
(10)
   4-(2-methylpyrimidin-5-yl)-2-(pyridin-2-ylmethoxy)-6,7-dihydro-5H-cyclopenta[b]pyridine,
(11)
   5-[2-(pyridin-2-ylmethoxy)-6,7-dihydro-5H-cyclopent a[b]pyridin-4-yl]pyrimidine-2-carbonitrile,
(12)
   2-[(3-fluoropyridin-2-yl)methoxy]-4-(2-methylpyrimi din-5-yl)-6,7-dihydro-5H-cyclopenta[b]pyridine,
(13)
   2-[(3,5-difluoropyridin-2-yl)methoxy]-4-(2-methylpy rimidin-5-yl)-6,7-dihydro-5H-cyclopenta[b]pyridine,
(14)
   2-[(3,6-difluoropyridin-2-yl)methoxy]-4-(2-methylpy rimidin-5-yl)-6,7-dihydro-5H-cyclopenta[b]pyridine,
(15)
   5-{2-[(3,5-difluoropyridin-2-yl)methoxy]-6,7-dihydr o-5H-cyclopenta[b]pyridin-4-yl}pyrimidine-2-carboni trile,
(16)
   2-[(6-methylpyridin-2-yl)methoxy]-4-(2-methylpyrimi din-5-yl)-6,7-dihydro-5H-cyclopenta[b]pyridine,
(17)
   6-({[4-(2-methylpyrimidin-5-yl)-6,7-dihydro-5H-cycl openta[b]pyridin-2-yl]oxy}methyl)pyridine-2-carboni trile,
(18)
   6-({[4-(2-methylpyrimidin-5-yl)-6,7-dihydro-5H-cycl openta[b]pyridin-2-yl]oxy}methyl)pyridine-3-carboni trile,
(19)
   5-{2-[(3-fluoropyridin-2-yl)methoxy]-6,7-dihydro-5H -cyclopenta[b]pyridin-4-yl}pyrimidine-2-carbonitril e,
(20)
   2-fluoro-4-[2-(pyridin-2-ylmethoxy)-6,7-dihydro-5H-cyclopenta[b]pyridin-4-yl]benzonitrile,
(21)
   3-fluoro-5-[2-(pyridin-2-ylmethoxy)-6,7-dihydro-5H-cyclopenta[b]pyridin-4-yl]phenyl methanesulfonate,
(22)
   4-(pyridin-3-yl)-2-(pyrimidin-2-ylmethoxy)-5,6,7,8-tetrahydroquinoline,
(23) 2-[(4-methyl-1,3-oxazol-2-yl)methoxy]-4-(pyridin-3-yl)-5,6,7,8-tetrahydroquinoline,
(24) 4-(pyridin-3-yl)-2-(1,3-thiazol-2-ylmethoxy)-5,6,7, 8-tetrahydroquinoline,
(25)
   2-[(1-methyl-1H-imidazol-2-yl)methoxy]-4-(pyridin-3 -yl)-5,6,7,8-tetrahydroquinoline,
(26)
   4-(4-methylpyridin-3-yl)-2-(pyridin-2-ylmethoxy)-5, 6,7,8-tetrahydroquinoline,
(27)
   5-[2-(pyridin-2-ylmethoxy)-5,6,7,8-tetrahydroquinol in-4-yl]pyridine-2-carbonitrile,
(28)
   {5-[2-(pyridin-2-ylmethoxy)-5,6,7,8-tetrahydroquino lin-4-yl]pyridin-3-yl}methanol,
(29)
   N-methyl-5-[2-(pyridin-2-ylmethoxy)-5,6,7,8-tetrahy droquinolin-4-yl]pyridin-3-amine,
(30)
   N,N-dimethyl-5-[2-(pyridin-2-ylmethoxy)-5,6,7,8-tet rahydroquinolin-4-yl]pyridin-3-amine,
(31)
   5-{2-[(3-fluoropyridin-2-yl)methoxy]-5,6,7,8-tetrah ydroquinolin-4-yl}pyridine-2-carbonitrile,
(32)
   2-(pyridin-2-ylmethoxy)-4-(pyrimidin-5-yl)-5,6,7,8-tetrahydroquinoline,
(33) 2-[(3-fluoropyridin-2-yl)methoxy]-4-(pyrimidin-5-yl )-5,6,7,8-tetrahydroquinoline,
(34) 2-[(3,6-difluoropyridin-2-yl)methoxy]-4-(pyrimidin-5-yl)-5,6,7,8-tetrahydroquinoline,
(35) 6-({[4-(pyrimidin-5-yl)-5,6,7,8-tetrahydroquinolin-2-yl]oxy}methyl)pyridine-2-carbontrile,
(36) 2-[(6-methylpyridin-2-yl)methoxy]-4-(pyrimidin-5-yl )-5,6,7,8-tetrahydroquinoline,
(37)
   [6-({[4-(pyrimidin-5-yl)-5,6,7,8-tetrahydroquinolin -2-yl]oxy}methyl)pyridin-2-yl]methanol,
(38)
   5-[2-(pyridin-2-ylmethoxy)-5,6,7,8-tetrahydroquinol in-4-yl]pyrimidin-2-amine,
(39)
   5-[2-(pyridin-2-ylmethoxy)-5,6,7,8-tetrahydroquinol in-4-yl]pyrimidine-2-carbonitrile,
(40)
   2-[(6-methylpyridin-2-yl)methoxy]-4-(2-methylpyrimi din-5-yl)-5,6,7,8-tetrahydroquinoline,
(41)
   6-({[4-(2-methylpyrimidin-5-yl)-5,6,7,8-tetrahydroq uinolin-2-yl]oxy}methyl)pyridine-2-carbonitrile,
(42)
   6-({[4-(2-methylpyrimidin-5-yl)-5,6,7,8-tetrahydroq uinolin-2-yl]oxy}methyl)pyridine-3-carbonitrile,
(43)
   2-[(5-fluoropyridin-2-yl)methoxy]-4-(2-methylpyrimi din-5-yl)-5,6,7,8-tetrahydroquinoline,
(44)
   2-[(3-fluoropyridin-2-yl)methoxy]-4-(2-methylpyrimi din-5-yl)-5,6,7,8-tetrahydroquinoline,
(45)
   2-[(3,5-difluoropyridin-2-yl)methoxy]-4-(2-methylpy rimidin-5-yl)-5,6,7,8-tetrahydroquinoline,
(46)
   5-{2-[(3-fluoropyridin-2-yl)methoxy]-5,6,7,8-tetrah ydroquinolin-4-yl}pyrimidin-2-amine,
(47)
   3-[2-(pyridin-2-ylmethoxy)-5,6,7,8-tetrahydroquinol in-4-yl]pyrazine-2-carbonitrile,
(48)
   (6-{2-[(5-fluoropyridin-2-yl)methoxy]-5,6,7,8-tetra hydroquinolin-4-yl}pyrazin-2-yl)methanol,
(49)
   (6-{2-[(3-fluoropyridin-2-yl)methoxy]-5,6,7,8-tetra hydroquinolin-4-yl}pyrazin-2-yl)methanol,
(50)
   7-ethyl-2-(pyridin-2-ylmethoxy)-4-(pyrimidin-5-yl)-6,7-dihydro-5H-cyclopenta[b]pyridine,
(51)
   3-({[4-(pyridin-3-yl)-6,7-dihydro-5H-cyclopenta[b]p yridin-2-yl]oxy}methyl)benzonitrile,
(52)
   5-{2-[(3,5-difluoropyridin-2-yl)methoxy]-6,7-dihydr o-5H-cyclopenta[b]pyridin-4-yl}pyridine-2-carbonitr ile,
(53)
   4-[({4-[5-(hydroxymethyl)pyridin-3-yl]-6,7-dihydro-5H-cyclopenta[b]pyridin-2-yl}oxy)methyl]benzonitril e,
(54)
   (5-{2-[(4-fluorobenzyl)oxy]-6,7-dihydro-5H-cyclopen ta[b]pyridin-4-yl}pyridin-3-yl)methanol,
(55)
   (5-{2-[(3-fluorobenzyl)oxy]-6,7-dihydro-5H-cyclopen ta[b]pyridin-4-yl}pyridin-3-yl)methanol,
(56)
   6-({[4-(5-fluoropyridin-3-yl)-6,7-dihydro-5H-cyclop enta[b]pyridin-2-yl]oxy}methyl)pyridine-3-carbonitr ile,
(57)
   3-({[4-(2-fluoropyridin-4-yl)-6,7-dihydro-5H-cyclop enta[b]pyridin-2-yl]oxy}methyl)benzonitrile,
(58)
   6-({[4-(2-fluoropyridin-4-yl)-6,7-dihydro-5H-cyclop enta[b]pyridin-2-yl]oxy}methyl)pyridine-3-carbonitr ile,
(59)
   3-[({4-[5-(hydroxymethyl)pyridin-3-yl]-6,7-dihydro-5H-cyclopenta[b]pyridin-2-yl}oxy)methyl]benzonitril e,
(60)
   6-({[4-(pyrimidin-5-yl)-6,7-dihydro-5H-cyclopenta[b ]pyridin-2-yl]oxy}methyl)pyridine-3-carbonitrile,
(61)
   2-[(3,6-difluoropyridin-2-yl)methoxy]-4-(pyrimidin-5-yl)-6,7-dihydro-5H-cyclopenta[b]pyridine,
(62)
   2-[(2-methylpyridin-4-yl)methoxy]-4-(pyrimidin-5-yl )-6,7-dihydro-5H-cyclopenta[b]pyridine,
(63)
   2-[(4-fluorobenzyl)oxy]-4-(pyrimidin-5-yl)-6,7-dihy dro-5H-cyclopenta[b]pyridine,
(64)
   4-({[4-(pyrimidin-5-yl)-6,7-dihydro-5H-cyclopenta[b ]pyridin-2-yl]oxy}methyl)benzonitrile,
(65)
   5-[2-(pyridin-2-ylmethoxy)-6,7-dihydro-5H-cyclopent a[b]pyridin-4-yl]pyrimidin-2-amine,
(66)
   2-[(4-fluorobenzyl)oxy]-4-(2-methylpyrimidin-5-yl)-6,7-dihydro-5H-cyclopenta[b]pyridine,
(67)
   4-({[4-(2-methylpyrimidin-5-yl)-6,7-dihydro-5H-cycl openta[b]pyridin-2-yl]oxy}methyl)benzonitrile,
(68)
   2-[(2-methylpyridin-4-yl)methoxy]-4-(2-methylpyrimi din-5-yl)-6,7-dihydro-5H-cyclopenta[b]pyridine,
(69)
   5-{2-[(3-fluorobenzyl)oxy]-6,7-dihydro-5H-cyclopent a[b]pyridin-4-yl}pyrimidine-2-amine,
(70)
   4-({[4-(2-aminopyrimidin-5-yl)-6,7-dihydro-5H-cyclo penta[b]pyridin-2-yl]oxy}methyl)benzonitrile,
(71)
   3-({[4-(2-aminopyrimidin-5-yl)-6,7-dihydro-5H-cyclo penta[b]pyridin-2-yl]oxy}methyl)benzonitrile,
(72)
   2-[(3-fluorobenzyl)oxy]-4-(2-methylpyrimidin-5-yl)-6,7-dihydro-5H-cyclopenta[b]pyridine,
(73)
   2-({[4-(2-methylpyrimidin-5-yl)-6,7-dihydro-5H-cycl openta[b]pyridin-2-yl]oxy}methyl)-1,3-oxazole-4-car bonitrile,
(74)
   {6-[2-(pyridin-2-ylmethoxy)-6,7-dihydro-5H-cyclopen ta[b]pyridin-4-yl]pyrazin-2-yl}methanol,
(75)
   (6-{2-[(5-fluoropyridin-2-yl)methoxy]-6,7-dihydro-5 H-cyclopenta[b]pyridin-4-yl}pyrazin-2-yl)methanol,
(76)
   3-[({4-[6-(hydroxymethyl)pyrazin-2-yl]-6,7-dihydro-5H-cyclopenta[b]pyridin-2-yl}oxy)methyl]benzonitril e,
(77)
   4-(pyridazin-3-yl)-2-(pyridin-2-ylmethoxy)-6,7-dihy dro-5H-cyclopenta[b]pyridine,
(78)
   2-[(3-fluoropyridin-2-yl)methoxy]-4-(pyridazin-3-yl )-6,7-dihydro-5H-cyclopenta[b]pyridine,
(79)
   4-({[4-(pyridazin-3-yl)-6,7-dihydro-5H-cyclopenta[b ]pyridin-2-yl]oxy}methyl)benzonitrile,
(80)
   6-({[4-(1-methyl-1H-pyrazol-5-yl)-6,7-dihydro-5H-cy clopenta[b]pyridin-2-yl]oxy}methyl)pyridine-3-carbo nitrile,
(81)
   6-({[4-(pyridazin-3-yl)-6,7-dihydro-5H-cyclopenta[b ]pyridin-2-yl]oxy}methyl)pyridine-3-carbonitrile,
(82)
   6-[({4-[5-(hydroxymethyl)pyridin-3-yl]-5,6,7,8-tetr ahydroquinolin-2-yl}oxy)methyl]pyridine-3-carbonitr ile,
(83)
   2-[(3,5-difluoropyridin-2-yl)methoxy]-4-(pyrimidin-5-yl)-5,6,7,8-tetrahydroquinoline,
(84)
   4-({[4-(pyrimidin-5-yl)-5,6,7,8-tetrahydroquinolin-2-yl]oxy}methyl)benzonitrile,
(85)
   3-({[4-(pyrimidin-5-yl)-5,6,7,8-tetrahydroquinolin-2-yl]oxy}methyl)benzonitrile,
(86)
   2-[(4-fluorobenzyl)oxy]-4-(pyrimidin-5-yl)-5,6,7,8 - tetrahydroquinoline,
(87)
   2-[(3-fluorobenzyl)oxy]-4-(pyrimidin-5-yl)-5,6,7,8 - tetrahydroquinoline,
(88)
   4-({[4-(pyrimidin-5-yl)-5,6,7,8-tetrahydroquinolin-2-yl]oxy}methyl)pyridine-2-carbonitrile,
(89)
   5-{2-[(6-fluoropyridin-2-yl)methoxy]-5,6,7,8-tetrah ydroquinolin-4-yl}pyrimidin-2-amine,
(90)
   3-({[4-(2-methylpyrimidin-5-yl)-5,6,7,8-tetrahydroq uinolin-2-yl]oxy}methyl)benzonitrile,
(91)
   4-({[4-(2-methylpyrimidin-5-yl)-5,6,7,8-tetrahydroq uinolin-2-yl]oxy}methyl)benzonitrile,
(92)
   2-[(2-methylpyridin-4-yl)methoxy]-4-(2-methylpyrimi din-5-yl)-5,6,7,8-tetrahydroquinoline,
(93)
   4-({[4-(2-methylpyrimidin-5-yl)-5,6,7,8-tetrahydroq uinolin-2-yl]oxy}methyl)pyridine-2-carbonitrile,
(94)
   6-[({4-[6-(hydroxymethyl)pyrazin-2-yl]-5,6,7,8-tetr ahydroquinolin-2-yl}oxy)methyl]pyridine-3-carbonitr ile, and
(95)
   4-(pyridazin-3-yl)-2-(pyridin-2-ylmethoxy)-5,6,7,8-tetrahydroquinoline.

Specific terms used in this specification are hereunder described in detail.

Examples of "halogen" include fluorine, chlorine, bromine, and iodine.

"Alkyl" may be a linear or branched one having 1 to 8 carbon atoms, and specific examples thereof may include methyl, ethyl, n-propyl, isopropyl, n-butyl, isobutyl, sec-butyl, tert-butyl, n-pentyl, isopentyl, n-hexyl, isohexyl, n-heptyl, isoheptyl, and n-octyl. Among these, an alkyl having 1 to 6 carbon atoms is preferred, and an alkyl having 1 to 3 carbon atoms is more preferred.

Examples of an alkyl moiety in "alkylsulfonate", "alkylsulfonyl", "aminoalkyl", "monoalkylaminoalkyl", "dialkylaminoalkyl", "(cycloalkyl)alkyl", "alkylcarbonyl", "alkylcarbonyloxy", "alkoxycarbonylaminoalkyl", "hydroxyalkyl", "alkoxyalkyl", "monohalogenoalkyl", "dihalogenoalkyl", "trihalogenoalkyl", "monoalkylamino", or "dialkylamino" may include the same as those described above for the "alkyl".

"Alkoxy" may be a linear or branched one having 1 to 8 carbon atoms, and specific examples thereof may include methoxy, ethoxy, n-propoxy, isopropoxy, n-butoxy, isobutoxy, sec-butoxy, tert-butoxy, n-pentyloxy, n-hexyloxy, n-heptyloxy, and n-octyloxy.

Examples of an alkoxy moiety in "alkoxycarbonyl", "alkoxycarbonylaminoalkyl", or "alkoxyalkyl" may include the same as those described above for the "alkoxy".

Examples of "heteroaryl" may include a monocyclic or bicyclic, 5- to 10-membered aromatic heterocyclic group having 1 to 3 heteroatoms selected from the group consisting of nitrogen atom, oxygen atom, and sulfur atom as a ring constituting atom. Among these, a monocyclic or bicyclic, 5- to 10-membered aromatic heterocyclic group which contains at least one nitrogen atom as a ring constituting atom and which may further have one or two heteroatoms selected from the group consisting of nitrogen atom, oxygen atom, and sulfur atom is preferred. Specific examples thereof may include furyl (for example, 2-furyl and 3-furyl), thienyl (for example, 2-thienyl and 3-thienyl), pyrrolyl (for example, 1-pyrrolyl, 2-pyrrolyl, and 3-pyrrolyl), imidazolyl (for example, 2-imidazolyl and 4-imidazolyl), pyrazolyl (for example, 3-pyrazolyl and 4-pyrazolyl), triazolyl (for example, 1,2,4-triazol-3-yl and 1,2,4-triazol-4-yl), tetrazolyl (for example, 2-tetrazolyl and 5-tetrazolyl), oxazolyl (for example, 2-oxazolyl, 4-oxazolyl, and 5-oxazolyl), thiazolyl (for example, 2-thiazolyl, 4-thiazolyl, and 5-thiazolyl), pyridyl (for example, 2-pyridyl, 3-pyridyl, and 4-pyridyl), pyridazinyl (for example, 3-pyridazinyl and 4-pyridazinyl), pyrimidinyl (for example, 2-pyrimidinyl, 4-pyrimidinyl, and 5-pyrimidinyl), pyrazinyl (for example, 2-pyrazinyl), benzothiazolyl (for example, benzothiazol-2-yl, benzothiazol-4-yl, benzothiazol-5-yl, benzothiazol-6-yl, and benzothiazol-7-yl), indolyl (for example, indol-3-yl, indol-4-yl, indol-5-yl, indol-6-yl, and indol-7-yl), benzothiophenyl (for example, 1-benzothiophen-2-yl, 1-benzothiophen-3-yl, 1-benzothiophenyl-4-yl, 1-benzothiophen-5-yl, 1-benzothiophen-6-yl, and 1-benzothiophen-7-yl), quinolyl (for example, quinolin-2-yl, quinolin-3-yl, quinolin-4-yl, quinoline-5-yl, quinolin-6-yl, quinolin-7-yl, and quinolin-8-yl), benzo[d]imidazolyl (for example, benzo[d]imidazol-2-yl, benzo[d]imidazol-4-yl, benzo[d]imidazol-5-yl, benzo[d]imidazol-6-yl, and benzo[d]imidazol-7-yl), imidazo[1,2-a]pyridyl (for example, imidazo[1,2-a]pyridin-2-yl, imidazo[1,2-a]pyridin-3-yl, imidazo[1,2-a]pyridin-5-yl, imidazo[1,2-a]pyridin-6-yl, imidazo[1,2-a]pyridin-7-yl, and imidazo[1,2-a]pyridin-8-yl), imidazo[1,2-b]pyridazinyl (for example, imidazo[1,2-b]pyridazin-2-yl, imidazo[1,2-b]pyridazin-3-yl, imidazo[1,2-b]pyridazin-6-yl, imidazo[1,2-b]pyridazin-7-yl, and imidazo[1,2-b]pyridazin-8-yl), isoxazolyl (for example, 3-isoxazolyl, 4-isoxazolyl, and 5-isoxazolyl), and isothiazolyl (for example, 3-isothiazolyl, 4-isothiazolyl, and 5-isothiazolyl). In addition, in such a heteroaryl containing nitrogen atom as a ring constituting atom, oxygen atom may coordinate to the nitrogen atom.

Examples of "benzo[d][1,3]dioxolyl" may include benzo[d][1,3]dioxol-4-yl and benzo[d][1,3]dioxol-5-yl.

Examples of "saturated cyclic amino" may include 4- to 7-membered saturated cyclic amino having one or two nitrogen atoms, which may have one oxygen atom or sulfur atom as a ring constituting atom. Specific examples thereof may include 1-azetidinyl, 1-pyrrolidinyl, 1-imidazolidinyl, piperidino, 1-piperazinyl, 1-tetrahydropyrimidinyl, morpholino, and thiomorpholino. In addition, the saturated cyclic amino may be substituted with one or two oxos, and examples of the saturated cyclic amino substituted with one or two oxos may include (thiomorpholine 1,1-dioxide)-4-yl and 2-oxo-azetidin-1-yl.

"Cycloalkyl" may be one having 3 to 8 carbon atoms, and specific examples thereof may include cyclopropyl, cyclobutyl, cyclopentyl, cyclohexyl, cycloheptyl, and cyclooctyl.

Examples of a cycloalkyl moiety of the "(cycloalkyl)alkyl" may include the same as those described above for the "cycloalkyl".

Examples of the "alkenyl" may include a linear on branched alkenyl having 2 to 6 carbon atoms. Specific examples thereof may include vinyl, allyl, butenyl, and hexenyl.

### [Mode for Carrying Out the Invention]

The compound of the present invention can be produced according to, for example, the following method, the Examples as described later, or a known method from a known compound or an intermediate which can be easily synthesized. In the production of the compound of the present invention, in the case where a starting material has a substituent which affects a reaction, the reaction is generally performed after the starting material is protected with an appropriate protective group according to a known method in advance. The protective group can be removed by a known method after the reaction.

### Production Method 1: Production method (1) of a compound represented by the following general formula [1A] (hereinafter referred to as "compound [1A]"), which is the compound of the present invention represented by the general formula [1], wherein R^{3a} is hydrogen, and R^{3b} is hydrogen, alkyl, halogen, or alkoxy

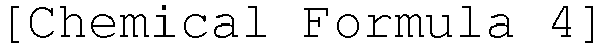

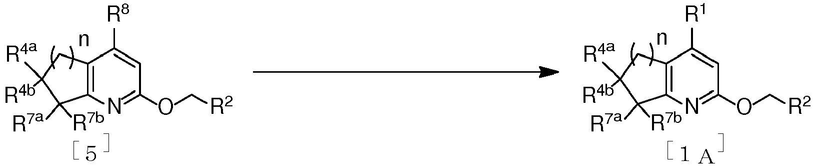

(R¹, R², R^{4a}, R^{4b}, and n mean the same as described above. R^{7a} represents hydrogen, and R^{7b} represents hydrogen, alkyl, halogen, or alkoxy. R⁸ represents halogen, trifluoromethanesulfonate, or a group represented by the following general formula [6] (hereinafter referred to as "substituent [6]"). 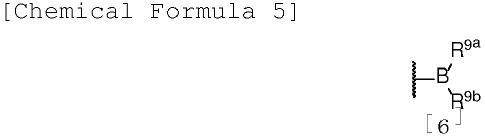 (R^{9a} and R^{9b} each represent hydroxy, or R^{9a} and R^{9b} together represent -O-C(CH₃)₂-C(CH₃)₂-O-, -O-(CH₂)₃-O-, or -O-CH₂-C(CH₃)₂-CH₂-O-.))

This reaction is a cross-coupling reaction of a compound represented by the foregoing general formula [5] (hereinafter referred to as "compound [5]") with (i) a compound represented by the following general formula [7A] (hereinafter referred to as "compound [7A]"), which is commercially available or may be produced by a known method, (ii) a compound represented by the following general formula [7B] (hereinafter referred to as "compound [7B]"), or (iii) a compound represented by the following general formula [8] (hereinafter referred to as "compound [8]"), which is commercially available or may be produced by a known method, using a palladium catalyst, and this reaction can be, for example, performed in the presence of an appropriate base and/or an inorganic salt in an appropriate solvent. 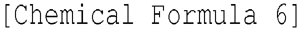 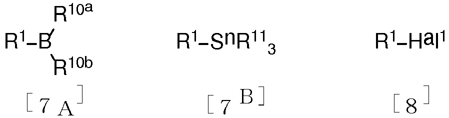 (R¹ means the same as described above. R^{10a} and R^{10b} each represents hydroxy, or R^{10a} and R^{10b} together represent -O-C(CH₃)₂-C(CH₃)₂-O-, -O-(CH₂)₃-O-, or -O-CH₂-C(CH₃)₂-CH₂-O-. R¹¹ represents methyl or n-butyl. Hal¹ represents halogen.)

In detail, the compound [1A] can be produced by a cross-coupling reaction of the compound [5], wherein R⁸ is halogen or trifluoromethanesulfonate, with the compound [7A] or compound [7B], or the compound [5], wherein R⁸ is the substituent [6], and the compound [8]. In addition, if desired, a ligand may be added, and a microwave reaction apparatus (for example, a microwave synthesis system "Initiator" (available from Biotage Japan Ltd.)) may be used.

An amount of the compound [7A], compound [7B], or compound [8] to be used is suitably in a range of, for example, 1 to 3-fold molar amount with respect to the compound [5]. Examples of the usable palladium catalyst may include a tris(dibenzylideneacetone)bispalladium chloroform adduct (hereinafter referred to as "Pd₂(dba)₃·CHCl₃"), tris(dibenzylideneacetone)bispalladium (hereinafter referred to as "Pd₂(dba)₃"), tetrakistriphenylphosphine palladium (hereinafter referred to as "Pd(PPh₃)₄"), a [1,1'-bis(diphenylphosphino)ferrocene]-dichloropall adium(II) dichloromethane adduct (hereinafter referred to as "Pd(dppf)Cl₂·CH₂Cl₂"), bis(triphenylphosphine)palladium(II) dichloride (hereinafter referred to as "PdCl₂(PPh₃)₂"), [1,1'-bis(di-tert-butylphosphino)ferrocene]palladiu m(II) dichloride (hereinafter referred to as "Pd(dtbpf)Cl₂"), bis(tricyclohexylphosphine)palladium(II) dichloride (hereinafter referred to as "PdCl₂ (PCy₃)₂"), and palladium acetate (hereinafter referred to as "Pd (OAc)₂"). An amount of such a palladium catalyst is suitably in a range of, for example, 0.01 to 0.3-fold molar amount with respect to the compound [5]. Examples of the usable ligand may include 1,1'-bis(diphenylphosphino)ferrocene (hereinafter referred to as "dppf"), 4,5-bis(diphenylphosphino)-9,9-dimethylxanthene (hereinafter referred to as "Xantphos"), 2-dicyclohexylphosphino-2',4',6'-triisopropylbiphen yl (hereinafter referred to as "X-Phos"), 2,2'-bis(diphenylphosphino)-1,1'-binaphthyl (hereinafter referred to as "BINAP"), 2-dicyclohexylphosphino-2',6'-dimethoxybiphenyl (hereinafter referred to as "S-Phos"), 9,9-dimethyl-4,5-bis[di(tert-butylphosphino)]xanthe ne (hereinafter referred to as "t-Bu-X-Phos"), 2-(di-tert-butylphosphino)biphenyl, bis[2-(diphenylphosphino)phenyl] ether (hereinafter referred to as "DPEphos"), tri-tert-butylphosphine, and tricyclohexylphosphine. An amount of such a ligand is suitably in a range of, for example, 1 to 5-fold molar amount with respect to the palladium catalyst. Examples of the usable base may include inorganic bases such as sodium tert-butoxide (hereinafter referred to as "NaO-t-Bu"), potassium tert-butoxide (hereinafter referred to as "KO-t-Bu"), sodium carbonate, sodium hydrogen carbonate, potassium carbonate, tripotassium phosphate, cesium carbonate, and potassium acetate. An amount of such a base is suitably in a range of, for example, 1 to 3-fold molar amount with respect to the compound [5]. Examples of the usual inorganic salt may include lithium chloride (hereinafter referred to as "LiCl") and cesium fluoride. An amount of such an inorganic salt is suitably in a range of, for example, 1 to 3-fold molar amount with respect to the compound [5]. Although the usable solvent is not particularly limited so long as it does not participate in the reaction, examples thereof include hydrocarbons such as toluene and xylene; ethers such as 1,4-dioxane, tetrahydrofuran (hereinafter referred to as "THF"), and 1,2-dimethoxyethane (hereinafter referred to as "DME"); amides such as N,N-dimethylformamide (hereinafter referred to as "DMF"), N,N-dimethylacetamide (hereinafter referred to as "DMA"), and N-methylpyrrolidone (hereinafter referred to as "NMP"); alcohols such as ethanol and propanol; water; and mixed solvents thereof. A reaction temperature is suitably in a range of 20°C to 200°C. Although a reaction time varies depending on the type of a starting material to be used, the reaction temperature, or the like, in general, it is suitably in a range of 10 minutes to 24 hours.

Incidentally, the compound [5] that is a starting material can be, for example, produced according to the following Production Method A of Compound [5] to Production Method C of Compound [5].

### Production Method A of Compound [5] : Production method (1) of a compound represented by the following general formula [5Aa] (hereinafter referred to as "compound [5Aa]"), which is the compound [5], wherein R⁸ is halogen

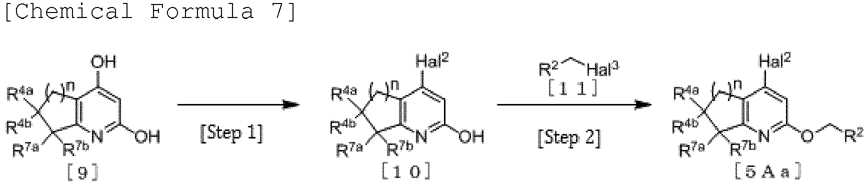

(R², R^{4a}, R^{4b}, R^{7a}, R^{7b}, and n mean the same as described above. Hal² and Hal³ each represents halogen.)

### Step 1

This reaction is a monohalogenation reaction of a compound represented by the foregoing general formula [9] (hereinafter referred to as "compound [9]"), and a compound represented by the foregoing general formula [10] (hereinafter referred to as "compound [10]") can be, for example, produced by allowing the compound [9] to react with a halogenating agent according to a method which is known itself as a halogenation reaction. In general, this reaction can be performed without using a solvent or in an appropriate solvent at a temperature ranging from 20°C to 200°C. In addition, if desired, a base may be added.

Examples of the usable halogenating agent may include phosphorus oxychloride, phosphorus oxybromide, phosphorus pentachloride, and phosphorus tribromide. An amount of such a halogenating agent is preferably in a range of, for example, 1 to 3-fold molar amount with respect to the compound [9]. Examples of the usable base may include organic bases such as N,N-dimethylaniline, N,N-diethylaniline, triethylamine (hereinafter referred to as "NEt₃"), and diisopropylethylamine (hereinafter referred to as "DIPEA"). Although the usable solvent is not particularly limited so long as it does not participate in the reaction, examples thereof may include hydrocarbons such as toluene, xylene, and benzene; ethers such as 1,4-dioxane and DME; and acetonitrile (hereinafter referred to as "MeCN"). A reaction temperature is preferably in a range of 20°C to 100°C. Although a reaction time varies depending on the type of a starting material to be used or the reaction temperature, in general, it is suitably in a range of 1 hour to 24 hours.

### Step 2

The compound [5Aa] can be produced by allowing the compound [10] to react with a compound represented by the foregoing general formula [11] (hereinafter referred to as "compound [11]"), which is commercially available or can be produced by a known method. This reaction can be performed in the presence of an appropriate base in an appropriate solvent at a temperature ranging from 0°C to 200°C. In addition, in the case of performing this reaction in the presence of a base, if desired, an additive may be added.

An amount of the compound [11] to be used is suitably in a range of, for example, 1 to 3-fold molar amount with respect to the compound [10]. Examples of the usable base include silver carbonate, potassium carbonate, cesium carbonate, potassium iodide, and sodium hydride. An amount of such a base is suitably in a range of, for example, 1 to 5-fold molar amount with respect to the compound [10]. Examples of the usable additive may include tetra-n-butylammonium iodide, tetra-n-butylammonium bromide, and 18-crown-6-ether. An amount of such an additive is suitably in a range of, for example, 1 to 5-fold molar amount with respect to the compound [10]. Although the usable solvent is not particularly limited so long as it does not participate in the reaction, examples thereof may include hydrocarbons such as toluene and xylene; ethers such as diethyl ether (hereinafter referred to as "Et₂O"), THF, 1,4-dioxane, DME, and cyclopentylmethyl ether (hereinafter referred to as "CPME"); amides such as DMF, DMA, and NMP; MeCN; acetone; and mixed solvents thereof. Although a reaction time varies depending on the type of a starting material to be used, a reaction temperature, or the like, in general, it is suitably in a range of 1 hour to 24 hours.

Incidentally, the compound [9] that is a starting material can be, for example, produced according to the following production method.

### Production Method of Compound [9]

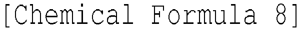

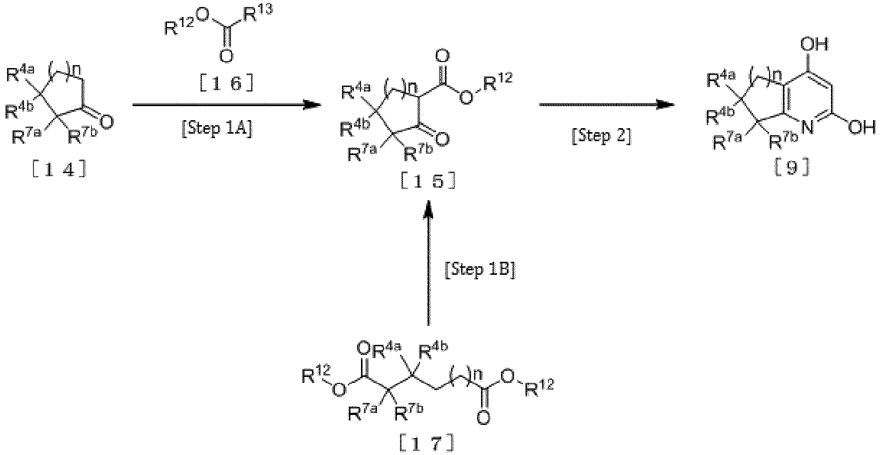

(R^{4a}, R^{4b}, R^{7a}, R^{7b}, and n mean the same as described above. R¹² represents alkyl. R¹³ represents OR¹² (R¹² means the same as described above) or cyano.)

### Step 1A

A compound represented by the foregoing general formula [15] (hereinafter referred to as "compound [15]") can be, for example, produced by allowing a compound represented by the foregoing general formula [14] (hereinafter referred to as "compound [14]"), which is commercially available or can be produced by a known method, to react with a compound represented by the foregoing general formula [16] (hereinafter referred to as "compound [16]"), which is commercially available or can be produced by a known method, according to a method described in Journal of Organic Chemistry, 1991, vol. 56, pp.6199-6205, US2005/38052, US2006/293364, or WO2009/47255.

### Step 1B

The compound [15] can be, for example, produced from a compound represented by the foregoing general formula [17] (hereinafter referred to as "compound [17]"), which is commercially available or may be produced by a known method, according to a method described in Organic Mass Spectrometry, 1988, vol. 23, pp.719-722, Canadian Journal of Chemistry, 1997, vol. 75, pp.965-974, or WO2006/35061.

### Step 2

The compound [9] can be, for example, produced from the compound [15] according to a method described in Helvetica Chimica Acta, 1945, vol. 28, pp.1684-1690 or Helvetica Chimica Acta, 1944, vol. 27, pp.1854-1858.

### Production Method B of Compound [5] : Production method (2) of the compound [5Aa], which is the compound [5], wherein R⁸ is halogen

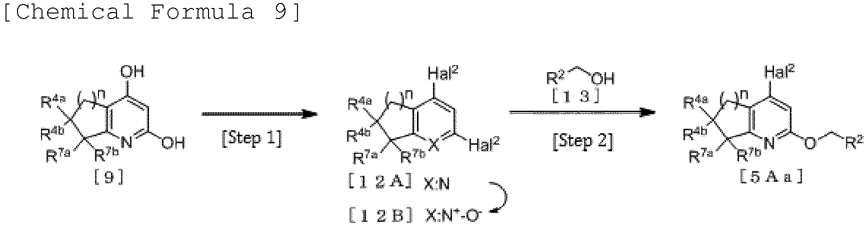

(R², R^{4a}, R^{4b}, R^{7a}, R^{7b}, Hal² and n mean the same as described above. X represents N or N⁺-O⁻.)

### Step 1

This reaction is a dihalogenation reaction of the compound [9], and a compound represented by the foregoing general formula [12A] (hereinafter referred to as "compound [12A]") can be, for example, produced by allowing the compound [9] to react with a halogenating agent according to a method which is known itself as a halogenation reaction. In general, this reaction can be performed without using a solvent or in an appropriate solvent at a temperature ranging from 20°C to 200°C. In addition, if desired, a base may be added.

Examples of the usable halogenating agent may include phosphorus oxychloride, phosphorus oxybromide, phosphorus pentachloride, and phosphorus tribromide. An amount of such a halogenating agent is preferably in a range of, for example, 1 to 3-fold molar amount with respect to the compound [9]. Examples of the usable base may include organic bases such as N,N-dimethylaniline, N,N-diethylaniline, NEt₃, and DIPEA. Although the usable solvent is not particularly limited so long as it does not participate in the reaction, examples thereof may include hydrocarbons such as toluene, xylene, and benzene; ethers such as 1,4-dioxane and dimethoxyethane; and MeCN. A reaction temperature is preferably in a range of 100°C to 200°C. Although a reaction time varies depending on the type of a starting material to be used or the reaction temperature, in general, it is suitably in a range of 1 hour to 24 hours.

Incidentally, a compound represented by the foregoing general formula [12B] (hereinafter referred to as "compound [12B]") can be produced by allowing the compound [12A] to react with an oxidizing agent. In general, this reaction can be performed in an appropriate solvent in the presence of an oxidizing agent, for example, 3-chloroperbenzoic acid (hereinafter referred to as "m-CPBA") or hydrogen peroxide, at a temperature ranging from 0°C to 100°C. In addition, if desired, an additive may be added.

Examples of the usable additive may include sodium hydrogen carbonate and trifluoroacetic anhydride (hereinafter referred to as "TFAA"). Although the usable solvent is not particularly limited so long as it does not participate in the reaction, examples thereof may include hydrocarbons such as toluene, xylene, and benzene; ethers such as 1,4-dioxane and DME; halogenated hydrocarbons such as dichloromethane (hereinafter referred to as "CH₂Cl₂") and chloroform; MeCN; water; and mixed solvents thereof. Although a reaction time varies depending on the type of a starting material to be used or the reaction temperature, in general, it is suitably in a range of 1 hour to 24 hours.

### Step 2

The compound [5Aa] can be produced by allowing the compound [12A] to react with a compound represented by the foregoing general formula [13] (hereinafter referred to as "compound [13]"), which is commercially available or can be produced by a known method. In general, this reaction can be performed in the presence of an appropriate base in an appropriate solvent at a temperature ranging from 0°C to 200°C.

An amount of the compound [13] to be used is suitably in a range of, for example, 1 to 3-fold molar amount with respect to the compound [12A]. Examples of the usable base may include NaO-t-Bu, KO-t-Bu, potassium carbonate, cesium carbonate, sodium hydroxide, and sodium hydride. An amount of such a base is suitably in a range of, for example, 1 to 5-fold molar amount with respect to the compound [12A]. Although the usable solvent is not particularly limited so long as it does not participate in the reaction, examples thereof may include hydrocarbons such as toluene and xylene; ethers such as Et₂O, THF, 1,4-dioxane, and DME; and amides such as DMF, DMA, and NMP. Although a reaction time varies depending on the type of a starting material to be used, the reaction temperature, or the like, in general, it is suitably in a range of 1 to 24 hours.

In addition, the compound [5Aa] can be produced by allowing the compound [12B] to react with the compound [13] which is commercially available or may be produced by a known method in the presence of an appropriate base in an appropriate solvent at a temperature ranging from 0°C to 200°C, followed by further performing a reduction reaction. In addition, if desired, an additive may be added.

An amount of the compound [13] to be used is suitably in a range of, for example, 1 to 3-fold molar amount with respect to the compound [12B]. Examples of the usable base may include NaO-t-Bu, KO-t-Bu, potassium carbonate, cesium carbonate, sodium hydroxide, and sodium hydride. An amount of such a base is suitably in a range of, for example, 1 to 5-fold molar amount with respect to the compound [12B]. Although the usable solvent is not particularly limited so long as it does not participate in the reaction, examples thereof may include halogenated hydrocarbons such as CH₂Cl₂ and chloroform; hydrocarbons such as hexane, toluene, and xylene; ethers such as Et₂O, THF, 1,4-dioxane, and DME; and amides such as DMF, DMA, and NMP. Examples of a usable reducing agent include phosphorus trichloride, phosphorus tribromide, lithium aluminum hydride, sodium borohydride (hereinafter referred to as "NaBH₄"), zinc, and iron. An amount of such a reducing agent is suitably in a range of, for example, 1 to 5-fold molar amount with respect to the compound [12B]. Examples of the usable additive include ammonium chloride and acetic acid. Although a reaction time varies depending on the type of a starting material to be used, the reaction temperature, or the like, in general, it is suitably in a range of 1 hour to 24 hours.

Production Method C of Compound [5] : Production method of a compound represented by the following general formula [5Ab] (hereinafter referred to as "compound [5Ab]"), which is the compound [5] wherein R⁸ is trifluoromethanesulfonate, and a compound represented by the following general formula [5B] (hereinafter referred to as "compound [5B]"), which is the compound [5], wherein R⁸ is the substituent [6] 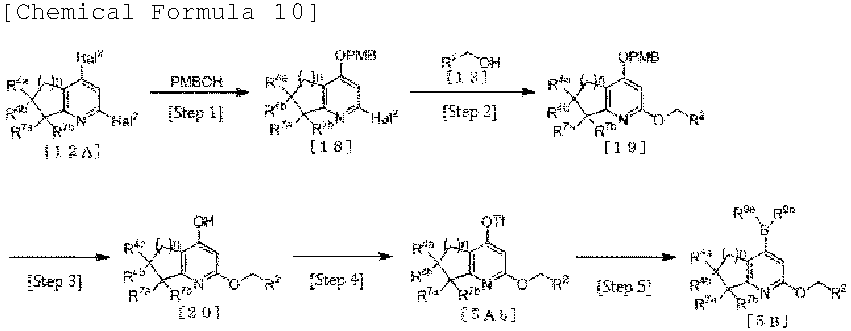 (R², R^{4a}, R^{4b}, R^{7a}, R^{7b}, R^{9a}, R^{9b}, Hal², and n mean the same as described above. PMB represents p-methoxybenzyl, and Tf represents trifluoromethanesulfonyl.)

### Step 1

A compound represented by the foregoing general formula [18] (hereinafter referred to as "compound [18]") can be produced by allowing the compound [12A] to react with p-methoxybenzyl alcohol. Therefore, this reaction can be performed according to a method which is known itself as a p-methoxybenzyl alcoholation reaction. This reaction can be performed in the presence of p-methoxybenzyl alcohol in an appropriate solvent using a base such as sodium hydride at a temperature ranging from 0°C to 150°C. In addition, if desired, for example, 15-crown-5-ether may be added.

An amount of the p-methoxybenzyl alcohol to be used is suitably in a range of, for example, 1 to 2-fold molar amount with respect to the compound [12A]. Although the usable solvent is not particularly limited so long as it does not participate in the reaction, examples thereof may include hydrocarbons such as toluene and xylene; ethers such as Et₂O, THF, 1,4-dioxane, and DME; and amides such as DMF, DMA, and NMP. Although a reaction time varies depending on the type of a starting material to be used, the reaction temperature, or the like, in general, it is suitably in a range of 1 hour to 24 hours.

### Step 2

A compound [19] can be produced by allowing the compound [18] to react with the compound [13] which is commercially available or can be produced by a known method.

In general, this reaction can be, for example, performed in the presence of a palladium catalyst and an appropriate base or inorganic salt in an appropriate solvent by optionally adding a ligand at a temperature ranging from 20°C to 200°C. In addition, if desired, this reaction can be performed using a microwave reaction apparatus.

An amount of the compound [13] to be used is suitably in a range of, for example, 1 to 3-fold molar amount with respect to the compound [18]. Examples of the usable palladium catalyst may include Pd₂(dba)₃·CHCl₃, Pd₂(dba)₃, Pd(PPh₃)₄, Pd(dppf)Cl₂·CH₂Cl₂, PdCl₂(PPh₃)₂, Pd(dtbpf)Cl₂, PdCl₂(PCy₃)₂, and Pd(OAc)₂. An amount of such a palladium catalyst is suitably in a range of, for example, 0.01 to 0.3-fold molar amount with respect to the compound [18]. Examples of the usable ligand may include dppf, Xantphos, X-Phos, BINAP, S-Phos, t-Bu-X-Phos, 2-(di-tert-butylphosphino)biphenyl, DPEphos, tri-tert-butylphosphine, and tricyclohexylphosphine. An amount of such a ligand is suitably in a range of, for example, 1 to 5-fold molar amount with respect to the palladium catalyst. Examples of the usable base may include inorganic bases such as NaO-t-Bu, KO-t-Bu, sodium carbonate, sodium hydrogen carbonate, potassium carbonate, tripotassium phosphate, cesium carbonate, and potassium acetate. An amount of such a base is suitably in a range of, for example, 1 to 3-fold molar amount with respect to the compound [18]. Examples of the usable inorganic salt may include LiCl and cesium fluoride. An amount of such an inorganic salt is suitably in a range of, for example, 1 to 3-fold molar amount with respect to the compound [18]. Although the usable solvent is not particularly limited so long as it does not participate in the reaction, examples thereof may include hydrocarbons such as toluene and xylene; ethers such as 1,4-dioxane, THF, and DMF; amides such as DMF, DMA, and NMP; alcohols such as ethanol and propanol; water; and mixed solvents thereof. Although a reaction time varies depending on the type of a starting material to be used, the reaction temperature, or the like, in general, it is suitably in a range of 1 hour to 24 hours.

### Step 3

A compound represented by the foregoing general formula [20] (hereinafter referred to as "compound [20]") can be produced by deprotection of p-methoxybenzyl of the compound [19]. Therefore, this reaction can be performed according to a known method which is known itself as the deprotection of p-methoxybenzyl. This reaction can be performed in the presence of a deprotecting agent of p-methoxybenzyl in an appropriate solvent at a temperature ranging from 0°C to 100°C.

Examples of the usable deprotecting agent of p-methoxybenzyl may include trifluoroacetic acid (hereinafter referred to as "TFA"), hydrochloric acid, AMBERLYST-15, cerium(IV) ammonium nitrate, and iodine. Although the usable solvent is not particularly limited so long as it does not participate in the reaction, examples thereof may include halogenated hydrocarbons such as CH₂Cl₂ and chloroform; hydrocarbons such as toluene and xylene; ethers such as Et₂O, THF, 1,4-dioxane, and DME; alcohols such as methanol, ethanol, and propanol; MeCN; water; and mixed solvents thereof. Although a reaction time varies depending on the type of a starting material to be used, the reaction temperature, or the like, in general, it is suitably in a range of 1 to 24 hours.

### Step 4

The compound [5Ab] can be produced by allowing the compound [20] to react with a trifluoromethanesulfonylatingagent. Therefore, this reaction can be performed according to a method which is known itself as a trifluoromethanesulfonylation reaction. In general, this reaction is performed in an appropriate solvent in the presence of a base at -78°C to 20°C.

Examples of the usable trifluoromethanesulfonylating agent may include trifluoromethanesulfonic anhydride (hereinafter referred to as "Tf₂O") and N-phenylbis(trifluoromethanesulfonimide) (hereinafter referred to as "Tf₂NPh"). Examples of the usable base may include Et₃N, pyridine, DIPEA, 2,6-lutidine, N,N-dimethylaminopyridine (hereinafter referred to as "DMAP"), and mixtures thereof. Although the usable solvent is not particularly limited so long as it does not participate in the reaction, for example, halogenated hydrocarbons such as CH₂Cl₂ and chloroform are preferred. Although a reaction time varies depending on the type of a starting material to be used, the reaction temperature, or the like, in general, it is suitably in a range of 1 to 24 hours.

### Step 5

The compound [5B] can be produced by subjecting the compound [5Ab] to boration. Therefore, this reaction can be performed according to a method which is known itself as a boration reaction. In general, this reaction can be performed in the presence of a palladium catalyst and an appropriate base or inorganic salt in an appropriate solvent by optionally adding a ligand at a temperature ranging from 20°C to 200°C. In addition, if desired, this reaction can be performed using a microwave reaction apparatus.

Examples of the usable borating agent include bis(pinacolato)diboron, pinacol borane, and the like. An amount of such a borating agent is suitably in a range of, for example, 1 to 2-fold molar amount with respect to the compound [5Ab]. Examples of the usable palladium catalyst may include Pd₂(dba)₃·CHCl₃, Pd₂(dba)₃, Pd(PPh₃)₄, Pd (dppf) Cl₂·CH₂Cl₂, PdCl₂(PPh₃)₂, Pd(dtbpf)Cl₂, PdCl₂(PCy₃)₂, and Pd(OAc)₂. An amount of such a palladium catalyst is suitably in a range of, for example, 0.01 to 0.3-fold molar amount with respect to the compound [5Ab]. Examples of the usable ligand may include dppf, Xantphos, X-Phos, BINAP, S-Phos, t-Bu-X-Phos, 2-(di-tert-butylphosphino)biphenyl, DPEphos, tri-tert-butylphosphine, and tricyclohexylphosphine. An amount of such a ligand is suitably in a range of, for example, 1 to 5-fold molar amount with respect to the palladium catalyst. Examples of the usable base may include inorganic bases such as potassium carbonate, tripotassium phosphate, cesium carbonate, and potassium acetate; and organic bases such as NEt₃ and DIPEA. An amount of such a base is suitably in a range of, for example, 1 to 3-fold molar amount with respect to the compound [5Ab]. Examples of the usable inorganic salt may include LiCl and cesium fluoride. An amount of such an inorganic salt is suitably in a range of, for example, 1 to 3-fold molar amount with respect to the compound [5Ab]. Although the usable solvent is not particularly limited so long as it does not participate in the reaction, examples thereof may include halogenated hydrocarbons such as 1,2-dichloroethane and chloroform; hydrocarbons such as toluene and xylene; ethers such as 1,4-dioxane, THF, and DME; amides such as DMF, DMA, and NMP; alcohols such as ethanol and propanol; water; and mixed solvents thereof. Although a reaction time varies depending on the type of a starting material to be used, the reaction temperature, or the like, in general, it is suitably in a range of 1 hour to 24 hours.

Production Method 2: Production method (2) of the compound [1A] 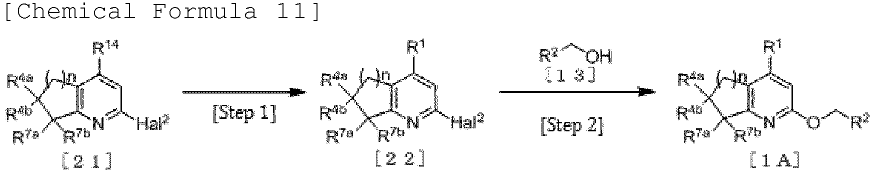 (R¹, R^{4a}, R^{4b}, R^{7a}, R^{7b}, Hal², and n mean the same as described above. R¹⁴ represents halogen, trifluoromethanesulfonate, or the substituent [6].)

### Step 1

This reaction is a cross-coupling reaction of a compound represented by the foregoing general formula [21] (hereinafter referred to as "compound [21]") with the compound [7A], the compound [7B], or the compound [8] using a palladium catalyst, and this reaction can be, for example, performed in the presence of an appropriate base and/or inorganic salt in an appropriate solvent.

A compound represented by the foregoing general formula [22] (hereinafter referred to as "compound [22] ") can be, for example, produced by subjecting the compound [21] wherein R¹⁴ is halogen or trifluoromethanesulfonate and the compound [7A] or compound [7B], or the compound [21] wherein R¹⁴ is the substituent [6] and the compound [8], to a cross-coupling reaction. In addition, a microwave reaction apparatus may be used.

An amount of the compound [7A], compound [7B], or compound [8] to be used is suitably in a range of, for example, 1 to 3-fold molar amount with respect to the compound [21]. Examples of the usable palladium catalyst may include Pd₂(dba)₃·CHCl₃, Pd₂(dba)₃, Pd(PPh₃)₄, Pd(dppf)Cl₂·CH₂Cl₂, PdCl₂(PPh₃)₂, Pd(dtbpf)Cl₂, PdCl₂(PCy₃)₂, and Pd(OAc)₂. An amount of such a palladium catalyst is suitably in a range of, for example, 0.01 to 0.3-fold molar amount with respect to the compound [21]. Examples of the usable base may include inorganic bases such as potassium carbonate, sodium carbonate, sodium hydrogen carbonate, tripotassium phosphate, cesium carbonate, and potassium acetate. An amount of such a base is suitably in a range of, for example, 1 to 3-fold molar amount with respect to the compound [21]. Examples of the usable inorganic salt may include LiCl and cesium fluoride. An amount of such an inorganic salt is suitably in a range of, for example, 1 to 3-fold molar amount with respect to the compound [21]. Although the usable solvent is not particularly limited so long as it does not participate in the reaction, examples thereof may include hydrocarbons such as toluene and xylene; ethers such as 1,4-dioxane, THF, and DME; amides such as DMF, DMA, and NMP; alcohols such as ethanol and propanol; MeCN; water; and mixed solvents thereof. A reaction temperature is suitably in a range of 20°C to 200°C. Although a reaction time varies depending on the type of a starting material to be used, the reaction temperature, or the like, in general, it is suitably in a range of 0.5 to 24 hours.

### Step 2

The compound [1A] can be, for example, produced by allowing the compound [22] to react with the compound [13] which is commercially available or can be produced by a known method according to the method described in Step 2 of Production Method C as described above.

Incidentally, the compound [21] that is a starting compound can be, for example, produced according to the following production method.
Production Method of Compound [21]: Production method of a compound represented by the following general formula [21A] (hereinafter referred to as "compound [21A]"), which is the compound [21], wherein R¹⁴ is trifluoromethanesulfonate, and a compound represented by the following general formula [21B] (hereinafter referred to as "compound [21B]"), which is the compound [21], wherein R¹⁴ is the substituent [6] 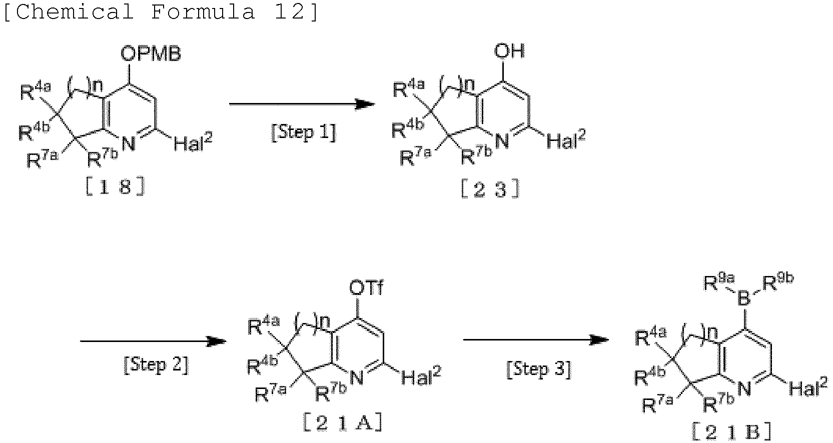 (R^{4a}, R^{4b}, R^{7a}, R^{7b}, R^{9a}, R^{9b}, Hal², PMB, Tf, and n mean the same as described above.)

### Step 1

This reaction is a p-methoxybenzyl deprotection reaction, and a compound represented by the foregoing general formula [23] (hereinafter referred to as "compound [23]") can be, for example, produced by deprotection of p-methoxybenzyl of the compound [18] according to the method described in Step 3 of Production Method C of the compound [5] as described above.

### Step 2

This reaction is a trifluoromethanesulfonylation reaction, and the compound [21A] can be, for example, produced by allowing the compound [23] to react with a trifluoromethanesulfonylating agent according to the method described in Step 4 of Production Method C of the compound [5] as described above.

### Step 3

This reaction is a boration reaction, and the compound [21B] can be, for example, produced by subjecting the compound [21A] to boration according to the method described in Step 5 of Production Method C of the compound [5] as described above.

Incidentally, the compound [21] wherein R¹⁴ is halogen can be produced according to the method described in Step 1 of Production Method B of the compound [5] as described above.
Production Method 3: Production method of a compound represented by the following general formula [1B] (hereinafter referred to as "compound [1B]"), which is the compound of the present invention represented by the general formula (1), wherein R^{3a} is hydrogen, and R^{3b} is alkylcarbonyloxy; a compound represented by the following general formula [1C] (hereinafter referred to as "compound [1C]"), which is the compound of the present invention represented by the general formula (1), wherein R^{3a} is hydrogen, and R^{3b} is hydroxy; a compound represented by the following general formula [1D] (hereinafter referred to as "compound [1D]"), which is the compound of the present invention represented by the general formula (1), wherein R^{3a} and R^{3b} taken together with the adjacent carbon atom represent the group [3]; and a compound represented by the following general formula [1E] (hereinafter referred to as "compound [1E]"), which is the compound of the present invention represented by the general formula (1), wherein R^{3a} and R^{3b} taken together with the adjacent carbon atom represent the group [4] 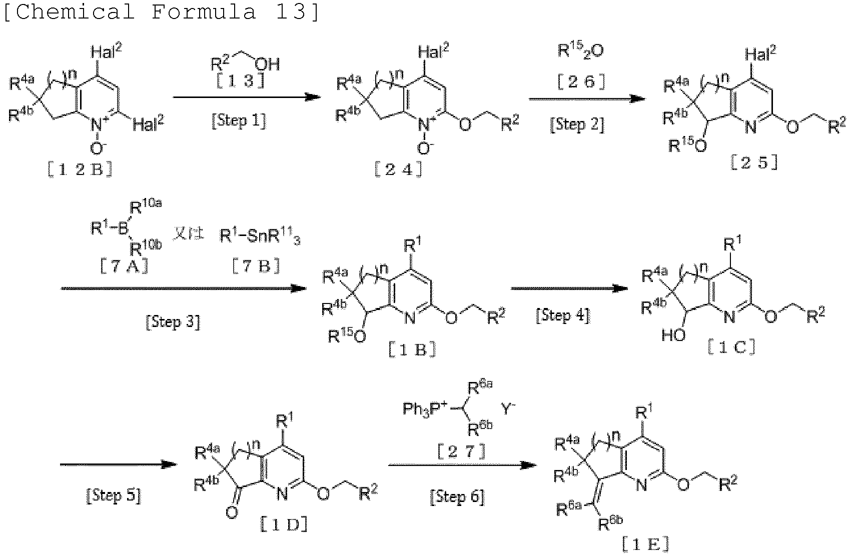 (R¹, R², R^{4a}, R^{4b}, R^{6a}, R^{6b}, R^{10a}, R^{10b}, R¹¹, Hal², and n mean the same as described above. R¹⁵ represents alkylcarbonyl. Ph represents phenyl. Y represents halogen.)

### Step 1

A compound represented by the foregoing general formula [24] (hereinafter referred to as "compound [24]") can be produced by allowing the compound [12B] to react with the compound [13] which is commercially available or can be produced according to a known method. In general, this reaction can be performed in the presence of an appropriate base in an appropriate solvent at a temperature ranging from 0°C to 200°C.

An amount of the compound [13] to be used is suitably in a range of, for example, 1 to 3-fold molar amount with respect to the compound [12B]. Examples of the usable base may include NaO-t-Bu, KO-t-Bu, potassium carbonate, cesium carbonate, sodium hydroxide, and sodium hydride. An amount of such a base is suitably in a range of, for example, 1 to 5-fold molar amount with respect to the compound [12B]. Although the usable solvent is not particularly limited so long as it does not participate in the reaction, examples thereof may include hydrocarbons such as toluene and xylene; ethers such as Et₂O, THF, 1,4-dioxane, and DME; and amides such as DMF, DMA, and NMP. Although a reaction time varies depending on the type of a starting material to be used, the reaction temperature, or the like, in general, it is suitably in a range of 1 to 24 hours.

### Step 2

A compound represented by the foregoing general formula [25] (hereinafter referred to as "compound [25]") can be produced by allowing the compound [24] to react with a compound represented by the foregoing general formula [26] (hereinafter referred to as "compound [26] ") : R152O (R15 means the same as described above). In general, this reaction can be performed in the presence of the compound [26] in an appropriate solvent at a temperature ranging from 20°C to 150°C. In addition, if desired, a base may be added.

Examples of the usable base may include sodium carbonate, sodium hydrogen carbonate, and sodium hydroxide. Although the usable solvent is not particularly limited so long as it does not participate in the reaction, examples thereof may include halogenated hydrocarbons such as CH2Cl2, 1,2-dichloroethane, and chloroform; hydrocarbons such as benzene, toluene, and xylene; ethers such as Et2O, THF, 1,4-dioxane, and DME; ethyl acetate; water; and mixed solvents thereof. Although a reaction time varies depending on the type of a starting material to be used, the reaction temperature, or the like, in general, it is suitably in a range of 0.5 to 24 hours.

### Step 3

This reaction is a cross-coupling reaction of the compound [25] with the compound [7A] or compound [7B] which is commercially available or can be produced by a known method using a palladium catalyst, and the compound [1B] can be, for example, produced according to the method described in Production Method 1 as described above.

### Step 4

This reaction is a alkylcarbonyl deprotection reaction, and the compound [1C] can be, for example, produced by allowing the compound [1B] to react with an appropriate base. In general, this reaction can be performed in the presence of an appropriate base in an appropriate solvent at a temperature ranging from 0°C to 100°C.

Examples of the usable base may include sodium hydroxide, potassium hydroxide, lithium hydroxide, potassium carbonate, sodium carbonate, ammonia, sodium methoxide, and potassium methoxide. Although the usable solvent is not particularly limited so long as it does not participate in the reaction, examples thereof may include halogenated hydrocarbons such as CH₂Cl₂ and chloroform; ethers such as Et₂O, THF, 1,4-dioxane, and DME; alcohols such as methanol, ethanol, and propanol; MeCN; water; and mixed solvents thereof. Although a reaction time varies depending on the type of a starting material to be used, the reaction temperature, or the like, in general, it is suitably in a range of 1 to 24 hours.

### Step 5

This reaction is an oxidation reaction of a secondary hydroxyl group, and the compound [1D] can be, for example, produced by allowing the compound [1C] to react with an oxidizing agent according to a method described in "Dai 4-han Jikken Kagaku Kouza, Vol. 23 (Oxidation Reaction)", Maruzen (1991), pp.37-78 and pp.299-346, edited by The Chemical Society of Japan; Tetrahedron, 1978, vol. 34, pp.1651-1660; or Tetrahedron Letters, 1994, vol. 35, pp.8019-8022.

Examples of the usable oxidizing agent may include Jones reagent, Sarett reagent, Collins reagent, and Dess-Martin reagent.

### Step 6

This reaction is the Wittig reaction, and the compound [1E] can be produced by allowing the compound [1D] to react with a compound represented by the foregoing general formula [27] which is commercially available or can be produced by a known method, according to a method described in Organic Reactions, 1965, vol. 14, p.270.

Although the compound of the present invention may be used as a medicinal agent as it is, it may also be used in a form of a pharmaceutically acceptable salt according to a known method. Examples of such a salt may include salts of mineral acids such as hydrochloric acid, hydrobromic acid, sulfuric acid, and phosphoric acid; salts of organic acids such as acetic acid, citric acid, oxalic acid, tartaric acid, maleic acid, succinic acid, fumaric acid, p-toluenesulfonic acid, benzenesulfonic acid, methanesulfonic acid, trifluoroacetic acid, and methanesulfonamide; salts of alkali metals or alkaline earth metals such as sodium, potassium, and calcium; and salts of organic amine compounds such as L-arginine, choline, L-lysine, t-butylamine, ethylenediamine, ammonia, dimethylaminoethanol, N-methylglucamine, tromethamine, and hydroxyethyl morpholine.

For example, a hydrochloride of the compound of the present invention can be obtained by dissolving the compound of the present invention in a solution of hydrogen chloride (hereinafter referred to as "HCl") in an alcohol, ethyl acetate, or Et₂O.

Although some of the compounds of the present invention have asymmetric carbon, all of their respective optical isomers and mixtures thereof fall within the scope of the present invention. For example, an optical isomer can be produced by optically resolving a racemate with an optically active acid (e.g., tartaric acid, dibenzoyltartaric acid, mandelic acid, 10-camphor sulfonic acid, etc.) while utilizing basicity thereof according to a known method, or by using, an optically active compound having been prepared in advance as a starting material. Besides, the optical isomer can also be produced by optical resolution using a chiral column or asymmetric synthesis.

In addition, among the compounds of the present invention, with respect to those which may form a tautomer, all of their respective tautomers and mixtures thereof fall within the scope of the present invention.

The compound of the present invention or a pharmaceutically acceptable salt thereof has mGluR5 inhibitory activity as shown in the Test Examples as described later.

Accordingly, the compound of the present invention or a pharmaceutically acceptable salt thereof can be used as a preventive agent or therapeutic agent for diseases in which mGluR5 participates.

Examples of a disease to which the compound of the present invention or a pharmaceutically acceptable salt thereof is applicable may include as follows:
(1) pain
(2) pruritus (see, for example, Patent Literature 1)
(3) a lower urinary tract symptom or lower urinary tract dysfunction
(4) gastroesophageal reflux disease (GERD) (see, for example, Non-Patent Literature 11) or gastroesophageal reflux disease associated with transient lower esophageal sphincter relaxation (TLESR) (see, for example, Non-Patent Literature 11),
(5) central nervous system disease
(6) drug-induced liver damage (see, for example, Non-Patent Literature 11)
(7) hypoxia-induced liver damage (see, for example, Non-Patent Literature 11)
(8) obesity (see, for example, Non-Patent Literature 23)
(9) diabetes (see, for example, Non-Patent Literature 11)
(10) oral squamous cell carcinoma (see, for example, Non-Patent Literature 11)
(11) liver cancer (see, for example, Non-Patent Literature 24)

Examples of "pain" may include acute pain (see, for example, Non-Patent Literature 7), chronic pain (see, for example, Non-Patent Literature 7), inflammatory pain (see, for example, Non-Patent Literatures 4 and 7), neuropathic pain (see, for example, Non-Patent Literatures 5 and 7), hyperalgesia (see, for example, Non-Patent Literatures 4 and 6), thermal hyperalgesia, allodynia (see, for example, Non-Patent Literature 6), pain by noxious thermal stimulation (see, for example, Non-Patent Literature 7), pain by noxious mechanical stimulation (see, for example, Non-Patent Literature 7), pain in the lower urinary tract or reproductive organs or migraine (see, for example, Non-Patent Literature 8).

Examples of "acute pain" may include herpetic pain, pain after tooth extraction, postoperative pain or a pain associated with ureteral calculus.

Examples of "chronic pain" may include cancer pain, pain associated with fibromyalgia, pain in complex regional pain syndrome or pain in postoperative scar pain syndrome.

Examples of "inflammatory pain" may include pain associated with rheumatoid arthritis or pain associated with osteoarthritis.

Examples of "neuropathic pain" may include trigeminal neuralgia, postherpetic neuralgia, pain associated with painful diabetic neuropathy or pain associated with carcinomatous neuropathy.

Examples of "pain in the lower urinary tract or reproductive organs" may include pain associated with bacterial cystitis (see, for example, Non-Patent Literatures 9, 12, 25 and 26), pain associated with interstitial cystitis (see, for example, Non-Patent Literatures 9, 12, 25 and 26), pain associated with cystitis, pain associated with acute prostatitis, pain associated with chronic prostatitis, pain associated with pelvic pain syndrome (see, for example, Non-Patent Literature 10), coital pain, bladder pain, urethral pain, vulvodynia, vaginal pain, scrotal pain, perineal pain or pelvic pain.

Examples of "central nervous system disease" may include L-dopa-induced dyskinesia (see, for example, Non-Patent Literatures 13 and 15), psychosis (see, for example, Non-Patent Literature 14), anxiety (see, for example, Non-Patent Literatures 6, 14 and 15), depression (see, for example, Non-Patent Literature 14), Alzheimer's disease (see, for example, Non-Patent Literature 16), fragile X syndrome (see, for example, Non-Patent Literature 17), Parkinson's disease, Huntington's disease (see, for example, Non-Patent Literature 18), morphine tolerance (see, for example, Non-Patent Literature 20), alcohol dependence (see, for example, Non-Patent Literature 21) or food addiction (see, for example, Non-Patent Literatures 9, 12, 25 and 22).

Examples of "lower urinary tract symptom or lower urinary tract dysfunction" may include frequent urination, diurnal frequency, nocturia, urinary urgency, urinary incontinence, bladder perception, symptom associated with pelvic organ prolapse, neurogenic bladder, lower urinary tract obstruction, discomfort in bladder, discomfort in lower urinary tract or discomfort in genital tract.

Examples of "disease accompanied by lower urinary tract symptom or lower urinary tract dysfunction" may include overactive bladder (see, for example, Non-Patent Literature 12), cystitis, urethritis, bacterial cystitis (see, for example, Non-Patent Literatures 9, 12, 25 and 26), interstitial cystitis (see, for example, Non-Patent Literatures 9, 12, 25 and 26), prostatic hyperplasia, prostate cancer, urolithiasis, acute prostatitis, chronic prostatitis, bladder tumor, poorly compliant bladder, pelvic organ prolapse, uterine fibroid, polyuria, stress urinary incontinence, cerebrovascular accident, Parkinson disease, multiple system atrophy, brain tumor, dementia, spinal cord injury, multiple sclerosis, Degenerative Myelopathy, spinal vascular disorder, spina bifida, peripheral neuropathy or pelvic pain syndrome (see, for example, Non-Patent Literature 10).

When administered as a medicinal agent, the compound of the present invention or a pharmaceutically acceptable salt thereof is administered as it is or as a pharmaceutical composition containing, for example, 0.001% to 99.5%, and preferably 0.1% to 90% thereof, in a pharmaceutically acceptable non-toxic and inactive carrier to a mammal including human.

A diluent, a bulking agent, and one or more of other formulation additives in the form of a solid, a semi-solid, or a liquid can be used as a carrier. It is desirable that the pharmaceutical composition of the present invention is administered in a unit dosage form. The pharmaceutical composition may be administered by intra-tissue administration, oral administration, intravenous administration, local administration (e.g., dermal administration, ocular instillation, intraperitoneal administration, intrathoracic administration, etc.), or transrectal administration. As a matter of course, the composition is administered in a dosage form suitable for these administration routes.

It is desirable that the dosage as a medicinal agent is adjusted while taking into consideration conditions of the patient such as age, body weight, nature, severity, and the like of the disease, route of administration, the compound of the present invention to be administered, whether or not the compound is a salt, the kind of the salt, and the like. For the oral administration, a daily dosage of the compound of the present invention or a pharmaceutically acceptable salt thereof as an active ingredient for adult is typically within the range of 10 mg to 3,000 mg, and preferably from 30 mg to 600 mg per adult. However, a dosage below the foregoing range may be sufficient in some cases, or a dosage above the foregoing range may be needed in other cases. In general, a daily dosage is administered once per day or may be administered by divided doses. Alternatively, a daily dosage can be administered intravenously by bolus administration or continuous infusion within 24 hours.

### [Example]

The present invention is hereunder described in more detail by reference to the following Examples, Test Examples, and Formulation Example, but it should not be construed that the present invention is limited thereto.

Incidentally, the measurement conditions for high-performance liquid chromatography mass spectrometry are as follows.
Analytical instrument: ACUITY UPLC MS/PDA System (available from Waters)
Mass spectrometer: Waters 3100 MS detector
Photodiode array detector: ACUITY PDA detector (UV detection wavelength: 210 to 400 nm)
Column: Acuity BEH C18, 1.7 µm, 2.1 x 50 mm
Flow rate: 0.5 mL/min
Column temperature: 40°C
Solvent:
Solvent A: 0.1% formic acid/water (v/v)
Solvent B: 0.1% formic acid/MeCN (v/v)

### Example 1

### 4-(Pyridin-3-yl)-2-(pyridin-2-ylmethoxy)-6,7-dihydr o-5H-cyclopenta[b]pyridine dihydrochloride

### [Step 1]

### Production of 4-chloro-2-(pyridin-2-ylmethoxy)-6,7-dihydro-5H-cyc lopenta[b]pyridine

Toluene (14 mL) was added to pyridine-2-ylmethanol (0.924 mL), followed by the addition of sodium hydride (60%, dispersion in oil, 640 mg) (hereinafter referred to as "NaH".) under ice water cooling and the mixture was stirred for 30 minutes. After the reaction mixture was allowed to return to room temperature, 2,4-dichloro-6,7-dihydro-5H-cyclopenta[b]pyridine (see for example, Helvetica Chimica Acta, 1945, vol.28, p.1684-1690) (2 g) was added, the reaction mixture was stirred at 170°C for 1 hour. After the reaction mixture was allowed to return to room temperature, filtered through Celite, and the filtrate was evaporated under reduced pressure. The resulting residue was purified by silica gel column chromatography to give the title compound (1.06 g) as a white solid.
[Rf value (TLC silica gel plate 60F₂₅₄, developing solvent; hexane : ethyl acetate =2:1) : 0.3]

### [Step 2]

### Production of 4-(pyridin-3-yl)-2-(pyridin-2-ylmethoxy)-6,7-dihydr o-5H-cyclopenta[b]pyridine dihydrochloride

To 4-chloro-2-(pyridin-2-ylmethoxy)-6,7-dihydro-5H-cyc lopenta[b]pyridine (20 mg), pyridin-3-yl boronic acid (19 mg), Pd(dppf)Cl₂·CH₂Cl₂ (5 mg), and potassium carbonate (32 mg) was added 1,4-dioxane/water (3/1, 1.9 mL), and the mixture was stirred at 100°C for 5 hours. After the solvent was evaporated under reduced pressure, the residue was purified by silica gel column chromatography to give 4-(pyridin-3-yl)-2-(pyridin-2-ylmethoxy)-6,7-dihydr o-5H-cyclopenta[b]pyridine(16 mg).

The resulting compound was dissolved in ethyl acetate (10 mL) and 1N HCl/Et₂O solution (0.26 mL) was added, and the mixture was stirred for 0.5 hour. The resulting precipitate was collected by filtration to give the title compound (10 mg) as a white amorphous form.
[MS (ESI) m/z 304.3 (M+H)⁺]

### Example 2

### 4-(2-Fluoropyridin-3-yl)-2-(pyridin-2-ylmethoxy)-6, 7-dihydro-5H-cyclopenta[b]pyridine hydrochloride

To 4-chloro-2-(pyridin-2-ylmethoxy)-6,7-dihydro-5H-cyc lopenta[b]pyridine (100 mg), 2-fluoropyridin-3-ylboronic acid (70 mg), Pd(OAc)₂ (9 mg), X-Phos (37 mg) and potassium phosphate (244 mg) was added 1,4-dioxane/water (3/1, 2 mL), and the mixture was stirred at 100°C for 6 hours. After the reaction mixture was allowed to return to room temperature, diluted with ethyl acetate, filtered through Celite, and the filtrate was evaporated under reduced pressure. The resulting residue was purified by silica gel column chromatography to give 4-(2-fluoropyridin-3-yl)-2-(pyridin-2-ylmethoxy)-6, 7-dihydro-5H-cyclopenta[b]pyridine(48 mg).
[MS (ESI) m/z 322.0 (M+H)⁺]

The resulting compound was dissolved in ethyl acetate and 1N HCl/Et₂O solution (0.12 mL) was added, and the mixture was stirred for 0.5 hour. The resulting precipitate was collected by filtration to give the title compound (10 mg) as a white powder.
[MS (ESI) m/z 322.0 (M+H)⁺]

### Example 3

### 3-[2-(Pyridin-2-ylmethoxy)-6,7-dihydro-5H-cyclopent a[b]pyridin-4-yl]pyridine-2-carbonitrile

To a solution of 4-(2-fluoropyridin-3-yl)-2-(pyridin-2-ylmethoxy)-6, 7-dihydro-5H-cyclopenta[b]pyridine (8 mg) in DMSO (0.2 mL) was added NaCN (4.9 mg), and the mixture was stirred at 150°C for 24 hours. After the reaction mixture was allowed to return to room temperature, added with water, filtered through Celite, and the filtrate was evaporated under reduced pressure. The resulting precipitate was collected by filtration to give the title compound (5 mg) as a white powder.
[MS (ESI) m/z 329.2 (M+H)⁺]

### Example 4

### 5-[2-(Pyridin-2-ylmethoxy)-6,7-dihydro-5H-cyclopent a[b]pyridin-4-yl]pyridine-2-carbonitrile hydrochloride

### [Step 1]

### Production of 2-(pyridin-2-ylmethoxy)-6,7-dihydro-5H-cyclopenta[b ]pyridin-4-ol

4-Chloro-2-(pyridin-2-ylmethoxy)-6,7-dihydro-5 H-cyclopenta[b]pyridine (5 g), potassium hydroxide (3.23 g), Pd₂(dba)₃ (352 mg) and t-Bu-XPhos (408 mg) was added 1,4-dioxane (20 mL) and water (20 mL), and the mixture was degassed, then stirred under argon (hereinafter referred to as "Ar") atmosphere at 100°C for 2 hours. The reaction mixture was filtered through Celite, and the filtrate was evaporated under reduced pressure. The residue was neutralized with hydrochloric acid and then the mixture was weakly basified with sodium hydrogen carbonate. The resulting mixture was extracted with water and ethyl acetate. The organic layer was washed with saturated brine, dried over anhydrous sodium sulfate, filtered off, and the solvent was evaporated under reduced pressure. The resulting residue was purified by silica gel column chromatography to give the title compound (4.55 g).

### [Step 2]

### Production of 2-(pyridin-2-ylmethoxy)-6,7-dihydro-5H-cyclopenta[b ]pyridin-4-yl trifluoromethanesulfonate

To 2-(pyridin-2-ylmethoxy)-6,7-dihydro-5H-cyclopenta[b ]pyridin-4-ol (4.55g) was added CH₂Cl₂(100 mL), Et₃N (5.3 mL) and Tf₂NPh (8.05 g), and the mixture was stirred at room temperature for 5 hours. After the solvent of the reaction mixture was evaporated under reduced pressure, the residue was extracted with water and ethyl acetate. The organic layer was washed with saturated brine, dried over anhydrous sodium sulfate, filtered off, and the solvent was evaporated under reduced pressure. The resulting residue was purified by silica gel column chromatography to give the title compound (6.46 g).

### [Step 3]

### Production of 5-[2-(pyridin-2-ylmethoxy)-6,7-dihydro-5H-cyclopent a[b]pyridin-4-yl]pyridine-2-carbonitrile hydrochloride

To 2-(pyridin-2-ylmethoxy)-6,7-dihydro-5H-cyclopenta[b ]pyridin-4-yl trifluoromethanesulfonate (100 mg), 5-(4,4,5,5-tetramethyl-1,3,2-dioxaborolan-2-yl)pyri dine-2-carbonitrile (74 mg), Pd (dppf)Cl₂·CH₂Cl₂ (22 mg) and potassium carbonate (111 mg) was added 1,4-dioxane/water (3/1, 4 mL), and the mixture was degassed, then stirred under Ar atmosphere at 80°C for 1 hour. After the reaction mixture was allowed to return to room temperature, the mixture was added with water and ethyl acetate, and subjected to extraction. The organic layer was washed with saturated brine, dried over anhydrous sodium sulfate, filtered off, and the solvent was evaporated under reduced pressure. The resulting residue was purified by silica gel column chromatography to give 5-[2-(pyridin-2-ylmethoxy)-6,7-dihydro-5H-cyclopent a[b]pyridin-4-yl]pyridine-2-carbonitrile (97 mg).

The resulting compound was dissolved in ethyl acetate (3 mL), added with 1N HCl/Et₂O solution (0.32 mL) under ice water cooling , and the mixture was stirred for 0.5 hour under ice water cooling. The resulting precipitate was collected by filtration to give the title compound (70 mg) as a pale yellow powder.
[MS (ESI) m/z 329.5 (M+H)⁺]

### Example 5

### 5-[2-(Pyridin-2-ylmethoxy)-6,7-dihydro-5H-cyclopent a[b]pyridin-4-yl]pyridin-3-ol

### [Step 1]

### Production of 2-(pyridin-2-ylmethoxy)-4-(4,4,5,5-tetramethyl-1,3, 2-dioxaborolan-2-yl)-6,7-dihydro-5H-cyclopenta[b]py ridine

To 2-(pyridin-2-ylmethoxy)-6,7-dihydro-5H-cyclopenta[b ]pyridin-4-yl trifluoromethanesulfonate (273 mg), bis(pinacolato)diboron (1.87 g), potassium acetate (1.97 g) and Pd(dppf)Cl₂·CH₂Cl₂ (273 mg) was added 1,4-dioxane (50 mL), and the mixture was degassed, then stirred under Ar atmosphere at 100°C for 1 hour. After the reaction mixture was allowed to return to room temperature, diluted with ethyl acetate, filtered through Celite, and the filtrate was evaporated under reduced pressure. The resulting residue was purified by silica gel column chromatography to give the title compound (48 mg) as a white solid.

### [Step2]

### Production of 5-[2-(pyridin-2-ylmethoxy)-6,7-dihydro-5H-cyclopent a[b]pyridin-4-yl]pyridin-3-ol

To 2-(pyridin-2-ylmethoxy)-4-(4,4,5,5-tetramethyl-1,3, 2-dioxaborolan-2-yl)-6,7-dihydro-5H-cyclopenta[b]py ridine (100 mg), 5-bromopyridin-3-ol (74 mg), Pd(dppf)Cl₂·CH₂Cl₂ (19 mg) and potassium carbonate (118 mg) was added 1,4-dioxane/water (3/1, 1.4 mL), and the mixture was degassed and stirred under Ar atmosphere at 100°C for 2 hours. After the reaction mixture was allowed to return to room temperature, diluted with ethyl acetate, filtered through Celite, and the filtrate was evaporated under reduced pressure. The resulting residue was purified by silica gel column chromatography to give the title compound (16 mg) as a pale yellow powder.
[MS (ESI) m/z 320.3 (M+H)⁺]

### Example 6

### 5-[2-(Pyridin-2-ylmethoxy)-6,7-dihydro-5H-cyclopent a[b]pyridin-4-yl]pyridin-3-yl methanesulfonate

To a solution of 5-[2-(pyridin-2-ylmethoxy)-6,7-dihydro-5H-cyclopent a[b]pyridin-4-yl]pyridin-3-ol (13 mg) in CH₂Cl₂ (1 mL) was added Et₃N (0.023 mL), methanesulfonyl chloride (hereinafter referred to as "MsCl") (0.007 mL) sequentially, and the mixture was stirred at room temperature overnight. The reaction mixture was purified by silica gel column chromatography to give the title compound (4 mg) as colorless oil.
[MS (ESI) m/z 398.3 (M+H)⁺]

### Example 7

### N-{3-Fluoro-5-[2-(pyridin-2-ylmethoxy)-6,7-dihydro-5H-cyclopenta[b]pyridin-4-yl]phenyl methanesulfonamide} methanesulfonamide

### [Step 1]

### Production of 3-fluoro-5-[2-(pyridin-2-ylmethoxy)-6,7-dihydro-5H-cyclopenta[b]pyridin-4-yl]phenol

To 2-(pyridin-2-ylmethoxy)-4-(4,4,5,5-tetramethyl-1,3, 2-dioxaborolan-2-yl)-6,7-dihydro-5H-cyclopenta[b]py ridine (100 mg), 3-bromo-5-fluorophenol (82 mg), Pd(OAc)₂ (2.6 mg) and S-Phos (9.4 mg) was added ethanol (1.4mL), and the mixture was degassed, and stirred under Ar atmosphere at 100°C for 2 hours. After the reaction mixture was allowed to return to room temperature, diluted with ethyl acetate, filtered through Celite, and the filtrate was evaporated under reduced pressure. The resulting residue was purified by silica gel column chromatography to give the title compound (63 mg) as a white powder.

### [Step 2]

### Production of 3-fluoro-5-[2-(pyridin-2-ylmethoxy)-6,7-dihydro-5H-cyclopenta[b]pyridin-4-yl]phenyl trifluoromethanesulfonate

To 3-fluoro-5-[2-(pyridin-2-ylmethoxy)-6,7-dihydro-5H-cyclopenta[b]pyridin-4-yl]phenol (36 mg) and pyridine (4 mg) was added CH₂Cl₂ (2 mL). The mixture was stirred under Ar atmosphere under ice water cooling, and then added with Tf₂O (0.035 mL) at the same temperature for 2 hours. The solvent was removed under reduced pressure, and the resulting residue was purified by silica gel column chromatography to give the title compound (45 mg) as colorless oil.

### [Step 3]

### Production of N-{3-fluoro-5-[2-(pyridin-2-ylmethoxy)-6,7-dihydro-5H-cyclopenta[b]pyridin-4-yl]phenyl methane sulfonamide} methanesulfonamide

To 3-fluoro-5-[2-(pyridin-2-ylmethoxy)-6,7-dihydro-5H-cyclopenta[b]pyridin-4-yl]phenyl trifluoromethanesulfonate (10 mg), methanesulfonamide (18 mg), Pd₂ (dba)₃·CHCl₃ (10 mg), t-Bu-X-Phos (8 mg) and potassium phosphate (41 mg) was added toluene (1 mL), and the mixture was degassed, and stirred under Ar atmosphere at 100°C overnight. After the reaction mixture was allowed to return to room temperature, diluted with ethyl acetate, filtered through Celite, and the filtrate was evaporated under reduced pressure. The resulting residue was purified by silica gel column chromatography to give the title compound (40 mg) as a white powder.
[MS (ESI) m/z 414.4 (M+H)⁺]

### Example 8

### 6-({[4-(2-Fluoropyridin-3-yl)-6,7-dihydro-5H-cyclop enta[b]pyridin-2-yl]oxy}methyl)pyridine-2-carbonitr ile

### [Step 1]

### Production of 2-[(1-oxidopyridin-2-yl)methoxy)-6,7-dihydro-5H-cyc lopenta[b]pyridin-4-yl trifluoromethanesulfonate

To a solution of 2-(pyridin-2-ylmethoxy)-6,7-dihydro-5H-cyclopenta[b ]pyridin-4-yl trifluoromethanesulfonate in CH₂Cl₂ (70 mL) was added m-CPBA (with abs. 25% water, 2.9 g) under ice water cooling, and the mixture was stirred at room temperature for 1 hour. The solvent of the reaction mixture was removed under reduced pressure, and the resulting residue was purified by silica gel column chromatography to give the title compound (3.52 g).

### [Step 2]

### Production of 2-[(6-cyanopyridin-2-yl)methoxy]-6,7-dihydro-5H-cyc lopenta[b]pyridin-4-yl trifluoromethanesulfonate

To a solution of 2-[(1-oxidopyridin-2-yl)methoxy)-6,7-dihydro-5H-cyc lopenta[b]pyridin-4-yl trifluoromethanesulfonate (3.52 g) in CH₂Cl₂ (9 mL) was added N, N-dimethylcarbamoyl chloride (1.93g), trimethylsilyl cyanide (1.78 g), and the mixture was stirred at room temperature for 1 hour. The solvent was removed under reduced pressure, and the resulting residue was purified by silica gel column chromatography to give the title compound (3.93 g) as colorless oil.

### [Step 3]

### Production of 6-({[4-(4,4,5,5-tetramethyl-1,3,2-dioxaborolan-2-yl )-6,7-dihydro-5H-cyclopenta[b]pyridin-2-yl]oxy}meth yl)pyridine-2-carbonitrile

The title compound was prepared as a white solid according to the procedure described in Example 5 Step 1 using 2-[(6-cyanopyridin-2-yl)methoxy]-6,7-dihydro-5H-cyc lopenta[b]pyridin-4-yl trifluoromethanesulfonate instead of 2-(pyridin-2-ylmethoxy)-6,7-dihydro-5H-cyclopenta[b ]pyridin-4-yl trifluoromethanesulfonate.

### [Step 4]

### Production of 6-({[4-(2-fluoropyridin-3-yl)-6,7-dihydro-5H-cyclop enta[b]pyridin-2-yl]oxy}methyl)pyridine-2-carbonitr ile

To 6-({[4-(4,4,5,5-tetramethyl-1,3,2-dioxaborolan-2-yl )-6,7-dihydro-5H-cyclopenta[b]pyridin-2-yl]oxy}meth yl)pyridine-2-carbonitrile (100 mg), 2-fluoro-3 iodopyridine (71 mg), Pd(dppf)Cl₂·CH₂Cl₂ (17 mg) and potassium carbonate (110 mg) was added 1,4-dioxane/water (3/1, 2.6 mL), and the mixture was degassed, then stirred under Ar atmosphere at 100°C for 4 hours. After the reaction mixture was allowed to return to room temperature, diluted with ethyl acetate, dried over anhydrous sodium sulfate, filtered through Celite, and the filtrate was evaporated under reduced pressure. The resulting residue was purified by silica gel column chromatography to give the title compound (65 mg) as a white solid.
[MS (ESI) m/z 347.4 (M+H)⁺]

### Example 9

### 5-{2-[(5-Fluoropyridin-2-yl)methoxy]-6,7-dihydro-5H -cyclopenta[b]pyridin-4-yl}pyridine-2-carbonitrile

### [Step 1]

### Production of 2-chloro-4-(pyridin-2-ylmethoxy)-6,7-dihydro-5H-cyc lopenta[b]pyridine

To pyridine-2-ylmethanol (0.694 mL) was added toluene (12 mL) and then added NaH (60% dispersion in oil, 480 mg) under ice water cooling, and the mixture was stirred for 30 minutes. After the reaction mixture was allowed to return to room temperature, 2,4-dichloro-6,7-dihydro-5H-cyclopenta[b]pyridine (1.5 g) was added to the mixture, and the mixture was stirred at 170°C for 1 hour. After the reaction mixture was allowed to return to room temperature, added with water and ethyl acetate, and subjected to extraction. The organic layer was washed with saturated brine, dried over anhydrous sodium sulfate, and the solvent was evaporated under reduced pressure. The resulting residue was purified by silica gel column chromatography to give the title compound (623 mg) as a white solid.
[Rf value (TLC silica gel plate 60F₂₅₄, developing solvent: hexane : ethyl acetate =1 : 1) : 0.3]

### [Step 2]

### Production of 2-chloro-6,7-dihydro-5H-cyclopenta[b]pyridin-4-ol

To 2-chloro-4-(pyridin-2-ylmethoxy)-6,7-dihydro-5H-cyc lopenta[b]pyridine (608 mg) and sulfuric acid (0.24 mL) were added MeCN (15 mL) and sodium iodide (hereinafter referred to as "NaI") (1.33 g), and the mixture was heated under reflux for 12 hours. After the solvent of the reaction mixture was removed under reduced pressure, the resulting residue was added with ethyl acetate and aqueous sodium thiosulfate solution, and subjected to extraction. The organic layer was washed with saturated brine, dried over anhydrous sodium sulfate, filtered off, and the solvent was evaporated under reduced pressure to give the title compound (470 mg) as a white solid.

### [Step 3]

### Production of 2-chloro-6,7-dihydro-5H-cyclopenta[b]pyridin-4-yl trifluoromethanesulfonate

To 2-chloro-6,7-dihydro-5H-cyclopenta[b]pyridin-4-ol (269 mg), 2,6-lutidine (0.34 mL) and DMAP (18 mg) was added CH₂Cl₂ (15 mL), and the mixture was added with Tf₂O (0.3 mL) under ice water cooling, and stirred at room temperature for 3 hours. After the solvent of the reaction mixture was removed under reduced pressure, the resulting residue was added with Et₂O and water, and subjected to extraction. The organic layer was dried over anhydrous sodium sulfate, filtered, and the solvent was evaporated under reduced pressure. The resulting residue was purified by silica gel column chromatography to give the title compound (439 mg) as colorless oil.

### [Step 4]

### Production of 2-chloro-4-(4,4,5,5-tetramethyl-1,3,2-dioxaborolan-2-yl)-6,7-dihydro-5H-cyclopenta[b]pyridine

The title compound was prepared as a white powder according to the procedure described in Example 5 Step 1 using 2-chloro-6,7-dihydro-5H-cyclopenta[b]pyridin-4-yl trifluoromethanesulfonate instead of 2-(pyridin-2-ylmethoxy)-6,7-dihydro-5H-cyclopenta[b ]pyridin-4-yl trifluoromethanesulfonate.

### [Step 5]

### Production of 5-(2-chloro-6,7-dihydro-5H-cyclopenta[b]pyridin-4-y l)pyridine-2-carbonitrile

The title compound was prepared as a white powder according to the procedure described in Example 5 Step 2 using 2-chloro-4-(4,4,5,5-tetramethyl-1,3,2-dioxaborolan-2-yl)-6,7-dihydro-5H-cyclopenta[b]pyridine instead of 2-(pyridin-2-ylmethoxy)-4-(4,4,5,5-tetramethyl-1,3, 2-dioxaborolan-2-yl)-6,7-dihydro-5H-cyclopenta[b]py ridine, and using 5-bromopyridine-2-carbonitrile instead of 5-bromopyridin-3-ol.

### [Step 6]

### Production of 5-{2-[(5-fluoropyridin-2-yl)methoxy]-6,7-dihydro-5H -cyclopenta[b]pyridin-4-yl}pyridine-2-carbonitrile

To 5-(2-chloro-6,7-dihydro-5H-cyclopenta[b]pyridin-4-y l)pyridine-2-carbonitrile (60 mg), (5-fluoropyridin-2-yl)methanol (36 mg), t-Bu-X-Phos (24 mg), NaO-t-Bu (45 mg), Pd₂(dba)₃·CHCl₃ (10 mg) and molecular sieve 4Å (hereinafter referred to as "MS4A") was added toluene (1 mL), and the mixture was degassed, then stirred under Ar atmosphere at 100°C for 1 hour. After the reaction mixture was allowed to return to room temperature, diluted with diethyl acetate filtered through Celite, and the filtrate was evaporated under reduced pressure. The resulting residue was dissolved in CH₂Cl₂ (3 mL), and then Et₃N (0.1 mL) and TFAA (0.05 mL) were added to the mixture under ice water cooling under Ar atmosphere, and the mixture was stirred at the same temperature for 2 hours. The resulting mixture was added with water and ethyl acetate, and subjected to extraction. The organic layer was washed with saturated brine, dried over anhydrous sodium sulfate, filtered off, and the solvent was evaporated under reduced pressure. The resulting residue was purified by silica gel column chromatography to give the title compound (11 mg).
[MS (ESI) m/z 347.3 (M+H)⁺]

### Example 10

### 5-{2-[(6-Methylpyridin-2-yl)methoxy]-6,7-dihydro-5H -cyclopenta[b]pyridin-4-yl}pyridine-2-carbonitrile

The title compound was prepared as a white solid according to the procedure described in Example 9 Step 6 using (6-methylpyridin-2-yl)methanol instead of (5-fluoropyridin-2-yl)methanol.
[MS (ESI) m/z 343.4 (M+H)⁺]

### Example 11

### 2-(Benzyloxy)-4-(pyrimidin-5-yl)-6,7-dihydro-5H-cyc lopenta[b]pyridine

### [Step 1]

### Production of 2-chloro-4-(pyrimidin-5-yl)-6,7-dihydro-5H-cyclopen ta[b]pyridine

To 2-chloro-6,7-dihydro-5H-cyclopenta[b]pyridin-4-yl trifluoromethanesulfonate (439 mg), Pd(dppf)Cl₂·CH₂Cl₂ (59 mg), potassium carbonate (603 mg) and pyrimidin-5-yl boronic acid (198 mg) was added THF/water (3/1, 16 mL), and the mixture was degassed, then stirred under Ar atmosphere at 60°C for 1 hour. The solvent of the reaction mixture was evaporated under reduced pressure, diluted with ethyl acetate, filtered through Celite, and the filtrate was evaporated under reduced pressure. The resulting residue was purified by silica gel column chromatography to give the title compound (315 mg).

### [Step 2]

### Production of 2-(benzyloxy)-4-(pyrimidin-5-yl)-6,7-dihydro-5H-cyc lopenta[b]pyridine

To 2-chloro-4-(pyrimidin-5-yl)-6,7-dihydro-5H-cyclopen ta[b]pyridine (50 mg), benzylalcohol (35 mg), t-Bu-X-Phos (15 mg) and NaO-t-Bu (41 mg) and Pd₂ (dba)₃·CHCl₃ (9 mg) was added toluene (2 mL), and the mixture was degassed, then stirred under Ar atmosphere at 100°C for 1 hour. After the reaction mixture was allowed to return to room temperature, diluted with ethyl acetate, filtered through a short column, and the solvent was evaporated under reduced pressure. The resulting residue was purified by silica gel column chromatography to give the title compound (11 mg).
[MS (ESI) m/z 304.4 (M+H)⁺]

### Example 12

### 2-(Pyridin-2-ylmethoxy)-4-(pyrimidin-5-yl)-6,7-dihy dro-5H-cyclopenta[b]pyridine hydrochloride

To 4-chloro-2-(pyridin-2-ylmethoxy)-6,7-dihydro-5H-cyc lopenta[b]pyridine (100 mg), pyrimidin-5-yl boronic acid (143 mg), Pd(OAc)₂ (9 mg), S-Phos (31 mg) and potassium carbonate (158 mg) was added 1,4-dioxane/water (3/1, 1.9 mL), and the mixture was degassed, then stirred under Ar atmosphere at 100°C for 6 hours. After the reaction mixture was allowed to return to room temperature, diluted with ethyl acetate, dried over anhydrous sodium sulfate, filtered through Celite, and the solvent was evaporated under reduced pressure. The resulting residue was purified by silica gel column chromatography to give 2-(pyridin-2-ylmethoxy)-4-(pyrimidin-5-yl)-6,7-dihy dro-5H-cyclopenta[b]pyridine (76 mg).

The resulting compound was dissolved in ethyl acetate (4.8 mL) and 1N HCl/Et₂O solution (0.24 mL) was added under ice water cooling, and the mixture was stirred at the same temperature for 1 hour. The resulting precipitate was collected by filtration to give the title compound (47 mg) as a white powder.
Elementary analysis as C₁₈H₁₆N₄O·HCl+0.2H₂O
Calcd. (%) C: 62.77.; H: 5.09.; N: 16.27
Found. (%) C: 62.81.; H: 4.85.; N: 15.80

### Example 13

### 2-(Pyridin-3-ylmethoxy)-4-(pyrimidin-5-yl)-6,7-dihy dro-5H-cyclopenta[b]pyridine

The title compound was prepared as a white powder according to the procedure described in Example 11 Step 2 using pyridin-3-ylmethanol instead of benzylalcohol.
[MS (ESI) m/z 305.4 (M+H)⁺]

### Example 14

### 2-(Pyridin-4-ylmethoxy)-4-(pyrimidin-5-yl)-6,7-dihy dro-5H-cyclopenta[b]pyridine

The title compound was prepared as a white powder according to the procedure described in Example 11 Step 2 using pyridin-4-ylmethanol instead of benzylalcohol.
[MS (ESI) m/z 305.4 (M+H)⁺]

### Example 15

### 2-[(3-Fluorobenzyl)oxy]-4-(pyrimidin-5-yl)-6,7-dihy dro-5H-cyclopenta[b]pyridine

The title compound was prepared as a white powder according to the procedure described in Example 11 Step 2 using 3-fluorobenzylalcohol instead of benzylalcohol.
[MS (ESI) m/z 322.3 (M+H)⁺]

### Example 16

### 2-[(2-Fluorobenzyl)oxy]-4-(pyrimidin-5-yl)-6,7-dihy dro-5H-cyclopenta[b]pyridine

The title compound was prepared as a white powder according to the procedure described in Example 11 Step 2 using 2-fluorobenzylalcohol instead of benzylalcohol.
[MS (ESI) m/z 322.3 (M+H)⁺]

### Example 17

### 2-[(2,6-Difluorobenzyl)oxy]-4-(pyrimidin-5-yl)-6,7-dihydro-5H-cyclopenta[b]pyridine

The title compound was prepared as a white powder according to the procedure described in Example 11 Step 2 using 2,6-difluorobenzylalcohol instead of benzylalcohol.
[MS (ESI) m/z 340.3 (M+H)⁺]

### Example 18

### 2-[(2,4-Difluorobenzyl)oxy]-4-(pyrimidin-5-yl)-6,7-dihydro-5H-cyclopenta[b]pyridine

The title compound was prepared as a white powder according to the procedure described in Example 11 Step 2 using 2,4-difluorobenzylalcohol instead of benzylalcohol.
[MS (ESI) m/z 340.3 (M+H)⁺]

### Example 19

### 2-[(3,5-Difluorobenzyl)oxy]-4-(pyrimidin-5-yl)-6,7-dihydro-5H-cyclopenta[b]pyridine

The title compound was prepared as a white powder according to the procedure described in Example 11 Step 2 using 3,5-difluorobenzylalcohol instead of benzylalcohol.
[MS (ESI) m/z 340.3 (M+H)⁺]

### Example 20

### 2-[(3,4-Difluorobenzyl)oxy]-4-(pyrimidin-5-yl)-6,7-dihydro-5H-cyclopenta[b]pyridine

The title compound was prepared as a white powder according to the procedure described in Example 11 Step 2 using 3,4-difluorobenzylalcohol instead of benzylalcohol.
[MS (ESI) m/z 340.3 (M+H)⁺]

### Example 21

### 2-[(4-Chloro-2-fluorobenzyl)oxy]-4-(pyrimidin-5-yl) -6,7-dihydro-5H-cyclopenta[b]pyridine

The title compound was prepared as a white powder according to the procedure described in Example 11 Step 2 using 4-chloro-2-fluorobenzylalcohol instead of benzylalcohol.
[MS (ESI) m/z 356.3 (M+H)⁺]

### Example 22

### 2-({[4-(Pyrimidin-5-yl)-6,7-dihydro-5H-cyclopenta[b ]pyridin-2-yl]oxy}methyl)benzonitrile

### [Step 1]

### Production of 2-[(2-bromobenzyl)oxy]-4-(pyrimidin-5-yl)-6,7-dihyd ro-5H-cyclopenta[b]pyridine

To a solution of 2-bromobenzylalcohol (97 mg) in DMF (3 mL) was added KO-t-Bu (72 mg) and 2-chloro-4-(pyrimidin-5-yl)-6,7-dihydro-5H-cyclopen ta[b]pyridine (100 mg), and the mixture was stirred at 70°C for 2 hours. After the reaction mixture was allowed to return to room temperature, the mixture was added with water and Et₂O, and subjected to extraction. The organic layer was dried over anhydrous sodium sulfate, filtered off, and the solvent was evaporated under reduced pressure. The resulting residue was purified by silica gel column chromatography to give the title compound (47 mg) as a white solid.

### [Step 2]

### Production of 2-({[4-(pyrimidin-5-yl)-6,7-dihydro-5H-cyclopenta[b ]pyridin-2-yl]oxy}methyl)benzonitrile

To 2-[(2-bromobenzyl)oxy]-4-(pyrimidin-5-yl)-6,7-dihyd ro-5H-cyclopenta[b]pyridine (45 mg), Pd(dppf)Cl₂·CH₂Cl₂ (10 mg), zinc cyanide (83 mg) and zinc powder (0.9 mg) was added DMF (1 mL), and the mixture was reacted under microwave irradiation at 180°C for 1 hour. After the reaction mixture was allowed to return to room temperature, diluted with ethyl acetate, filtered through Celite, and the filtrate was evaporated under reduced pressure. The resulting residue was purified by silica gel column chromatography to give the title compound (24 mg) as a white solid.
[MS (ESI) m/z 329.3 (M+H)⁺]

### Example 23

### 3-({[4-(Pyrimidin-5-yl)-6,7-dihydro-5H-cyclopenta[b ]pyridin-2-yl]oxy}methyl)benzonitrile

The title compound was prepared as a white powder according to the procedure described in Example 11 Step 2 using 3-(hydroxymethyl)benzonitrile instead of benzylalcohol.
[MS (ESI) m/z 329.3 (M+H)⁺]

### Example 24

### 2-[(6-Fluoropyridin-2-yl)methoxy]-4-(pyrimidin-5-yl )-6,7-dihydro-5H-cyclopenta[b]pyridine

The title compound was prepared according to the procedure described in Example 11 Step 2 using (6-fluoropyridin-2-yl)methanol instead of benzylalcohol, and using cesium carbonate instead of NaO-t-Bu.
[MS (ESI) m/z 323.4 (M+H)⁺]

### Example 25

### 2-[(5-Fluoropyridin-2-yl)methoxy]-4-(pyrimidin-5-yl )-6,7-dihydro-5H-cyclopenta[b]pyridine

The title compound was prepared as a white powder according to the procedure described in Example 11 Step 2 using (5-fluoropyridin-2-yl)methanol instead of benzylalcohol, and using cesium carbonate instead of NaO-t-Bu.
[MS (ESI) m/z 323.4 (M+H)⁺]

### Example 26

### 2-[(3-Fluoropyridin-2-yl)methoxy]-4-(pyrimidin-5-yl )-6,7-dihydro-5H-cyclopenta[b]pyridine

To 2-chloro-4-(pyrimidin-5-yl)-6,7-dihydro-5H-cyclopen ta[b]pyridine (70 mg), (3-fluoropyridin-2-yl)methanol (46 mg), t-Bu-X-Phos (31 mg), Pd₂(dba)₃·CHCl₃ (19 mg) and cesium carbonate (295 mg) was added toluene (3 mL), and the mixture was degassed, then stirred under Ar atmosphere at 100°C for 5 hours. After the reaction mixture was allowed to return to room temperature, diluted with ethyl acetate, filtered through a short column, and the solvent was evaporated under reduced pressure. The resulting residue was purified by silica gel column chromatography to give the title compound (47 mg) as a white solid.
[MS (ESI) m/z 323.4 (M+H)⁺]

### Example 27

### 2-[(3,5-Difluoropyridin-2-yl)methoxy]-4-(pyrimidin5-yl)-6,7-dihydro-5H-cyclopenta[b]pyridine

The title compound was prepared as a white solid according to the procedure described in Example 11 Step 2 using (3,5-difluoropyridin-2-yl)methanol instead of benzylalcohol.
[MS (ESI) m/z 341.3 (M+H)⁺]

### Example 28

### 2-[(6-Methylpyridin-2-yl)methoxy]-4-(pyrimidin-5-yl )-6,7-dihydro-5H-cyclopenta[b]pyridine

The title compound was prepared as a white powder according to the procedure described in Example 11 Step 2 using (6-methylpyridin-2-yl)methanol instead of benzylalcohol.
[MS (ESI) m/z 319.4 (M+H)⁺]

### Example 29

### 2-({[4-(Pyrimidin-5-yl)-6,7-dihydro-5H-cyclopenta[b ]pyridin-2-yl]oxy}methyl)quinoline

The title compound was prepared as a white solid according to the procedure described in Example 11 Step 2 using quinolin-2-ylmethanol instead of benzylalcohol.
[MS (ESI) m/z 425.5 (M+H)⁺]

### Example 30

### 2-[(5-Fluoropyridin-3-yl)methoxy]-4-(pyrimidin-5-yl )-6,7-dihydro-5H-cyclopenta[b]pyridine

The title compound was prepared as a white powder according to the procedure described in Example 11 Step 2 using (5-fluoropyridin-3-yl)methanol instead of benzylalcohol.
[MS (ESI) m/z 323.4 (M+H)⁺]

### Example 31

### 2-[(6-Methylpyridin-3-yl)methoxy]-4-(pyrimidin-5-yl )-6,7-dihydro-5H-cyclopenta[b]pyridine

The title compound was prepared as yellow oil according to the procedure described in Example 11 Step 2 using (6-methylpyridin-3-yl)methanol instead of benzylalcohol.
[MS (ESI) m/z 319.4 (M+H)⁺]

### Example 32

### 2-[(3-Fluoropyridin-4-yl)methoxy]-4-(pyrimidin-5-yl )-6,7-dihydro-5H-cyclopenta[b]pyridine

The title compound was prepared as a white powder according to the procedure described in Example 11 Step 2 using (3-fluoropyridin-4-yl)methanol instead of benzylalcohol.
[MS (ESI) m/z 323.3 (M+H)⁺]

### Example 33

### 2-[(2-Cyclopropylpyridin-4-yl)methoxy]-4-(pyrimidin -5-yl)-6,7-dihydro-5H-cyclopenta[b]pyridine

### [Step 1]

### Production of methyl 2-cyclopropylpyridine-4-carboxylate

To methyl 2-chloropyrimidine-4-carboxylate (200 mg), cyclopropylboronic acid (150 mg), PdCl₂(PCy₃)₂ (172 mg) and potassium phosphate (742 mg) was added toluene (0.9 mL), and the mixture was degassed, then stirred under Ar atmosphere at 100°C overnight. After the reaction mixture was allowed to return to room temperature, diluted with ethyl acetate, filtered through Celite, and the solvent was evaporated under reduced pressure. The resulting residue was purified by silica gel column chromatography to give the title compound as yellow oil (218 mg).

### [Step 2]

### Production of (2-cyclopropylpyridin-4-yl)methanol

To a solution of methyl 2-cyclopropylpyridine-4-carboxylate (218 mg) in methanol (8 mL) was added NaBH₄ (140 mg) under Ar atmosphere and the mixture was stirred at room temperature overnight. The reaction mixture was added with water and ethyl acetate, and subjected to extraction. The organic layer was washed saturated brine, dried over anhydrous sodium sulfate, filtered off, and the solvent was evaporated under reduced pressure. The resulting residue was purified by silica gel column chromatography to give the title compound (51 mg) as pale yellow oil.

### [Step 3]

### Production of 2-[(2-cyclopropylpyridin-4-yl)methoxy]-4-(pyrimidin -5-yl)-6,7-dihydro-5H-cyclopenta[b]pyridine

The title compound was prepared as a white powder according to the procedure described in Example 11 Step 2 using (2-cyclopropylpyridin-4-yl)methanol instead of benzylalcohol.
[MS (ESI) m/z 345.3 (M+H)⁺]

### Example 34

### 4-(Pyrimidin-5-yl)-2-(thiophen-2-ylmethoxy)-6,7-dih ydro-5H-cyclopenta[b]pyridine

The title compound was prepared as a white solid according to the procedure described in Example 11 Step 2 using thiophen-2-ylmethanol instead of benzylalcohol.
[MS (ESI) m/z 310.3 (M+H)⁺]

### Example 35

### 2-[(5-Chlorothiophen-2-yl)methoxy]-4-(pyrimidin-5-y l)-6,7-dihydro-5H-cyclopenta[b]pyridine

The title compound was prepared as a white solid according to the procedure described in Example 11 Step 2 using (5-chlorothiophen-2-yl)methanol instead of benzylalcohol.
[MS (ESI) m/z 344.2 (M+H)⁺]

### Example 36

### 4-(2-Methylpyrimidin-5-yl)-2-(pyridin-2-ylmethoxy)-6,7-dihydro-5H-cyclopenta[b]pyridine hydrochloride

4-(2-methylpyrimidin-5-yl)-2-(pyridin-2-ylmeth oxy)-6,7-dihydro-5H-cyclopenta[b]pyridine (429 mg) was prepared according to the procedure described in Example 5 Step 2 using 5-bromo-2-methylpyrimidine instead of 5-bromopyridin-3-ol. The resulting compound was dissolved in ethyl acetate and 1N HCl/Et₂O solution (1.48 mL) was added under ice water cooling, and the mixture was stirred at the same temperature for 0.5 hour. The resulting precipitate was collected by filtration to give the title compound (451 mg) as a white powder.
[MS (ESI) m/z 319.4 (M+H)⁺]
Elementary analysis as C₁₉H₁₈N₄O·HCl+0.3H₂O
Calcd. (%) C: 63.35.; H: 5.48.; N: 15.55
Found. (%) C: 63.26.; H: 5.35.; N: 15.39

### Example 37

### 5-[2-(Pyridin-2-ylmethoxy)-6,7-dihydro-5H-cyclopent a[b]pyridin-4-yl]pyrimidine-2-carbonitrile hydrochloride

5-[2-(Pyridin-2-ylmethoxy)-6,7-dihydro-5H-cycl openta[b]pyridin-4-yl]pyrimidine-2-carbonitrile was prepared (65 mg) according to the procedure described in Example 5 Step 2 using 5-bromopyrimidine-2 carbonitrile instead of 5-bromopyrimidin-3-ol. The resulting compound was dissolved in ethyl acetate (2 mL) and 1N HCl/Et₂O solution (0.22 mL) was added under ice water cooling, then the mixture was stirred at the same temperature for 0.5 hour. The resulting precipitate was collected by filtration to give the title compound (63 mg) as a white powder.
[MS (ESI) m/z 330.3 (M+H)⁺]

### Example 38

### 2-(Benzyloxy)-4-(2-methylpyrimidin-5-yl)-6,7-dihydr o-5H-cyclopenta[b]pyridine

### [Step 1]

### Production of 2-chloro-4-(2-methylpyrimidin-5-yl)-6,7-dihydro-5H-cyclopenta[b]pyridine

The title compound was prepared as a white solid according to the procedure described in Example 5 Step 2 using 2-chloro-4-(4,4,5,5-tetramethyl-1,3,2-dioxaborolan-2-yl)-6,7-dihydro-5H-cyclopenta[b]pyridine instead of 2-(pyridin-2-ylmethoxy)-4-(4,4,5,5-tetramethyl-1,3, 2-dioxaborolan-2-yl)-6,7-dihydro-5H-cyclopenta[b]py ridine, and using 5-bromo-2-methylpyrimidine instead of 5-bromopyridin-3-ol, and using THF/water (3/1) instead of 1,4-dioxane/water (3/1).

### [Step 2]

### Production of 2-(benzyloxy)-4-(2-methylpyrimidin-5-yl)-6,7-dihydr o-5H-cyclopenta[b]pyridine

The title compound was prepared as a white solid according to the procedure described in Example 11 Step 2 using 2-chloro-4-(2-methylpyrimidin-5-yl)-6,7-dihydro-5H-cyclopenta[b]pyridine instead of 2-chloro-4-(pyrimidin-5-yl)-6,7-dihydro-5H-cyclopen ta[b]pyridine, and using cesium carbonate instead of NaO-t-Bu.
[MS (ESI) m/z 318.3 (M+H)⁺]

### Example 39

### 2-[(6-Fluoropyridin-2-yl)methoxy]-4-(2-methylpyrimi din-5-yl)-6,7-dihydro-5H-cyclopenta[b]pyridine

To 2-chloro-4-(2-methylpyrimidin-5-yl)-6,7-dihydro-5H-cyclopenta[b]pyridine (100 mg), (6-fluoropyridin-2-yl)methanol (62 mg), t-Bu-X-Phos (41 mg), NaO-t-Bu (78 mg), Pd₂(dba)₃·CHCl₃ (25 mg) and MS4A was added toluene (2 mL), and the mixture was degassed, then stirred under Ar atmosphere at 100°C for 4 hours. After the reaction mixture was allowed to return to room temperature, diluted with ethyl acetate, filtered through Celite, and the filtrate was evaporated under reduced pressure. The resulting residue was purified by silica gel column chromatography to give the title compound (22 mg) as a white powder.
[MS (ESI) m/z 337.4 (M+H)⁺]

### Example 40

### 2-[(5-Fluoropyridin-2-yl)methoxy]-4-(2-methylpyrimi din-5-yl)-6,7-dihydro-5H-cyclopenta[b]pyridine hydrochloride

2-[(5-Fluoropyridin-2-yl)methoxy]-4-(2-methylp yrimidin-5-yl)-6,7-dihydro-5H-cyclopenta[b]pyridine (46 mg) was prepared according to the procedure described in Example 39 using (5-fluoropyridin-2-yl)methanol instead of (6-fluoropyridin-2-yl)methanol.
[MS (ESI) m/z 337.4 (M+H)⁺]

The resulting compound was dissolved in ethyl acetate (2.6 mL) and 1N HCl/Et₂O solution (0.22 mL) was added under ice water cooling, then the mixture was stirred at the same temperature for 0.5 hour. The resulting precipitate was collected by filtration to give the title compound (24 mg) as a white powder.
[MS (ESI) m/z 337.4 (M+H)⁺]

### Example 41

### 2-[(3-Fluoropyridin-2-yl)methoxy]-4-(2-methylpyrimi din-5-yl)-6,7-dihydro-5H-cyclopenta[b]pyridine

The title compound was prepared as a white powder according to the procedure described in Example 39 using (3-fluoropyridin-2-yl)methanol instead of (6-fluoropyridin-2-yl)methanol.
[MS (ESI) m/z 337.4 (M+H)⁺]

### Example 42

### 2-[(3,5-Difluoropyridin-2-yl)methoxy]-4-(2-methylpy rimidin-5-yl)-6,7-dihydro-5H-cyclopenta[b]pyridine

The title compound was prepared as a white powder according to the procedure described in Example 39 using (3,5-difluoropyridin-2-yl)methanol instead of (6-fluoropyridin-2-yl)methanol.
[MS (ESI) m/z 355.4 (M+H)⁺]

### Example 43

### 2-[(3,6-Difluoropyridin-2-yl)methoxy]-4-(2-methylpy rimidin-5-yl)-6,7-dihydro-5H-cyclopenta[b]pyridine

The title compound was prepared as a white powder according to the procedure described in Example 39 using (3,6-difluoropyridin-2-yl)methanol instead of (6-fluoropyridin-2-yl)methanol.
[MS (ESI) m/z 355.3 (M+H)⁺]

### Example 44

### 5-{2-[(3,5-Difluoropyridin-2-yl)methoxy]-6,7-dihydr o-5H-cyclopenta[b]pyridin-4-yl}pyrimidine-2-carboni trile

### [Step 1]

### Production of 2,4-dichloro-6,7-dihydro-5H-cyclopenta[b]pyridine 1-oxide

To a solution of 2,4-dichloro-6,7-dihydro-5H-cyclopenta[b]pyridine (1 g) in CH₂Cl₂ (20 mL) was added m-CPBA (with abs. 25% water, 1.69 g), and the mixture was stirred at room temperature for 24 hours. The resulting residue was added with ethyl acetate, aqueous sodium hydrogen carbonate solution, and aqueous sodium thiosulfate solution, and subjected to extraction. The organic layer was dried over anhydrous sodium sulfate, filtered off, and the solvent was evaporated under reduced pressure. The resulting residue was purified by silica gel column chromatography to give the title compound (852 mg) as a white powder.

### [Step 2]

### Production of 4-chloro-2-[(3,5-difluoropyridin-2-yl)methoxy]-6,7-dihydro-5H-cyclopenta[b]pyridine

To 2,4-dichloro-6,7-dihydro-5H-cyclopenta[b]pyridine 1-oxide (200 mg) and (3,5-difluoropyridin-2-yl)methanol(185 mg) was added THF (8 mL) and then added NaH (60% dispersion in oil, 59 mg) under ice water cooling, then the mixture was stirred at room temperature for 3 hours. After that phosphorus trichloride (175 mg) was added to the mixture under ice water cooling, and the mixture was stirred for 30 minutes. The reaction mixture was added with aqueous sodium bicarbonate solution, water and ethyl acetate, and subjected to extraction. The organic layer was dried over anhydrous sodium sulfate, filtered off, and the solvent was evaporated under reduced pressure. The resulting residue was purified by silica gel column chromatography to give the title compound (63 mg) as a white powder.

### [Step 3]

### Production of 2-[(3,5-difluoropyridin-2-yl)methoxy]-6,7-dihydro-5 H-cyclopenta[b]pyridin-4-ol

To 4-chloro-2-[(3,5-difluoropyridin-2-yl)methoxy]-6,7-dihydro-5H-cyclopenta[b]pyridine (6.2 mg), potassium hydroxide (29 mg), Pd₂(dba)₃ (10 mg) and t-Bu-X-Phos (9 mg) were added 1,4-dioxane (1 mL) and water (1 mL), and the mixture was degassed, then stirred under Ar atmosphere at 100°C for 1 minute. After the reaction mixture was allowed to return to room temperature, and added with ethyl acetate, saturated aqueous ammonium chloride solution and water, and subjected to extraction. The organic layer was dried over anhydrous sodium sulfate, filtered off, and the solvent was evaporated under reduced pressure. The resulting residue was purified by silica gel column chromatography to give the title compound (67 mg) as pale brown oil.

### [Step 4]

### Production of 2-[(3,5-difluoropyridin-2-yl)methoxy]-6,7-dihydro-5 H-cyclopenta[b]pyridin-4-yl trifluoromethanesulfonate

To a solution of 2-[(3,5-difluoropyridin-2-yl)methoxy]-6,7-dihydro-5 H-cyclopenta[b]pyridin-4-ol (65 mg) in CH₂Cl₂ (2 mL) was sequentially added Et₃N (5.3 mL), Tf₂NPh (92 mg) and DMAP (3 mg), and the mixture was stirred at room temperature for 2 hours. The reaction mixture was extracted with water and ethyl acetate. The organic layer was washed with saturated brine, dried over magnesium sulfate, filtered off, and the solvent was evaporated under reduced pressure. The resulting residue was purified by silica gel column chromatography to give the title compound (70 mg).

### [Step 5]

### Production of 5-{2-[(3,5-difluoropyridin-2-yl)methoxy]-6,7-dihydr o-5H-cyclopenta[b]pyridin-4-yl}pyrimidine-2-carboni trile

To 2-[(3,5-difluoropyridin-2-yl)methoxy]-6,7-dihydro-5 H-cyclopenta[b]pyridin-4-yl trifluoromethanesulfonate (50 mg), 5-(4,4,5,5-tetramethyl-1,3,2-dioxaborolan-2-yl)pyri midine-2-carbonitrile (31 mg), Pd(dppf)Cl₂·CH₂Cl₂ (10 mg) and potassium carbonate (50 mg) was added 1,4-dioxane/water (3/1, 1 mL), and the mixture was degassed, then stirred under Ar atmosphere at 70°C for 1 hour. After the reaction mixture was allowed to return to room temperature, the mixture was added with water and ethyl acetate, and subjected to extraction. The organic layer was dried over anhydrous sodium sulfate, filtered off and the solvent was evaporated under reduced pressure. The resulting residue was purified by silica gel column chromatography to give the title compound (31 mg) as a white solid.
[MS (ESI) m/z 366.3 (M+H)⁺]

### Example 45

### 2-[(6-Methylpyridin-2-yl)methoxy]-4-(2-methylpyrimi din-5-yl)-6,7-dihydro-5H-cyclopenta[b]pyridine hydrochloride

To 2-chloro-4-(2-methylpyrimidin-5-yl)-6,7-dihydro-5H-cyclopenta[b]pyridine (100 mg), (6-methylpyridin-2-yl)methanol (52 mg), t-Bu-X-Phos (55 mg), and cesium carbonate (398 mg) and Pd₂ (dba)₃·CHCl₃ (34 mg) was added toluene (2.2 mL), and the mixture was degassed, then stirred under Ar atmosphere at 100°C for 6 hours. After the reaction mixture was allowed to return to room temperature, diluted with ethyl acetate, filtered through Celite, and the filtrate was evaporated under reduced pressure. The resulting residue was purified by silica gel column chromatography to give 2-[(6-methylpyridin-2-yl)methoxy]-4-(2-methylpyrimi din-5-yl)-6,7-dihydro-5H-cyclopenta[b]pyridine (49 mg). The resulting compound was dissolved in ethyl acetate (2 mL) and 1N HCl/Et₂O solution (0.147 mL) was added, then the mixture was stirred under ice water cooling for 3 hours. The resulting precipitate was collected by filtration to give the title compound (43 mg) as a white powder.
[MS (ESI) m/z 333.4 (M+H)⁺]

### Example 46

### 6-({[4-(2-Methylpyrimidin-5-yl)-6,7-dihydro-5H-cycl openta[b]pyridin-2-yl]oxy}methyl)pyridine-2-carboni trile

The title compound was prepared according to the procedure described in Example 8 Step 4 using 5-bromo-2-methylpyrimidine instead of 2-fluoro-3-iodopyridine.
[MS (ESI) m/z 344.4 (M+H)⁺]

### Example 47

### 6-({[4-(2-Methylpyrimidin-5-yl)-6,7-dihydro-5H-cycl openta[b]pyridin-2-yl]oxy}methyl)pyridine-3-carboni trile

To 2-chloro-4-(2-methylpyrimidin-5-yl)-6,7-dihydro-5H-cyclopenta[b]pyridine (100 mg), 6-(hydroxymethyl)pyridine-3-carbonitrile (57 mg), t-Bu-X-Phos (55 mg), cesium carbonate (398 mg) and Pd₂(dba)₃·CHCl₃ (34 mg) was added toluene (2.2 mL), and the mixture was degassed, then stirred under Ar atmosphere at 100°C for 6 hours. After the reaction mixture was allowed to return to room temperature, diluted with ethyl acetate, filtered through Celite, and the filtrate was evaporated under reduced pressure. The resulting residue was purified by silica gel column chromatography to give the title compound (30 mg) as a white powder.
[MS (ESI) m/z 344.4 (M+H)⁺]

### Example 48

### 5-{2-[(6-Fluoropyridin-2-yl)methoxy]-6,7-dihydro-5H -cyclopenta[b]pyridin-4-yl}pyrimidine-2-carbonitril e

### [Step 1]

### Production of 5-(2-chloro-6,7-dihydro-5H-cyclopenta[b]pyridin-4-y l)pyrimidine-2-carbonitrile

The title compound was prepared according to the procedure described in Example 11 Step 1 using 5-(4,4,5,5-tetramethyl-1,3,2-dioxaborolan-2-yl)pyri midine-2-carbonitrile instead of pyrimidin-5-ylboronic acid.

### [Step 2]

### Production of 5-{2-[(6-fluoropyridin-2-yl)methoxy]-6,7-dihydro-5H -cyclopenta[b]pyridin-4-yl}pyrimidine-2-carbonitril e

The title compound was prepared as a white powder according to the procedure described in Example 47 using 5-(2-chloro-6,7-dihydro-5H-cyclopenta[b]pyridin-4-y l)pyrimidine-2-carbonitrile instead of 2-chloro-4-(2-methylpyrimidin-5-yl)-6,7-dihydro-5H-cyclopenta[b]pyridine, and using (6-fluoropyridin-2-yl)methanol instead of 6-(hydroxymethyl)pyridine-3-carbonitrile.
[MS (ESI) m/z 348.3 (M+H)⁺]

### Example 49

### 5-{2-[(5-Fluoropyridin-2-yl)methoxy]-6,7-dihydro-5H -cyclopenta[b]pyridin-4-yl}pyrimidine-2-carbonitril e

The title compound was prepared as a yellow solid according to the procedure described in Example 48 Step2 using (5-fluoropyridin-2-yl)methanol instead of (6-fluoropyridin-2-yl)methanol.
[MS (ESI) m/z 348.4 (M+H)⁺]

### Example 50

### 5-{2-[(3-Fluoropyridin-2-yl)methoxy]-6,7-dihydro-5H -cyclopenta[b]pyridin-4-yl}pyrimidine-2-carbonitril e

### [Step 1]

### Production of 4-chloro-2-[(3-fluoropyridin-2-yl)methoxy]-6,7-dihy dro-5H-cyclopenta[b]pyridine

The title compound was prepared as a white powder according to the procedure described in Example 44 Step2 using (3-fluoropyridin-2-yl)methanol instead of (3,5-difluoropyridin-2-yl)methanol.

### [Step 2]

### Production of 2-[(3-fluoropyridin-2-yl)methoxy]-6,7-dihydro-5H-cy clopenta[b]pyridin-4-ol

The title compound was prepared as a white solid according to the procedure described in Example 44 Step3 using 4-chloro-2-[(3-fluoropyridin-2-yl)methoxy]-6,7-dihy dro-5H-cyclopenta[b]pyridine instead of 4-chloro-2-[(3,5-difluoropyridin-2-yl)methoxy]-6,7-dihydro-5H-cyclopenta[b]pyridine.

### [Step 3]

### Production of 2-[(3-fluoropyridin-2-yl)methoxy]-6,7-dihydro-5H-cy clopenta[b]pyridin-4-yl trifluoromethanesulfonate

To a solution of 2-[(3-fluoropyridin-2-yl)methoxy]-6,7-dihydro-5H-cy clopenta[b]pyridin-4-ol (157 mg) and 2,6-lutidine (0.11 mL) in CH₂Cl₂ (6 mL) was added Tf₂O (0.12 mL) under ice water cooling, and the mixture was stirred at room temperature for 1 hour. The reaction mixture was added with water and Et₂O, and subjected to extraction. The organic layer was washed with saturated brine, dried over anhydrous sodium sulfate, filtered off, and the solvent was evaporated under reduced pressure. The resulting residue was purified by silica gel column chromatography to give the title compound (220 mg) as colorless oil.

### [Step 4]

### Production of 5-{2-[(3-fluoropyridin-2-yl)methoxy]-6,7-dihydro-5H -cyclopenta[b]pyridin-4-yl}pyrimidine-2-carbonitril e

The title compound was prepared as a white solid according to the procedure described in Example 44 Step 5 using 2-[(3-fluoropyridin-2-yl)methoxy]-6,7-dihydro-5H-cy clopenta[b]pyridin-4-yl trifluoromethanesulfonate instead of 2-[(3,5-difluoropyridin-2-yl)methoxy]-6,7-dihydro-5 H-cyclopenta[b]pyridin-4-yl trifluoromethanesulfonate.
[MS (ESI) m/z 348.3 (M+H)⁺]

### Example 51

### 5-{2-[(6-Cyanopyridin-2-yl)methoxy]-6,7-dihydro-5H-cyclopenta[b]pyridin-4-yl}pyrimidine-2-carbonitrile

The title compound was prepared according to the procedure described in Example 8 Step 4 using 5-bromopyrimidine-2-carbonitrile instead of 2-fluoro-3-iodopyridine.
[MS (ESI) m/z 355.3 (M+H)⁺]

### Example 52

### 6-({[4-(2-Aminopyrimidin-5-yl)-6,7-dihydro-5H-cyclo penta[b]pyridin-2-yl]oxy}methyl)pyridine-2-carbonit rile hydrochloride

6-({[4-(2-aminopyrimidin-5-yl)-6,7-dihydro-5H-cyclopenta[b]pyridin-2-yl]oxy}methyl)pyridine-2-car bonitrile (46 mg) was prepared according to the procedure described in Example 8 Step 4 using 5-bromopyrimidine-2-amine instead of 2-fluoro-3-iodopyridine.
[MS (ESI) m/z 345.4 (M+H)⁺]

The resulting compound was dissolved in Et₂O (2.6 mL) and 1N HCl/Et₂O solution (0.134 mL) was added, then the mixture was stirred for 0.5 hour. The resulting precipitate was collected by filtration to give the title compound (41 mg) as a white powder.
[MS (ESI) m/z 345.4 (M+H)⁺]

### Example 53

### 4-(Pyrazin-2-yl)-2-(pyridin-2-ylmethoxy)-6,7-dihydr o-5H-cyclopenta[b]pyridine hydrochloride

To 4-chloro-2-(pyridin-2-ylmethoxy)-6,7-dihydro-5H-cyc lopenta[b]pyridine (50 mg), 2-(tributylstannyl)pyrazine (211 mg) and Pd(PPh₃)₄ (88 mg) was added DMF (0.9 mL), and the mixture was reacted under microwave irradiation at 180°C for 30 minutes. After the reaction mixture was allowed to return to room temperature, diluted with ethyl acetate, filtered through Celite, and the filtrate was evaporated under reduced pressure. The resulting residue was purified by silica gel column chromatography to give 4-(pyrazin-2-yl)-2-(pyridin-2-ylmethoxy)-6,7-dihydr o-5H-cyclopenta[b]pyridine (94 mg). The resulting compound was dissolved in ethyl acetate (3 mL) and 1N HCl/Et₂O solution (0.3 mL) was added under ice water cooling, then the mixture was stirred at the same temperature for 2 hours under ice water cooling. The resulting precipitate was collected by filtration to give the title compound (86 mg) as a white powder.
[MS (ESI) m/z 305.2 (M+H)⁺]
Elementary analysis as C₁₈H₁₆N₄O·HCl+0.8H₂O
Calcd. (%) C: 60.86.; H: 5.28.; N: 15.77
Found. (%) C: 60.84.; H: 5.18.; N: 15.57

### Example 54

### 6-[2-(Pyridin-2-ylmethoxy)-6,7-dihydro-5H-cyclopent a[b]pyridin-4-yl]pyrazine-2-carbonitrile hydrochloride

### [Step 1]

### Production of 4-(6-chloropyrazin-2-yl)-2-(pyridin-2-ylmethoxy)-6, 7-dihydro-5H-cyclopenta[b]pyridine

The title compound was prepared according to the procedure described in Example 5 Step 2 using 2,6-dichloropyrazine instead of 5-bromopyridin-3-ol.

### [Step 2]

### Production of 6-[2-(pyridin-2-ylmethoxy)-6,7-dihydro-5H-cyclopent a[b]pyridin-4-yl]pyrazine-2-carbonitrile hydrochloride

To 4-(6-chloropyrazin-2-yl)-2-(pyridin-2-ylmethoxy)-6, 7-dihydro-5H-cyclopenta[b]pyridine (100 mg), Pd(dppf)Cl₂·CH₂Cl₂ (24 mg), zinc cyanide (21 mg) and zinc powder (2 mg) was added DMF (2 mL), then the mixture was reacted under microwave irradiation at 190°C for 1 hour. After the reaction mixture was allowed to return to room temperature, added with water and ethyl acetate, and subjected to extraction. The organic layer was dried over anhydrous sodium sulfate, filtered off, and the solvent was evaporated under reduced pressure. The resulting residue was purified by silica gel column chromatography to give 6-[2-(pyridin-2-ylmethoxy)-6,7-dihydro-5H-cyclopent a[b]pyridin-4-yl]pyrazine-2-carbonitrile (16 mg). The resulting compound was dissolved in ethyl acetate (1 mL) and 1N HCl/Et₂O solution (0.055 mL) was added under ice water cooling, then the mixture was stirred at the same temperature for 0.5 hour. The resulting precipitate was collected by filtration to give the title compound (18 mg) as a white powder.
[MS (ESI) m/z 330.3 (M+H)⁺]

### Example 55

### 2-{6-[2-(Pyridin-2-ylmethoxy)-6,7-dihydro-5H-cyclop enta[b]pyridin-4-yl]pyrazin-2-yl}propan-2-ol

### [Step 1]

### Production of ethyl 6-[2-(pyridin-2-ylmethoxy)-6,7-dihydro-5H-cyclopent a[b]pyridin-4-yl]pyrazine-2-carboxylate

A mixture of 4-(6-chloropyrazin-2-yl)-2-(pyridin-2-ylmethoxy)-6, 7-dihydro-5H-cyclopenta[b]pyridine (170 mg), Pd(dppf)Cl₂·CH₂Cl₂ (32 mg) and DIPEA (0.26 mL) inethanol (2.5 mL) and DMF (2.5 mL) was stirred under carbon monooxide atmosphere at 100°C overnight. After the reaction mixture was allowed to return to room temperature, diluted with Et₂O, filtered through Celite, and the filtrate was evaporated under reduced pressure. The resulting residue was purified by silica gel column chromatography to give the title compound (66 mg)

### [Step 2]

### Production of 2-{6-[2-(pyridin-2-ylmethoxy)-6,7-dihydro-5H-cyclop enta[b]pyridin-4-yl]pyrazin-2-yl}propan-2-ol

To a solution of ethyl 6-[2-(pyridin-2-ylmethoxy)-6,7-dihydro-5H-cyclopent a[b]pyridin-4-yl]pyrazine-2-carboxylate (66 mg) in THF (3.5 mL) was added dropwise methylmagnesium iodide (in Et₂O solution, 0.83 mL) under Ar atmosphere under ice water cooling, and the mixture was stirred at room temperature for 5 hours. The reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (5 mg) as a white powder.
[MS (ESI) m/z 363.4 (M+H)⁺]

### Example 56

### 6-[({4-[6-(Hydroxymethyl)pyrazin-2-yl]-6,7-dihydro-5H-cyclopenta[b]pyridin-2-yl}oxy)methyl]pyridine-2-carbonitrile

To 6-({[4-(4,4,5,5-tetramethyl-1,3,2-dioxaborolan-2-yl )-6,7-dihydro-5H-cyclopenta[b]pyridin-2-yl]oxy}meth yl)pyridine-2-carbonitrile (100 mg), (6-chloropyrazin-2-yl)methanol (46 mg), Pd₂(dba)₃ (12 mg), S-Phos (22 mg) and potassium carbonate (110 mg) was added 1,4-dioxane/water (3/1, 4 mL), and the mixture was degassed, then stirred under Ar atmosphere at 100°C for 2 hours. After the reaction mixture was allowed to return to room temperature, added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (72 mg) as a white powder.
[MS (ESI) m/z 360.3 (M+H)⁺]

### Example 57

### 6-({[4-(6-Methylpyrazin-2-yl)-6,7-dihydro-5H-cyclop enta[b]pyridin-2-yl]oxy}methyl)pyridine-2-carbonitr ile

The title compound was prepared as a white powder according to the procedure described in Example 56 using 2-chloro-6-methylpyrazine instead of (6-chloropyrazin-2-yl)methanol.
[MS (ESI) m/z 344.3 (M+H)⁺]

### Example 58

### 4-(3-Fluorophenyl)-2-(pyridin-2-ylmethoxy)-6,7-dihy dro-5H-cyclopenta[b]pyridine

To 4-chloro-2-(pyridin-2-ylmethoxy)-6,7-dihydro-5H-cyc lopenta[b]pyridine (100 mg), (3-fluorophenyl)boronic acid (70 mg), Pd(OAc)₂ (1.7 mg), X-Phos (7.3 mg) and potassium carbonate (106 mg) was added ethanol (1.9 mL), and the mixture was degassed, then stirred under Ar atmosphere at 95°C for 2 hours. After the reaction mixture was allowed to return to room temperature, diluted with ethyl acetate, filtered through Celite, and the filtrate was evaporated under reduced pressure. The resulting residue was purified by silica gel column chromatography to give the title compound (106 mg) as a white solid.
[MS (ESI) m/z 322.3 (M+H)⁺]

### Example 59

### 4-(4-Fluorophenyl)-2-(pyridin-2-ylmethoxy)-6,7-dihy dro-5H-cyclopenta[b]pyridine

The title compound was prepared as a beige powder according to the procedure described in Example 58 using (4-fluorophenyl)boronic acid instead of (3-fluorophenyl)boronic acid.
[MS (ESI) m/z 321.2 (M+H)⁺]

### Example 60

### 4-(4-Methylphenyl)-2-(pyridin-2-ylmethoxy)-6,7-dihy dro-5H-cyclopenta[b]pyridine

The title compound was prepared as a white powder according to the procedure described in Example 58 using (4-methylphenyl)boronic acid instead of (3-fluorophenyl)boronic acid.
[MS (ESI) m/z 317.3 (M+H)⁺]

### Example 61

### 2-(Pyridin-2-ylmethoxy)-4-[3-(trifluoromethyl)pheny l]-6,7-dihydro-5H-cyclopenta[b]pyridine

The title compound was prepared as colorless oil according to the procedure described in Example 58 using [3-(trifluoromethyl)phenyl]boronic acid instead of (3-fluorophenyl)boronic acid.
[MS (ESI) m/z 372.2 (M+H)⁺]

### Example 62

### 2-(Pyridin-2-ylmethoxy)-4-[2-(trifluoromethyl)pheny l]-6,7-dihydro-5H-cyclopenta[b]pyridine

The title compound was prepared as colorless oil according to the procedure described in Example 58 using [2-(trifluoromethyl)phenyl]boronic acid instead of (3-fluorophenyl)boronic acid.
[MS (ESI) m/z 371.2 (M+H)⁺]

### Example 63

### 4-(3-Nitrophenyl)-2-(pyridin-2-ylmethoxy)-6,7-dihyd ro-5H-cyclopenta[b]pyridine

The title compound was prepared as a white powder according to the procedure described in Example 58 using (3-nitrophenyl)boronic acid instead of (3-fluorophenyl)boronic acid.
[MS (ESI) m/z 349.3 (M+H)⁺]

### Example 64

### 4-(3-Fluoro-4-methylphenyl)-2-(pyridin-2-ylmethoxy) -6,7-dihydro-5H-cyclopenta[b]pyridine

The title compound was prepared as colorless oil according to the procedure described in Example 5 Step2 using 4-bromo-2-fluoro-1-methylbenzene instead of 5-bromopyridin-3-ol.
[MS (ESI) m/z 335.3 (M+H)⁺]

### Example 65

### 4-(3-Fluoro-5-methylphenyl)-2-(pyridin-2-ylmethoxy) -6,7-dihydro-5H-cyclopenta[b]pyridine

The title compound was prepared as a white powder according to the procedure described in Example 5 Step2 using 1-bromo-3-fluoro-5-methylbenzene instead of 5-bromopyridin-3-ol.
[MS (ESI) m/z 335.4 (M+H)⁺]

### Example 66

### 2-Fluoro-4-[2-(pyridin-2-ylmethoxy)-6,7-dihydro-5H-cyclopenta[b]pyridin-4-yl]benzonitrile

The title compound was prepared as a white powder according to the procedure described in Example 5 Step2 using 4-bromo-2-fluorobenzonitrile instead of 5-bromopyridin-3-ol.
[MS (ESI) m/z 346.3 (M+H)⁺]

### Example 67

### 2-Fluoro-4-[2-(pyridin-2-ylmethoxy)-6,7-dihydro-5H-cyclopenta[b]pyridin-4-yl]benzamide

To 2-fluoro-4-[2-(pyridin-2-ylmethoxy)-6,7-dihydro-5H-cyclopenta[b]pyridin-4-yl]benzonitrile (25 mg) was added tert-butanol (1 mL), further added excess amounts of potassium fluoride on alumina, and the mixture was stirred under at 95°C overnight. After the reaction mixture was allowed to return to room temperature, diluted with ethyl acetate, filtered through Celite, and the filtrate was evaporated under reduced pressure. The resulting residue was purified by silica gel column chromatography to give the title compound (15 mg) as a white powder.
[MS (ESI) m/z 364.3 (M+H)⁺]

### Example 68

### 3-Fluoro-5-[2-(pyridin-2-ylmethoxy)-6,7-dihydro-5H-cyclopenta[b]pyridin-4-yl]phenol

The title compound was prepared as a white powder according to the procedure described in Example 7 Step1.
[MS (ESI) m/z 337.3 (M+H)⁺]

### Example 69

### 3-Fluoro-5-[2-(pyridin-2-ylmethoxy)-6,7-dihydro-5H-cyclopenta[b]pyridin-4-yl]phenyl methanesulfonate

To a solution of 3-fluoro-5-[2-(pyridin-2-ylmethoxy)-6,7-dihydro-5H-cyclopenta[b]pyridin-4-yl]phenol (16 mg) in CH₂Cl₂ (1 mL) was added MsCl (0.008 mL), and the mixture was stirred at room temperature overnight. The reaction mixture was purified by silica gel column chromatography to give the title compound (4 mg) as colorless oil.
[MS (ESI) m/z 415.3 (M+H)⁺]

### Example 70

### 4-(3,4-Dimethoxyphenyl)-2-(pyridin-2-ylmethoxy)-6,7 -dihydro-5H-cyclopenta[b]pyridine

The title compound was prepared as colorless oil according to the procedure described in Example 58 using (3,4-dimethoxyphenyl)boronic acid instead of (3-fluorophenyl)boronic acid.
[MS (ESI) m/z 363.3 (M+H)⁺]

### Example 71

### 4-(5-Methylpyridin-2-yl)-2-(pyridin-2-ylmethoxy)-6, 7-dihydro-5H-cyclopenta[b]pyridine

To 4-chloro-2-(pyridin-2-ylmethoxy)-6,7-dihydro-5H-cyc lopenta[b]pyridine (100 mg), 5-methyl-2-(tributylstannyl)pyridine (146 mg) and Pd(PPh₃)₄ (44 mg) was added DMF (0.5 mL), and the mixture was reacted under microwave irradiation at 160°C for 1 hour. After the reaction mixture was allowed to return to room temperature, diluted with ethyl acetate, filtered through Celite, and the filtrate was evaporated under reduced pressure. The resulting residue was purified by silica gel column chromatography to give the title compound (24 mg) as a white powder.
[MS (ESI) m/z 318.3 (M+H)⁺]

### Example 72

### 4-(6-Methylpyridin-2-yl)-2-(pyridin-2-ylmethoxy)-6, 7-dihydro-5H-cyclopenta[b]pyridine

To 4-chloro-2-(pyridin-2-ylmethoxy)-6,7-dihydro-5H-cyc lopenta[b]pyridine (100 mg), 2-methyl-6-(tributylstannyl)pyridine (147 mg) and Pd(PPh₃)₄ (44 mg) was added 1,4-dioxane (0.5 mL), and the mixture was reacted under microwave irradiation at 160°C for 1 hour. After the reaction mixture was allowed to return to room temperature, diluted with ethyl acetate, filtered through Celite, and the filtrate was evaporated under reduced pressure. The resulting residue was purified by silica gel column chromatography to give the title compound (24 mg) as a white powder.
[MS (ESI) m/z 318.3 (M+H)⁺]

### Example 73

### 4-(2-Fluoropyridin-4-yl)-2-(pyridin-2-ylmethoxy)-6, 7-dihydro-5H-cyclopenta[b]pyridine

The title compound was prepared as a white powder according to the procedure described in Example 58 using (2-fluoropyridin-2-yl)boronic acid instead of (3-fluorophenyl)boronic acid.
[MS (ESI) m/z 322.3 (M+H)⁺]

### Example 74

### 4-(Furan-2-yl)-2-(pyridin-2-ylmethoxy)-6,7-dihydro-5H-cyclopenta[b]pyridine

The title compound was prepared as a white solid according to the procedure described in Example 58 using (furan-2-yl)boronic acid instead of (3-fluorophenyl)boronic acid.
[MS (ESI) m/z 294.3 (M+H)⁺]

### Example 75

### 4-(1-Methyl-1H-pyrrol-2-yl)-2-(pyridin-2-ylmethoxy) -6,7-dihydro-5H-cyclopenta[b]pyridine

The title compound was prepared as a white powder according to the procedure described in Example 72 using 1-methyl-2-(tributylstannanyl)-1H-pyrrole instead of 2-methyl-6-(tributylstannanyl)pyridine.
[MS (ESI) m/z 306.3 (M+H)⁺]

### Example 76

### 3-[2-(Pyridin-2-ylmethoxy)-6,7-dihydro-5H-cyclopent a[b]pyridin-4-yl]quinoline

The title compound was prepared as a beige powder according to the procedure described in Example 58 using quinolin-3-ylboronic acid instead of (3-fluorophenyl)boronic acid.
[MS (ESI) m/z 354.3 (M+H)⁺]

### Example 77

### 1-Methyl-6-[2-(pyridin-2-ylmethoxy)-6,7-dihydro-5H-cyclopenta[b]pyridin-4-yl]-1H-benzimidazole

The title compound was prepared as colorless oil according to the procedure described in Example 58 using 1-methyl-6-(4,4,5,5-tetramethyl-1,3,2-dioxaborolan-2-yl)-1H-benzimidazole (see, for example, WO2009/14637) insteadof (3-fluorophenyl)boronic acid, and using Pd₂(dba)₃ instead of Pd(OAc)₂
[MS (ESI) m/z 357.3 (M+H)⁺]

### Example 78

### 1-Methyl-5-[2-(pyridin-2-ylmethoxy)-6,7-dihydro-5H-cyclopenta[b]pyridin-4-yl]-1H-benzimidazole hydrochloride

1-Methyl-5-[2-(pyridin-2-ylmethoxy)-6,7-dihydr o-5H-cyclopenta[b]pyridin-4-yl]-1H-benzimidazole (129 mg) was prepared as colorless oil according to the procedure described in Example 58 using (1-methyl-1H-benzimidazol-5-yl)boronic acid instead of (3-fluorophenyl)boronic acid.
[MS (ESI) m/z 357.3 (M+H)⁺]

The resulting compound was dissolved in ethyl acetate (4 mL), followed by the addition of 1N HCl/Et₂O solution (0.353 mL), and the mixture was stirred for 0.5 hour. The resulting precipitate was collected by filtration to give the title compound (128 mg) as a white powder.
[MS (ESI) m/z 357.3 (M+H)⁺]

### Example 79

### 4-(1,3-Benzodioxol-5-yl)-2-(pyridin-2-ylmethoxy)-6, 7-dihydro-5H-cyclopenta[b]pyridine

The title compound was prepared as a beige powder according to the procedure described in Example 58 using 1,3-benzodioxol-5-ylboronic acid instead of (3-fluorophenyl)boronic acid.
[MS (ESI) m/z 347.3 (M+H)⁺]

### Example 80

### 6-[2-(Pyridin-2-ylmethoxy)-6,7-dihydro-5H-cyclopent a[b]pyridin-4-yl]imidazo[1,2-a]pyridine

The title compound was prepared as a brown powder according to the procedure described in Example 5 Step2 using 6-bromoimidazo[1,2-a]pyridine instead of 5-bromopyridin-3-ol.
[MS (ESI) m/z 343.4 (M+H)⁺]

### Example 81

### 6-[2-(Pyridin-2-ylmethoxy)-6,7-dihydro-5H-cyclopent a[b]pyridin-4-yl]imidazo[1,2-b]pyridazine

The title compound was prepared as a white powder according to the procedure described in Example 5 Step2 using 6-chloroimidazo[1,2-b]pyridazine instead of 5-bromopyridin-3-ol.
[MS (ESI) m/z 344.4 (M+H)⁺]

### Example 82

### 4-(Pyridin-3-yl)-2-(pyridin-2-ylmethoxy)-5,6,7,8-te trahydroquinoline

### [Step 1]

### Production of 4-chloro-2-(pyridin-2-ylmethoxy)-5,6,7,8-tetrahydro quinoline

To pyridin-2-ylmethanol (119 mg) was added toluene (2 mL), followed by the addition of NaH (60% dispersion in oil, 48 mg) under ice water cooling, and the mixture was stirred for 30 minutes. After the reaction mixture was allowed to return to room temperature, 2,4-dichloro-5,6,7,8-tetrahydroquinoline (see, for example, Helvetica Chimica Acta, 1945, vol. 28, p.1684-1690) (200 mg) was added to the mixture, and the mixture was stirred at 100°C for 3 hours. After the reaction mixture was allowed to return to room temperature, added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (44 mg) as colorless oil.
[Rf value (TLC silica gel plate 60F₂₅₄, developing solvent: hexane : ethyl acetate = 2:1) : 0.5]

### [Step 2]

### Production of 4-(pyridin-3-yl)-2-(pyridin-2-ylmethoxy)-5,6,7,8-te trahydroquinoline

To 4-chloro-2-(pyridin-2-ylmethoxy)-5,6,7,8-tetrahydro quinoline (22 mg), pyridin-3-yl boronic acid (15 mg), Pd(OAc)₂ (2 mg), S-Phos (7 mg) and potassium phosphate (51 mg) was added 1,4-dioxane/water (3/1, 2 mL), and the mixture was stirred at 100°C for 2 hours. After the reaction mixture was allowed to return to room temperature, diluted with ethyl acetate, filtered through Celite, and the filtrate was evaporated under reduced pressure. The resulting residue was purified by silica gel column chromatography to give the title compound (17 mg) as colorless oil.
[MS (ESI) m/z 318.3 (M+H)⁺]

### Example83

### 2-[(6-Methylpyridin-2-yl)methoxy]-4-(pyridin-3-yl)-5,6,7,8-tetrahydroquinoline

### [Step 1]

### Production of 2-chloro-4-(pyridin-2-ylmethoxy)-5,6,7,8-tetrahydro quinoline

The title compound was prepared according to the procedure described in Example 9 Step 1 using 2,4-dichloro-5,6,7,8-tetrahydroquinoline instead of 2,4-dichloro-6,7-dihydro-5H-cyclopenta[b]pyridine.
[Rf value (TLC silica gel plate 60F₂₅₄, developing solvent: hexane : ethyl acetate = 2 : 1) : 0.3]

### [Step 2]

### Production of 2-chloro-5,6,7,8-tetrahydroquinolin-4-ol

The title compound was prepared according to the procedure described in Example 9 Step 2 using 2-chloro-4-(pyridin-2-ylmethoxy)-5,6,7,8-tetrahydro quinoline instead of 2-chloro-4-(pyridin-2-ylmethoxy)-6,7-dihydro-5H-cyc lopenta[b]pyridine.

### [Step 3]

### Production of 2-chloro-5,6,7,8-tetrahydroquinolin-4-yl trifluoromethanesulfonate

The title compound was prepared according to the procedure described in Example 9 Step 3 using 2-chloro-5,6,7,8-tetrahydroquinolin-4-ol instead of 2-chloro-6,7-dihydro-5H-cyclopenta[b]pyridin-4-ol.

### [Step 4]

### Production of 2-chloro-4-(pyridin-3-yl)-5,6,7,8-tetrahydroquinoli ne

To 2-chloro-5,6,7,8-tetrahydroquinolin-4-yl trifluoromethanesulfonate (200 mg), pyridin-3-ylboronic acid (86 mg), Pd(PPh₃)₄ (73 mg) and potassium carbonate (262 mg) was added THF/ water (3/1, 8 mL), and the mixture was degassed, then stirred under Ar atmosphere at 60°C for 3 hours. After the reaction mixture was allowed to return to room temperature, the reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (144 mg).

### [Step 5]

### Production of 2-[(6-methylpyridin-2-yl)methoxy]-4-(pyridin-3-yl)-5,6,7,8-tetrahydroquinoline

(6-Methylpyridin-2-yl)methanol (15 mg) and NaH (60% dispersion in oil, 10 mg) was dissolved in DMSO (1 mL), and the mixture was stirred for 30 minutes. Then, 2-chloro-4-(pyridin-3-yl)-5,6,7,8-tetrahydroquinoli ne (20 mg) was added to the mixture, and the mixture was stirred at 100°C for 1 hour. After the reaction mixture was allowed to return to room temperature, the reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (5 mg) as pale yellow oil.
[MS (ESI) m/z 332.2 (M+H)⁺]

### Example 84

### 2-[(5-Methylpyridin-2-yl)methoxy]-4-(pyridin-3-yl)-5,6,7,8-tetrahydroquinoline

### [Step 1]

### Production of 4-(pyridin-3-yl)-5,6,7,8-tetrahydroquinolin-2-ol

To a solution of 4-(pyridin-3-yl)-2-(pyridin-2-ylmethoxy)-5,6,7,8-te trahydroquinoline (640 mg) and NaI (1.21 g) in MeCN (20 mL) and water (2 mL) was added sulfuric acid (0.22 mL), and the mixture was heated under reflux for 1 hour. After the reaction mixture was allowed to return to room temperature, added with aqueous sodium hydrogen carbonate solution and aqueous sodium thiosulfate solution, and then the solvent was evaporated under reduced pressure. The resulting residue was diluted with chloroform/methanol (7/1), filtered through Celite, and the solvent was evaporated under reduced pressure. The residue was purified by silica gel column chromatography to give the title compound (336 mg).

### [Step 2]

### Production of 2-[(5-methylpyridin-2-yl)methoxy]-4-(pyridin-3-yl)-5,6,7,8-tetrahydroquinoline

To 4-(pyridin-3-yl)-5,6,7,8-tetrahydroquinolin-2-ol (20 mg), 2-(chloromethyl)-5-methylpyridine hydrochloride (32 mg) and silver(I) carbonate (73 mg) was added DMF (1 mL), and the mixture was stirred at 100°C for 3 hours. After the reaction mixture was allowed to return to room temperature, diluted with ethyl acetate, filtered through Celite, and the filtrate was evaporated under reduced pressure. The resulting residue was purified by silica gel column chromatography to give the title compound (7.5 mg) as colorless oil.
[MS (ESI) m/z 332.1 (M+H)⁺]

### Example 85

### 2-[(4-Methylpyridin-2-yl)methoxy]-4-(pyridin-3-yl)-5,6,7,8-tetrahydroquinoline

To 2-chloro-4-(pyridin-3-yl)-5,6,7,8-tetrahydroquinoli ne (30 mg), (4-methylpyridin-2-yl)methanol (23 mg), Pd₂(dba)₃ (7 mg), t-Bu-X-Phos (8 mg) and cesium carbonate (80 mg) was added toluene (1.5 mL), and the mixture was degassed, then stirred under Ar atmosphere at 100°C for 2 hours. After the reaction mixture was allowed to return to room temperature, the mixture was added with water ethyl acetate, and subjected to extraction. The organic layer was dried over anhydrous sodium sulfate, filtered off, and the filtrate was evaporated under reduced pressure. The resulting residue was purified by silica gel column chromatography to give the title compound (29 mg) as colorless oil.
[MS (ESI) m/z 331.9 (M+H)⁺]

### Example 86

### 2-[(6-Fluoropyridin-2-yl)methoxy]-4-(pyridin-3-yl)-5,6,7,8-tetrahydroquinoline

To 2-chloro-4-(pyridin-3-yl)-5,6,7,8-tetrahydroquinoli ne (65 mg), (6-fluoropyridin-2-yl)methanol (34 mg), Pd₂(dba)₃·CHCl₃ (17 mg), t-Bu-X-Phos (18 mg) and cesium carbonate (174 mg) was added toluene (2.6 mL), and the mixture was degassed, then stirred at 100°C for 4 hours under Ar atmosphere. The mixture was filtered through Celite and the solvent was evaporated under reduced pressure. The resulting residue was purified by silica gel column chromatography to give the title compound (26 mg) as colorless oil.
[MS (ESI) m/z 336.2 (M+H)⁺]

### Example 87

### 2-[(6-Methoxypyridin-2-yl)methoxy]-4-(pyridin-3-yl) -5,6,7,8-tetrahydroquinoline hydrochloride

2-[(6-Methoxypyridin-2-yl)methoxy]-4-(pyridin-3-yl)-5,6,7,8-tetrahydroquinoline (16 mg) was prepared according to the procedure described in Example 86 using (6-methoxypyridin-2-yl)methanol instead of (6-fluoropyridin-2-yl)methanol, and using NaO-t-Bu instead of cesium carbonate.
[MS (ESI) m/z 348.2 (M+H)⁺]

The resulting compound was dissolved in ethyl acetate (1 mL), 1N HCl/Et₂O solution (0.046 mL) was added to the solution under ice water cooling, and the mixture was stirred under ice water cooling for 0.5 hour. The resulting precipitate was collected by filtration to give the title compound (4 mg) as a pale green powder.
[MS (ESI) m/z 348.2 (M+H)⁺]

### Example 88

### [6-({[4-(Pyridin-3-yl)-5,6,7,8-tetrahydroquinolin-2 -yl]oxy}methyl)pyridin-2-yl]methanol

The title compound was prepared as a white solid according to the procedure described in Example 86 using (pyridine-2,6-diyl)bismethanol instead of (6-fluoropyridin-2-yl)methanol and using NaO-t-Bu instead of cesium carbonate.
[MS (ESI) m/z 348.2 (M+H)⁺]

### Example 89

### 2-{[6-(Methoxymethyl)pyridin-2-yl]methoxy}-4-(pyrid in-3-yl)-5,6,7,8-tetrahydroquinoline

To [6-({[4-(pyridin-3-yl)-5,6,7,8-tetrahydroquinolin-2 -yl]oxy}methyl)pyridin-2-yl]methanol (7 mg) was added THF (0.5 mL), followed by the addition of NaH (60% dispersion in oil, 2.4 mg) under ice water cooling, and the mixture was stirred for 30 minutes and then was added with methyl iodide (hereinafter referred to as "MeI") (0.0015 mL) and stirred at room temperature for 4 hours. Additionally, NaH (60% dispersion in oil, 2.4 mg) was added to the solution, and the mixture was further stirred for 2 hours. The reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (4 mg) as pale green oil.
[MS (ESI) m/z 362.3 (M+H)⁺]

### Example 90

### 4-(Pyridin-3-yl)-2-(pyrimidin-2-ylmethoxy)-5,6,7,8-tetrahydroquinoline

The title compound was prepared as colorless oil according to the procedure described in Example 86 using pyrimidin-2-ylmethanol instead of (6-fluoropyridin-2-yl)methanol.
[MS (ESI) m/z 319.2 (M+H)⁺]

### Example 91

### 2-(1,3-Oxazol-2-ylmethoxy)-4-(pyridin-3-yl)-5,6,7,8 -tetrahydroquinoline

To a solution of 2-chloro-4-(pyridin-3-yl)-5,6,7,8-tetrahydroquinoli ne (20 mg), 1,3-oxazol-2-ylmethanol (12 mg) in DMSO (1 mL) was added NaH (60% dispersion in oil, 7 mg), and the mixture was stirred at 100°C for 3 hours. After the reaction mixture was allowed to return to room temperature, the reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (11 mg) as a white powder.
[MS (ESI) m/z 308.3 (M+H)⁺]

### Example 92

### 2-[(4-Methyl-1,3-oxazol-2-yl)methoxy]-4-(pyridin-3-yl)-5,6,7,8-tetrahydroquinoline

The title compound was prepared as colorless oil according to the procedure described in Example 85 using (4-methyl-1,3-oxazol-2-yl)methanol instead of (4-methylpyridin-2-yl)methanol.
[MS (ESI) m/z 322.2 (M+H)⁺]

### Example 93

### 2-[(2-Methyl-1,3-oxazol-4-yl)methoxy]-4-(pyridin-3-yl)-5,6,7,8-tetrahydroquinoline hydrochloride

2-[(2-methyl-1,3-oxazol-4-yl)methoxy]-4-(pyrid in-3-yl)-5,6,7,8-tetrahydroquinoline (25 mg) was prepared according to the procedure described in Example 85 using (2-methyl-1,3-oxazol-4-yl)methanol instead of (4-methylpyridin-2-yl)methanol. The resulting compound was dissolved in ethyl acetate and 1N HCl/Et₂O solution (0.078 mL) was added under ice water cooling, then the mixture was stirred at the same temperature. The resulting precipitate was collected by filtration to give the title compound (20 mg) as a white powder.
[MS (ESI) m/z 322.3 (M+H)⁺]

### Example 94

### 4-(Pyridin-3-yl)-2-(1,3-thiazol-2-ylmethoxy)-5,6,7, 8-tetrahydroquinoline hydrochloride

The title compound was prepared as a white powder according to the procedure described in Example 87 using 1,3-thiazol-2-ylmethanol instead of (6-methoxypyridin-2-yl)methanol, and using 1,4-dioxane instead of toluene.
[MS (ESI) m/z 324.1 (M+H)⁺]

### Example 95

### 2-[(4-Methyl-1,3-thiazol-2-yl)methoxy]-4-(pyridin-3 -yl)-5,6,7,8-tetrahydroquinoline

The title compound was prepared as colorless oil according to the procedure described in Example 85 using (4-methyl-1,3-thiazol-2-yl)methanol instead of (4-methylpyridin-2-yl)methanol, and using NaO-t-Bu instead of cesium carbonate.
[MS (ESI) m/z 338.0 (M+H)⁺]

### Example 96

### 2-[(2-Methyl-1,3-thiazol-4-yl)methoxy]-4-(pyridin-3 -yl)-5,6,7,8-tetrahydroquinoline

The title compound was prepared as colorless oil according to the procedure described in Example 86 using (2-methyl-1,3-thiazol-4-yl)methanol instead of (4-methylpyridin-2-yl)methanol.
[MS (ESI) m/z 338.0 (M+H)⁺]

### Example 97

### 2-(1H-Imidazol-2-ylmethoxy)-4-(pyridin-3-yl)-5,6,7, 8-tetrahydroquinoline

### [Step 1]

### Production of 4-(pyridin-3-yl)-2-[(1-{[2-(trimethylsilyl)ethoxy]m ethyl}-1H-imidazol-2-yl)methoxy]-5,6,7,8-tetrahydro quinoline

The title compound was prepared according to the procedure described in Example 85 using (1-{[2-(trimethylsilyl)ethoxy]methyl}-1H-imidazol-2 -yl)methanol (see, for example, WO2007/000582) instead of (6-fluoropyridin-2-yl)methanol and using NaO-t-Bu instead of cesium carbonate.

### [Step2]

### Production of 2-(1H-imidazol-2-ylmethoxy)-4-(pyridin-3-yl)-5,6,7, 8-tetrahydroquinoline

To 4-(pyridin-3-yl)-2-[(1-{[2-(trimethylsilyl)ethoxy]m ethyl}-1H-imidazol-2-yl)methoxy]-5,6,7,8-tetrahydro quinoline (90 mg) was added CH₂Cl₂ (2 mL), ethanol (0.01 mL) and TFA (0.5 mL), and the mixture was stirred at 40°C for 10 hours and further stirred at 60°C for 2 hours. Additionally, the reaction mixture was added with TFA (0.45 mL) was added, and was further stirred at room temperature for 2 hours. The reaction mixture was added with water, ethyl acetate and potassium carbonate, and extracted with chloroform. The organic layer was dried over anhydrous sodium sulfate, filtered off, and the filtrate was evaporated under reduced pressure. The resulting residue was purified by silica gel column chromatography to give the title compound (35 mg) as colorless oil.
[MS (ESI) m/z 307.2 (M+H)⁺]

### Example 98

### 2-[(1-Methyl-1H-imidazol-2-yl)methoxy]-4-(pyridin-3 -yl)-5,6,7,8-tetrahydroquinoline

To a solution of 2-(1H-imidazol-2-ylmethoxy)-4-(pyridin-3-yl)-5,6,7, 8-tetrahydroquinoline (10 mg) in DMSO (0.5 mL) were added potassium carbonate (14 mg) and MeI (0.003 mL), and the mixture was stirred at room temperature overnight. The reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (8 mg) as colorless oil.
[MS (ESI) m/z 321.2 (M+H)⁺]

### Example 99

### 4-(4-Methylpyridin-3-yl)-2-(pyridin-2-ylmethoxy)-5, 6,7,8-tetrahydroquinoline

To 4-chloro-2-(pyridin-2-ylmethoxy)-5,6,7,8-tetrahydro quinoline (40 mg), (4-methylpyridin-3-yl)boronic acid (40 mg), Pd(dppf)Cl₂·CH₂Cl₂ (6 mg) and potassium carbonate (104 mg) was added 1,4-dioxane/water (3/1, 0.6 mL), and the mixture was stirred at 180°C for 15 minutes. After the reaction mixture was allowed to return to room temperature, diluted with ethyl acetate, filtered through Celite, and the filtrate was evaporated under reduced pressure. The resulting residue was purified by silica gel column chromatography to give the title compound (16 mg) as colorless oil.
[MS (ESI) m/z 332.3 (M+H)⁺]

### Example 100

### 4-(2-Methylpyridin-3-yl)-2-(pyridin-2-ylmethoxy)-5, 6,7,8-tetrahydroquinoline hydrochloride

To 4-chloro-2-(pyridin-2-ylmethoxy)-5,6,7,8-tetrahydro quinoline, 2-methyl-3-(4,4,5,5-tetramethyl-1,3,2-dioxaborolan-2-yl)pyridine Pd₂(dba)₃·CHCl₃ (19 mg), t-Bu-X-Phos (19 mg) and potassium carbonate (76 mg) was added 1,4-dioxane/water (3/1, 1.8 mL), and the mixture was degassed, then stirred under Ar atmosphere at 80°C for 5 hours. After the reaction mixture was allowed to return to room temperature, diluted with ethyl acetate, dried over anhydrous sodium sulfate, filtered through Celite, and the filtrate was evaporated under reduced pressure. The resulting residue was purified by silica gel column chromatography to give 4-(2-methylpyridin-3-yl)-2-(pyridin-2-ylmethoxy)-5, 6,7,8-tetrahydroquinoline. The resulting compound was dissolved in ethyl acetate, followed by the addition of 1N HCl/Et₂O solution (0.03 mL), and the mixture was stirred at room temperature overnight. The resulting precipitate was collected by filtration to give the title compound as a white powder.
[MS (ESI) m/z 332.3 (M+H)⁺]

### Example 101

### 4-(6-Fluoropyridin-3-yl)-2-(pyridin-2-ylmethoxy)-5, 6,7,8-tetrahydroquinoline dihydrochloride

To 5-bromo-2-fluoropyridine (67 mg), bis (pinacolato) diboron (116 mg), Pd₂(dba)₃·CHCl₃ (20 mg), X-Phos (36 mg) and potassium carbonate (80 mg) was added 1,4-dioxane (3.8 mL), and the mixture was degassed, then stirred under Ar atmosphere at 100°C for 2 hours. After the reaction mixture was allowed to return to room temperature, diluted with ethyl acetate, filtered through Celite, and the filtrate was evaporated under reduced pressure. The resulting residue was purified by silica gel column chromatography to give 2-fluoro-5-(4,4,5,5-tetramethyl-1,3,2-dioxaborolan-2-yl)pyridine. To the resulting compound, 4-chloro-2-(pyridin-2-ylmethoxy)-5,6,7,8-tetrahydro quinoline (70 mg), Pd(OAc)₂ (6 mg), S-Phos (21 mg), potassium carbonate (104 mg) was added 1,4-dioxane/water (3/1, 1.8 mL), and the mixture was degassed, then stirred under Ar atmosphere at 100°C for 5 hours. After the reaction mixture was allowed to return to room temperature, diluted with ethyl acetate, dried over anhydrous sodium sulfate, filtered through Celite, and the filtrate was evaporated under reduced pressure. The resulting residue was purified by silica gel column chromatography to give 4-(6-fluoropyridin-3-yl)-2-(pyridin-2-ylmethoxy)-5, 6,7,8-tetrahydroquinoline (90 mg).
[MS (ESI) m/z 336.3 (M+H)⁺]

The resulting compound was dissolved in ethyl acetate, followed by the addition of 1N HCl/Et₂O (0.268 mL), and the resulting precipitate was collected by filtration to give the title compound (38 mg) as a white powder.
[MS (ESI) m/z 336.3 (M+H)⁺]

### Example 102

### 4-(5-Methoxypyridin-3-yl)-2-(pyridin-2-ylmethoxy)-5 ,6,7,8-tetrahydroquinoline hydrochloride

The title compound was prepared as a white powder according to the procedure described in Example 101 using 3-bromo-5-methoxypyridine instead of 5-bromo-2-fluoropyridine.
[MS (ESI) m/z 348.2 (M+H)⁺]

### Example 103

### 4-(2-Methoxypyridin-3-yl)-2-(pyridin-2-ylmethoxy)-5 ,6,7,8-tetrahydroquinoline hydrochloride

### [Step 1]

### Production of 4-(2-fluoropyridin-3-yl)-2-(pyridin-2-ylmethoxy)-5, 6,7,8-tetrahydroquinoline

To 4-chloro-2-(pyridin-2-ylmethoxy)-5,6,7,8-tetrahydro quinoline (170 mg), 2-fluoro-3-(4,4,5,5-tetramethyl-1,3,2-dioxaborolan-2-yl)pyridine (207 mg), Pd(OAc)₂ (14 mg), S-Phos (51 mg) and potassium carbonate (257 mg) was added 1,4-dioxane/water (3/1, 6 mL), and the mixture was degassed, then stirred under Ar atmosphere at 100°C for 4 hours. After the reaction mixture was allowed to return to room temperature, diluted with ethyl acetate, dried over anhydrous sodium sulfate, filtered through Celite, and the solvent was evaporated under reduced pressure. The resulting residue was purified by silica gel column chromatography to give the title compound (124 mg) as a white powder.

### [Step 2]

### Production of 4-(2-methoxypyridin-3-yl)-2-(pyridin-2-ylmethoxy)-5 ,6,7,8-tetrahydroquinoline hydrochloride

To 4-(2-fluoropyridin-3-yl)-2-(pyridin-2-ylmethoxy)-5, 6,7,8-tetrahydroquinoline (25 mg) were added methanol (2 mL) and KO-t-Bu (42 mg), and the mixture was heated under reflux for 12 hours. The reaction mixture was evaporated under reduced pressure. The resulting residue was purified by silica gel column chromatography to give 4-(2-methoxypyridin-3-yl)-2-(pyridin-2-ylmethoxy)-5 ,6,7,8-tetrahydroquinoline (25 mg) as colorless oil. The resulting compound was dissolved in ethyl acetate (2 mL), followed by the addition of 1N HCl/Et₂O solution (0.075 mL), and the mixture was stirred at room temperature for 30 minutes. The resulting precipitate was collected by filtration to give the title compound (4 mg) as a white solid.
[MS (ESI) m/z 348.2 (M+H)⁺]

### Example 104

### 5-[2-(Pyridin-2-ylmethoxy)-5,6,7,8-tetrahydroquinol in-4-yl]pyridine-2-carbonitrile hydrochloride

5-[2-(pyridin-2-ylmethoxy)-5,6,7,8-tetrahydroq uinolin-4-yl]pyridine-2-carbonitrile was prepared as colorless oil according to the procedure described in Example 103 Step 1 using 5-(4,4,5,5-tetramethyl-1,3,2-dioxaborolan-2-yl)pyri dine-2-carbonitrile instead of 2-fluoro-3-(4,4,5,5-tetramethyl-1,3,2-dioxaborolan-2-yl)pyridine. The resulting compound (12 mg) was dissolved in ethyl acetate (2 mL), followed by the addition of 1N HCl/Et₂O solution (0.035 mL), and the resulting precipitate was collected by filtration to give the title compound (9.1 mg) as a white powder.
[MS (ESI) m/z 344.2 (M+H)⁺]

### Example 105

### 4-(4-Chloropyridin-3-yl)-2-(pyridin-2-ylmethoxy)-5, 6,7,8-tetrahydroquinoline hydrochloride

### [Step 1]

### Production of 2-(pyridin-2-ylmethoxy)-5,6,7,8-tetrahydroquinolin-4-ol

To 4-chloro-2-(pyridin-2-ylmethoxy)-5,6,7,8-tetrahydro quinoline (1 g), potassium hydroxide (613 mg), Pd₂(dba)₃ (33 mg) and t-Bu-X-Phos (38 mg) were added 1,4-dioxane (5 mL) and water (1 mL), and the mixture was degassed, then stirred under Ar atmosphere at 100°C for 2 hours. After the reaction mixture was allowed to return to room temperature, the mixture was added with ethyl acetate and water, and subjected to extraction. The organic layer was dried over anhydrous magnesium sulfate, filtered off, and the filtrate was evaporated under reduced pressure. The resulting residue was purified by silica gel column chromatography to give the title compound (739 mg).

### [Step 2]

### Production of 2-(pyridin-2-ylmethoxy)-5,6,7,8-tetrahydroquinolin-4-yl trifluoromethanesulfonate

The title compound was prepared according to the procedure described in Example 4 Step2 using 2-(pyridin-2-ylmethoxy)-5,6,7,8-tetrahydroquinolin-4-ol instead of 2-(pyridin-2-ylmethoxy)-6,7-dihydro-5H-cyclopenta[b ]pyridin-4-ol.

### [Step 3]

### Production of 4-(4-chloropyridin-3-yl)-2-(pyridin-2-ylmethoxy)-5, 6,7,8-tetrahydroquinoline hydrochloride

To 2-(pyridin-2-ylmethoxy)-5,6,7,8-tetrahydroquinolin-4-yl trifluoromethanesulfonate (40 mg), 4-chloro-3-(tributylstannanyl)pyridine (60 mg), LiCl (13 mg), Pd(PPh₃)₄ (24 mg) and copper iodide (3.9 mg) was added DMF (0.6 mL), and the mixture was reacted under microwave irradiation at 160°C for 30 minutes. After the reaction mixture was allowed to return to room temperature, diluted with ethyl acetate, filtered through Celite, and the filtrate was evaporated under reduced pressure. The resulting residue was purified by silica gel column chromatography to give 4-(4-chloropyridin-3-yl)-2-(pyridin-2-ylmethoxy)-5, 6,7,8-tetrahydroquinoline (15 mg) as a white powder.
[MS (ESI) m/z 352.1 (M+H)⁺]

The resulting compound was dissolved in ethyl acetate, followed by addition of 1N HCl/Et₂O solution (0.043 mL) under ice water cooling, then and the mixture was stirred at room temperature overnight. The resulting precipitate was collected by filtration to give the title compound (7 mg) as a white powder.
[MS (ESI) m/z 352.1 (M+H)⁺]

### Example 106

### 4-(6-Methoxypyridin-3-yl)-2-(pyridin-2-ylmethoxy)-5 ,6,7,8-tetrahydroquinoline hydrochloride

To 4-(6-methoxypyridin-3-yl)-2-(pyridin-2-ylmethoxy)-5 ,6,7,8-tetrahydroquinoline (45 mg) was added methanol (4 mL) and NaH (60% dispersion in oil, 16 mg), and the mixture was stirred at room temperature for 72 hours, and then at 40°C for 5 hours, further and at room temperature for 24 hours. The reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give 4-(6-methoxypyridin-3-yl)-2-(pyridin-2-ylmethoxy)-5 ,6,7,8-tetrahydroquinoline (13 mg). The resulting compound was dissolved in ethyl acetate, followed by the addition of 1N HCl/Et₂O solution (0.039 mL) under ice water cooling, and the mixture was stirred at room temperature for 1 hour. The resulting precipitate was collected by filtration to give the title compound (5 mg) as a white powder.
[MS (ESI) m/z 348.2 (M+H)⁺]

### Example 107

### 4-(5-Fluoropyridin-3-yl)-2-(pyridin-2-ylmethoxy)-5, 6,7,8-tetrahydroquinoline hydrochloride

The title compound was prepared as a white solid according to the procedure described in Example 100 using 3-fluoro-5-(4,4,5,5-tetramethyl-1,3,2-dioxaborolan-2-yl)pyridine instead of 2-methyl-3-(4,4,5,5-tetramethyl-1,3,2-dioxaborolan-2-yl)pyridine.
[MS (ESI) m/z 336.0 (M+H)⁺]

### Example 108

### 4-(2-Fluoro-5-methylpyridin-3-yl)-2-(pyridin-2-ylme thoxy)-5,6,7,8-tetrahydroquinoline

### [Step 1]

### Production of 2-(pyridin-2-ylmethoxy)-4-(4,4,5,5-tetramethyl-1,3, 2-dioxaborolan-2-yl)-5,6,7,8-tetrahydroquinoline

The title compound was prepared according to the procedure described in Example 5 Step 1 using 2-(pyridin-2-ylmethoxy)-5,6,7,8-tetrahydroquinolin-4-yl trifluoromethanesulfonate instead of 2-(pyridin-2-ylmethoxy)-6,7-dihydro-5H-cyclopenta[b ]pyridin-4-yl trifluoromethanesulfonate.

### [Step 2]

### Production of 4-(2-fluoro-5-methylpyridin-3-yl)-2-(pyridin-2-ylme thoxy)-5,6,7,8-tetrahydroquinoline

To 2-(pyridin-2-ylmethoxy)-4-(4,4,5,5-tetramethyl-1,3, 2-dioxaborolan-2-yl)-5,6,7,8-tetrahydroquinoline (50 mg), 3-bromo-2-fluoro-5-methylpyridine (34 mg), Pd(dppf)Cl₂·CH₂Cl₂ (9 mg) and potassium carbonate (57 mg) was added 1,4-dioxane/water (3/1, 1.3 mL), and the mixture was degassed, then stirred under Ar atmosphere at 100°C for 6 hours. After the reaction mixture was allowed to return to room temperature, diluted with AcOEt, dried over anhydrous sodium sulfate, filtered through Celite, and the filtrate was evaporated under reduced pressure. The resulting residue was purified by silica gel column chromatography to give the title compound (30 mg) as a white solid.
[MS (ESI) m/z 350.4 (M+H)⁺]

### Example 109

### 5-Methyl-3-[2-(pyridin-2-ylmethoxy)-5,6,7,8-tetrahy droquinolin-4-yl]pyridine-2-carbonitrile

To 4-(2-fluoro-5-methylpyridin-3-yl)-2-(pyridin-2-ylme thoxy)-5,6,7,8-tetrahydroquinoline (20 mg) and sodium cyanide (hereinafter referred to as "NaCN") (6 mg) was added DMSO (0.6 mL), and the mixture was stirred at 150°C for 4 hours. The reaction mixture was added with water and Et₂O, and subjected to extraction. The organic layer was washed with saturated brine, dried over anhydrous sodium sulfate, filtered off, and the filtrate was evaporated under reduced pressure. The resulting residue was purified by silica gel column chromatography to give the title compound (10 mg) as colorless oil.
[MS (ESI) m/z 357.4 (M+H)⁺]

### Example 110

### 4-(5-Chloro-2-fluoropyridin-3-yl)-2-(pyridin-2-ylme thoxy)-5,6,7,8-tetrahydroquinoline hydrochloride

To 2-(pyridin-2-ylmethoxy)-4-(4,4,5,5-tetramethyl-1,3, 2-dioxaborolan-2-yl)-5,6,7,8-tetrahydroquinoline (100 mg), 3-bromo-5-chloro-2-fluoropyridine (76 mg), Pd(dppf)Cl₂·CH₂Cl₂ (18 mg) and potassium carbonate (116 mg) was added 1,4-dioxane/water (3/1, 2.8 mL). The mixture was degassed, and then stirred under Ar atmosphere at 100°C for 4 hours. After the reaction mixture was allowed to return to room temperature, diluted with ethyl acetate, filtered through Celite, and the filtrate was evaporated under reduced pressure. The resulting residue was purified by silica gel column chromatography to give the title compound (64 mg) as a white powder.
[MS (ESI) m/z 370.3 (M+H)⁺]

The resulting compound (25 mg) was dissolved in Et₂O (2 mL), followed by the addition of 1N HCl/Et₂O solution (0.067 mL), and the resulting precipitate was collected by filtration to give the title compound (21 mg) as a white powder.
[MS (ESI) m/z 370.3 (M+H)⁺]

### Example 111

### 6-Fluoro-5-[2-(pyridin-2-ylmethoxy)-5,6,7,8-tetrahy droquinolin-4-yl]pyridine-3-carbonitrile

To 4-(5-chloro-2-fluoropyridin-3-yl)-2-(pyridin-2-ylme thoxy)-5,6,7,8-tetrahydroquinoline (25 mg), zinc cyanide (17 mg), Pd₂(dba)₃·CHCl₃ (4.2 mg) and S-Phos (7 mg) were added DMF (0.6 mL) and water (0.006 mL), and the mixture was reacted under microwave irradiation at 180°C for 30 minutes. After the reaction mixture was allowed to return to room temperature, added with water and ethyl acetate, and subjected to extraction. The organic layer was washed with saturated brine and saturated aqueous sodium hydrogen carbonate solution, dried over anhydrous sodium sulfate, filtered off, and the filtrate was evaporated under reduced pressure. The resulting residue was purified by silica gel column chromatography to give the title compound (8 mg) as a colorless solid.
[MS (ESI) m/z 361.4 (M+H)⁺]

### Example 112

### 3-[2-(Pyridin-2-ylmethoxy)-5,6,7,8-tetrahydroquinol in-4-yl]pyridine-2-carboxamide

### [Step 1]

### Production of 3-[2-(pyridin-2-ylmethoxy)-5,6,7,8-tetrahydroquinol in-4-yl]pyridine-2-carbonitrile

The title compound was prepared as yellow oil according to the procedure described in Example 109 using 4-(2-fluoropyridin-3-yl)-2-(pyridin-2-ylmethoxy)-5, 6,7,8-tetrahydroquinoline instead of 4-(2-fluoro-5-methylpyridin-3-yl)-2-(pyridin-2-ylme thoxy)-5,6,7,8-tetrahydroquinoline.

### [Step 2]

### Production of 3-[2-(pyridin-2-ylmethoxy)-5,6,7,8-tetrahydroquinol in-4-yl]pyridine-2-carboxamide

To 3-[2-(pyridin-2-ylmethoxy)-5,6,7,8-tetrahydroquinol in-4-yl]pyridine-2-carbonitrile (10 mg) was added tert-butanol (1 mL), further added potassium fluoride on alumina (100 mg), and the mixture was stirred at 90°C for 4 hours. After the reaction mixture was allowed to return to room temperature, diluted with ethyl acetate, filtered through Celite, and the filtrate was evaporated under reduced pressure. The resulting residue was purified by silica gel column chromatography to give the title compound (9 mg) as a white solid.
[MS (ESI) m/z 362.3 (M+H)⁺]

### Example 113

### 5-[2-(Pyridin-2-ylmethoxy)-5,6,7,8-tetrahydroquinol in-4-yl]pyridine-3-carbonitrile hydrochloride

The title compound was prepared as a white powder according to the procedure described in Example 101 using 5-bromonicotinonitrile instead of 5-bromo-2-fluoropyridine.
[MS (ESI) m/z 344.3 (M+H)⁺]
Elementary analysis as C₂₁H₁₈N₄O·HCl+0.1H₂O
Calcd. (%) C: 66.26.; H: 5.08.; N: 14.72
Found. (%) C: 66.22.; H: 5.04.; N: 14.55

### Example 114

### 2-(Pyridin-2-ylmethoxy)-4-[5-(trifluoromethyl)pyrid in-3-yl]-5,6,7,8-tetrahydroquinoline hydrochloride

To 3-bromo-5-(trifluoromethyl)pyridine (86 mg), bis (pinacolato) diboron (116 mg), Pd₂(dba)₃·CHCl₃ (20 mg), X-Phos (36 mg) and potassium carbonate (80 mg) was added 1,4-dioxane (3.8 mL), and the mixture was degassed, then stirred under Ar atmosphere at 100°C for 2 hours. After the reaction mixture was allowed to return to room temperature, diluted with ethyl acetate, filtered through Celite, and the filtrate was evaporated to give 3-(4,4,5,5-tetramethyl-1,3,2-dioxaborolan-2-yl)-5-( trifluoromethyl)pyridine. To the resulting compound (70 mg) were added Pd(OAc)₂₂ (6 mg), S-Phos (21 mg), potassium carbonate (104 mg) and 1,4-dioxane/water (3/1, 1.8 mL). The mixture was degassed, and then stirred under Ar atmosphere at 100°C for 5 hours. After the reaction mixture was allowed to return to room temperature, diluted with ethyl acetate, dried over anhydrous sodium sulfate, filtered through Celite, and the filtrate was evaporated under reduced pressure. The resulting residue was purified by silica gel column chromatography to give 2-(pyridin-2-ylmethoxy)-4-[5-(trifluoromethyl)pyrid in-3-yl]-5,6,7,8-tetrahydroquinoline (90 mg).
[MS (ESI) m/z 386.1 (M+H)⁺]

The resulting compound was dissolved in ethyl acetate (5.8 mL), followed by the addition of 1N HCl/Et₂O solution (0.288 mL), and the mixture was stirred under ice water cooling for 1 hour. The resulting precipitate was collected by filtration to give the title compound (78 mg) as a white powder.
[MS (ESI) m/z 386.3 (M+H)⁺]

### Example 115

### 1-{5-[2-(Pyridin-2-ylmethoxy)-5,6,7,8-tetrahydroqui nolin-4-yl]pyridin-3-yl}ethanone dihydrochloride

The title compound was prepared as a white powder according to the procedure described in Example 101 using 1-(5-bromopyridin-3-yl)ethanone instead of 5-bromo-2-fluoropyridine.
[MS (ESI) m/z 360.2 (M+H)⁺]
Elementary analysis as C₂₂H₂₁N₃O₂·2HCl+0.7H₂O
Calcd. (%) C: 59.38.; H: 5.53.; N: 9.44
Found. (%) C: 59.13.; H: 5.17.; N: 9.31

### Example 116

### Methyl 5-[2-(Pyridin-2-ylmethoxy)-5,6,7,8-tetrahydroquinol in-4-yl]pyridine-3-carboxylate

To 4-chloro-2-(pyridin-2-ylmethoxy)-5,6,7,8-tetrahydro quinoline (59 mg), ethyl 5-(4,4,5,5-tetramethyl-1,3,2-dioxaborolan-2-yl)pyri dine-3-carboxylate (78 mg), Pd₂(dba)₃·CHCl₃ (8.2 mg) and potassium carbonate (140 mg) was added 1,4-dioxane/water (3/1, 1 mL), and the mixture was stirred at 100°C for 24 hours. After the reaction mixture was allowed to return to room temperature, diluted with ethyl acetate, filtered through Celite, and the filtrate was evaporated under reduced pressure. The resulting residue was purified by silica gel column chromatography eluting ethyl acetate/methanol to give the title compound (3 mg) as colorless oil.
[MS (ESI) m/z 376.3 (M+H)⁺]

### Example 117

### 5-[2-(Pyridin-2-ylmethoxy)-5,6,7,8-tetrahydroquinol in-4-yl]pyridine-3-carboxylic acid

### [Step 1]

### Production of ethyl 5-[2-(pyridin-2-ylmethoxy)-5,6,7,8-tetrahydroquinol in-4-yl]pyridine-3-carboxylate

The title compound was prepared as pale yellow oil according to the procedure described in Example 103 Step1 using ethyl 5-(4,4,5,5-tetramethyl-1,3,2-dioxaborolan-2-yl)pyri dine-3-carboxylate instead of 2-fluoro-3-(4,4,5,5-tetramethyl-1,3,2-dioxaborolan-2-yl)pyridine.

### [Step 2]

### Production of 5-[2-(pyridin-2-ylmethoxy)-5,6,7,8-tetrahydroquinol in-4-yl]pyridine-3-carboxylic acid

To ethyl 5-[2-(pyridin-2-ylmethoxy)-5,6,7,8-tetrahydroquinol in-4-yl]pyridine-3-carboxylate (13 mg) was added dimethylamine/water (40%, 1 mL), and the mixture was reacted under microwave irradiation at 140°C for 0.5 hour. The reaction mixture was purified by silica gel column chromatography to give the title compound (6 mg) as a white powder.
[MS (ESI) m/z 362.2 (M+H)⁺]

### Example 118

### N-Methyl-5-[2-(pyridin-2-ylmethoxy)-5,6,7,8-tetrahy droquinolin-4-yl]pyridine-3-carboxamide hydrochloride

A mixture of ethyl 5-[2-(pyridin-2-ylmethoxy)-5,6,7,8-tetrahydroquinol in-4-yl]pyridine-3-carboxylate (15 mg) and methylamine/methanol (1 mL) was reacted under microwave irradiation at 140°C for 0.5 hour. The solvent of the reaction mixture was evaporated under reduced pressure and the resulting residue was purified by silica gel column chromatography to give N-methyl-5-[2-(pyridin-2-ylmethoxy)-5,6,7,8-tetrahy droquinolin-4-yl]pyridine-3-carboxamide (14 mg) as colorless oil.
[MS (ESI) m/z 375.3 (M+H)⁺]

The resulting compound was dissolved in ethyl acetate (1 mL), followed by addition of 1N HCl/Et₂O solution (0.039 mL), and then the resulting precipitate was collected by filtration to give the title compound (9 mg) as a white powder.
[MS (ESI) m/z 375.3 (M+H)⁺]

### Example 119

### N-Cyclopropyl-5-[2-(pyridin-2-ylmethoxy)-5,6,7,8-te trahydroquinolin-4-yl]pyridine-3-carboxamide hydrochloride

To 5-[2-(pyridin-2-ylmethoxy)-5,6,7,8-tetrahydroquinol in-4-yl]pyridine-3-carboxylic acid (20 mg), cyclopropylamine (16 mg), 2-(7-aza-1H-benzotriazole-1-yl)-1,1,3,3-tetramethyl uronium hexafluorophosphate (hereinafter referred to as "HATU") (44 mg) and Et₃N (0.04 mL) was added DMF (4 mL), and then the mixture was stirred at room temperature for 24 hours. The reaction mixture was added with water and ethyl acetate, and subjected to extraction. The organic layer was washed with saturated brine, dried over anhydrous sodium sulfate, filtered off, and the filtrate was evaporated under reduced pressure. The resulting residue was purified by silica gel column chromatography to give N-cyclopropyl-5-[2-(pyridin-2-ylmethoxy)-5,6,7,8-te trahydroquinolin-4-yl]pyridine-3-carboxamide (17 mg) as a white powder.
[MS (ESI) m/z 401.3 (M+H)⁺]

The resulting compound was dissolved in ethyl acetate (2 mL), followed by addition of 1N HCl/Et₂O solution (0.039 mL), then the solvent of the reaction mixture was evaporated under reduced pressure. Ethyl acetate was added to the resulting residue, and the insoluble portion was collected by filtration to give the title compound (5 mg) as a white powder.
[MS (ESI) m/z 401.3 (M+H)⁺]

### Example 120

### N,N-Dimethyl-5-[2-(pyridin-2-ylmethoxy)-5,6,7,8-tet rahydroquinolin-4-yl]pyridine-3-carboxamide hydrochloride

To ethyl 5-[2-(pyridin-2-ylmethoxy)-5,6,7,8-tetrahydroquinol in-4-yl]pyridine-3-carboxylate (12 mg) was added dimethylamine/water (0.5 mL), and the mixture was reacted under microwave irradiation at 140°C for 15 minutes. The reaction mixture was filtered through Celite, and the filtrate was evaporated under reduced pressure. To the resulting residue were added dimethylamine, HATU, Et₃N and methanol, then the mixture was reacted under microwave irradiation at 140°C for 15 minutes. The reaction mixture was filtered through Celite, and the filtrate was evaporated under reduced pressure. The resulting residue was purified by silica gel column chromatography to give N,N-dimethyl-5-[2-(pyridin-2-ylmethoxy)-5,6,7,8-tet rahydroquinolin-4-yl]pyridine-3-carboxamide (14 mg) as colorless oil.
[MS (ESI) m/z 389.3 (M+H)⁺]

The resulting compound was dissolved in ethyl acetate, followed by the addition of 1N HCl/Et₂O solution (0.036 mL). The resulting precipitate was collected by filtration to give the title compound (4 mg) as a white powder.
[MS (ESI) m/z 389.3 (M+H)⁺]

### Example 121

### N-(Cyclopropylmethyl)-5-[2-(pyridin-2-ylmethoxy)-5, 6,7,8-tetrahydroquinolin-4-yl]pyridine-3-carboxamid e

To 5-[2-(pyridin-2-ylmethoxy)-5,6,7,8-tetrahydroquinol in-4-yl]pyridine-3-carboxylic acid (25 mg), cyclopropylmethylamine (49 mg), HATU (52 mg) and DIPEA (0.12 mL) were added DMF (4 mL) and MeCN (0.004 mL), and the mixture was stirred at 40°C for 24 hours. Cyclopropylmethylamine (49 mg), HATU (52 mg) and DIPEA (0.12 mL) were further added, and the mixture was stirred at 40°C for 24 hours. The reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compounds (7 mg) as colorless oil.
[MS (ESI) m/z 415.3 (M+H)⁺]

### Example 122

### {5-[2-(Pyridin-2-ylmethoxy)-5,6,7,8-tetrahydroquino lin-4-yl]pyridin-3-yl}methanol hydrochloride

To ethyl 5-[2-(pyridin-2-ylmethoxy)-5,6,7,8-tetrahydroquinol in-4-yl]pyridine-3-carboxylate (35 mg) were added THF (1 mL), NaBH₄ (23 mg) and MeOH, and the mixture was stirred at 50°C for 3 hours. The reaction mixture was added with saturated aqueous potassium carbonate solution, and stirred at room temperature for 1 hour. The reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give {5-[2-(pyridin-2-ylmethoxy)-5,6,7,8-tetrahydroq uinolin-4-yl]pyridin-3-yl}methanol (23 mg) as colorless oil.
[MS (ESI) m/z 348.3 (M+H)⁺]

The resulting compound (10 mg) was dissolved in ethyl acetate (1 mL),followed by the addition of 1N HCl/Et₂O solution (0.029 mL). The resulting precipitate was collected by filtration to give the title compound (6 mg) as a white powder.
[MS (ESI) m/z 348.3 (M+H)⁺]

### Example 123

### 4-[5-(Methoxymethyl)pyridin-3-yl]-2-(pyridin-2-ylme thoxy)-5,6,7,8-tetrahydroquinoline hydrochloride

To {5-[2-(pyridin-2-ylmethoxy)-5,6,7,8-tetrahydroquino lin-4-yl]pyridin-3-yl}methanol (10 mg) were added THF (0.5mL), followed by the addition of NaH (60% dispersion in oil, 2.4 mg) under ice water cooling, and the mixture was stirred for 30 minutes. After that, methyl iodide (hereinafter referred to as "MeI") (0.0015 mL) was added, and stirred at room temperature overnight. The reaction mixture was diluted with ethyl acetate, filtered through Celite, and the filtrate was evaporated under reduced pressure. The resulting residue was purified by silica gel column chromatography to give 4-[5-(methoxymethyl)pyridin-3-yl]-2-(pyridin-2-ylme thoxy)-5,6,7,8-tetrahydroquinoline (12 mg) as yellow oil. The resulting compound was dissolved in ethyl acetate, followed by the addition of 1N HCl/Et₂O solution (0.028 mL). The resulting precipitate was collected by filtration to give the title compound (5 mg) as a pale yellow powder.
[MS (ESI) m/z 362.3 (M+H)⁺]

### Example 124

### 5-[2-(Pyridin-2-ylmethoxy)-5,6,7,8-tetrahydroquinol in-4-yl]pyridin-3-amine

The title compound (47 mg) was prepared as a white solid according to the procedure described in Example 103 Step 1 using 5-(4,4,5,5-tetramethyl-1,3,2-dioxaborolan-2-yl)pyri din-3-amine instead of 2-fluoro-3-(4,4,5,5-tetramethyl-1,3,2-dioxaborolan-2-yl)pyridine.
[MS (ESI) m/z 333.2 (M+H)⁺]

### Example 125

### N-Methyl-5-[2-(pyridin-2-ylmethoxy)-5,6,7,8-tetrahy droquinolin-4-yl]pyridin-3-amine

To 5-[2-(pyridin-2-ylmethoxy)-5,6,7,8-tetrahydroquinol in-4-yl]pyridin-3-amine (24 mg), formaldehyde (0.008 mL) and sodium triacetoxyborohydride (61 mg) was added MeCN (1.5 mL), and the mixture was stirred at room temperature overnight. The reaction mixture was added with water, ethyl acetate and saturated aqueous sodium hydrogen carbonate solution, and subjected to extraction. The organic layer was dried over anhydrous sodium sulfate, filtered off, and the filtrate was evaporated under reduced pressure. The resulting residue was purified by silica gel column chromatography to give the title compound (3 mg) as a white powder.
[MS (ESI) m/z 347.2 (M+H)⁺]

### Example 126

### N,N-Dimethyl-5-[2-(pyridin-2-ylmethoxy)-5,6,7,8-tet rahydroquinolin-4-yl]pyridin-3-amine

To 5-[2-(pyridin-2-ylmethoxy)-5,6,7,8-tetrahydroquinol in-4-yl]pyridin-3-amine (24 mg), formaldehyde (0.033 mL) and sodium triacetoxyborohydride (92 mg) was added MeCN (1.5 mL), and the mixture was stirred at room temperature overnight. The reaction mixture was added with water, ethyl acetate and saturated aqueous sodium hydrogen carbonate solution, and subjected to extraction. The organic layer was dried over anhydrous sodium sulfate, filtered off, and the filtrate was evaporated under reduced pressure. The resulting residue was purified by silica gel column chromatography to give the title compound (8 mg) as pale yellow oil.
[MS (ESI) m/z 361.2 (M+H)⁺]

### Example 127

### tert-Butyl [2-({5-[2-(pyridin-2-ylmethoxy)-5,6,7,8-tetrahydroq uinolin-4-yl]pyridin-3-yl}amino)ethyl]carbamate

To tert-butyl {2-[(5-bromopyridin-3-yl)amino]ethyl}carbamate (see, for example, US2003/187026)(120 mg), bis (pinacolato) diboron (116 mg), Pd₂(dba)₃·CHCl₃ (20 mg), X-Phos (36 mg) and potassium acetate (80 mg) was added 1,4-dioxane (7.6 mL), and the mixture was stirred at 100°C for 4 hours. After the reaction mixture was allowed to return to room temperature, diluted with ethyl acetate, filtered through Celite, and the filtrate was evaporated. To the resulting residue were added 4-chloro-2-(pyridin-2-ylmethoxy)-5,6,7,8-tetrahydro quinoline (35 mg) Pd(OAc)₂ (6 mg), S-Phos(21 mg), potassium carbonate (100 mg) and 1,4-dioxane/water (3/1, 3.6 mL) . The mixture was stirred at 100°C for 4 hours. After the reaction mixture was allowed to return to room temperature, diluted with ethyl acetate, dried over anhydrous sodium sulfate, filtered through Celite, and the filtrate was evaporated under reduced pressure. The resulting residue was purified by silica gel column chromatography to give the title compound (49 mg) as colorless oil.
[MS (ESI) m/z 476.3 (M+H)⁺]

### Example 128

### N-{5-[2-(Pyridin-2-ylmethoxy)-5,6,7,8-tetrahydroqui nolin-4-yl]pyridin-3-yl}ethane-1,2-diamine

To tert-butyl [2-({5-[2-(pyridin-2-ylmethoxy)-5,6,7,8-tetrahydroq uinolin-4-yl]pyridin-3-yl}amino)ethyl]carbamate (37 mg) was added THF (2 mL), followed by the addition of 10% hydrochloric acid (2 mL) under ice water cooling, and the reaction mixture was stirred at room temperature for 10 hours. The reaction mixture was added with water, CHCl₃ and potassium carbonate, and subjected to extraction. The organic layer was dried over anhydrous sodium sulfate, filtered off, and the filtrate was evaporated under reduced pressure to give the title compound (24 mg) as pale yellow oil.
[MS (ESI) m/z 376.2 (M+H)⁺]

### Example 129

### N,N-Dimethyl-N'-{5-[2-(pyridin-2-ylmethoxy)-5,6,7,8 -tetrahydroquinolin-4-yl]pyridin-3-yl}ethane-1,2-di amine

To N-{5-[2-(pyridin-2-ylmethoxy)-5,6,7,8-tetrahydroqui nolin-4-yl]pyridin-3-yl}ethane-1,2-diamine (20 mg) were added MeCN (2 mL), Et₃N (0.074 mL) and methyl p-toluenesulfonate (60 mg), and the reaction mixture was stirred 50°C for 10 hours. The reaction mixture was added with saturated aqueous sodium hydrogen carbonate solution and chloroform, and subjected to extraction. The organic layer was dried over anhydrous sodium sulfate, filtered off, and the filtrate was evaporated under reduced pressure to give the title compound (3 mg) as yellow oil.
[MS (ESI) m/z 404.3 (M+H)⁺]

### Example 130

### 4-(5-Cyclopropylpyridin-3-yl)-2-(pyridin-2-ylmethox y)-5,6,7,8-tetrahydroquinoline hydrochloride

The title compound was prepared as a white powder according to the procedure described in Example 100 using 3-cyclopropyl-5-(4,4,5,5-tetramethyl-1,3,2-dioxabor olan-2-yl)pyridine instead of 2-methyl-3-(4,4,5,5-tetramethyl-1,3,2-dioxaborolan-2-yl)pyridine.
[MS (ESI) m/z 358.3 (M+H)⁺]

### Example 131

### 1-{5-[2-(Pyridin-2-ylmethoxy)-5,6,7,8-tetrahydroqui nolin-4-yl]pyridin-3-yl}azetidin-2-one hydrochloride

### [Step 1]

### Production of 1-(5-bromopyridin-3-yl)azetidin-2-one

To 3,5-dibromopyridine, azetidin-2-one copper iodide and potassium carbonate was added toluene, and the mixture was stirred at 100°C for 6 hours. After the reaction mixture was allowed to return to room temperature, diluted with ethyl acetate, dried over anhydrous sodium sulfate, filtered through Celite, and the filtrate was evaporated under reduced pressure to give the title compound as yellow oil.

### [Step 2]

### Production of 1-{5-[2-(pyridin-2-ylmethoxy)-5,6,7,8-tetrahydroqui nolin-4-yl]pyridin-3-yl}azetidin-2-one hydrochloride

To 1-(5-bromopyridin-3-yl)azetidin-2-one (62 mg), bis (pinacolato) diboron (83 mg), Pd₂(dba)₃·CHCl₃ (14 mg), X-Phos (26 mg) and potassium acetate (80 mg) was added 1,4-dioxane (5.5 mL), and the mixture was stirred at 100°C. After the reaction mixture was allowed to return to room temperature, diluted with ethyl acetate, filtered through Celite, and the filtrate was evaporated under reduced pressure. To the resulting residue were added 4-chloro-2-(pyridin-2-ylmethoxy)-5,6,7,8-tetrahydro quinoline (56 mg), Pd₂(dba)₃·CHCl₃ (14 mg), t-Bu-X-Phos (23 mg), potassium carbonate and 1,4-dioxane/water (3/1, 3.6 mL) . The mixture was stirred at 100°C for 5 hours. After the reaction mixture was allowed to return to room temperature, diluted with ethyl acetate, dried over anhydrous sodium sulfate, filtered through Celite, and the filtrate was evaporated under reduced pressure. The resulting residue was purified by silica gel column chromatography to give 1-{5-[2-(pyridin-2-ylmethoxy)-5,6,7,8-tetrahydroqui nolin-4-yl]pyridin-3-yl}azetidin-2-one (58 mg) as yellow oil.
[MS (ESI) m/z 387.2 (M+H)⁺]

The resulting compound was dissolved in ethyl acetate, followed by the addition of 1N HCl/Et₂O solution (0.148 mL), and the resulting precipitate was collected by filtration to give the title compound (63 mg) as a white powder.
[MS (ESI) m/z 387.2 (M+H)⁺]

### Example 132

### N-{5-[2-(Pyridin-2-ylmethoxy)-5,6,7,8-tetrahydroqui nolin-4-yl]pyridin-3-yl}acetamide hydrochloride

To 5-[2-(pyridin-2-ylmethoxy)-5,6,7,8-tetrahydroquinol in-4-yl]pyridin-3-amine (20 mg) were added THF (1 mL), Et₃N (0.025 mL) and acetyl chloride (0.007 mL), and the mixture was stirred at room temperature for 3 hours. The reaction mixture was added with saturated aqueous sodium hydrogen carbonate solution and ethyl acetate, and subjected to extraction. The organic layer was dried over anhydrous sodium sulfate, filtered off, and the filtrate was evaporated under reduced pressure. The resulting residue was purified by silica gel column chromatography to give N-{5-[2-(pyridin-2-ylmethoxy)-5,6,7,8-tetrahydroqui nolin-4-yl]pyridin-3-yl}acetamide (20 mg) as colorless oil. The resulting compound was dissolved in ethyl acetate, followed by the addition of 1N HCl/Et₂O solution (0.148 mL), and the resulting precipitate was collected by filtration to give the title compound (9 mg) as a white powder.
[MS (ESI) m/z 375.2 (M+H)⁺]

### Example 133

### 4-[5-(1,1-Dioxidothiomorpholin-4-yl)pyridin-3-yl]-2 -(pyridin-2-ylmethoxy)-5,6,7,8-tetrahydroquinoline hydrochloride

### [Step 1]

### Production of 4-(5-bromopyridin-3-yl)thiomorpholine 1,1-dioxide

To 3,5-dibromopyridine (500 mg), thiomorpholine 1,1-dioxide (343 mg), Pd₂(dba)₃·CHCl₃ (110 mg), 4,5-bis(diphenylphosphino)-9,9-dimethylxanthene (123 mg) and NaO-t-Bu (305 mg) was added toluene (6 mL) under Ar atmosphere and the reaction mixture was stirred at 100°C for 1 hour. The mixture was filtered through Celite, and the filtrate was evaporated under reduced pressure. The resulting residue was purified by silica gel column chromatography to give the title compound (324 mg) as a brown solid.

### [Step 2]

### Production of 4-[5-(1,1-dioxidothiomorpholin-4-yl)pyridin-3-yl]-2 -(pyridin-2-ylmethoxy)-5,6,7,8-tetrahydroquinoline hydrochloride

4-[5-(1,1-Dioxidothiomorpholin-4-yl)pyridin-3-yl]-2-(pyridin-2-ylmethoxy)-5,6,7,8-tetrahydroquino line was prepared as yellow oil according to the procedure described in Example 127 using 4-(5-bromopyridin-3-yl)thiomorpholine 1,1-dioxide instead of tert-butyl {2-[(5-bromopyridin-3-yl)amino]ethyl}carbamate.
[MS (ESI) m/z 451.2 (M+H)⁺]

The resulting compound (92 mg) was dissolved in ethyl acetate, followed by the addition of 1N HCl/Et₂O solution (0.204 mL), and the resulting precipitate was collected by filtration to give the title compound (50 mg) as a white powder.
[MS (ESI) m/z 451.2 (M+H)⁺]

### Example 134

### 4-[5-(Azetidin-3-yloxy)pyridin-3-yl]-2-(pyridin-2-y lmethoxy)-5,6,7,8-tetrahydroquinoline hydrochloride

### [Step 1]

### Production of tert-butyl 3-[(5-bromopyridin-3-yl)oxy]azetidine-1-carboxylate

To 5-bromopyridin-3-ol (500 mg), tert-butyl 3-hydroxyazetidine-1-carboxylate (747 mg) and PPh₃ (1.13 g) were added toluene (15 mL) and diethyl azodicarboxylate (2.2 M in toluene solution, 2 mL), and the mixture was stirred at 90°C for 2 hours. The solvent of the reaction mixture was evaporated under reduced pressure. The resulting residue was purified by silica gel column chromatography to give the title compound (820 mg) as a pink solid.

### [Step 2]

### Production of tert-butyl 3-({5-[2-(pyridin-2-ylmethoxy)-5,6,7,8-tetrahydroqu inolin-4-yl]pyridin-3-yl}oxy)azetidine-1-carboxylat e

The title compound was prepared as yellow oil according to the procedure described in Example 127 using tert-butyl 3-[(5-bromopyridin-3-yl)oxy]azetidine-1-carboxylate instead of tert-butyl {2-[(5-bromopyridin-3-yl)amino]ethyl}carbamate.

### [Step 3]

### Production of 4-[5-(azetidin-3-yloxy)pyridin-3-yl]-2-(pyridin-2-y lmethoxy)-5,6,7,8-tetrahydroquinoline hydrochloride

To tert-butyl 3-({5-[2-(pyridin-2-ylmethoxy)-5,6,7,8-tetrahydroqu inolin-4-yl]pyridin-3-yl}oxy)azetidine-1-carboxylat e (130 mg) were added THF (2.7 mL) and 10% hydrochloric acid (2.7 mL) and the mixture was stirred at room temperature overnight. The reaction mixture was neutralized by addition of potassium carbonate, and then extracted with CHCl₃. The organic layer was dried over anhydrous sodium sulfate, filtered off, and the filtrate was evaporated under reduced pressure. The resulting residue was purified by silica gel column chromatography to give 4-[5-(azetidin-3-yloxy)pyridin-3-yl]-2-(pyridin-2-y lmethoxy)-5,6,7,8-tetrahydroquinoline.
[MS (ESI) m/z 389.2 (M+H)⁺]

The resulting compound (18 mg) was dissolved in ethyl acetate, followed by the addition of 1N HCl/Et₂O solution (0.046 mL), and the resulting precipitate was collected by filtration to give the title compound (11 mg) as a white powder.
[MS (ESI) m/z 389.2 (M+H)⁺]

### Example 135

### 1-[3-({5-[2-(Pyridin-2-ylmethoxy)-5,6,7,8-tetrahydr oquinolin-4-yl]pyridin-3-yl}oxy)azetidin-1-yl]ethan one

To 4-[5-(azetidin-3-yloxy)pyridin-3-yl]-2-(pyridin-2-y lmethoxy)-5,6,7,8-tetrahydroquinoline (34 mg) were added THF (1 mL), Et₃N (0.037 mL) and acetyl chloride (0.01 mL), and then the mixture was stirred at room temperature overnight. The reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (30 mg) as a white amorphous.
[MS (ESI) m/z 431.2 (M+H)⁺]

### Example 136

### 4-{5-[(1-Methylazetidin-3-yl)oxy]pyridin-3-yl}-2-(p yridin-2-ylmethoxy)-5,6,7,8-tetrahydroquinoline

To 4-[5-(azetidin-3-yloxy)pyridin-3-yl]-2-(pyridin-2-y lmethoxy)-5,6,7,8-tetrahydroquinoline (34 mg) were added THF (1 mL) and cesium carbonate (51 mg), and followed by addition of MeI (0.0065 mL) under ice water cooling, and the mixture was stirred at room temperature for 12 hours. The reaction mixture was filtered through Celite, and the filtrate was evaporated under reduced pressure. The resulting residue was purified by silica gel column chromatography to give the title compound (10 mg) as colorless oil.
[MS (ESI) m/z 403.2 (M+H)⁺]

### Example 137

### 4-(2-Fluoropyridin-3-yl)-2-[(6-fluoropyridin-2-yl)m ethoxy]-5,6,7,8-tetrahydroquinoline

### [Step 1]

### Production of 2-chloro-4-(4,4,5,5-tetramethyl-1,3,2-dioxaborolan-2-yl)-5,6,7,8-tetrahydroquinoline

The title compound was prepared as a white solid according to the procedure described in Example 9 Step 4 using 2-chloro-5,6,7,8-tetrahydroquinolin-4-yl trifluoromethanesulfonate instead of 2-chloro-6,7-dihydro-5H-cyclopenta[b]pyridin-4-yl trifluoromethanesulfonate.

### [Step 2]

### Production of 2-chloro-4-(2-fluoropyridin-3-yl)-5,6,7,8-tetrahydr oquinoline

To 2-chloro-4-(4,4,5,5-tetramethyl-1,3,2-dioxaborolan-2-yl)-5,6,7,8-tetrahydroquinoline (400 mg), 2-fluoro-3-iodopyridine (365 mg), Pd(dppf)Cl₂·CH₂Cl₂ (89 mg) and potassium carbonate (565 mg) was added THF/water (3/1, 6.2 mL) and the mixture was degassed, then stirred under Ar atmosphere at 60°C for 4 hours. After the reaction mixture was allowed to return to room temperature, diluted with ethyl acetate, filtered through Celite, and the filtrate was evaporated under reduced pressure. The resulting residue was purified by silica gel column chromatography to give the title compound (262 mg) as a white powder.

### [Step 3]

### Production of 4-(2-fluoropyridin-3-yl)-2-[(6-fluoropyridin-2-yl)m ethoxy]-5,6,7,8-tetrahydroquinoline

To 2-chloro-4-(2-fluoropyridin-3-yl)-5,6,7,8-tetrahydr oquinoline (62 mg), (6-fluoropyridin-2-yl)methanol (36 mg), Pd₂(dba)₃·CHCl₃ (15 mg), t-Bu-X-Phos (24 mg) and cesium carbonate (231 mg) was added toluene (2.6 mL), and the mixture was degassed, then stirred under Ar atmosphere at 100°C for 4 hours. After the reaction mixture was allowed to return to room temperature, diluted with ethyl acetate, filtered through Celite, and the filtrate was evaporated under reduced pressure. The resulting residue was purified by silica gel column chromatography to give the title compound as colorless oil.
[MS (ESI) m/z 354.4 (M+H)⁺]

### Example 138

### 4-(2-Fluoropyridin-3-yl)-2-[(5-fluoropyridin-2-yl)m ethoxy]-5,6,7,8-tetrahydroquinoline hydrochloride

4-(2-Fluoropyridin-3-yl)-2-[(5-fluoropyridin-2 -yl)methoxy]-5,6,7,8-tetrahydroquinoline was prepared as colorless oil according to the procedure described in Example 137 Step 3 using (5-fluoropyridin-2-yl)methanol instead of (6-fluoropyridin-2-yl)methanol. The resulting compound (96 mg) was dissolved in ethyl acetate (2.7 mL), followed by the addition of 1N HCl/Et₂O solution (0.3 mL), and the mixture was stirred at room temperature for 0.5 hour. The resulting precipitate was collected by filtration to give the title compound (113 mg) as a white powder.
[MS (ESI) m/z 354.4 (M+H)⁺]

### Example 139

### 4-(2-Fluoropyridin-3-yl)-2-[(3-fluoropyridin-2-yl)m ethoxy]-5,6,7,8-tetrahydroquinoline

The title compound was prepared as colorless oil according to the procedure described in Example 137 Step 3 using (3-fluoropyridin-2-yl)methanol instead of (6-fluoropyridin-2-yl)methanol.
[MS (ESI) m/z 354.4 (M+H)⁺]

### Example 140

### 5-{2-[(6-Fluoropyridin-2-yl)methoxy]-5,6,7,8-tetrah ydroquinolin-4-yl}pyridine-2-carbonitrile

### [Step 1]

### Production of 5-(2-chloro-5,6,7,8-tetrahydroquinolin-4-yl)pyridin e-2-carbonitrile

The title compound was prepared as a white powder according to the procedure described in Example 137 Step 2 using 5-bromopyridine-2-carbonitrile instead of 2-fluoro-3-iodopyridine and using 1,4-dioxane instead of THF.

### [Step 2]

### Production of 5-{2-[(6-fluoropyridin-2-yl)methoxy]-5,6,7,8-tetrah ydroquinolin-4-yl}pyridine-2-carbonitrile

To 5-(2-chloro-5,6,7,8-tetrahydroquinolin-4-yl)pyridin e-2-carbonitrile (90 mg), (6-fluoropyridin-2-yl)methanol (55 mg), Pd₂(dba)₃ (31 mg), t-Bu-X-Phos (28 mg) and cesium carbonate (218 mg) was added toluene (3 mL) and the mixture was degassed, then stirred under Ar atmosphere at 100°C for 8 hours. After the reaction mixture was allowed to return to room temperature, diluted with ethyl acetate, filtered through Celite, and the filtrate was evaporated under reduced pressure. The resulting residue was purified by silica gel column chromatography to give the title compound (57 mg) as a white powder.
[MS (ESI) m/z 361.3 (M+H)⁺]

### Example 141

### 5-{2-[(3-Fluoropyridin-2-yl)methoxy]-5,6,7,8-tetrah ydroquinolin-4-yl}pyridine-2-carbonitrile

The title compound was prepared as colorless oil according to the procedure described in Example 140 Step 2 using (3-fluoropyridin-2-yl)methanol instead of (6-fluoropyridin-2-yl)methanol, and using NaO-t-Bu instead of cesium carbonate.
[MS (ESI) m/z 361.3 (M+H)⁺]

### Example 142

### 5-{2-[(3,5-Difluoropyridin-2-yl)methoxy]-5,6,7,8-te trahydroquinolin-4-yl}pyridine-2-carbonitrile

The title compound was prepared as colorless oil according to the procedure described in Example 140 Step 2 using (3,5-difluoropyridin-2-yl)methanol instead of (6-fluoropyridin-2-yl)methanol, and using NaO-t-Bu instead of cesium carbonate.
[MS (ESI) m/z 379.3 (M+H)⁺]

### Example 143

### 2-(Pyridin-2-ylmethoxy)-4-(pyrimidin-5-yl)-5,6,7,8-tetrahydroquinoline hydrochloride

2-(Pyridin-2-ylmethoxy)-4-(pyrimidin-5-yl)-5,6 ,7,8-tetrahydroquinoline was prepared as a pale yellow solid according to the procedure described in Example 82 Step 2 using pyrimidin-5-ylboronic acid instead of pyridin-3-ylboronic acid, and using potassium carbonate instead of potassium phosphate. The resulting compound (100 mg) was dissolved in ethyl acetate (6.2 mL), followed by addition of 1N HCl/Et₂O solution (0.3 mL) under ice water cooling, and the mixture was stirred for 1 hour. The resulting precipitate was collected by filtration to give the title compound (91 mg) as a white powder.
[MS (ESI) m/z 318.9 (M+H)⁺]
Elementary analysis as C₁₉H₁₈N₄O·HCl+0.1H₂O
Calcd. (%) C: 63.99.; H: 5.43.; N: 15.71
Found. (%) C: 63.83.; H: 5.42.; N: 15.53

### Example 144

### 2-[(6-Fluoropyridin-2-yl)methoxy]-4-(pyrimidin-5-yl )-5,6,7,8-tetrahydroquinoline

### [Step 1]

### Production of 2-chloro-4-(pyrimidin-5-yl)-5,6,7,8-tetrahydroquino line

The title compound was prepared as a beige powder according to the procedure described in Example 83 Step 4 using pyrimidin-5-ylboronic acid instead of pyridin-3-ylboronic acid and using Pd(dppf)Cl₂·CH₂Cl₂ instead of Pd(PPh₃)₄.

### [Step 2]

### Production of 2-[(6-fluoropyridin-2-yl)methoxy]-4-(pyrimidin-5-yl )-5,6,7,8-tetrahydroquinoline

To 2-chloro-4-(pyrimidin-5-yl)-5,6,7,8-tetrahydroquino line (71 mg), (6-fluoropyridin-2-yl)methanol (41 mg), Pd₂(dba)₃·CHCl₃ (20 mg), t-Bu-X-Phos (33 mg) and cesium carbonate (315 mg) was added toluene (2.9 mL), and the mixture was degassed, then stirred under Ar atmosphere at 100°C for 14 hours. After the reaction mixture was allowed to return to room temperature, diluted with ethyl acetate, filtered through Celite, and the filtrate was evaporated under reduced pressure. The resulting residue was purified by silica gel column chromatography to give the title compound (59 mg) as a pale yellow powder.
[MS (ESI) m/z 337.3 (M+H)⁺]

### Example 145

### 2-[(5-Fluoropyridin-2-yl)methoxy]-4-(pyrimidin-5-yl )-5,6,7,8-tetrahydroquinoline

The title compound was prepared as yellow oil according to the procedure described in Example 144 Step 2 using (5-fluoropyridin-2-yl)methanol instead of (6-fluoropyridin-2-yl)methanol.
[MS (ESI) m/z 337.4 (M+H)⁺]

### Example 146

### 2-[(4-Fluoropyridin-2-yl)methoxy]-4-(pyrimidin-5-yl )-5,6,7,8-tetrahydroquinoline

### [Step 1]

### Production of ethyl 4-fluoropyridine-2-carboxylate

To 2-chloro-4-fluoropyridine (300 mg), Pd(dppf)Cl₂·CH₂Cl₂ (149 mg) and DIPEA (1.18 mL)were added ethanol (2 mL) and DMF (2 mL), and the mixture was stirred under carbon monooxide atmosphere at 80°C overnight. After the reaction mixture was allowed to return to room temperature, evaporated under reduced pressure and the reaction mixture was added with ethyl acetate and water, and subjected to extraction. The organic layer was dried over anhydrous sodium sulfate, filtered off, and the filtrate was evaporated under reduced pressure. The resulting residue was purified by silica gel column chromatography to give the title compound (254 mg) as brown oil.

### [Step 2]

### Production of (4-fluoropyridin-2-yl)methanol

To ethyl 4-fluoropyridine-2-carboxylate (254 mg) were added MeOH and NaBH₄ (170 mg) sequentially, then the mixture was stirred for 4 hours. After the solvent of the reaction mixture was evaporated under reduced pressure, the residue was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (126 mg) as colorless oil.

### [Step 3]

### Production of 2-[(4-fluoropyridin-2-yl)methoxy]-4-(pyrimidin-5-yl )-5,6,7,8-tetrahydroquinoline

The title compound was prepared as a white powder according to the procedure described in Example 144 Step 2 using (4-fluoropyridin-2-yl)methanol instead of (6-fluoropyridin-2-yl)methanol.
[MS (ESI) m/z 337.3 (M+H)⁺]

### Example 147

### 2-[(3-Fluoropyridin-2-yl)methoxy]-4-(pyrimidin-5-yl )-5,6,7,8-tetrahydroquinoline

The title compound was prepared as yellow oil according to the procedure described in Example 144 Step 2 using (3-fluoropyridin-2-yl)methanol instead of (6-fluoropyridin-2-yl)methanol.
[MS (ESI) m/z 337.2 (M+H)⁺]

### Example 148

### 2-[(3,6-Difluoropyridin-2-yl)methoxy]-4-(pyrimidin-5-yl)-5,6,7,8-tetrahydroquinoline

### [Step 1]

### Production of (3,6-difluoropyridin-2-yl)methanol

To a solution of methyl 3,6-difluoropyridine-2-carboxylate (300 mg) in MeOH (5 mL) was added NaBH₄ (170 mg) under ice water cooling, then the reaction mixture was stirred at room temperature for 4 hours. Hydrochloric acid was added to the reaction mixture under ice water cooling, and the solvent was evaporated under reduced pressure. The resulting residue was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (105 mg) as colorless oil.

### [Step 2]

### Production of 2-[(3,6-difluoropyridin-2-yl)methoxy]-4-(pyrimidin-5-yl)-5,6,7,8-tetrahydroquinoline

The title compound was prepared as a pale yellow powder according to the procedure described in Example 144 Step 2 using (3,6-difluoropyridin-2-yl)methanol instead of (6-fluoropyridin-2-yl)methanol and using Pd₂(dba)₃ instead of Pd₂(dba)₃·CHCl₃.
[MS (ESI) m/z 355.4 (M+H)⁺]

### Example 149

### 6-({[4-(Pyrimidin-5-yl)-5,6,7,8-tetrahydroquinolin-2-yl]oxy}methyl)pyridine-2-carbonitrile

The title compound was prepared as a pale yellow powder according to the procedure described in Example 144 Step 2 using 6-(hydroxymethyl)pyridine-2-carbonitrile instead of (6-fluoropyridin-2-yl)methanol.
[MS (ESI) m/z 344.4 (M+H)⁺]

### Example 150

### 6-({[4-(Pyrimidin-5-yl)-5,6,7,8-tetrahydroquinolin-2-yl]oxy}methyl)pyridine-3-carbonitrile

To 2-chloro-4-(pyrimidin-5-yl)-5,6,7,8-tetrahydroquino line (80 mg), 6-(hydroxymethyl)pyridine-3-carbonitrile (46 mg), Pd₂(dba)₃ (30 mg), t-Bu-X-Phos (55 mg), NaO-t-Bu (63 mg) and MS4A (150 mg) was added toluene (2 mL), and the mixture was degassed, then stirred under Ar atmosphere at 100°C for 5 hours. After the reaction mixture was allowed to return to room temperature, diluted with ethyl acetate, washed with water and saturated brine, dried over anhydrous magnesium sulfate, filtered off, and the filtrate was evaporated under reduced pressure. The resulting residue was purified by silica gel column chromatography to give the title compound (20 mg) as a pale yellow powder.
[MS (ESI) m/z 344.4 (M+H)⁺]

### Example 151

### 2-({[4-(Pyrimidin-5-yl)-5,6,7,8-tetrahydroquinolin-2-yl]oxy}methyl)pyridine-4-carbonitrile

The title compound was prepared as a white powder according to the procedure described in Example 150 using 2-(hydroxymethyl)pyridine-4-carbonitrile instead of 6-(hydroxymethyl)pyridine-3-carbonitrile.
[MS (ESI) m/z 344.3 (M+H)⁺]

### Example 152

### 2-[(6-Methylpyridin-2-yl)methoxy]-4-(pyrimidin-5-yl )-5,6,7,8-tetrahydroquinoline

### [Step 1]

### Production of 4-(pyrimidin-5-yl)-5,6,7,8-tetrahydroquinolin-2-ol

To 2-(pyridin-2-ylmethoxy)-4-(pyrimidin-5-yl)-5,6,7,8-tetrahydroquinoline (550 mg) were added THF (12 mL) and 15% hydrochloric acid (3 mL), and the mixture was heated under reflux for 3 days. After the reaction mixture was allowed to return to room temperature, the mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (240 mg).

### [Step 2]

### Production of 2-[(6-methylpyridin-2-yl)methoxy]-4-(pyrimidin-5-yl )-5,6,7,8-tetrahydroquinoline

To 4-(pyrimidin-5-yl)-5,6,7,8-tetrahydroquinolin-2-ol (20 mg), 2- (chloromethyl) -6-methylpyridine (25 mg) and silver carbonate (24 mg) was added toluene (1 mL), and the mixture was reacted under microwave irradiation at 160°C for 50 minutes. The reaction mixture was filtered through Celite, and the filtrate was evaporated under reduced pressure. The resulting residue was purified by silica gel column chromatography to give the title compound (7 mg) as a white powder.
[MS (ESI) m/z 333.3 (M+H)⁺]

### Example 153

### 2-[(3-Methylpyridin-2-yl)methoxy]-4-(pyrimidin-5-yl )-5,6,7,8-tetrahydroquinoline

The title compound was prepared as a white powder according to the procedure described in Example 144 Step 2 using (3-methylpyridin-2-yl)methanol instead of (6-fluoropyridin-2-yl)methanol.
[MS (ESI) m/z 333.4 (M+H)⁺]

### Example 154

### 4-(Pyrimidin-5-yl)-2-{[6-(trifluoromethyl)pyridin-2 -yl]methoxy}-5,6,7,8-tetrahydroquinoline hydrochloride

To 2-chloro-4-(pyrimidin-5-yl)-5,6,7,8-tetrahydroquino line (30 mg), [6-(trifluoromethyl)pyridin-2-yl]methanol (28 mg), Pd₂(dba)₃·CHCl₃ (8.3 mg), t-Bu-X-Phos (8.3 mg) and cesium carbonate (80 mg) was added toluene (1.6 mL), and the mixture was degassed, then stirred under Ar atmosphere at 100°C overnight. After the reaction mixture was allowed to return to room temperature, diluted with ethyl acetate, filtered through Celite, and the filtrate was evaporated under reduced pressure. The resulting residue was purified by silica gel column chromatography to give the title compound (37 mg) as a pink solid.
[MS (ESI) m/z 388.2 (M+H)⁺]

Et₂O and 1N HCl/Et₂O solution were added to the resulting compound, then the mixture was evaporated under reduced pressure to give the title compound (26 mg) as a white powder.
[MS (ESI) m/z 388.2 (M+H)⁺]

### Example 155

### 2-[(3-Methoxypyridin-2-yl)methoxy]-4-(pyrimidin-5-y l)-5,6,7,8-tetrahydroquinoline

To 2-[(3-fluoropyridin-2-yl)methoxy]-4-(pyrimidin-5-yl )-5,6,7,8-tetrahydroquinoline (5 mg) were added methanol (1 mL) and NaH (60% dispersion in oil, excess amount) and the mixture was stirred at room temperature overnight and then at 50°C for 2 days, and reacted under microwave irradiation at 120°C for 1 hour, and then 100°C for 3 days. The solvent of the reaction mixture was evaporated under reduced pressure and the resulting residue was purified by silica gel column chromatography to give the title compound (3 mg) as colorless oil.
[MS (ESI) m/z 349.3 (M+H)⁺]

### Example 156

### 6-({[4-(Pyrimidin-5-yl)-5,6,7,8-tetrahydroquinolin-2-yl]oxy}methyl)pyridine-2-carboxamide

To 6-({[4-(pyrimidin-5-yl)-5,6,7,8-tetrahydroquinolin-2-yl]oxy}methyl)pyridine-2-carbonitrile (20 mg) was added tert-butanol (1 mL),followed by the addition of potassium fluoride on alumina (10 mg), and the mixture was stirred at 90°C for 4 hours. After the reaction mixture was allowed to return to room temperature, diluted with ethyl acetate, filtered through Celite, and the filtrate was evaporated under reduced pressure. The resulting residue was purified by silica gel column chromatography to give the title compound (25 mg) as a white powder.
[MS (ESI) m/z 362.3 (M+H)⁺]

### Example 157

### 6-({[4-(Pyrimidin-5-yl)-5,6,7,8-tetrahydroquinolin-2-yl]oxy}methyl)pyridin-2-amine

To 2-[(6-fluoropyridin-2-yl)methoxy]-4-(pyrimidin-5-yl )-5,6,7,8-tetrahydroquinoline (20 mg) was added concentrated ammonia/methanol solution (1 mL), and the mixture was stirred in sealed tube at 110°C for 2 days. The solvent of the reaction mixture was evaporated under reduced pressure, and the resulting residue was purified by silica gel column chromatography to give the title compound (2.3 mg) as yellow oil.
[MS (ESI) m/z 334.3 (M+H)⁺]

### Example 158

### N-Methyl-6-({[4-(pyrimidin-5-yl)-5,6,7,8-tetrahydro quinolin-2-yl]oxy}methyl)pyridin-2-amine

The title compound was prepared as a white powder according to the procedure described in Example 157 using 2N monomethylamine/methanol solution instead of concentrated ammonia/methanol solution.
[MS (ESI) m/z 348.4 (M+H)⁺]

### Example 159

### [6-({[4-(Pyrimidin-5-yl)-5,6,7,8-tetrahydroquinolin -2-yl]oxy}methyl)pyridin-2-yl]methanol

The title compound was prepared as a pale yellow powder according to the procedure described in Example 144 Step 2 using (pyridine-2,6-diyl)bismethanol instead of (6-fluoropyridin-2-yl)methanol.
[MS (ESI) m/z 349.4 (M+H)⁺]

### Example 160

### 2-{[6-(Methoxymethyl)pyridin-2-yl]methoxy}-4-(pyrim idin-5-yl)-5,6,7,8-tetrahydroquinoline hydrochloride

To [6-({[4-(pyrimidin-5-yl)-5,6,7,8-tetrahydroquinolin -2-yl]oxy}methyl)pyridin-2-yl]methanol (50 mg) was added THF (1 mL), followed by the addition of NaH (60% dispersion in oil, 10 mg) under ice water cooling, and then the mixture was stirred at room temperature for 10 minutes. MeI (0.013 mL) was added to the mixture under ice water cooling, and then stirred at room temperature for 2 hours. After the reaction mixture was diluted with ethyl acetate, washed with water and saturated brine, dried over anhydrous magnesium sulfate, filtered off, and the filtrate was evaporated under reduced pressure. The resulting residue was purified by silica gel column chromatography to give the title compound (41 mg) as a white powder. To the resulting compound was added Et₂O (2 mL) and followed by addition of 1N HCl/Et₂O solution (0.31 mL) under ice water cooling, and the mixture was stirred at room temperature for 0.5 hour. The resulting precipitate was collected by filtration to give the title compound (12 mg) as a white powder.
[MS (ESI) m/z 363.4 (M+H)⁺]

### Example 161

### 2-[(6-Ethenylpyridin-2-yl)methoxy]-4-(pyrimidin-5-y l)-5,6,7,8-tetrahydroquinoline

### [Step 1]

### Production of 2-bromo-6-({[tert-butyl(dimethyl)silyl]oxy}methyl)p yridine

To (6-bromopyridin-2-yl)methanol (2 g) were added CH₂Cl₂ (50 mL), tert-butylchlorodimethylsilane (1.6 g), imidazole (54 mg) and DIPEA (5.5 mL), and the mixture was stirred at room temperature overnight. After the reaction mixture was diluted with Et₂O, washed with water and saturated brine, dried over anhydrous magnesium sulfate, filtered off, and the filtrate was evaporated under reduced pressure. The resulting residue was purified by silica gel column chromatography to give the title compound (3.11 g) as colorless oil.

### [Step 2]

### Production of 2-({[tert-butyl(dimethyl)silyl]oxy}methyl)-6-etheny lpyridine

2-Bromo-6-({[tert-butyl(dimethyl)silyl]oxy}met hyl)pyridine (500 mg) was added 1,4-dioxane (3 mL), tributylvinyltin (1.05 g) and Pd(PPh₃)₄ (96 mg) under Ar atmosphere, and the mixture was reacted under microwave irradiation at 120°C for 20 minutes. Pd(PPh₃)₄ (96 mg) was further added to the reaction mixture, and the mixture was further reacted under microwave irradiation at 120°C for 1 hour. After the reaction mixture was allowed to return to room temperature, the mixture was diluted with ethyl acetate, washed with water and saturated brine, dried over anhydrous magnesium sulfate, filtered off, and the filtrate was evaporated under reduced pressure. The resulting residue was purified by silica gel column chromatography to give the title compound (416 mg) as colorless oil.

### [Step 3]

### Production of (6-ethenylpyridin-2-yl)methanol

To 2-({[tert-butyl(dimethyl)silyl]oxy}methyl)-6-etheny lpyridine (150 mg) was added THF (3 mL), followed by the addition of and 1.0M tetrabutylammonium fluoride (hereinafter referred to as "TBAF") in THF solution (0.35 mL) under ice water cooling, and the mixture was stirred at room temperature for 3 hours. After the reaction mixture was diluted with ethyl acetate, washed with water and saturated brine, dried over anhydrous magnesium sulfate, filtered off, and the filtrate was evaporated under reduced pressure. The resulting residue was purified by silica gel column chromatography to give the title compound (68 mg) as colorless oil.

### [Step 4]

### Production of 2-[(6-ethenylpyridin-2-yl)methoxy]-4-(pyrimidin-5-y l)-5,6,7,8-tetrahydroquinoline

The title compound was prepared as a white powder according to the procedure described in Example 144 Step 2 using (6-ethenylpyridin-2-yl)methanol instead of (6-fluoropyridin-2-yl)methanol.
[MS (ESI) m/z 345.4 (M+H)⁺]

### Example 162

### 2-[(6-Propylpyridin-2-yl)methoxy]-4-(pyrimidin-5-yl )-5,6,7,8-tetrahydroquinoline hydrochloride

The title compound was prepared as a white powder according to the procedure described in Example 144 Step 2 using (6-propylpyridin-2-yl)methanol instead of (6-fluoropyridin-2-yl)methanol, and using Pd₂(dba)₃ instead of Pd₂(dba)₃·CHCl₃. To the resulting compound (64 mg) was added Et₂O (4 mL), followed by the addition of 1N HCl/Et₂O solution (0.178 mL) under ice water cooling, and the mixture was stirred at room temperature for 0.5 hour. The resulting precipitate was collected by filtration to give the title compound (57 mg) as a white powder.
[MS (ESI) m/z 361.5 (M+H)⁺]

### Example 163

### 2-(Pyrazin-2-ylmethoxy)-4-(pyrimidin-5-yl)-5,6,7,8-tetrahydroquinoline

The title compound was prepared as colorless oil according to the procedure described in Example 152 Step 2 using 2-(chloromethyl)pyrazine instead of 2-(chloromethyl)-6-methylpyridine.
[MS (ESI) m/z 320.2 (M+H)⁺]

### Example 164

### 4-(Pyrimidin-5-yl)-2-(1,3-thiazol-2-ylmethoxy)-5,6, 7,8-tetrahydroquinoline

The title compound was prepared as yellow oil according to the procedure described in Example 144 Step 2 using 1,3-thiazol-2-ylmethanol instead of (6-fluoropyridin-2-yl)methanol.
[MS (ESI) m/z 325.2 (M+H)⁺]

### Example 165

### 2-(1,3-Oxazol-2-ylmethoxy)-4-(pyrimidin-5-yl)-5,6,7 ,8-tetrahydroquinoline

The title compound was prepared as a white solid according to the procedure described in Example 144 Step 2 using 1,3-oxazol-2-ylmethanol instead of (6-fluoropyridin-2-yl)methanol.
[MS (ESI) m/z 310.2 (M+H)⁺]

### Example 166

### 2-[(1-Oxidopyridin-2-yl)methoxy]-4-(pyrimidin-5-yl) -5,6,7,8-tetrahydroquinoline

To 2-(pyridin-2-ylmethoxy)-4-(pyrimidin-5-yl)-5,6,7,8-tetrahydroquinoline (63 mg) was added chloroform (100 mL), followed by the addition of m-CPBA (with abs. 25% water, 46 mg) under ice water cooling, and the mixture was stirred at the same temperature for 1 hour, and then stirred at room temperature overnight. The reaction mixture was purified by silica gel column chromatography to give the title compound (12 mg) as a white powder.
[MS (ESI) m/z 335.3 (M+H)⁺]

### Example 167

### 4-(2-Fluoropyrimidin-5-yl)-2-(pyridin-2-ylmethoxy)-5,6,7,8-tetrahydroquinoline hydrochloride

### [Step 1]

### Production of 2-fluoro-5-(trimethylstannanyl)pyrimidine

To 5-bromo-2-fluoropyrimidine (300 mg), hexamethylditin (841 mg) and Pd(PPh₃)₄ (202 mg) was added 1,4-dioxane (33 mL), and the mixture was stirred under Ar atmosphere at 100°C for 10 hours, and stirred at room temperature for 2 days. The solvent of the reaction mixture was evaporated under reduced pressure. The resulting residue was purified by silica gel column chromatography to give the title compound (361 mg) as colorless oil.

### [Step 2]

### Production of 4-(2-fluoropyrimidin-5-yl)-2-(pyridin-2-ylmethoxy)-5,6,7,8-tetrahydroquinoline hydrochloride

To 4-chloro-2-(pyridin-2-ylmethoxy)-5,6,7,8-tetrahydro quinoline (100 mg), 2-fluoro-5-(trimethylstannanyl)pyrimidine (95 mg), LiCl (46 mg) and Pd(PPh₃)₄ (42 mg) was added DMF (0.6 mL), and the mixture was reacted under microwave irradiation at 180°C for 1 hour. After the reaction mixture was allowed to return to room temperature, diluted with ethyl acetate, filtered through Celite, and the filtrate was evaporated under reduced pressure. The resulting residue was purified by silica gel column chromatography to give 4-(2-fluoropyrimidin-5-yl)-2-(pyridin-2-ylmethoxy)-5,6,7,8-tetrahydroquinoline (14 mg) as colorless oil. The resulting compound was dissolved in ethyl acetate, followed by the addition of 1N HCl/Et₂O solution and the resulting precipitate was collected by filtration to give the title compound (12 mg) as a white powder.
[MS (ESI) m/z 337.1 (M+H)⁺]

### Example 168

### 4-(2-Methylpyrimidin-5-yl)-2-(pyridin-2-ylmethoxy)-5,6,7,8-tetrahydroquinoline

To 2-(pyridin-2-ylmethoxy)-5,6,7,8-tetrahydroquinolin-4-yl trifluoromethanesulfonate (50 mg), 2-methyl-5-(trimethylsilyl)pyrimidine (33 mg) and Pd(PPh₃)₄ (15 mg) was added NMP (1 mL), and the mixture was stirred at 100°C overnight. After the reaction mixture was allowed to return to room temperature, the mixture was added with Et₂O and water, and subjected to extraction. The organic layer was dried over anhydrous sodium sulfate, filtered off, and the filtrate was evaporated under reduced pressure. The resulting residue was purified by silica gel column chromatography to give the title compound (12 mg). To the resulting compound was added Et₂O (1mL) and 1N HCl/Et₂O solution (0.036 mL). The reaction mixture was washed by decantation, and the solvent was evaporated under reduced pressure to give the title compound (11 mg) as a white powder.
[MS (ESI) m/z 333.4 (M+H)⁺]

### Example 169

### 5-[2-(Pyridin-2-ylmethoxy)-5,6,7,8-tetrahydroquinol in-4-yl]pyrimidin-2-amine

To 4-(2-fluoropyrimidin-5-yl)-2-(pyridin-2-ylmethoxy)-5,6,7,8-tetrahydroquinoline (8 mg) was added concentrated ammonia/methanol solution (2 mL) and the mixture was stirred at 40°C for 3 hours. The solvent of the reaction mixture was evaporated under reduced pressure, and the resulting residue was purified by silica gel column chromatography to give the title compound (5 mg) as a white powder.
[MS (ESI) m/z 334.3 (M+H)⁺]

### Example 170

### N-Methyl-5-[2-(pyridin-2-ylmethoxy)-5,6,7,8-tetrahy droquinolin-4-yl]pyrimidin-2-amine

The title compound was prepared as a white powder according to the procedure described in Example 169 using 2N monomethylamine/methanol solution instead of concentrated ammonia/methanol solution.
[MS (ESI) m/z 348.3 (M+H)⁺]

### Example 171

### N,N-Dimethyl-5-[2-(pyridin-2-ylmethoxy)-5,6,7,8-tet rahydroquinolin-4-yl]pyrimidin-2-amine

To 4-chloro-2-(pyridin-2-ylmethoxy)-5,6,7,8-tetrahydro quinoline (100 mg), 2-fluoro-5-(trimethylstannanyl)pyrimidine (95 mg), LiCl (46 mg) and Pd(PPh₃)₄ (42 mg) was added DMF (0.6 mL), and the mixture was reacted under microwave irradiation at 180°C for 1 hour. After the reaction mixture was allowed to return to room temperature, diluted with ethyl acetate, filtered through Celite, and the filtrate was evaporated under reduced pressure. The resulting residue was purified by silica gel column chromatography to give the title compound (4.8 mg) as colorless oil.
[MS (ESI) m/z 362.2 (M+H)⁺]

### Example 172

### 5-[2-(Pyridin-2-ylmethoxy)-5,6,7,8-tetrahydroquinol in-4-yl]pyrimidin-2-ol

To 4-(2-fluoropyrimidin-5-yl)-2-(pyridin-2-ylmethoxy)-5,6,7,8-tetrahydroquinoline (6 mg) were added t-butyl alcohol (0.5 mL) and sodium hydroxide solution (0.5mL), and the mixture was stirred at 90°C for 4 hours. After the reaction mixture was allowed to return to room temperature, added with chloroform and water, and neutralized with saturated aqueous ammonium chloride solution, and then subjected to extraction. The organic layer was dried over anhydrous sodium sulfate, filtered off, and the filtrate was evaporated under reduced pressure. The resulting residue was purified by silica gel column chromatography to give the title compound (7.5 mg) as a white powder.
[MS (ESI) m/z 335.3 (M+H)⁺]

### Example 173

### 5-[2-(Pyridin-2-ylmethoxy)-5,6,7,8-tetrahydroquinol in-4-yl]pyrimidine-2-carbonitrile hydrochloride

5-[2-(pyridin-2-ylmethoxy)-5,6,7,8-tetrahydroq uinolin-4-yl]pyrimidine-2-carbonitrile was prepared as a white solid according to the procedure described in Example 108 Step 2 using 5-bromopyrimidine-2-carbonitrile instead of 3-bromo-2-fluoro-5-methylpyridine. The resulting compound (831 mg) was dissolved in ethyl acetate (25 mL), followed by the addition of 1N HCl/Et₂O solution (2.16 mL) under ice water cooling. The resulting precipitate was collected by filtration to give the title compound (697 mg) as a white powder.
[MS (ESI) m/z 344.4 (M+H)⁺]
Elementary analysis as C₂₀H₁₇N₅O·HCl+water
Calcd. (%) C: 60.38.; H: 5.07.; N: 17.60
Found. (%) C: 60.98.; H: 5.14.; N: 16.93

### Example 174

### 5-[2-(Pyridin-2-ylmethoxy)-5,6,7,8-tetrahydroquinol in-4-yl]pyrimidine-4-carbonitrile hydrochloride

### [Step 1]

### Production of 5-iodo-4-{[2-(trimethylsilyl)ethoxy]methoxy}pyrimid ine

To 5-iodopyrimidin-4-ol (850 mg) were added DMF (9 mL), DIPEA (2 mL) and DMAP (46 mg). 2-(Chloromethyl)ethyltrimethylsilane (1 mL) was added to the mixture under ice water cooling, and the mixture was stirred at room temperature overnight. The reaction mixture was added with Et₂O and water, and subjected to extraction. The organic was dried over anhydrous sodium sulfate, filtered off, and the filtrate was evaporated under reduced pressure. The resulting residue was purified by silica gel column chromatography to give the title compound (1 g) as a yellow solid.

### [Step 2]

### Production of 2-(pyridin-2-ylmethoxy)-4-(4-{[2-(trimethylsilyl)et hoxy]methoxy}pyrimidin-5-yl)-5,6,7,8-tetrahydroquin oline

The title compound was prepared as yellow oil according to the procedure described in Example 108 Step 2 using 5-iodo-4-{[2-(trimethylsilyl)ethoxy]methoxy}pyrimid ine instead of 3-bromo-2-fluoro-5-methylpyridine.

### [Step 3]

### Production of 5-[2-(pyridin-2-ylmethoxy)-5,6,7,8-tetrahydroquinol in-4-yl]pyrimidin-4-ol

To 2-(pyridin-2-ylmethoxy)-4-(4-{[2-(trimethylsilyl)et hoxy]methoxy}pyrimidin-5-yl)-5,6,7,8-tetrahydroquin oline (237 mg) were added CH₂Cl₂ (3.4 mL) and ethanol (0.034 mL), followed by the addition of TFA (0.5 mL) under ice water cooling, then the mixture was stirred at room temperature for 3 hours. After the reaction mixture was neutralized with water and potassium carbonate, extracted with chloroform. The organic was dried over anhydrous sodium sulfate, filtered off, and the filtrate was evaporated under reduced pressure. Et₂O was added to the residue, and the resulting suspension was stirred for a while, and then filtered to give the title compound (110 mg) as a white powder.

### [Step 4]

### Production of 4-(4-chloropyrimidin-5-yl)-2-(pyridin-2-ylmethoxy)-5,6,7,8-tetrahydroquinoline

To 5-[2-(pyridin-2-ylmethoxy)-5,6,7,8-tetrahydroquinol in-4-yl]pyrimidin-4-ol (69 mg) was added dichloroethane (2mL) and PPh₃ (109 mg), and the mixture was stirred for 1 hour. Carbon tetrachloride (0.059 mL) was added to the mixture, and then the mixture was stirred at 60°C for 6 hours. The solvent of the reaction mixture was evaporated under reduced pressure. The resulting residue was purified by silica gel column chromatography to give the title compound (24 mg) as colorless oil.
[MS (ESI) m/z 353.3 (M+H)⁺]

### [Step 5]

### Production of 5-[2-(pyridin-2-ylmethoxy)-5,6,7,8-tetrahydroquinol in-4-yl]pyrimidine-4-carbonitrile hydrochloride

To 4-(4-chloropyrimidin-5-yl)-2-(pyridin-2-ylmethoxy)-5,6,7,8-tetrahydroquinoline (30 mg), Pd(dppf)Cl₂·CH₂Cl₂ (3.5 mg) and zinc cyanide (10 mg) was added DMF (0.5 mL) and the mixture was reacted under microwave irradiation at 180°C for 1 hour. After the reaction mixture was allowed to return to room temperature, diluted with ethyl acetate, filtered through Celite, and the filtrate was evaporated under reduced pressure. The resulting residue was purified by silica gel column chromatography to give the title compound (22 mg) as colorless oil.
[MS (ESI) m/z 344.4 (M+H)⁺]

The resulting compound was dissolved in ethyl acetate (2 mL), followed by addition of 1N HCl/Et₂O solution (0.064 mL) was added, and the resulting precipitate was collected by filtration to give the title compound (17 mg) as a white powder.
[MS (ESI) m/z 344.4 (M+H)⁺]

### Example 175

### 5-[2-(Pyridin-2-ylmethoxy)-5,6,7,8-tetrahydroquinol in-4-yl]pyrimidin-4-ol

The title compound was prepared as a white powder according to the procedure described in Example 174 Step 3
[MS (ESI) m/z 335.3 (M+H)⁺]

### Example 176

### 4-(4-Chloropyrimidin-5-yl)-2-(pyridin-2-ylmethoxy)-5,6,7,8-tetrahydroquinoline hydrochloride

4-(4-chloropyrimidin-5-yl)-2-(pyridin-2-ylmeth oxy)-5,6,7,8-tetrahydroquinoline (24 mg) was dissolved in Et₂O (1 mL), followed by the addition of 1N HCl/Et₂O solution (0.034 mL), and the resulting precipitate was collected by filtration to give the title compound (7 mg) as a white powder.
[MS (ESI) m/z 353.3 (M+H)⁺]

### Example 177

### 5-[2-(Pyridin-2-ylmethoxy)-5,6,7,8-tetrahydroquinol in-4-yl]pyrimidin-4-amine

To 4-(4-chloropyrimidin-5-yl)-2-(pyridin-2-ylmethoxy)-5,6,7,8-tetrahydroquinoline (30 mg) was added concentrated ammonia/methanol solution (2 mL), and the mixture was stirred in sealed tube at 60°C overnight. The solvent of the reaction mixture was evaporated under reduced pressure, and the resulting residue was purified by silica gel column chromatography to give the title compound (5.6 mg) as a white powder.
[MS (ESI) m/z 334.4 (M+H)⁺]

### Example 178

### 4-(4-Methoxypyrimidin-5-yl)-2-(pyridin-2-ylmethoxy) -5,6,7,8-tetrahydroquinoline

To 4-(4-chloropyrimidin-5-yl)-2-(pyridin-2-ylmethoxy)-5,6,7,8-tetrahydroquinoline (30 mg) was added concentrated ammonia/methanol solution (2 mL), and the mixture was stirred in sealed tube at 60°C overnight. The solvent of the reaction mixture was evaporated under reduced pressure, and the resulting residue was purified by silica gel column chromatography to give the title compound (7.8 mg) as a white powder.
[MS (ESI) m/z 349.4 (M+H)⁺]

### Example 179

### 2-(Benzyloxy)-4-(2-methylpyrimidin-5-yl)-5,6,7,8-te trahydroquinoline

### [Step 1]

### Production of 2-chloro-4-(2-methylpyrimidin-5-yl)-5,6,7,8-tetrahy droquinoline

The title compound was prepared as a white powder according to the procedure described in Example 137 Step 2 using 5-bromo-2-methylpyrimidine instead of 2-fluoro-3-iodopyridine.

### [Step 2]

### Production of 2-(benzyloxy)-4-(2-methylpyrimidin-5-yl)-5,6,7,8-te trahydroquinoline

To 2-chloro-4-(2-methylpyrimidin-5-yl)-5,6,7,8-tetrahy droquinoline (30 mg), benzylalcohol (0.016 mL), Pd₂(dba)₃ (11 mg), t-Bu-X-Phos (12 mg) and cesium carbonate (94 mg) was added toluene (1.2 mL), and the mixture was degassed, then stirred under Ar atmosphere at 100°C for 6 hours. After the reaction mixture was allowed to return to room temperature, and the solvent of the reaction mixture was evaporated under reduced pressure. The resulting residue was purified by silica gel column chromatography to give the title compound (22 mg) as a white powder.
[MS (ESI) m/z 332.4 (M+H)⁺]

### Example 180

### 4-(2-Methylpyrimidin-5-yl)-2-(pyridin-4-ylmethoxy)-5,6,7,8-tetrahydroquinoline

The title compound was prepared as a white solid according to the procedure described in Example 179 Step 2 using pyridin-4-ylmethanol instead of benzyl alcohol.
[MS (ESI) m/z 333.4 (M+H)⁺]

### Example 181

### 4-(2-Methylpyrimidin-5-yl)-2-(pyridin-3-ylmethoxy)-5,6,7,8-tetrahydroquinoline

The title compound was prepared as a white solid according to the procedure described in Example 179 Step 2 using pyridin-3-ylmethanol instead of benzyl alcohol.
[MS (ESI) m/z 333.4 (M+H)⁺]

### Example 182

### 2-[(6-Methylpyridin-2-yl)methoxy]-4-(2-methylpyrimi din-5-yl)-5,6,7,8-tetrahydroquinoline hydrochloride

2-[(6-methylpyridin-2-yl)methoxy]-4-(2-methylp yrimidin-5-yl)-5,6,7,8-tetrahydroquinoline was prepared as a white powder according to the procedure described in Example 179 Step 2 using (6-methylpyridin-2-yl)methanol instead of benzyl alcohol. To the resulting compound (44 mg) was added Et₂O (3 mL), followed by the addition of 1N HCl/Et₂O solution (0.14 mL) under ice water cooling, and the mixture was stirred at room temperature for 0.5 hour. The resulting precipitate was collected by filtration to give the title compound (30 mg) as a white powder.
[MS (ESI) m/z 347.4 (M+H)⁺]

### Example 183

### 6-({[4-(2-Methylpyrimidin-5-yl)-5,6,7,8-tetrahydroq uinolin-2-yl]oxy}methyl)pyridine-2-carbonitrile

### [Step 1]

### Production of 2-[(1-oxidopyridin-2-yl)methoxy]-5,6,7,8-tetrahydro quinolin-4-yl trifluoromethanesulfonate

The title compound was prepared according to the procedure described in Example 8 Step 1 using 2-(pyridin-2-ylmethoxy)-5,6,7,8-tetrahydroquinolin-4-yl trifluoromethanesulfonate instead of 2-(pyridin-2-ylmethoxy)-6,7-dihydro-5H-cyclopenta[b ]pyridin-4-yl trifluoromethanesulfonate.

### [Step 2]

### Production of 2-[(6-cyanopyridin-2-yl)methoxy]-5,6,7,8-tetrahydro quinolin-4-yl trifluoromethanesulfonate

The title compound was prepared according to the procedure described in Example 8 Step 2 using 2-[(1-oxidopyridin-2-yl)methoxy]-5,6,7,8-tetrahydro quinolin-4-yl trifluoromethanesulfonate instead of 2-[(1-oxidopyridin-2-yl)methox)-6,7-dihydro-5H-cycl openta[b]pyridin-4-yl trifluoromethanesulfonate.

### [Step 3]

### Production of 6-({[4-(4,4,5,5-tetramethyl-1,3,2-dioxaborolan-2-yl )-5,6,7,8-tetrahydroquinolin-2-yl]oxy}methyl)pyridi ne-2-carbonitrile

The title compound was prepared as a white solid according to the procedure described in Example 8 Step 3 using 2-[(6-cyanopyridin-2-yl)methoxy]-5,6,7,8-tetrahydro quinolin-4-yl trifluoromethanesulfonate instead of 2-[(6-cyanopyridin-2-yl)methoxy]-6,7-dihydro-5H-cyc lopenta[b]pyridin-4-yl trifluoromethanesulfonate.

### [Step 4]

### Production of 6-({[4-(2-methylpyrimidin-5-yl)-5,6,7,8-tetrahydroq uinolin-2-yl]oxy}methyl)pyridine-2-carbonitrile

The title compound was prepared as a white powder according to the procedure described in Example 108 Step 2 using 6-({[4-(4,4,5,5-tetramethyl-1,3,2-dioxaborolan-2-yl )-5,6,7,8-tetrahydroquinolin-2-yl]oxy}methyl)pyridi ne-2-carbonitrile instead of 2-(pyridin-2-ylmethoxy)-4-(4,4,5,5-tetramethyl-1,3, 2-dioxaborolan-2-yl)-5,6,7,8-tetrahydroquinoline, and using 5-bromo-2-methylpyrimidine instead of 3-bromo-2-fluoro-5-methylpyridine.
[MS (ESI) m/z 358.4 (M+H)⁺]

### Example 184

### 6-({[4-(2-Methylpyrimidin-5-yl)-5,6,7,8-tetrahydroq uinolin-2-yl]oxy}methyl)pyridine-3-carbonitrile hydrochloride

6-({[4-(2-Methylpyrimidin-5-yl)-5,6,7,8-tetrah ydroquinolin-2-yl]oxy}methyl)pyridine-3-carbonitril e was prepared as a white powder according to the procedure described in Example 179 Step 2 using 6-(hydroxymethyl)pyridine-3-carbonitrile instead of benzyl alcohol. To the resulting compound (44 mg) was added ethyl acetate (3 mL) and followed by addition of 1N HCl/Et₂O solution (0.18 mL) under ice water cooling, and the mixture was stirred for 0.5 hour. The resulting precipitate was collected by filtration to give the title compound (18 mg) as a white powder.
[MS (ESI) m/z 358.4 (M+H)⁺]

### Example 185

### 2-[(6-Fluoropyridin-2-yl)methoxy]-4-(2-methylpyrimi din-5-yl)-5,6,7,8-tetrahydroquinoline

The title compound was prepared as a white powder according to the procedure described in Example 179 Step 2 using (6-fluoropyridin-2-yl)methanol instead of benzyl alcohol.
[MS (ESI) m/z 351.3 (M+H)⁺]

### Example 186

### 2-[(5-Fluoropyridin-2-yl)methoxy]-4-(2-methylpyrimi din-5-yl)-5,6,7,8-tetrahydroquinoline

The title compound was prepared as a white powder according to the procedure described in Example 179 Step 2 using (5-fluoropyridin-2-yl)methanol instead of benzyl alcohol.
[MS (ESI) m/z 351.4 (M+H)⁺]

### Example 187

### 2-[(3-Fluoropyridin-2-yl)methoxy]-4-(2-methylpyrimi din-5-yl)-5,6,7,8-tetrahydroquinoline

To 2-chloro-4-(2-methylpyrimidin-5-yl)-5,6,7,8-tetrahy droquinoline (60 mg), (3-fluoropyridin-2-yl)methanol (38 mL), Pd₂(dba)₃ (21 mg), t-Bu-X-Phos (24 mg) and cesium carbonate (150 mg) was added toluene (2 mL), and the mixture was degassed, then stirred under Ar atmosphere at 100°C for 11 hours. After the reaction mixture was allowed to return to room temperature, the reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography and by recycling preparative gel permeation chromatography (Japan Analytical Industry, Co. Ltd., LC-9201) to give the title compound (27 mg) as a white powder.
[MS (ESI) m/z 351.3 (M+H)⁺]

### Example 188

### 2-[(3,5-Difluoropyridin-2-yl)methoxy]-4-(2-methylpy rimidin-5-yl)-5,6,7,8-tetrahydroquinoline

The title compound was prepared as a pale yellow powder according to the procedure described in Example 150 using 2-chloro-4-(2-methylpyrimidin-5-yl)-5,6,7,8-tetrahy droquinoline instead of 2-chloro-4-(pyrimidin-5-yl)-5,6,7,8-tetrahydroquino line, and using (3,5-difluoropyridin-2-yl)methanol instead of 6-(hydroxymethyl)pyridine-3-carbonitrile
[MS (ESI) m/z 369.4 (M+H)⁺]

### Example 189

### 2-[(3,6-Difluoropyridin-2-yl)methoxy]-4-(2-methylpy rimidin-5-yl)-5,6,7,8-tetrahydroquinoline

The title compound was prepared as a white powder according to the procedure described in Example 179 Step 2 using (3,6-difluoropyridin-2-yl)methanol instead of benzyl alcohol.
[MS (ESI) m/z 369.3 (M+H)⁺]

### Example 190

### 2-[(3-Fluoropyridin-2-yl)methoxy]-4-(2-methoxypyrim idin-5-yl)-5,6,7,8-tetrahydroquinoline

### [Step 1]

### Production of 2-chloro-4-(2-methoxypyrimidin-5-yl)-5,6,7,8-tetrah ydroquinoline

The title compound was prepared as a white powder according to the procedure described in Example 137 Step 2 using 5-bromo-2-methoxypyrimidine instead of 2-fluoro-3-iodopyridine, and using 1,4-dioxane instead of THF.

### [Step 2]

### Production of 2-[(3-fluoropyridin-2-yl)methoxy]-4-(2-methoxypyrim idin-5-yl)-5,6,7,8-tetrahydroquinoline

The title compound was prepared as a white powder according to the procedure described in Example 179 Step 2 using 2-chloro-4-(2-methoxypyrimidin-5-yl)-5,6,7,8-tetrah ydroquinoline instead of 2-chloro-4-(2-methylpyrimidin-5-yl)-5,6,7,8-tetrahy droquinoline, and using (3-fluoropyridin-2-yl)methanol instead of benzyl alcohol.
[MS (ESI) m/z 367.4 (M+H)⁺]

### Example 191

### 5-{2-[(3-Fluoropyridin-2-yl)methoxy]-5,6,7,8-tetrah ydroquinolin-4-yl}pyrimidin-2-amine hydrochloride

### [Step 1]

### Production of 2-chloro-4-[(4-methoxybenzyl)oxy]-5,6,7,8-tetrahydr oquinoline

To 4-methoxybenzylalcohol (2.05 g) were added DMF (50 mL) and NaH (60% dispersion in oil, 891 mg) under ice water cooling, and the mixture was stirred for 30 minutes. After the reaction mixture was allowed to return to room temperature, 2,4-dichloro-5,6,7,8-tetrahydroquinoline (see, for example, Helvetica Chimica Acta, 1945, vol. 28, p. 1684-1690) (3 g) was added to the mixture, and the mixture was stirred at 60°C for 4 hours. After the reaction mixture was allowed to return to room temperature, the reaction mixture was added with water and ethyl acetate, and subjected to extraction. The organic layer was washed with saturated brine, dried over anhydrous sodium sulfate, filtered off, and the filtrate was evaporated under reduced pressure. The resulting residue was purified by silica gel column chromatography to give the title compound (3.31 g) as colorless oil.

### [Step 2]

### Production of 2-[(3-fluoropyridin-2-yl)methoxy]-4-[(4-methoxybenz yl)oxy]-5,6,7,8-tetrahydroquinoline

To 2-chloro-4-[(4-methoxybenzyl)oxy]-5,6,7,8-tetrahydr oquinoline (200 mg), (3-fluoropyridin-2-yl)methanol (109 mL), Pd₂(dba)₃ (60 mg), t-Bu-X-Phos (67 mg) and potassium phosphate (280 mg) was added toluene (5 mL), and the mixture was degassed, then stirred under Ar atmosphere at 100°C for 4 hours. The reaction mixture was added with NaO-t-Bu (127 mg), and further stirred at 100°C for 2 hours. The solvent of the reaction mixture was evaporated under reduced pressure and the resulting residue was purified by silica gel column chromatography to give the title compound (113 mg) as red oil.

### [Step 3]

### Production of 2-[(3-fluoropyridin-2-yl)methoxy]-5,6,7,8-tetrahydr oquinolin-4-ol

To 2-[(3-fluoropyridin-2-yl)methoxy]-4-[(4-methoxybenz yl)oxy]-5,6,7,8-tetrahydroquinoline (270 mg) and anisole (148 mg) were added CH₂Cl₂ (3 mL), followed by the addition of TFA (3 mL) under ice water cooling, then the mixture was stirred at room temperature for 2 hours. After toluene was added to the reaction mixture, the solvent of the reaction mixture was evaporated under reduced pressure. The resulting residue was purified by silica gel column chromatography to give the title compound (149 mg) as a white powder.

### [Step 4]

### Production of 2-[(3-fluoropyridin-2-yl)methoxy]-5,6,7,8-tetrahydr oquinolin-4-yl trifluoromethanesulfonate

To 2-[(3-fluoropyridin-2-yl)methoxy]-5,6,7,8-tetrahydr oquinolin-4-ol (147 mg) were added CH₂Cl₂ (5 mL), Et₃N (5.3 mL), DMAP (6.5 mg) and Tf₂NPh (92 mg) under ice water cooling, and the mixture was stirred at room temperature overnight. The reaction mixture was added with water and ethyl acetate, and subjected to extraction. The organic layer was washed with saturated brine, dried over anhydrous sodium sulfate, filtered off, and the filtrate was evaporated under reduced pressure. The resulting residue was purified by silica gel column chromatography to give the title compound (220 mg) as colorless oil.

### [Step 5]

### Production of 5-{2-[(3-fluoropyridin-2-yl)methoxy]-5,6,7,8-tetrah ydroquinolin-4-yl}pyrimidin-2-amine hydrochloride

5-{2-[(3-Fluoropyridin-2-yl)methoxy]-5,6,7,8-t etrahydroquinolin-4-yl}pyrimidin-2-amine was prepared as a white powder according to the procedure described in Example 44 Step 5 using 2-[(3-fluoropyridin-2-yl)methoxy]-5,6,7,8-tetrahydr oquinolin-4-yl trifluoromethanesulfonate instead of 2-[(3,5-difluoropyridin-2-yl)methoxy]-6,7-dihydro-5 H-cyclopenta[b]pyridin-4-yl trifluoromethanesulfonate, and using 5-(4,4,5,5-tetramethyl-1,3,2-dioxaborolan-2-yl)pyri midin-2-amine instead of 5-(4,4,5,5-tetramethyl-1,3,2-dioxaborolan-2-yl)pyri midine-2-carbonitrile. To the resulting compound (50 mg) were added chloroform (3 mL) and methanol (1 mL), and followed by the addition of 1N HCl/Et₂O solution (0.156 mL), and the mixture was stirred for 0.5 hour. Ethyl acetate was added to the reaction mixture, and the resulting precipitate was collected by filtration to give the title compound (30 mg) as a pale yellow powder.
[MS (ESI) m/z 352.3 (M+H)⁺]

### Example 192

### 5-{2-[(5-Fluoropyridin-2-yl)methoxy]-5,6,7,8-tetrah ydroquinolin-4-yl}pyrimidin-2-amine hydrochloride

### [Step 1]

### Production of 2-[(5-fluoropyridin-2-yl)methoxy]-4-[(4-methoxybenz yl)oxy]-5,6,7,8-tetrahydroquinoline

To 2-chloro-4-[(4-methoxybenzyl)oxy]-5,6,7,8-tetrahydr oquinoline (450 mg), (5-fluoropyridin-2-yl)methanol (226 mL), Pd₂(dba)₃ (136 mg), t-Bu-X-Phos (151 mg) and NaO-t-Bu (285 mg) was added toluene (10 mL), and the mixture was degassed, then stirred under Ar atmosphere at 100°C for 2 hours. After the reaction mixture was allowed to return to room temperature, the reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (287 mg) as a yellow solid.

### [Step 2]

### Production of 2-[(5-fluoropyridin-2-yl)methoxy]-5,6,7,8-tetrahydr oquinolin-4-ol

The title compound was prepared as a white powder according to the procedure described in Example 191 Step 3 using 2-[(5-fluoropyridin-2-yl)methoxy]-4-[(4-methoxybenz yl)oxy]-5,6,7,8-tetrahydroquinoline instead of 2-[(3-fluoropyridin-2-yl)methoxy]-4-[(4-methoxybenz yl)oxy]-5,6,7,8-tetrahydroquinoline.

### [Step 3]

### Production of 2-[(5-fluoropyridin-2-yl)methoxy]-5,6,7,8-tetrahydr oquinolin-4-yl trifluoromethanesulfonate

The title compound was prepared as colorless oil according to the procedure described in Example 191 Step 4 using 2-[(5-fluoropyridin-2-yl)methoxy]-5,6,7,8-tetrahydr oquinolin-4-ol instead of 2-[(3-fluoropyridin-2-yl)methoxy]-5,6,7,8-tetrahydr oquinolin-4-ol.

### [Step 4]

### Production of 5-{2-[(5-fluoropyridin-2-yl)methoxy]-5,6,7,8-tetrah ydroquinolin-4-yl}pyrimidin-2-amine hydrochloride

The title compound (10 mg) was prepared as a white powder according to the procedure described in Example 4 Step 3 using 2-[(5-fluoropyridin-2-yl)methoxy]-5,6,7,8-tetrahydr oquinolin-4-yl trifluoromethanesulfonate instead of 2-(pyridin-2-ylmethoxy)-6,7-dihydro-5H-cyclopenta[b ]pyridin-4-yl trifluoromethanesulfonate, and using 5-(4,4,5,5-tetramethyl-1,3,2-dioxaborolan-2-yl)pyri midin-2-amine instead of 5-(4,4,5,5-tetramethyl-1,3,2-dioxaborolan-2-yl)pyri dine-2-carbonitrile
[MS (ESI) m/z 352.3 (M+H)⁺]

### Example 193

### 5-{2-[(6-Methylpyridin-2-yl)methoxy]-5,6,7,8-tetrah ydroquinolin-4-yl}pyrimidine-2-carbonitrile

### [Step 1]

### Production of 4-chloro-5,6,7,8-tetrahydroquinolin-2-ol

To 5,6,7,8-tetrahydroquinolin-2,4-diol (see, for example, Helvetica Chimica Acta, 1945, vol. 28, p. 1684-1690) (65 g) was added phosphoryl chloride (150 mL) and the mixture was 180°C for 8 hour in sealed tube. After the mixture was cooled under ice water cooling, toluene was added to the reaction mixture, and it was evaporated under reduced pressure to remove solvent and phosphory chloride. The residue was neutralized with ethyl acetate and saturated aqueous potassium carbonate solution, filtered through Celite and the Celite was washed with methanol. The resulting filtrate was evaporated under reduced pressure to give the title compound (16.6 g) as a white powder.

### [Step 2]

### Production of 4-chloro-5,6,7,8-tetrahydroquinolin-2-yl trifluoromethanesulfonate

The title compound was prepared as colorless oil according to the procedure described in Example 50 Step 3 using 4-chloro-5,6,7,8-tetrahydroquinolin-2-ol instead of 2-[(3-fluoropyridin-2-yl)methoxy]-6,7-dihydro-5H-cy clopenta[b]pyridin-4-ol.

### [Step 3]

### Production of 4-chloro-2-[(6-methylpyridin-2-yl)methoxy]-5,6,7,8-tetrahydroquinoline

To 4-chloro-5,6,7,8-tetrahydroquinolin-2-yl trifluoromethanesulfonate (300 mg), (6-methylpyridin-2-yl)methanol (117 mL), Pd₂(dba)₃·CHCl₃ (59 mg), t-Bu-X-Phos (97 mg) and cesium carbonate (929 mg) was added toluene (9 mL), and the mixture was degassed, then stirred under Ar atmosphere at 100°C for 5 hours. After the reaction mixture was allowed to return to room temperature, diluted with ethyl acetate, filtered through Celite, and the filtrate was evaporated under reduced pressure. The resulting residue was purified by silica gel column chromatography to give the title compound (90 mg).

### [Step 4]

### Production of 2-[(6-methylpyridin-2-yl)methoxy]-5,6,7,8-tetrahydr oquinolin-4-ol

To 4-chloro-2-[(6-methylpyridin-2-yl)methoxy]-5,6,7,8-tetrahydroquinoline (90 mg) were added DMSO (1 mL), water (1 mL) and potassium hydroxide (52 mg), and the mixture was stirred under Ar atmosphere at 100°C for 12 hours. t-Bu-X-Phos (32 mg) and Pd₂(dba)₃·CHCl₃ (19 mg) was added to the reaction mixture, and the mixture was further stirred at 100°C for 4 hours. After the reaction mixture was allowed to return to room temperature, diluted with ethyl acetate, filtered through Celite, and the filtrate was evaporated under reduced pressure. The resulting residue was purified by silica gel column chromatography to give the title compound (40 mg) as a yellow solid.

### [Step 5]

### Production of 2-[(6-methylpyridin-2-yl)methoxy]-5,6,7,8-tetrahydr oquinolin-4-yl trifluoromethanesulfonate

The title compound was prepared according to the procedure described in Example 50 Step 3 using 2-[(6-methylpyridin-2-yl)methoxy]-5,6,7,8-tetrahydr oquinolin-4-ol instead of 2-[(3-fluoropyridin-2-yl)methoxy]-6,7-dihydro-5H-cy clopenta[b]pyridin-4-ol.

### [Step 6]

### Production of 5-{2-[(6-methylpyridin-2-yl)methoxy]-5,6,7,8-tetrah ydroquinolin-4-yl}pyrimidine-2-carbonitrile

The title compound was prepared as a white solid according to the procedure described in Example 44 Step 5 using 2-[(6-methylpyridin-2-yl)methoxy]-5,6,7,8-tetrahydr oquinolin-4-yl trifluoromethanesulfonate instead of 2-[(3,5-difluoropyridin-2-yl)methoxy]-6,7-dihydro-5 H-cyclopenta[b]pyridin-4-yl trifluoromethanesulfonate.
[MS (ESI) m/z 358.4 (M+H)⁺]

### Example 194

### 4-(Pyrazin-2-yl)-2-(pyridin-2-ylmethoxy)-5,6,7,8-te trahydroquinoline hydrochloride

To 4-chloro-2-(pyridin-2-ylmethoxy)-5,6,7,8-tetrahydro quinoline (50 mg), 2-(tributylstannanyl)pyrazine (100 mg) and Pd(PPh₃)₄ (41 mg) was added DMF (0.6 mL), and the mixture was reacted under microwave irradiation at 180°C for 15 minutes. After the reaction mixture was allowed to return to room temperature, diluted with ethyl acetate, filtered through Celite, and the filtrate was evaporated under reduced pressure. The resulting residue was purified by silica gel column chromatography to give 4-(pyrazin-2-yl)-2-(pyridin-2-ylmethoxy)-5,6,7,8-te trahydroquinoline (20 mg).
[MS (ESI) m/z 319.3 (M+H)⁺]

The resulting compound was dissolved in ethyl acetate (2.5 mL), followed by the addition of 1N HCl/Et₂O solution (0.063 mL) under ice water cooling, and the resulting precipitate was collected by filtration to give the title compound (15 mg) as a white solid.
[MS (ESI) m/z 319.3 (M+H)⁺]

### Example 195

### 4-(6-Chloropyrazin-2-yl)-2-(pyridin-2-ylmethoxy)-5, 6,7,8-tetrahydroquinoline

The title compound was prepared as colorless oil according to the procedure described in Example 108 Step 2 using 2,6-dichloropyrazine instead of 3-bromo-2-fluoro-5-methylpyridine.
[MS (ESI) m/z 353.2 (M+H)⁺]

### Example 196

### 4-(5-Chloropyrazin-2-yl)-2-(pyridin-2-ylmethoxy)-5, 6,7,8-tetrahydroquinoline hydrochloride

4-(5-chloropyrazin-2-yl)-2-(pyridin-2-ylmethox y)-5,6,7,8-tetrahydroquinoline was prepared according to the procedure described in Example 108 Step 2 using 2,5-dichloropyrazine instead of 3-bromo-2-fluoro-5-methylpyridine. The resulting compound (20 mg) was dissolved in Et₂O, followed by addition of 1N HCl/Et₂O solution (0.057 mL), and the resulting precipitate was collected by filtration to give the title compound (18 mg) as a white powder.
[MS (ESI) m/z 353.0 (M+H)⁺]

### Example 197

### 4-(3-Chloropyrazin-2-yl)-2-(pyridin-2-ylmethoxy)-5, 6,7,8-tetrahydroquinoline hydrochloride

4-(3-chloropyrazin-2-yl)-2-(pyridin-2-ylmethox y)-5,6,7,8-tetrahydroquinoline was prepared according to the procedure described in Example 108 Step 2 using 2,3-dichloropyrazine instead of 3-bromo-2-fluoro-5-methylpyridine. The resulting compound (20 mg) was dissolved in Et₂O, followed by the addition of 1N HCl/Et₂O solution (0.057 mL), and the resulting precipitate was collected by filtration to give the title compound (15 mg) as a white powder.
[MS (ESI) m/z 353.3 (M+H)⁺]

### Example 198

### 3-[2-(Pyridin-2-ylmethoxy)-5,6,7,8-tetrahydroquinol in-4-yl]pyrazine-2-carbonitrile hydrochloride

The title compound was prepared as a white powder according to the procedure described in Example 54 Step 2 using 4-(3-chloropyrazin-2-yl)-2-(pyridin-2-ylmethoxy)-5, 6,7,8-tetrahydroquinoline instead of 4-(6-chloropyrazin-2-yl)-2-(pyridin-2-ylmethoxy)-6, 7-dihydro-5H-cyclopenta[b]pyridine.
[MS (ESI) m/z 344.6 (M+H)⁺]

### Example 199

### 4-(6-Fluoropyrazin-2-yl)-2-(pyridin-2-ylmethoxy)-5, 6,7,8-tetrahydroquinoline hydrochloride

To 4-(6-chloropyrazin-2-yl)-2-(pyridin-2-ylmethoxy)-5, 6,7,8-tetrahydroquinoline (20 mg), cesium fluoride (86 mg) and 1,4,7,10,13,16-hexaoxacyclooctadecane (7 mg) was added MeCN (1 mL), and the mixture was heated under reflux for 15 hours. After the reaction mixture was allowed to return to room temperature, the reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give 4-(6-fluoropyrazin-2-yl)-2-(pyridin-2-ylmethoxy)-5, 6,7,8-tetrahydroquinoline (13 mg). The resulting compound was dissolved in Et₂O, followed by the addition of 1N HCl/Et₂O solution (0.057 mL), and the resulting precipitate was collected by filtration to give the title compound (15 mg) as a white powder.
[MS (ESI) m/z 336.9 (M+H)⁺]

### Example 200

### 4-(3-Fluoropyrazin-2-yl)-2-(pyridin-2-ylmethoxy)-5, 6,7,8-tetrahydroquinoline hydrochloride

The title compound was prepared as a white powder according to the procedure described in Example 199 using 4-(3-chloropyrazin-2-yl)-2-(pyridin-2-ylmethoxy)-5, 6,7,8-tetrahydroquinoline instead of 4-(6-chloropyrazin-2-yl)-2-(pyridin-2-ylmethoxy)-5, 6,7,8-tetrahydroquinoline.
[MS (ESI) m/z 336.9 (M+H)⁺]

### Example 201

### 4-(6-methylpyrazin-2-yl)-2-(pyridin-2-ylmethoxy)-5, 6,7,8-tetrahydroquinoline

The title compound was prepared as a white powder according to the procedure described in Example 124 using 2-methyl-6-(4,4,5,5-tetramethyl-1,3,2-dioxaborolan-2-yl)pyrazine instead of 5-(4,4,5,5-tetramethyl-1,3,2-dioxaborolan-2-yl)pyri din-3-amine.
[MS (ESI) m/z 333.3 (M+H)⁺]

### Example 202

### 4-(6-Ethylpyrazin-2-yl)-2-(pyridin-2-ylmethoxy)-5,6 ,7,8-tetrahydroquinoline

To 4-(6-chloropyrazin-2-yl)-2-(pyridin-2-ylmethoxy)-5, 6,7,8-tetrahydroquinoline (20 mg) and Pd(dppf)Cl₂·CH₂Cl₂ (5 mg) were added THF (1 mL) and 1M diethylzinc/hexane solution (0.074 mL), and the mixture was degassed, then stirred under Ar atmosphere at 50°C for 0.5 hour. After the reaction mixture was allowed to return to room temperature, the reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (19 mg) as colorless oil.
[MS (ESI) m/z 347.3 (M+H)⁺]

### Example 203

### 4-[6-(Propan-2-yl)pyrazin-2-yl]-2-(pyridin-2-ylmeth oxy)-5,6,7,8-tetrahydroquinoline

To 4-(6-chloropyrazin-2-yl)-2-(pyridin-2-ylmethoxy)-5, 6,7,8-tetrahydroquinoline (20 mg), and dichloro[1,3-bis(diphenylphosphino)propane]nickel (II) (3 mg) were added THF (1 mL) and 0.5M 2-propylzinc bromide/THF solution (0.17 mL), and the mixture was degassed, then stirred under Ar atmosphere at 80°C for 5 hours and then stirred at 100°C for 2 hours. After the reaction mixture was allowed to return to room temperature, the reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (10 mg) as colorless oil.
[MS (ESI) m/z 361.4 (M+H)⁺]

### Example 204

### {6-[2-(Pyridin-2-ylmethoxy)-5,6,7,8-tetrahydroquino lin-4-yl]pyrazin-2-yl}methanol

### [Step 1]

### Production of 6-[2-(pyridin-2-ylmethoxy)-5,6,7,8-tetrahydroquinol in-4-yl]pyrazine-2-carbaldehyde

The title compound was prepared according to the procedure described in Example 108 Step 2 using 6-chloropyrazine-2-carbaldehyde instead of 3-bromo-2-fluoro-5-methylpyridine.

### [Step 2]

### Production of {6-[2-(pyridin-2-ylmethoxy)-5,6,7,8-tetrahydroquino lin-4-yl]pyrazin-2-yl}methanol

To 6-[2-(pyridin-2-ylmethoxy)-5,6,7,8-tetrahydroquinol in-4-yl]pyrazine-2-carbaldehyde (50 mg) was added MeOH (1.5 mL), followed by the addition of NaBH₄ (7 mg) under ice water cooling, and the mixture was stirred for 1 hour. Ethyl acetate, hydrochloric acid, aqueous sodium hydrogen carbonate solution and water were sequentially added to the reaction mixture, and the resulting mixture was subjected to extraction. The organic layer was dried over anhydrous sodium sulfate, filtered off, and the filtrate was evaporated under reduced pressure. The resulting residue was purified by silica gel column chromatography to give the title compound (43 mg) as a white powder.
[MS (ESI) m/z 349.3 (M+H)⁺]

### Example 205

### 4-(6-Methoxypyrazin-2-yl)-2-(pyridin-2-ylmethoxy)-5 ,6,7,8-tetrahydroquinoline hydrochloride

To 4-(6-chloropyrazin-2-yl)-2-(pyridin-2-ylmethoxy)-5, 6,7,8-tetrahydroquinoline (20 mg) was added 28% sodium methoxide/methanol solution (1mL) and the mixture was stirred at 60°C for 3 hours. After the reaction mixture was allowed to return to room temperature, the mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give 4-(6-methoxypyrazin-2-yl)-2-(pyridin-2-ylmethoxy)-5 ,6,7,8-tetrahydroquinoline (15 mg). The resulting compound was dissolved in Et₂O, followed by addition of 1N HCl/Et₂O solution (0.039 mL), and the resulting precipitate was collected by filtration to give the title compound (13 mg) as a white powder.
[MS (ESI) m/z 349.3 (M+H)⁺]

### Example 206

### 6-[2-(Pyridin-2-ylmethoxy)-5,6,7,8-tetrahydroquinol in-4-yl]pyrazin-2-amine hydrochloride

To 4-(6-chloropyrazin-2-yl)-2-(pyridin-2-ylmethoxy)-5, 6,7,8-tetrahydroquinoline (10 mg) was added 28% ammonia solution, and the mixture was reacted under microwave irradiation at 170°C for 2 hours. The solvent of the reaction mixture was evaporated under reduced pressure, and the resulting residue was purified by silica gel column chromatography to give the title compound (5 mg) as a white powder.
[MS (ESI) m/z 334.3 (M+H)⁺]

### Example 207

### 5-[2-(Pyridin-2-ylmethoxy)-5,6,7,8-tetrahydroquinol in-4-yl]pyrazin-2-amine

The title compound was prepared (5 mg) as a white powder according to the procedure described in Example 206 using 4-(5-chloropyrazin-2-yl)-2-(pyridin-2-ylmethoxy)-5, 6,7,8-tetrahydroquinoline instead of 4-(6-chloropyrazin-2-yl)-2-(pyridin-2-ylmethoxy)-5, 6,7,8-tetrahydroquinoline.
[MS (ESI) m/z 334.3 (M+H)⁺]

### Example 208

### N-MethylMethyl-6-[2-(pyridin-2-ylmethoxy)-5,6,7,8-t etrahydroquinolin-4-yl]pyrazin-2-amine hydrochloride

To 4-(6-chloropyrazin-2-yl)-2-(pyridin-2-ylmethoxy)-5, 6,7,8-tetrahydroquinoline (10 mg) was added 2N monomethylamine/methanol solution (1 mL), and the mixture was reacted under microwave irradiation at 190°C for 1 hour. The reaction mixture was evaporated under reduced pressure, and the resulting residue was purified by silica gel column chromatography to give N-methyl-6-[2-(pyridin-2-ylmethoxy)-5,6,7,8-tetrahy droquinolin-4-yl]pyrazin-2-amine (10 mg) as a white solid. The resulting compound was dissolved in chloroform, followed by addition of 1N HCl/Et₂O solution (0.029 mL) and the solvent of the reaction mixture was evaporated under reduced pressure. Et₂O was added to the resulting residue, and the insoluble portion was collected by filtration to give the title compound (10 mg) as a yellow powder.
[MS (ESI) m/z 348.2 (M+H)⁺]

### Example 209

### N,N-DimethylDimethyl-6-[2-(pyridin-2-ylmethoxy)-5,6 ,7,8-tetrahydroquinolin-4-yl]pyrazin-2-amine hydrochloride

To 4-(6-chloropyrazin-2-yl)-2-(pyridin-2-ylmethoxy)-5, 6,7,8-tetrahydroquinoline (10 mg), dimethylamine hydrochloride (12 mg) and cesium carbonate (46 mg) was DMF (0.5 mL), and the mixture was stirred at 120°C for 5 hours. After the reaction mixture was allowed to return to room temperature, diluted with ethyl acetate, filtered through Celite, and the filtrate of the reaction mixture was evaporated under reduced pressure. The resulting residue was purified by silica gel column chromatography to give N,N-dimethyl-6-[2-(pyridin-2-ylmethoxy)-5,6,7,8-tet rahydroquinolin-4-yl]pyrazin-2-amine (8 mg). The resulting compound was dissolved in chloroform, followed by the addition of 1N HCl/Et₂O solution (0.022 mL), and the solvent was evaporated under reduced pressure. Et₂O was added to the resulting residue, and the insoluble portion was collected by filtration to give the title compound (8 mg) as a yellow powder.
[MS (ESI) m/z 362.4 (M+H)⁺]

### Example 210

### N-MethylMethyl-1-{6-[2-(pyridin-2-ylmethoxy)-5,6,7, 8-tetrahydroquinolin-4-yl]pyrazin-2-yl}methanamine

To 6-[2-(pyridin-2-ylmethoxy)-5,6,7,8-tetrahydroquinol in-4-yl]pyrazine-2-carbaldehyde (15 mg) were added CH₂Cl₂ (0.5 mL) and 2N monomethyamine/THF solution (0.043 mL), and the mixture was stirred at room temperature for 0.5 hour. Sodium triacetoxyborohydride (18 mg) and acetic acid (0.007 mL) were added to the reaction mixture, and the mixture was further stirred at room temperature for 16 hours. The mixture was diluted with ethyl acetate, filtered through Celite, and the filtrate was evaporated under reduced pressure. The resulting residue was purified by silica gel column chromatography to give the title compound (9 mg) as colorless oil.
[MS (ESI) m/z 362.4 (M+H)⁺]

### Example 211

### N,N-DimethylDimethyl-1-{6-[2-(pyridin-2-ylmethoxy)-5,6,7,8-tetrahydroquinolin-4-yl]pyrazin-2-yl}methan amine

The title compound was prepared as colorless oil according to the procedure described in Example 210 using 2N dimethylamine/THF solution instead of 2N monomethylamine/THF solution.
[MS (ESI) m/z 376.2 (M+H)⁺]

### Example 212

### 2-[(6-Fluoropyridin-2-yl)methoxy]-4-(6-methylpyrazi n-2-yl)-5,6,7,8-tetrahydroquinoline

### [Step 1]

### Production of 2-chloro-4-(6-methylpyrazin-2-yl)-5,6,7,8-tetrahydr oquinoline

The title compound was prepared as a white powder according to the procedure described in Example 137 Step 2 using 2-chloro-6-methylpyrazine instead of 2-fluoro-3-iodopyridine.

### [Step 2]

### Production of 2-[(6-fluoropyridin-2-yl)methoxy]-4-(6-methylpyrazi n-2-yl)-5,6,7,8-tetrahydroquinoline

The title compound was prepared as colorless oil according to the procedure described in Example 140 Step 2 using 2-chloro-4-(6-methylpyrazin-2-yl)-5,6,7,8-tetrahydr oquinoline instead of 5-(2-chloro-5,6,7,8-tetrahydroquinolin-4-yl)pyridin e-2-carbonitrile.
[MS (ESI) m/z 351.4 (M+H)⁺]

### Example 213

### 2-[(5-FluoropyridinFluoropyridin-2-yl)methoxy]-4-(6 -methylpyrazin-2-yl)-5,6,7,8-tetrahydroquinoline

The title compound was prepared as a white powder according to the procedure described in Example 212 Step 2 using (5-fluoropyridin-2-yl)methanol instead of (6-fluoropyridin-2-yl)methanol.
[MS (ESI) m/z 351.4 (M+H)⁺]

### Example 214

### 2-[(3-Fluoropyridin-2-yl)methoxy]-4-(6-methylpyrazi n-2-yl)-5,6,7,8-tetrahydroquinoline

The title compound was prepared as colorless oil according to the procedure described in Example 212 Step 2 using (3-fluoropyridin-2-yl)methanol instead of (6-fluoropyridin-2-yl)methanol.
[MS (ESI) m/z 351.4 (M+H)⁺]

### Example 215

### (6-{2-[(6-Fluoropyridin-2-yl)methoxy]-5,6,7,8-tetra hydroquinolin-4-yl}pyrazin-2-yl)methanol

### [Step 1]

### Production of [6-(2-chloro-5,6,7,8-tetrahydroquinolin-4-yl)pyrazi n-2-yl]methanol

The title compound was prepared as a yellow solid according to the procedure described in Example 137 Step 2 using (6-chloropyradin-2-yl)methanol instead of 2-fluoro-3-iodopyridine, and using 1,4-dioxane instead of THF.

### [Step 2]

### Production of 4-[6-({[tert-butyl(dimethyl)silyl]oxy}methyl)pyrazi n-2-yl]-2-chloro-5,6,7,8-tetrahydroquinoline

The title compound was prepared as colorless oil according to the procedure described in Example 161 Step 1 using [6-(2-chloro-5,6,7,8-tetrahydroquinolin-4-yl)pyrazi n-2-yl]methanol instead of (6-bromopyridin-2-yl)methanol.

### [Step 3]

### Production of 4-[6-({[tert-butyl(dimethyl)silyl]oxy}methyl)pyrazi n-2-yl]-2-[(6-fluoropyridin-2-yl)methoxy]-5,6,7,8-t etrahydroquinoline

The title compound was prepared as yellow oil according to the procedure described in Example 140 Step 2 using 4-[6-({[tert-butyl(dimethyl)silyl]oxy}methyl)pyrazi n-2-yl]-2-chloro-5,6,7,8-tetrahydroquinoline instead of 5-(2-chloro-5,6,7,8-tetrahydroquinolin-4-yl)pyridin e-2-carbonitrile.

### [Step 4]

### Production of (6-{2-[(6-fluoropyridin-2-yl)methoxy]-5,6,7,8-tetra hydroquinolin-4-yl}pyrazin-2-yl)methanol

To 4-[6-({[tert-butyl(dimethyl)silyl]oxy}methyl)pyrazi n-2-yl]-2-[(6-fluoropyridin-2-yl)methoxy]-5,6,7,8-t etrahydroquinoline (60 mg) were added THF (2.4 mL) and 1.0M TBAF/THF solution (0.375 mL), and the mixture was stirred at room temperature for 12 hours. The solvent of the reaction mixture was evaporated under reduced pressure. The resulting residue was purified by silica gel column chromatography to give the title compound (29 mg) as yellow oil.
[MS (ESI) m/z 367.4 (M+H)⁺]

### Example 216

### (6-{2-[(5-Fluoropyridin-2-yl)methoxy]-5,6,7,8-tetra hydroquinolin-4-yl}pyrazin-2-yl)methanol

### [Step 1] Production of

### 4-[6-({[tert-butyl(dimethyl)silyl]oxy}methyl)pyrazi n-2-yl]-2-[(5-fluoropyridin-2-yl)methoxy]-5,6,7,8-t etrahydroquinoline

To 4-[6-({[tert-butyl(dimethyl)silyl]oxy}methyl)pyrazi n-2-yl]-2-chloro-5,6,7,8-tetrahydroquinoline (80 mg), (5-fluoropyridin-2-yl)methanol (31 mg), Pd₂(dba)₃ (11 mg), t-Bu-X-Phos (21 mg) and NaO-t-Bu (39 mg) was added toluene (2 mL), and the mixture was degassed, and then stirred under Ar atmosphere at 100°C overnight. After the reaction mixture was allowed to return to room temperature, diluted with ethyl acetate, filtered through Celite, and the filtrate was evaporated under reduced pressure. The resulting residue was purified by silica gel column chromatography to give the title compound (59 mg) as yellow oil.

### [Step 2]

### Production of (6-{2-[(5-fluoropyridin-2-yl)methoxy]-5,6,7,8-tetra hydroquinolin-4-yl}pyrazin-2-yl)methanol

The title compound was prepared as yellow oil according to the procedure described in Example 215 Step 4 using 4-[6-({[tert-butyl(dimethyl)silyl]oxy}methyl)pyrazi n-2-yl]-2-[(5-fluoropyridin-2-yl)methoxy]-5,6,7,8-t etrahydroquinoline instead of 4-[6-({[tert-butyl(dimethyl)silyl]oxy}methyl)pyrazi n-2-yl]-2-[(6-fluoropyridin-2-yl)methoxy]-5,6,7,8-t etrahydroquinoline.
[MS (ESI) m/z 367.4 (M+H)⁺]

### Example 217

### (6-{2-[(3-Fluoropyridin-2-yl)methoxy]-5,6,7,8-tetra hydroquinolin-4-yl}pyrazin-2-yl)methanol

### [Step 1]

### Production of 4-[6-({[tert-butyl(dimethyl)silyl]oxy}methyl)pyrazi n-2-yl]-2-[(3-fluoropyridin-2-yl)methoxy]-5,6,7,8-t etrahydroquinoline

The title compound was prepared according to the procedure described in Example 216 Step 1 using (3-fluoropyridin-2-yl)methanol instead of (5-fluoropyridin-2-yl)methanol.

### [Step 2]

### Production of (6-{2-[(3-fluoropyridin-2-yl)methoxy]-5,6,7,8-tetra hydroquinolin-4-yl}pyrazin-2-yl)methanol

The title compound was prepared as yellow oil according to the procedure described in Example 215 Step 4 using 4-[6-({[tert-butyl(dimethyl)silyl]oxy}methyl)pyrazi n-2-yl]-2-[(3-fluoropyridin-2-yl)methoxy]-5,6,7,8-t etrahydroquinoline instead of 4-[6-({[tert-butyl(dimethyl)silyl]oxy}methyl)pyrazi n-2-yl]-2-[(6-fluoropyridin-2-yl)methoxy]-5,6,7,8-t etrahydroquinoline.
[MS (ESI) m/z 367.4 (M+H)⁺]

### Example 218

### (6-{2-[(3,5-Difluoropyridin-2-yl)methoxy]-5,6,7,8-t etrahydroquinolin-4-yl}pyrazin-2-yl)methanol

### [Step 1]

### Production of 4-[6-({[tert-butyl(dimethyl)silyl]oxy}methyl)pyrazi n-2-yl]-2-[(3,5-difluoropyridin-2-yl)methoxy]-5,6,7 ,8-tetrahydroquinoline

The title compound was prepared according to the procedure described in Example 216 Step 1 using (3,5-difluoropyridin-2-yl)methanol instead of (5-fluoropyridin-2-yl)methanol.

### [Step 2]

### Production of (6-{2-[(3,5-difluoropyridin-2-yl)methoxy]-5,6,7,8-t etrahydroquinolin-4-yl}pyrazin-2-yl)methanol

The title compound was prepared as yellow oil according to the procedure described in Example 215 Step 4 using 4-[6-({[tert-butyl(dimethyl)silyl]oxy}methyl)pyrazi n-2-yl]-2-[(3,5-difluoropyridin-2-yl)methoxy]-5,6,7 ,8-tetrahydroquinoline instead of 4-[6-({[tert-butyl(dimethyl)silyl]oxy}methyl)pyrazi n-2-yl]-2-[(6-fluoropyridin-2-yl)methoxy]-5,6,7,8-t etrahydroquinoline.
[MS (ESI) m/z 385.4 (M+H)⁺]

### Example 219

### 6-({[4-(6-Methoxypyrazin-2-yl)-5,6,7,8-tetrahydroqu inolin-2-yl]oxy}methyl)pyridine-2-carbonitrile

The title compound was prepared as a white powder according to the procedure described in Example 108 Step 2 using 6-({[4-(4,4,5,5-tetramethyl-1,3,2-dioxaborolan-2-yl )-5,6,7,8-tetrahydroquinolin-2-yl]oxy}methyl)pyridi ne-2-carbonitrile instead of 2-(pyridin-2-ylmethoxy)-4-(4,4,5,5-tetramethyl-1,3, 2-dioxaborolan-2-yl)-5,6,7,8-tetrahydroquinoline, and using 2-chloro-6-methoxypyrazine instead of 3-bromo-2-fluoro-5-methylpyridine.
[MS (ESI) m/z 374.4 (M+H)⁺]

### Example 220

### 4-(3,4-Difluorophenyl)-2-(pyridin-2-ylmethoxy)-5,6, 7,8-tetrahydroquinoline

The title compound was prepared as a white solid according to the procedure described in Example 108 Step 2 using 4-bromo-1,2-difluorobenzene instead of 3-bromo-2-fluoro-5-methylpyridine.
[MS (ESI) m/z 353.0 (M+H)⁺]

### Example 221

### 4-(3-Methoxyphenyl)-2-(pyridin-2-ylmethoxy)-5,6,7,8 -tetrahydroquinoline

The title compound was prepared as a white solid according to the procedure described in Example 108 Step 2 using 1-bromo-3-methoxybenzene instead of 3-bromo-2-fluoro-5-methylpyridine.
[MS (ESI) m/z 348.3 (M+H)⁺]

### Example 222

### 4-(4-Methylpyridin-2-yl)-2-(pyridin-2-ylmethoxy)-5, 6,7,8-tetrahydroquinoline

To 4-chloro-2-(pyridin-2-ylmethoxy)-5,6,7,8-tetrahydro quinoline (100 mg), 4-methyl-2-(tributylstannanyl)pyridine (139 mg) and Pd(PPh₃)₄ (42 mg) was added 1,4-dioxane (0.5 mL), and the mixture was reacted under microwave irradiation at 160°C for 1 hour. After the reaction mixture was allowed to return to room temperature, diluted with ethyl acetate, filtered through Celite, and the filtrate was evaporated under reduced pressure. The resulting residue was purified by silica gel column chromatography to give the title compound (54 mg) as colorless oil.
[MS (ESI) m/z 332.3 (M+H)⁺]

### Example 223

### 4-(4-Fluoropyridin-2-yl)-2-(pyridin-2-ylmethoxy)-5, 6,7,8-tetrahydroquinoline

The title compound was prepared as a white solid according to the procedure described in Example 108 Step 2 using 2-chloro-4-fluoropyridine instead of 3-bromo-2-fluoro-5-methylpyridine.
[MS (ESI) m/z 336.3 (M+H)⁺]

### Example 224

### 2-(Pyridin-2-ylmethoxy)-4-(1,3-thiazol-5-yl)-5,6,7, 8-tetrahydroquinoline

To 4-chloro-2-(pyridin-2-ylmethoxy)-5,6,7,8-tetrahydro quinoline (30 mg), 5-(tributylstannanyl)-1,3-thiazole (82 mg) and Pd(PPh₃)₄ (25 mg) was added DMF (0.8 mL), and the mixture was stirred at 100°C for 6 hours. The mixture was filtered through Celite, and the filtrate was evaporated under reduced pressure. The resulting residue was purified by silica gel column chromatography to give the title compound (13 mg) as colorless oil.
[MS (ESI) m/z 324.3 (M+H)⁺]

### Example 225

### 4-(2-Methyl-1,3-thiazol-5-yl)-2-(pyridin-2-ylmethox y)-5,6,7,8-tetrahydroquinoline hydrochloride

4-(2-Methyl-1,3-thiazol-5-yl)-2-(pyridin-2-ylm ethoxy)-5,6,7,8-tetrahydroquinoline was prepared as yellow oil according to the procedure described in Example 222 using 2-methyl-5-(tributylstannanyl)-1,3-thiazole instead of 4-methyl-2-(tributylstannanyl)pyridine, and using DMF instead of 1,4-dioxane. The resulting compound (68 mg) was dissolved in ethyl acetate (4 mL), followed by the addition of 1N HCl/Et₂O solution (0.2 mL) under ice water cooling, and the mixture was stirred at the same temperature for 2 hours. The resulting precipitate was collected by filtration to give the title compound (65 mg) as a white powder.
[MS (ESI) m/z 338.2 (M+H)⁺]

### Example 226

### {5-[2-(Pyridin-2-ylmethoxy)-5,6,7,8-tetrahydroquino lin-4-yl]-1,3-thiazol-2-yl}methanol hydrochloride

To 2-(pyridin-2-ylmethoxy)-4-(1,3-thiazol-5-yl)-5,6,7, 8-tetrahydroquinoline (84 mg) was added THF (2.6 mL) and 1.1M lithium hexamethyldisilazide/THF solution (0.71 mL) was added to the mixture at -78°C, then the mixture was stirred for 0.5 hour. Then, DMF (0.06 mL) was added to the reaction mixture, and the mixture was further stirred for 1 hour. The reaction mixture was added with water and ethyl acetate, and subjected to extraction. The organic layer was dried over anhydrous sodium sulfate, filtered off, and the filtrate was evaporated under reduced pressure. The resulting residue was dissolved in MeOH (2 mL), and NaBH₄ (13 mg) was added to the mixture, and then the mixture was stirred for 1 hour. The reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give {5-[2-(pyridin-2-ylmethoxy)-5,6,7,8-tetrahydroquino lin-4-yl]-1,3-thiazol-2-yl}methanol (19 mg) as colorless oil.
The resulting compound (9 mg) was dissolved in ethyl acetate (2 mL), followed by the addition of 1N HCl/Et₂O solution (0.04 mL) and the resulting precipitate was collected by filtration to give the title compound (4.3 mg) as a white powder.
[MS (ESI) m/z 354.1 (M+H)⁺]

### Example 227

### 4-[2-(Methoxymethyl)-1,3-thiazol-5-yl]-2-(pyridin-2 -ylmethoxy)-5,6,7,8-tetrahydroquinoline

To {5-[2-(pyridin-2-ylmethoxy)-5,6,7,8-tetrahydroquino lin-4-yl]-1,3-thiazol-2-yl}methanol (10 mg) were added THF (1 mL), NaH (60% dispersion in oil, 3 mg), and MeI (0.0053 mL) sequentially under ice water cooling, and the mixture was stirred at room temperature for 4 hours. The reaction mixture was purified by silica gel column chromatography to give the title compound (3 mg) as colorless oil.
[MS (ESI) m/z 368.3 (M+H)⁺]

### Example 228

### 4-{2-[(Propan-2-yloxy)methyl]-1,3-thiazol-5-yl}-2-( pyridin-2-ylmethoxy)-5,6,7,8-tetrahydroquinoline

To {5-[2-(pyridin-2-ylmethoxy)-5,6,7,8-tetrahydroquino lin-4-yl]-1,3-thiazol-2-yl}methanol (39 mg) were added CH₂Cl₂ (2mL), carbon tetrabromide (43 mg), and PPh₃ (35 mg), and the mixture was stirred at room temperature for 3 hours. The reaction mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography. To the obtained product (colorless oil, 10 mg) was added 2-propanol (2 mL), followed by the addition of NaH (60% dispersion in oil, 4 mg) under ice water cooling, and the mixture was stirred at room temperature overnight. The solvent of the reaction mixture was evaporated under reduced pressure, and then the residue was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (6 mg) as colorless oil.
[MS (ESI) m/z 396.3 (M+H)⁺]

### Example 229

### 2-[2-(Pyridin-2-ylmethoxy)-5,6,7,8-tetrahydroquinol in-4-yl]-1,3-thiazole-5-carbonitrile

The title compound was prepared as a white solid according to the procedure described in Example 108 Step 2 using 2-bromo-1,3-thiazole-5-carbonitrile instead of 3-bromo-2-fluoro-5-methylpyridine.
[MS (ESI) m/z 349.3 (M+H)⁺]

### Example 230

### 6-(2-(Pyridin-2-ylmethoxy)-5,6,7,8-tetrahydroquinol in-4-yl)pyrazin-2-ol

To 4-(6-chloropyrazin-2-yl)-2-(pyridin-2-ylmethoxy)-5, 6,7,8-tetrahydroquinoline (10 mg), potassium hydroxide (5 mg), Pd₂(dba)₃ (1.3 mg) and t-Bu-X-Phos (2 mg) were added 1,4-dioxane (0.5 mL) and water (0.5 mL), and the mixture was degassed, then stirred under Ar atmosphere at 100°C for 1 hour. After the reaction mixture was allowed to return to room temperature, the mixture was dried over anhydrous sodium sulfate, filtered through Celite, and the filtrate was evaporated under reduced pressure. The resulting residue was purified by silica gel column chromatography to give the title compound (10 mg) as a white powder.
[MS (ESI) m/z 335.1 (M+H)⁺]

### Example 231

### 4-(1-Oxidopyrimidin-5-yl)-2-(pyridin-2-ylmethoxy)-5 ,6,7,8-tetrahydroquinoline

The title compound was prepared as a pale brown solid according to the procedure described in Example 108 Step 2 using 5-bromopyrimidine 1-oxide instead of 3-bromo-2-fluoro-5-methylpyridine.
[MS (ESI) m/z 335.3 (M+H)⁺]

### Example 232

### 4-(Pyridin-3-yl)-2-(pyridin-2-ylmethoxy)-6,7,8,9-te trahydro-5H-cyclohepta[b]pyridine

### [Step 1]

### Production of 4-chloro-2-(pyridin-2-ylmethoxy)-6,7,8,9-tetrahydro -5H-cyclohepta[b]pyridine

The title compound was prepared as yellow oil according to the procedure described in Example 1 Step 1 using 2,4-dichloro-6,7,8,9-tetrahydro-5H-cyclohepta[b]pyr idine (see, for example, Helvetica Chimica Acta, 1944, vol. 27, p.1854-1858) instead of 2,4-dichloro-6,7-dihydro-5H-cyclopenta[b]pyridine. [Rf value (TLC silica gel plate 60F₂₅₄, developing solvent: hexane : ethyl acetate =2:1) : 0.6]

### [Step 2]

### Production of 2-(pyridin-2-ylmethoxy)-4-(pyrimidin-5-yl-6,7,8,9-t etrahydro-5H-cyclohepta[b]pyridine

To 4-chloro-2-(pyridin-2-ylmethoxy)-6,7,8,9-tetrahydro -5H-cyclohepta[b]pyridine (18 mg), pyridin-3-ylboronic acid (15 mg), Pd(dppf)Cl₂·CH₂Cl₂ (10 mg) and potassium carbonate (26 mg) was added 1,4-dioxane/water (3/1, 1 mL), and the mixture was stirred under at 100°C for 4 hours. After the reaction mixture was allowed to return to room temperature, diluted with ethyl acetate, filtered through Celite, and the filtrate was evaporated under reduced pressure. The resulting residue was purified by silica gel column chromatography to give the title compound (5 mg) as a white solid.
[MS (ESI) m/z 332.3 (M+H)⁺]

### Example 233

### 2-(Pyridin-2-ylmethoxy)-4-(pyrimidin-5-yl)-6,7,8,9-tetrahydro-5H-cyclohepta[b]pyridine hydrochloride

The title compound was prepared as a white powder according to the procedure described in Example 12 using 4-chloro-2-(pyridin-2-ylmethoxy)-6,7,8,9-tetrahydro -5H-cyclohepta[b]pyridine instead of 4-chloro-2-(pyridin-2-ylmethoxy)-6,7-dihydro-5H-cyc lopenta[b]pyridine.
[MS (ESI) m/z 333.3 (M+H)⁺]

### Example 234

### 4-(Pyrazin-2-yl)-2-(pyridin-2-ylmethoxy)-6,7,8,9-te trahydro-5H-cyclohepta[b]pyridine hydrochloride

To 4-chloro-2-(pyridin-2-ylmethoxy)-6,7,8,9-tetrahydro -5H-cyclohepta[b]pyridine (70 mg), 2-(tributylstannanyl)pyrazine (166 mg) and Pd(PPh₃)₄ (55 mg) was added DMF (0.6 mL), and the mixture was reacted under microwave irradiation at 180°C for 30 minutes. After the reaction mixture was allowed to return to room temperature, diluted with ethyl acetate, filtered through Celite, and the filtrate was evaporated under reduced pressure. The resulting residue was purified by silica gel column chromatography to give 4-(pyrazin-2-yl)-2-(pyridin-2-ylmethoxy)-6,7,8,9-te trahydro-5H-cyclohepta[b]pyridine (94 mg).
[MS (ESI) m/z 333.2 (M+H)⁺]

The resulting compound was dissolved in ethyl acetate (3.1 mL), followed by the addition of 1N HCl/Et₂O solution (0.078 mL) under ice water cooling, then stirred at the same temperature for 1 hour. The resulting precipitate was collected by filtration to give the title compound (23 mg) as a white powder.

### Example 235

### 2-(Pyridin-2-ylmethoxy)-4-(pyrimidin-5-yl)-6,7-dihy dro-5H-cyclopenta[b]pyridin-7-ol hydrochloride

### [Step 1]

### Production of 4-chloro-2-(pyridin-2-ylmethoxy)-6,7-dihydro-5H-cyc lopenta[b]pyridine 1-oxide

To 2,4-dichloro-6,7-dihydro-5H-cyclopenta[b]pyridine 1-oxide (1 g) and pyridin-2-ylmethanol (0.521 mL) were added DMF (10 mL) and NaH (60% dispersion in oil, 274 mg) under Ar atmosphere under ice water cooling, and the mixture was stirred for 1 hour. The reaction mixture was purified by silica gel column chromatography to give the title compound (1.28 g).

### [Step 2]

### Production of 4-chloro-2-(pyridin-2-ylmethoxy)-6,7-dihydro-5H-cyc lopenta[b]pyridin-7-yl acetate

To 4-chloro-2-(pyridin-2-ylmethoxy)-6,7-dihydro-5H-cyc lopenta[b]pyridine 1-oxide (1.28 g) was added acetic anhydride (15 mL), and the mixture was stirred at 90°C for 2 hours. After the reaction mixture was allowed to return to room temperature, excess acetic anhydride was evaporated under reduced pressure. The resulting residue was neutralized with Et₂O and saturated aqueous sodium hydrogen carbonate solution, and then extracted with Et₂O. The organic layer was dried over anhydrous sodium sulfate, filtered off, and the filtrate was evaporated under reduced pressure. The resulting residue was purified by silica gel column chromatography to give the title compound (1.25 mg) as yellow oil.

### [Step 3]

### Production of 2-(pyridin-2-ylmethoxy)-4-(pyrimidin-5-yl)-6,7-dihy dro-5H-cyclopenta[b]pyridin-7-ol hydrochloride

2-(Pyridin-2-ylmethoxy)-4-(pyrimidin-5-yl)-6,7 -dihydro-5H-cyclopenta[b]pyridin-7-ol (95 mg) was prepared as a white powder according to the procedure described in Example 58 using 4-chloro-2-(pyridin-2-ylmethoxy)-6,7-dihydro-5H-cyc lopenta[b]pyridin-7-yl acetate instead of 4-chloro-2-(pyridin-2-ylmethoxy)-6,7-dihydro-5H-cyc lopenta[b]pyridine, and using pyrimidin-5-ylboronic acid instead of (3-fluorophenyl)boronic acid. The resulting compound was dissolved in ethyl acetate (8 mL), followed by the addition of 1N HCl/Et₂O solution (0.3 mL), and then stirred for 0.5 hour. The resulting precipitate was collected by filtration to give the title compound (93.4 mg) as a white powder.
[MS (ESI) m/z 321.2 (M+H)⁺]

### Example 236

### 2-(Pyridin-2-ylmethoxy)-4-(pyrimidin-5-yl)-5,6-dihy dro-7H-cyclopenta[b]pyridin-7-one

To 2-(pyridin-2-ylmethoxy)-4-(pyrimidin-5-yl)-6,7-dihy dro-5H-cyclopenta[b]pyridin-7-ol (3 g) and 1,1,1-triacetoxy-1,1-dihydro-1,2-benziodoxol-3(1H)-one (Dess-Martin reagent, 4.37 g) was added CH₂Cl₂ (100 mL), and the mixture was stirred under Ar atmosphere at room temperature overnight. Chloroform and saturated aqueous sodium hydrogen carbonate solution was added to the reaction mixture, and the resulting precipitate was removed by filtration, and the filtrate was extracted with chloroform. The organic layer was dried over anhydrous sodium sulfate, filtered off, and the filtrate was evaporated under reduced pressure. The resulting residue was purified by silica gel column chromatography to give the title compound (2.2 g) as a pale yellow powder.
[MS (ESI) m/z 319.3 (M+H)⁺]

### Example 237

### 7-Methylidene-2-(pyridin-2-ylmethoxy)-4-(pyrimidin-5-yl)-6,7-dihydro-5H-cyclopenta[b]pyridine

To methyltriphenylphosphonium bromide (281 mg) were added THF (8mL) and KO-t-Bu (90 mg) under ice water cooling, and the mixture was stirred under Ar atmosphere for 1 hour. 2-(Pyridin-2-ylmethoxy)-4-(pyrimidin-5-yl)-5,6-dihy dro-7H-cyclopenta[b]pyridin-7-one (50 mg) was added to the reaction mixture, and the mixture was further stirred at room temperature for 2 hours. The mixture was added with water and ethyl acetate, and subjected to extraction. The organic layer was washed with saturated brine, dried over anhydrous sodium sulfate, filtered off, and the filtrate was evaporated under reduced pressure. The resulting residue was purified by silica gel column chromatography to give the title compound (24 mg) as a white powder.
[MS (ESI) m/z 317.3 (M+H)⁺]

### Example 238

### 7-Methyl-2-(pyridin-2-ylmethoxy)-4-(pyrimidin-5-yl) -6,7-dihydro-5H-cyclopenta[b]pyridine

### [Step 1]

### Production of 7-methyl-4-(pyrimidin-5-yl)-6,7-dihydro-5H-cyclopen ta[b]pyridin-2-ol

To 7-methylidene-2-(pyridin-2-ylmethoxy)-4-(pyrimidin-5-yl)-6,7-dihydro-5H-cyclopenta[b]pyridine (24 mg) were added methanol (4 mL) and palladium-activated carbon ethylenediamine complex (50 mg), and the mixture was stirred under hydrogen atmosphere at room temperature for 2 days. The reaction mixture was filtered through Celite, and the solvent was evaporated under reduced pressure to give the title compound (17 mg).

### [Step 2]

### Production of 7-methyl-2-(pyridin-2-ylmethoxy)-4-(pyrimidin-5-yl) -6,7-dihydro-5H-cyclopenta[b]pyridine

To 7-methyl-4-(pyrimidin-5-yl)-6,7-dihydro-5H-cyclopen ta[b]pyridin-2-ol (17 mg)and 2-(bromomethyl)pyridine hydrobromide (38 mg) were added toluene (10 mL) and silver carbonate (21 mg), and the mixture was stirred under Ar atmosphere at 100°C for 10 hours. After the reaction mixture was allowed to return to room temperature, diluted with ethyl acetate, filtered through Celite, and the filtrate was evaporated under reduced pressure. The resulting residue was purified by silica gel column chromatography to give the title compound (5 mg) as a white powder.
[MS (ESI) m/z 319.3 (M+H)⁺]

### Example 239

### 7-Ethylidene-2-(pyridin-2-ylmethoxy)-4-(pyrimidin-5 -yl)-6,7-dihydro-5H-cyclopenta[b]pyridine

THF (11 mL) was added to ethyltriphenylphosphonium bromide (416 mg), followed by the addition of 2.7 M of n-butyllithium/THF (0.41 mL) under ice water cooling, and the mixture was stirred under Ar atmosphere for 1 hour. 2-(Pyridin-2-ylmethoxy)-4-(pyrimidin-5-yl)-5,6-dihy dro-7H-cyclopenta[b]pyridin-7-one (70 mg) was added to the reaction mixture and the mixture was further stirred at room temperature for 2 hours. The mixture was added with water and ethyl acetate, and subjected to extraction. The organic layer was washed with saturated brine, dried over anhydrous sodium sulfate, filtered off, and the filtrate was evaporated under reduced pressure. The resulting residue was purified by silica gel column chromatography to give the title compound (63 mg) as a white powder.
[MS (ESI) m/z 331.4 (M+H)⁺]

### Example 240

### 7-Ethyl-2-(pyridin-2-ylmethoxy)-4-(pyrimidin-5-yl)-6,7-dihydro-5H-cyclopenta[b]pyridine

To 7-ethylidene-2-(pyridin-2-ylmethoxy)-4-(pyrimidin-5 -yl)-6,7-dihydro-5H-cyclopenta[b]pyridine (63 mg) were added methanol (4 mL) and palladium-activated carbon ethylenediamine complex (30 mg), and the mixture was stirred under hydrogen atmosphere at room temperature for one day. Palladium-activated carbon ethylenediamine complex (30 mg) was added to the reaction mixture, and the mixture was further stirred at room temperature for one day. The reaction mixture was filtered through Celite and the filtrate was evaporated under reduced pressure. The resulting residue was purified by silica gel column chromatography to give the title compound (6 mg) as colorless oil.
[MS (ESI) m/z 333.4 (M+H)⁺]

### Example 241

### 2-(Pyridin-2-ylmethoxy)-4-(pyrimidin-5-yl)-6,7-dihy dro-5H-cyclopenta[b]pyridin-7-yl acetate hydrochloride

2-(Pyridin-2-ylmethoxy)-4-(pyrimidin-5-yl)-6,7 -dihydro-5H-cyclopenta[b]pyridin-7-yl acetate (29 mg) was prepared as colorless oil according to the procedure described in Example 58 using 4-chloro-2-(pyridin-2-ylmethoxy)-6,7-dihydro-5H-cyc lopenta[b]pyridin-7-yl acetate instead of 4-chloro-2-(pyridin-2-ylmethoxy)-6,7-dihydro-5H-cyc lopenta[b]pyridine and using pyrimidin-5-ylboronic acid instead of (3-fluorophenyl)boronic acid.
[MS (ESI) m/z 363.1 (M+H)⁺]

The resulting compound was dissolved in ethyl acetate (1 mL), followed by the addition of 1N HCl/Et₂O solution (0.08 mL), and the mixture was stirred for 0.5 hour. The resulting precipitate was collected by filtration to give the title compound (10 mg) as a white powder.
[MS (ESI) m/z 363.3 (M+H)⁺]

### Example 242

### 7-Methoxy-2-(pyridin-2-ylmethoxy)-4-(pyrimidin-5-yl )-6,7-dihydro-5H-cyclopenta[b]pyridine hydrochloride

The title compound was prepared as a white powder according to the procedure described in Example 123 using 2-(pyridin-2-ylmethoxy)-4-(pyrimidin-5-yl)-6,7-dihy dro-5H-cyclopenta[b]pyridin-7-ol instead of {5-[2-(pyridin-2-ylmethoxy)-5,6,7,8-tetrahydroquino lin-4-yl]pyridin-3-yl}methanol.
[MS (ESI) m/z 335.3 (M+H)⁺]

### Example 243

### 6,6-Dimethyl-2-(pyridin-2-ylmethoxy)-4-(pyrimidin-5 -yl)-5,6-dihydro-7H-cyclopenta[b]pyridin-7-one

To 2-(pyridin-2-ylmethoxy)-4-(pyrimidin-5-yl)-5,6-dihy dro-7H-cyclopenta[b]pyridin-7-one (100 mg) was added THF (31 mL), and 1M lithium hexamethyldisilazide/THF solution (0.75 mL) was added to the mixture at -78°C, and the mixture was stirred under Ar atmosphere for 1 hour. Then, MeI (0.11 mL) was added to the reaction mixture, and the mixture was further stirred at room temperature for 2 hour. The reaction mixture was added with water and ethyl acetate, and subjected to extraction. The organic layer was washed with saturated brine, dried over anhydrous sodium sulfate, filtered off, and the filtrate was evaporated under reduced pressure. The resulting residue was purified by silica gel column chromatography to give the title compound (50 mg) as pale yellow oil.
[MS (ESI) m/z 347.4 (M+H)⁺]

### Example 244

### 6,6-Dimethyl-2-(pyridin-2-ylmethoxy)-4-(pyrimidin-5 -yl)-6,7-dihydro-5H-cyclopenta[b]pyridine

To 6,6-dimethyl-2-(pyridin-2-ylmethoxy)-4-(pyrimidin-5 -yl)-5,6-dihydro-7H-cyclopenta[b]pyridin-7-one (90 mg) were added ethylene glycol (5 mL), hydrazine monohydrate (30 mg) and potassium hydroxide (29 mg), and then the mixture was stirred under Ar atmosphere at 120°C for 2 hours. Potassium hydroxide (100 mg) was added to the reaction mixture, and the mixture was further stirred at 150°C for 5 hours. The reaction mixture was added with water and ethyl acetate, and subjected to extraction. The organic layer was washed with saturated brine, dried over anhydrous sodium sulfate, filtered off, and the filtrate was evaporated under reduced pressure. The resulting residue was purified by silica gel column chromatography to give the title compound (33 mg) as a white powder.
[MS (ESI) m/z 333.4 (M+H)⁺]

### Example 245

### 3-({[4-(Pyridin-3-yl)-6,7-dihydro-5H-cyclopenta[b]p yridin-2-yl]oxy}methyl)benzonitrile

### [Step 1]

### Production of 4-chloro-6,7-dihydro-5H-cyclopenta[b]pyridin-2-ol

To N,N-dimethylaniline (12.68 g) was added phosphoryl chloride (5.263 mL), and then 6,7-dihydro-5H-cyclopenta[b]pyridine-2,4-diol (see, for example, Helvetica Chimica Acta, 1945, vol. 28, p.1684-1690) (5 g) was added in portions and the mixture was stirred at room temperature overnight. 5N Sodium hydroxide solution was added dropwise under water cooling, and the resulting precipitate was collected by filtration to give the title compound (3.31 g) as a white solid.

### [Step 2]

### Production of 3-{[(4-chloro-6,7-dihydro-5H-cyclopenta[b]pyridin-2 -yl)oxy]methyl}benzonitrile

To 4-chloro-6,7-dihydro-5H-cyclopenta[b]pyridin-2-ol (500 mg), 3-(bromomethyl)benzonitrile (867 mg) and silver carbonate (813 mg) was added CPME (17 mL), and then the mixture was stirred under Ar atmosphere at 100°C for 4 hours. After the reaction mixture was allowed to return to room temperature, diluted with ethyl acetate, filtered through Celite, and the filtrate was evaporated under reduced pressure. The resulting residue was purified by silica gel column chromatography to give the title compound (960 mg) as colorless oil.

### [Step 3]

### Production of 3-({[4-(pyridin-3-yl)-6,7-dihydro-5H-cyclopenta[b]p yridin-2-yl]oxy}methyl)benzonitrile

To 3-{[(4-chloro-6,7-dihydro-5H-cyclopenta[b]pyridin-2 -yl)oxy]methyl}benzonitrile (43 mg), pyridin-3-ylboronic acid (18 mg), Pd(dtbpf)Cl₂ (3 mg) and potassium carbonate (44 mg) was added 1,4-dioxane/water (3/1, 1 mL), and the mixture was stirred at 100°C for 2 hours. After the reaction mixture was allowed to return to room temperature, diluted with ethyl acetate, filtered through Celite, and the filtrate was evaporated under reduced pressure. The resulting residue was purified by silica gel column chromatography to give the title compound (25 mg) as a white powder.
[MS (ESI) m/z 328.6 (M+H)⁺]

### Example 246

### 5-{2-[(3,5-Difluoropyridin-2-yl)methoxy]-6,7-dihydr o-5H-cyclopenta[b]pyridin-4-yl}pyridine-2-carbonitr ile

To 5-(2-chloro-6,7-dihydro-5H-cyclopenta[b]pyridin-4-y l)pyridine-2-carbonitrile (47 mg), (3,5-difluoropyridin-2-yl)methanol (32 mg), Pd₂(dba)₃·CHCl₃ (11 mg), cesium carbonate (180 mg) and t-Bu-X-Phos (19 mg) was added toluene (1 mL), and the mixture was degassed, then stirred under Ar atmosphere at 100°C for 4 hours. After the reaction mixture was allowed to return to room temperature, diluted with ethyl acetate, filtered through Celite, and the filtrate was evaporated under reduced pressure. The resulting residue was dissolved in CH₂Cl₂ (2 mL), and added with Et₃N (0.1 mL) and TFAA (0.05 mL) under ice water cooling, and the mixture was stirred at the same temperature for 2 hours. The reaction mixture was added with ethyl acetate and saturated aqueous sodium hydrogen carbonate solution, and subjected to extraction. The organic layer was washed with saturated brine, dried over anhydrous sodium sulfate, filtered off, and the filtrate was evaporated under reduced pressure. The resulting residue was purified by silica gel column chromatography and by recycling preparative gel permeation chromatography (Japan Analytical Industry, Co. Ltd., LC-9201) to give the title compound (27 mg) as colorless oil.
[MS (ESI) m/z 365.3 (M+H)⁺]

### Example 247

### 4-[({4-[5-(Hydroxymethyl)pyridin-3-yl]-6,7-dihydro-5H-cyclopenta[b]pyridin-2-yl}oxy)methyl]benzonitril e

### [Step 1]

### Production of 4-{[(4-chloro-6,7-dihydro-5H-cyclopenta[b]pyridin-2 -yl)oxy]methyl}benzonitrile

To 4-chloro-6,7-dihydro-5H-cyclopenta[b]pyridin-2-ol (200 mg), 4-(bromomethyl)benzonitrile (300 mg), and silver carbonate (325 mg) was added toluene (10 mL), and the mixture was stirred at 100°C for 2 hours. After the reaction mixture was allowed to return to room temperature, diluted with ethyl acetate, filtered through Celite, and the filtrate was evaporated under reduced pressure. The resulting residue was purified by silica gel column chromatography to give the title compound (343 mg) as a white solid.

### [Step 2]

### Production of 4-[({4-[5-(hydroxymethyl)pyridin-3-yl]-6,7-dihydro-5H-cyclopenta[b]pyridin-2-yl}oxy)methyl]benzonitril e

To 4-{[(4-chloro-6,7-dihydro-5H-cyclopenta[b]pyridin-2 -yl)oxy]methyl}benzonitrile (25 mg), [5-(4,4,5,5-tetramethyl-1,3,2-dioxaborolan-2-yl)pri ding-3-yl]methanol (31 mg), Pd(dtbpf)Cl₂ (3 mg) and potassium carbonate (36 mg) was added 1,4-dioxane/water (4/1, 1 mL), and the mixture was degassed, then stirred under Ar atmosphere at 100°C for 2 hours. The reaction mixture was purified by silica gel column chromatography to give the title compound (19 mg) as a white solid.
[MS (ESI) m/z 358.3 (M+H)⁺]

### Example 248

### (5-{2-[(4-Fluorobenzyl)oxy]-6,7-dihydro-5H-cyclopen ta[b]pyridin-4-yl}pyridin-3-yl)methanol

### [Step 1]

### Production of 4-chloro-2-[(4-fluorobenzyl)oxy]-6,7-dihydro-5H-cyc lopenta[b]pyridine

The title compound was prepared as a white solid according to the procedure described in Example 247 Step 1 using 1-(bromomethyl)-4-fluorobenzene instead of 4-(bromomethyl)benzonitrile.

### [Step 2]

### Production of 5-{2-[(4-fluorobenzyl)oxy]-6,7-dihydro-5H-cyclopent a[b]pyridin-4-yl}pyridin-3-yl)methanol

The title compound was prepared as a white solid according to the procedure described in Example 247 Step 2 using 4-chloro-2-[(4-fluorobenzyl)oxy]-6,7-dihydro-5H-cyc lopenta[b]pyridine instead of 4-{[(4-chloro-6,7-dihydro-5H-cyclopenta[b]pyridin-2 -yl)oxy]methyl}benzonitrile
[MS (ESI) m/z 351.3 (M+H)⁺]

### Example 249

### (5-{2-[(3-Fluorobenzyl)oxy]-6,7-dihydro-5H-cyclopen ta[b]pyridin-4-yl}pyridin-3-yl)methanol

### [Step 1]

### Production of 4-chloro-2-[(3-fluorobenzyl)oxy]-6,7-dihydro-5H-cyc lopenta[b]pyridine

The title compound was prepared as a white solid according to the procedure described in Example 247 Step 1 using 1-(bromomethyl)-3-fluorobenzene instead of 4-(bromomethyl)benzonitrile.

### [Step 2]

### Production of (5-{2-[(3-fluorobenzyl)oxy]-6,7-dihydro-5H-cyclopen ta[b]pyridin-4-yl}pyridin-3-yl)methanol

The title compound was prepared as a white solid according to the procedure described in Example 247 Step 2 using 4-chloro-2-[(3-fluorobenzyl)oxy]-6,7-dihydro-5H-cyc lopenta[b]pyridine instead of 4-{[(4-chloro-6,7-dihydro-5H-cyclopenta[b]pyridin-2 -yl)oxy]methyl}benzonitrile
[MS (ESI) m/z 351.3 (M+H)⁺]

### Example 250

### 6-({[4-(5-Fluoropyridin-3-yl)-6,7-dihydro-5H-cyclop enta[b]pyridin-2-yl]oxy}methyl)pyridine-3-carbonitr ile

### [Step 1]

### Production of 6-{[(4-chloro-6,7-dihydro-5H-cyclopenta[b]pyridin-2 -yl)oxy]methyl}pyridine-3-carbonitrile

The title compound was prepared as a pale yellow powder according to the procedure described in Example 247 Step 1 using 6-(bromomethyl)pyridine-3-carbonitrile instead of 4-(bromomethyl)benzonitrile.

### [Step 2]

### Production of 6-({[4-(5-fluoropyridin-3-yl)-6,7-dihydro-5H-cyclop enta[b]pyridin-2-yl]oxy}methyl)pyridine-3-carbonitr ile

The title compound was prepared as a white powder according to the procedure described in Example 247 Step 2 using 6-{[(4-chloro-6,7-dihydro-5H-cyclopenta[b]pyridin-2 -yl)oxy]methyl}pyridine-3-carbonitrile instead of 4-{[(4-chloro-6,7-dihydro-5H-cyclopenta[b]pyridin-2 -yl)oxy]methyl}benzonitrile, and using 3-fluoro-5-(4,4,5,5-tetramethyl-1,3,2-dioxaborolan-2-yl)pyridine instead of [5-(4,4,5,5-tetramethyl-1,3,2-dioxaborolan-2-yl)pyr idin-3-yl]methanol
[MS (ESI) m/z 347.3 (M+H)⁺]

### Example 251

### 3-({[4-(2-Fluoropyridin-4-yl)-6,7-dihydro-5H-cyclop enta[b]pyridin-2-yl]oxy}methyl)benzonitrile

The title compound was prepared as colorless oil according to the procedure described in Example 245 Step 3 using 2-fluoro-4-(4,4,5,5-tetramethyl-1,3,2-dioxaborolan-2-yl)pyridine instead of pyridin-3-ylboronic acid.
[MS (ESI) m/z 346.3 (M+H)⁺]

### Example 252

### 6-({[4-(2-Fluoropyridin-4-yl)-6,7-dihydro-5H-cyclop enta[b]pyridin-2-yl]oxy}methyl)pyridine-3-carbonitr ile

The title compound was prepared as a white powder according to the procedure described in Example 250 Step 2 using 2-fluoro-4-(4,4,5,5-tetramethyl-1,3,2-dioxaborolan-2-yl)pyridine instead of 3-fluoro-5-(4,4,5,5-tetramethyl-1,3,2-dioxaborolan-2-yl)pyridine.
[MS (ESI) m/z 347.3 (M+H)⁺]

### Example 253

### 3-[({4-[5-(Hydroxymethyl)pyridin-3-yl]-6,7-dihydro-5H-cyclopenta[b]pyridin-2-yl}oxy)methyl]benzonitril e

The title compound was prepared as a white solid according to the procedure described in Example 247 Step 2 using 3-{[(4-chloro-6,7-dihydro-5H-cyclopenta[b]pyridin-2 -yl)oxy]methyl}benzonitrile instead of 4-{[(4-chloro-6,7-dihydro-5H-cyclopenta[b]pyridin-2 -yl)oxy]methyl}benzonitrile.
[MS (ESI) m/z 358.4 (M+H)⁺]

### Example 254

### 6-({[4-(Pyrimidin-5-yl)-6,7-dihydro-5H-cyclopenta[b ]pyridin-2-yl]oxy}methyl)pyridine-3-carbonitrile hydrochloride

6-({[4-(Pyrimidin-5-yl)-6,7-dihydro-5H-cyclope nta[b]pyridin-2-ylloxylmethyl)pyridine-3-carbonitri le was prepared as a pale yellow solid (54 mg) according to the procedure described in Example 11 Step 2 using 6-(hydroxymethyl)pyridine-3-carbonitrile instead of benzylalcohol, and using cesium carbonate instead of NaO-t-Bu. The resulting compound was dissolved in ethyl acetate (10 mL), followed by the addition of 1N HCl/Et₂O solution (0.164 mL) under ice water cooling, and the mixture was stirred at the same temperature for 0.5 hour. The resulting precipitate was collected by filtration to give the title compound (44 mg) as a white powder.
[MS (ESI) m/z 330.4 (M+H)⁺]
Elementary analysis as C₁₉H₁₅N₅O·HCl+0.2H₂O
Calcd. (%) C: 61.77.; H: 4.48.; N: 18.96
Found. (%) C: 61.59.; H: 4.30.; N: 18.70

### Example 255

### 2-[(3,6-Difluoropyridin-2-yl)methoxy]-4-(pyrimidin-5-yl)-6,7-dihydro-5H-cyclopenta[b]pyridine

The title compound was prepared as a pale yellow powder according to the procedure described in Example 11 Step 2 using (3,6-difluoropyridin-2-yl)methanol instead of benzylalcohol, and using Pd₂(dba)₃ instead of Pd₂(dba)₃·CHCl₃, and using cesium carbonate instead of NaO-t-Bu.
[MS (ESI) m/z 341.3 (M+H)⁺]

### Example 256

### 2-[(2-Methylpyridin-4-yl)methoxy]-4-(pyrimidin-5-yl )-6,7-dihydro-5H-cyclopenta[b]pyridine

The title compound was prepared as a white powder according to the procedure described in Example 11 Step 2 using (2-methylpyridin-4-yl)methanol instead of benzylalcohol, and using Pd₂(dba)₃ instead of Pd₂(dba)₃·CHCl₃.
[MS (ESI) m/z 319.5 (M+H)⁺]

### Example 257

### 2-[(4-Fluorobenzyl)oxy]-4-(pyrimidin-5-yl)-6,7-dihy dro-5H-cyclopenta[b]pyridine

The title compound was prepared as a pale yellow solid according to the procedure described in Example 11 Step 2 using (4-fluorophenyl)methanol instead of benzylalcohol, and using Pd₂(dba)₃ instead of Pd₂(dba)₃·CHCl₃, and using potassium phosphate instead of NaO-t-Bu.
[MS (ESI) m/z 322.2 (M+H)⁺]

### Example 258

### 4-({[4-(Pyrimidin-5-yl)-6,7-dihydro-5H-cyclopenta[b ]pyridin-2-yl]oxy}methyl)benzonitrile

The title compound was prepared as a cream solid according to the procedure described in Example 247 Step 2 using pyrimidin-5-ylboronic acid instead of [5-(4,4,5,5-tetramethyl-1,3,2-dioxaborolan-2-yl)pyr idin-3-yl]methanol.
[MS (ESI) m/z 329.4 (M+H)⁺]

### Example 259

### 5-[2-(Pyridin-2-ylmethoxy)-6,7-dihydro-5H-cyclopent a[b]pyridin-4-yl]pyrimidin-2-amine hydrochloride

### [Step 1]

### Production of 4-(2-fluoropyrimidin-5-yl)-2-(pyridin-2-ylmethoxy)-6,7-dihydro-5H-cyclopenta[b]pyridine

To 2-(pyridin-2-ylmethoxy)-6,7-dihydro-5H-cyclopenta[b ]pyridin-4-yl trifluoromethanesulfonate (83 mg), 2-fluoro-5-(trimethylstannanyl)pyrimidine (69 mg) and Pd(PPh₃)₄ (38 mg) was added NMP (2 mL), and the mixture was stirred at 100°C for 4 hours. After the reaction mixture was allowed to return to room temperature, the mixture was extracted with water and ethyl acetate. The organic layer was washed with water and saturated brine in turn, dried over anhydrous sodium sulfate, filtered off, and the filtrate was evaporated under reduced pressure. The resulting residue was purified by silica gel column chromatography to give the title compound (53 mg) as a white solid.

### [Step 2]

### Production of 5-[2-(pyridin-2-ylmethoxy)-6,7-dihydro-5H-cyclopent a[b]pyridin-4-yl]pyrimidin-2-amine hydrochloride

To 4-(2-fluoropyrimidin-5-yl)-2-(pyridin-2-ylmethoxy)-6,7-dihydro-5H-cyclopenta[b]pyridine (20 mg) was added concentrated ammonia/methanol solution (1 mL), and then the mixture was stirred at 50°C for 3 hours. After the reaction mixture was allowed to return to room temperature, the solvent was evaporated under reduced pressure. The resulting residue was purified by silica gel column chromatography and by recycling preparative gel permeation chromatography (Japan Analytical Industry, Co. Ltd., LC-9201) to give 5-[2-(pyridin-2-ylmethoxy)-6,7-dihydro-5H-cyclopent a[b]pyridin-4-yl]pyrimidin-2-amine (8 mg) as a white powder.
[MS (ESI) m/z 320.3 (M+H)⁺]

The resulting compound was dissolved in ethyl acetate, followed by the addition of 1N HCl/Et₂O solution (0.164 mL) under ice water cooling, and the resulting precipitate was collected by filtration to give the title compound (5 mg) as a white powder.
[MS (ESI) m/z 320.3 (M+H)⁺]

### Example 260

### 2-[(4-Fluorobenzyl)oxy]-4-(2-methylpyrimidin-5-yl)-6,7-dihydro-5H-cyclopenta[b]pyridine

To 2-chloro-4-(2-methylpyrimidin-5-yl)-6,7-dihydro-5H-cyclopenta[b]pyridine (50 mg), (4-fluorophenyl) methanol (51 mL), t-Bu-X-Phos (21 mg), Pd₂(dba)₃ (19 mg) and NaO-t-Bu (39 mg) was added toluene (2 mL), and the mixture was degassed, then stirred under Ar atmosphere at 100°C for 6 hours. After the solvent of the reaction mixture was evaporated under reduced pressure and the resulting residue was purified by silica gel column chromatography to give the title compound (21 mg) as a white powder.
[MS (ESI) m/z 336.3 (M+H)⁺]

### Example 261

### 4-({[4-(2-Methylpyrimidin-5-yl)-6,7-dihydro-5H-cycl openta[b]pyridin-2-yl]oxy}methyl)benzonitrile

The title compound was prepared as a white solid according to the procedure described in Example 260 using 4-(hydroxymethyl)benzonitrile instead of (4-fluorophenyl)methanol.
[MS (ESI) m/z 343.3 (M+H)⁺]

### Example 262

### 2-[(2-Methylpyridin-4-yl)methoxy]-4-(2-methylpyrimi din-5-yl)-6,7-dihydro-5H-cyclopenta[b]pyridine

The title compound was prepared as yellow oil according to the procedure described in Example 260 using (2-methylpyridin-4-yl)methanol instead of (4-fluorophenyl)methanol.
[MS (ESI) m/z 333.6 (M+H)⁺]

### Example 263

### 5-{2-[(3-Fluorobenzyl)oxy]-6,7-dihydro-5H-cyclopent a[b]pyridin-4-yl}pyrimidin-2-amine

The title compound was prepared as a white solid according to the procedure described in Example 249 Step 2 using 5-(4,4,5,5-tetramethyl-1,3,2-dioxaborolan-2-yl)pyri midin-2-amine instead of [5-(4,4,5,5-tetramethyl-1,3,2-dioxaborolan-2-yl)pyr idin-3-yl]methanol.
[MS (ESI) m/z 337.3 (M+H)⁺]

### Example 264

### 4-({[4-(2-Aminopyrimidin-5-yl)-6,7-dihydro-5H-cyclo penta[b]pyridin-2-yl]oxy}methyl)benzonitrile

The title compound was prepared as a white solid according to the procedure described in Example 247 Step 2 using 5-(4,4,5,5-tetramethyl-1,3,2-dioxaborolan-2-yl)pyri midin-2-amine instead of [5-(4,4,5,5-tetramethyl-1,3,2-dioxaborolan-2-yl)pyr idin-3-yl]methanol.
[MS (ESI) m/z 344.3 (M+H)⁺]

### Example 265

### 3-({[4-(2-Aminopyrimidin-5-yl)-6,7-dihydro-5H-cyclo penta[b]pyridin-2-yl]oxy}methyl)benzonitrile

The title compound was prepared as a white solid according to the procedure described in Example 245 Step 3 using 5-(4,4,5,5-tetramethyl-1,3,2-dioxaborolan-2-yl)pyri midin-2-amine instead of pyridin-3-ylboronic acid.
[MS (ESI) m/z 344.4 (M+H)⁺]

### Example 266

### 2-[(3-Fluorobenzyl)oxy]-4-(2-methylpyrimidin-5-yl)-6,7-dihydro-5H-cyclopenta[b]pyridine

The title compound was prepared as a white powder according to the procedure described in Example 260 using (3-fluorophenyl)methanol instead of (4-fluorophenyl)methanol.
[MS (ESI) m/z 336.3 (M+H)⁺]

### Example 267

### 2-({[4-(2-Methylpyrimidin-5-yl)-6,7-dihydro-5H-cycl openta[b]pyridin-2-yl]oxy}methyl)-1,3-oxazole-4-car bonitrile

### [Step 1]

### Production of ethyl [(4-chloro-6,7-dihydro-5H-cyclopenta[b]pyridin-2-yl )oxy]acetate

The title compound was prepared as a white solid according to the procedure described in Example 247 Step 1 using bromoethyl acetate instead of 4-(bromomethyl)benzonitrile

### [Step 2]

### Production of [(4-chloro-6,7-dihydro-5H-cyclopenta[b]pyridin-2-yl )oxy]acetic acid

Ethyl [(4-chloro-6,7-dihydro-5H-cyclopenta[b]pyridin-2-yl )oxy]acetate was dissolved in ethanol (4 mL) and water (4 mL), followed by the addition of sodium hydroxide solution, and the mixture was stirred at 50°C for 3 hours. After the reaction mixture was allowed to return to room temperature, the reaction mixture was made weakly acidic with hydrochloric acid, and then the mixture was added with water and ethyl acetate, and subjected to extraction. The organic layer was washed with saturated brine, dried over anhydrous sodium sulfate, filtered off, and the filtrate was evaporated under reduced pressure. The resulting residue was purified by silica gel column chromatography to give the title compound (203 mg) as a white solid.

### [Step 3]

### Production of methyl N-{[(4-chloro-6,7-dihydro-5H-cyclopenta[b]pyridin-2 -yl)oxy]acetyl}serinate

To [(4-chloro-6,7-dihydro-5H-cyclopenta[b]pyridin-2-yl )oxy]acetic acid (200 mg), DL-serine methyl ester hydrochloride (205 mg), HOBt (118 mg) and triethylamine (0.184 mL) was added CH₂Cl₂ (8 mL), followed by the addition of 1-ethyl-3-(3-dimethylaminopropyl)carbodiimide (204 mg), and the mixture was stirred at room temperature overnight. The reaction mixture was added with water and ethyl acetate, and subjected to extraction. The organic layer was washed with saturated brine, dried over anhydrous magnesium sulfate, filtered off, and the filtrate was evaporated under reduced pressure. The resulting residue was purified by silica gel column chromatography to give the title compound (242 mg) as a white solid.

### [Step 4]

### Production of methyl 2-{[(4-chloro-6,7-dihydro-5H-cyclopenta[b]pyridin-2 -yl)oxy]methyl}-4,5-dihydro-1,3-oxazole-4-carboxyla te

To methyl N-{[(4-chloro-6,7-dihydro-5H-cyclopenta[b]pyridin-2 -yl)oxy]acetyl}serinate (240 mg) was added CH₂Cl₂ (6mL), and the reaction mixture was degassed, cooled to -78°C, followed by the addition of N,N-diethylaminosulfur trifluoride (176 mg) under Ar atmosphere, then the mixture was stirred at the same temperature for 1 hour. To the reaction mixture was added potassium carbonate (151 mg), the mixture was gradually allowed to warm up to room temperature, then stirred at room temperature overnight. The resulting mixture was added with water and ethyl acetate, and subjected to extraction. The organic layer was washed with saturated brine, dried over anhydrous magnesium sulfate, filtered off, and the filtrate was evaporated under reduced pressure. The resulting residue was purified by silica gel column chromatography to give the title compound (147 mg) as pale yellow oil.

### [Step 5]

### Production of methyl 2-{[(4-chloro-6,7-dihydro-5H-cyclopenta[b]pyridin-2 -yl)oxy]methyl}-1,3-oxazole-4-carboxylate

To methyl 2-{[(4-chloro-6,7-dihydro-5H-cyclope nta[b]pyridin-2-yl)oxy]methyl}-4,5-dihydro-1,3-oxaz ole-4-carboxylate (140 mg) was added CH₂Cl₂ (4 mL), followed by the addition of 1,8-diazabicyclo[5.4.0] -7-undecene (205 mg) and bromotrichloromethane (116 mg) and the mixture was stirred for 2 hours under i ce water cooling. Aqueous sodium sulfite solution w as added to the reaction mixture, and the reaction m ixture was added with water and ethyl acetate, and s ubjected to extraction. The organic layer was washe d with saturated brine, dried over anhydrous magnesi um sulfate, filtered off, and the filtrate was evapo rated under reduced pressure. The resulting residue was purified by silica gel column chromatography to give the title compound (72 mg) as a white solid.

### [Step 6]

### Production of 2-{[(4-chloro-6,7-dihydro-5H-cyclopenta[b]pyridin-2 -yl)oxy]methyl}-1,3-oxazole-4-carboxylic acid

The title compound was prepared as a white solid according to the procedure described in Example 267 Step 2 using methyl 2-{[(4-chloro-6,7-dihydro-5H-cyclopenta[b]pyridin-2 -yl)oxy]methyl}-1,3-oxazole-4-carboxylate instead of ethyl [(4-chloro-6,7-dihydro-5H-cyclopenta[b]pyridin-2-yl )oxy]acetate.

### [Step 7]

### Production of 2-{[(4-chloro-6,7-dihydro-5H-cyclopenta[b]pyridin-2 -yl)oxy]methyl}-1,3-oxazole-4-carboxamide

The title compound was prepared as a white solid according to the procedure described in Example 267 Step 3 using 2-{[(4-chloro-6,7-dihydro-5H-cyclopenta[b]pyridin-2 -yl)oxy]methyl}-1,3-oxazole-4-carboxylic acid instead of [(4-chloro-6,7-dihydro-5H-cyclopenta[b]pyridin-2-yl )oxy]acetic acid, and using ammonium chloride instead of DL-serine methyl ester hydrochloride.

### [Step 8]

### Production of 2-{[(4-chloro-6,7-dihydro-5H-cyclopenta[b]pyridin-2 -yl)oxy]methyl}-1,3-oxazole-4-carbonitrile

2-{[(4-Chloro-6,7-dihydro-5H-cyclopenta[b]pyridin-2 -yl)oxy]methyl}-1,3-oxazole-4-carboxamide (35 mg) was added CH₂Cl₂ (4 mL), followed by the addition of triethylamine (0.066 mL) and TFAA (0.022 mL) under ice water cooling, and the mixture was stirred at the same temperature for 1 hour. The reaction mixture was added with water and ethyl acetate, and subjected to extraction. The organic layer was washed with saturated brine, dried over anhydrous magnesium sulfate, filtered off, and the filtrate was evaporated under reduced pressure. The resulting residue was purified by silica gel column chromatography to give the title compound (29 mg) as a white solid.

### [Step 9]

### Production of 2-({[4-(2-methylpyrimidin-5-yl)-6,7-dihydro-5H-cycl openta[b]pyridin-2-yl]oxy}methyl)-1,3-oxazole-4-car bonitrile

The title compound was prepared as a white solid according to the procedure described in Example 247 Step 2 using 2-{[(4-chloro-6,7-dihydro-5H-cyclopenta[b]pyridin-2 -yl)oxy]methyl}-1,3-oxazole-4-carbonitrile instead of 4-{[(4-chloro-6,7-dihydro-5H-cyclopenta[b]pyridin-2 -yl)oxy]methyl}benzonitrile, and using 2-methyl-5-(4,4,5,5-tetramethyl-1,3,2-dioxaborolan-2-yl)pyrimidine instead of [5-(4,4,5,5-tetramethyl-1,3,2-dioxaborolan-2-yl)pyr idin-3-yl]methanol
[MS (ESI) m/z 334.4 (M+H)⁺]

### Example 268

### {6-[2-(Pyridin-2-ylmethoxy)-6,7-dihydro-5H-cyclopen ta[b]pyridin-4-yl]pyrazin-2-yl}methanol hydrochloride

To 2-(pyridin-2-ylmethoxy)-4-(4,4,5,5-tetramethyl-1,3, 2-dioxaborolan-2-yl)-6,7-dihydro-5H-cyclopenta[b]py ridine (100 mg), (6-chloropyrazin-2-yl)methanol (49 mg), Pd₂(dba)₃ (13 mg) and S-Phos (23 mg) was added 1,4-dioxane/water (3/1, 4 mL), and the mixture was degassed, then stirred under Ar atmosphere at 100°C for 2 hours. After the reaction mixture was allowed to return to room temperature, the mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography and by recycling preparative gel permeation chromatography (Japan Analytical Industry, Co. Ltd., LC-9201) to give {6-[2-(pyridin-2-ylmethoxy)-6,7-dihydro-5H-cyclopen ta[b]pyridin-4-yl]pyrazin-2-yl}methanol (63 mg) as a white powder. The resulting compound was dissolved in ethyl acetate (5 mL), followed by the addition of 1N HCl/Et₂O solution (0.21 mL) under ice water cooling, and the mixture was stirred at the same temperature for 0.5 hours. The resulting precipitate was collected by filtration to give the title compound (53 mg) as a white powder.
[MS (ESI) m/z 335.3 (M+H)⁺]
Elementary analysis as C₁₉H₁₈N₄O₂·HCl+0.1H₂O
Calcd. (%) C: 61.24.; H: 5.19.; N: 15.04
Found. (%) C: 61.24.; H: 4.98.; N: 15.07

### Example 269

### (6-{2-[(5-Fluoropyridin-2-yl)methoxy]-6,7-dihydro-5 H-cyclopenta[b]pyridin-4-yl}pyrazin-2-yl)methanol

### [Step 1]

### Production of 4-chloro-2-[(5-fluoropyridin-2-yl)methoxy]-6,7-dihy dro-5H-cyclopenta[b]pyridine

The title compound was prepared as a white powder according to the procedure described in Example 44 Step2 using (5-fluoropyridin-2-yl)methanol instead of (3,5-difluoropyridin-2-yl)methanol.

### [Step 2]

### Production of 2-[(5-fluoropyridin-2-yl)methoxy]-6,7-dihydro-5H-cy clopenta[b]pyridin-4-ol

The title compound was prepared as a pale brown powder according to the procedure described in Example 44 Step3 using 4-chloro-2-[(5-fluoropyridin-2-yl)methoxy]-6,7-dihy dro-5H-cyclopenta[b]pyridine instead of 4-chloro-2-[(3,5-difluoropyridin-2-yl)methoxy]-6,7-dihydro-5H-cyclopenta[b]pyridine.

### [Step 3]

### Production of 2-[(5-fluoropyridin-2-yl)methoxy]-6,7-dihydro-5H-cy clopenta[b]pyridin-4-yl trifluoromethanesulfonate

The title compound was prepared according to the procedure described in Example 44 Step 4 using 2-[(5-fluoropyridin-2-yl)methoxy]-6,7-dihydro-5H-cy clopenta[b]pyridin-4-ol instead of 2-[(3,5-difluoropyridin-2-yl)methoxy]-6,7-dihydro-5 H-cyclopenta[b]pyridin-4-ol.

### [Step 4]

### Production of 2-[(5-fluoropyridin-2-yl)methoxy]-4-(4,4,5,5-tetram ethyl-1,3,2-dioxaborolan-2-yl)-6,7-dihydro-5H-cyclo penta[b]pyridine

The title compound was prepared according to the procedure described in Example 5 Step 1 using 2-[(5-fluoropyridin-2-yl)methoxy]-6,7-dihydro-5H-cy clopenta[b]pyridin-4-yl trifluoromethanesulfonate instead of 2-(pyridin-2-ylmethoxy)-6,7-dihydro-5H-cyclopenta[b ]pyridin-4-yl trifluoromethanesulfonate.

### [Step 5]

### Production of (6-{2-[(5-fluoropyridin-2-yl)methoxy]-6,7-dihydro-5 H-cyclopenta[b]pyridin-4-yl}pyrazin-2-yl)methanol

To 2-[(5-fluoropyridin-2-yl)methoxy]-4-(4,4,5,5-tetram ethyl-1,3,2-dioxaborolan-2-yl)-6,7-dihydro-5H-cyclo penta[b]pyridine (37 mg), (6-chloropyrazin-2-yl)methanol (16 mg), Pd(dppf)Cl₂·CH₂Cl₂ (8 mg) and potassium carbonate (42 mg) was added 1,4-dioxane/water (3/1, 1 mL), and the reaction mixture was degassed, then stirred under Ar atmosphere at 80°C for 3 hours. After the reaction mixture was allowed to return to room temperature, the mixture was added with water and ethyl acetate, and subjected to extraction. The organic layer was dried over anhydrous sodium sulfate, filtered off, and the filtrate was evaporated under reduced pressure. The resulting residue was purified by silica gel column chromatography to give the title compound (22 mg) as a white solid.
[MS (ESI) m/z 353.1 (M+H)⁺]

### Example 270

### 3-[({4-[6-(Hydroxymethyl)pyrazin-2-yl]-6,7-dihydro-5H-cyclopenta[b]pyridin-2-yl}oxy)methyl]benzonitril e

### [Step 1]

### Production of [6-(2-chloro-6,7-dihydro-5H-cyclopenta[b]pyridin-4-yl)pyrazin-2-yl]methanol

The title compound was prepared as a white solid according to the procedure described in Example 9 Step 5 using (6-chloropyrazin-2-yl)methanol instead of 5-bromopyridine-2-carbonitrile.

### [Step 2]

### Production of 4-[6-({[tert-butyl(dimethyl)silyl]oxy}methyl)pyrazi n-2-yl]-2-chloro-6,7-dihydro-5H-cyclopenta[b]pyridi ne

The title compound was prepared as a white powder according to the procedure described in Example 161 Step 1 using [6-(2-chloro-6,7-dihydro-5H-cyclopenta[b]pyridin-4-yl)pyrazin-2-yl]methanol instead of (6-bromopyridin-2-yl)methanol.

### [Step 3]

### Production of 3-[({4-[6-({[tert-butyl(dimethyl)silyl]oxy}methyl)p yrazin-2-yl]-6,7-dihydro-5H-cyclopenta[b]pyridin-2-yl}oxy)methyl]benzonitrile

To 4-[6-({[tert-butyl(dimethyl)silyl]oxy}methyl)pyrazi n-2-yl]-2-chloro-6,7-dihydro-5H-cyclopenta[b]pyridi ne (30 mg), 3-(hydroxymethyl)benzonitrile (16 mg), t-Bu-X-Phos (7 mg), Pd₂(dba)₃ (4 mg) and NaO-t-Bu (15 mg) was added 1,4-dioxane (1 mL), and the mixture was degassed, then stirred under Ar atmosphere at 100°C for 2 hours. The reaction mixture was purified by silica gel column chromatography to give the title compound (15 mg) as colorless oil.

### [Step 4]

### Production of 3-[({4-[6-(hydroxymethyl)pyrazin-2-yl]-6,7-dihydro-5H-cyclopenta[b]pyridin-2-yl}oxy)methyl]benzonitril e

3-[({4-[6-({[tert-Butyl(dimethyl)silyl]oxy}met hyl)pyrazin-2-yl]-6,7-dihydro-5H-cyclopenta[b]pyrid in-2-yl}oxy)methyl]benzonitrile (15mg) was dissolved in THF (0.5 mL), followed by the addition of 1.0M TBAF/THF solution (0.05 mL) and the mixture was stirred at room temperature for 1 hour. The reaction mixture was purified by silica gel column chromatography to give the title compound (10 mg) as a white solid.
[MS (ESI) m/z 359.3 (M+H)⁺]

### Example 271

### 4-(Pyridazin-3-yl)-2-(pyridin-2-ylmethoxy)-6,7-dihy dro-5H-cyclopenta[b]pyridine

The title compound was prepared as a white powder according to the procedure described in Example 5 Step2 using pyridazin-3-yl trifluoromethanesulfonate (see, for example, Tetrahedron, 2009, 65, 8969-8980) instead of 5-bromopyridin-3-ol.
[MS (ESI) m/z 305.3 (M+H)⁺]

### Example 272

### 2-[(3-Fluoropyridin-2-yl)methoxy]-4-(pyridazin-3-yl )-6,7-dihydro-5H-cyclopenta[b]pyridine

### [Step 1]

### Production of 2-[(3-fluoropyridin-2-yl)methoxy]-4-(4,4,5,5-tetram ethyl-1,3,2-dioxaborolan-2-yl)-6,7-dihydro-5H-cyclo penta[b]pyridine

The title compound was prepared as pale yellow oil according to the procedure described in Example 5 Step 1 using 4-chloro-2-[(3-fluoropyridin-2-yl)methoxy]-6,7-dihy dro-5H-cyclopenta[b]pyridine instead of 2-(pyridin-2-ylmethoxy)-6,7-dihydro-5H-cyclopenta[b ]pyridin-4-yl trifluoromethanesulfonate, and using PdCl₂(PCy₃)₂ instead of Pd(dppf)Cl₂·CH₂Cl₂.

### [Step 2]

### Production of 2-[(3-fluoropyridin-2-yl)methoxy]-4-(pyridazin-3-yl )-6,7-dihydro-5H-cyclopenta[b]pyridine

The title compound was prepared as a white powder according to the procedure described in Example 271 using 2-[(3-fluoropyridin-2-yl)methoxy]-4-(4,4,5,5-tetram ethyl-1,3,2-dioxaborolan-2-yl)-6,7-dihydro-5H-cyclo penta[b]pyridine instead of 2-(pyridin-2-ylmethoxy)-4-(4,4,5,5-tetramethyl-1,3, 2-dioxaborolan-2-yl)-6,7-dihydro-5H-cyclopenta[b]py ridine
[MS (ESI) m/z 323.3 (M+H)⁺]

### Example 273

### 4-({[4-(Pyridazin-3-yl)-6,7-dihydro-5H-cyclopenta[b ]pyridin-2-yl]oxy}methyl)benzonitrile

### [Step 1]

### Production of 2-chloro-4-(pyridazin-3-yl)-6,7-dihydro-5H-cyclopen ta[b]pyridine

The title compound was prepared as a white solid according to the procedure described in Example 9 Step 5 using pyridazin-3-yl trifluoromethanesulfonate (see, for example, Tetrahedron, 2009, vol. 65, p. 8969-8980) instead of 5-bromopyridine-2-carbonitrile, and using THF/water (3/1) instead of 1,4-dioxane/water (3/1)

### [Step 2]

### Production of 4-({[4-(pyridazin-3-yl)-6,7-dihydro-5H-cyclopenta[b ]pyridin-2-yl]oxy}methyl)benzonitrile

The title compound was prepared as a white powder according to the procedure described in Example 261 using 2-chloro-4-(pyridazin-3-yl)-6,7-dihydro-5H-cyclopen ta[b]pyridine instead of 2-chloro-4-(2-methylpyrimidin-5-yl)-6,7-dihydro-5H-cyclopenta[b]pyridine, and using Pd₂(dba)₃·CHCl₃ instead of Pd₂(dba)₃.
[MS (ESI) m/z 329.6 (M+H)⁺]

### Example 274

### 6-({[4-(1-Methyl-1H-pyrazol-5-yl)-6,7-dihydro-5H-cy clopenta[b]pyridin-2-yl]oxy}methyl)pyridine-3-carbo nitrile

The title compound was prepared as a white powder according to the procedure described in Example 250 Step 2 using 1-methyl-5-(4,4,5,5-tetramethyl-1,3,2-dioxaborolan-2-yl)-1H-pyrazole instead of 3-fluoro-5-(4,4,5,5-tetramethyl-1,3,2-dioxaborolan-2-yl)pyridine.
[MS (ESI) m/z 332.3 (M+H)⁺]

### Example 275

### 6-({[4-(Pyridazin-3-yl)-6,7-dihydro-5H-cyclopenta[b ]pyridin-2-yl]oxy}methyl)pyridine-3-carbonitrile

The title compound was prepared as a white powder according to the procedure described in Example 273 Step 2 using 6-(hydroxymethyl)pyridine-3-carbonitrile instead of 4-(hydroxymethyl)benzonitrile.
[MS (ESI) m/z 330.3 (M+H)⁺]

### Example 276

### 6-[({4-[5-(Hydroxymethyl)pyridin-3-yl]-5,6,7,8-tetr ahydroquinolin-2-yl}oxy)methyl]pyridine-3-carbonitr ile

### [Step 1]

### Production of 6-{[(4-chloro-5,6,7,8-tetrahydroquinolin-2-yl)oxy]m ethyl}pyridine-3-carbonitrile

The title compound was prepared as a white solid according to the procedure described in Example 250 Step 1 using 4-chloro-5,6,7,8-tetrahydroquinolin-2-ol instead of 4-chloro-6,7-dihydro-5H-cyclopenta[b]pyridin-2-ol.

### [Step 2]

### Production of 6-[({4-[5-(hydroxymethyl)pyridin-3-yl]-5,6,7,8-tetr ahydroquinolin-2-yl}oxy)methyl]pyridine-3-carbonitr ile

The title compound was prepared as a white solid according to the procedure described in Example 247 Step 2 using 6-{[(4-chloro-5,6,7,8-tetrahydroquinolin-2-yl)oxy]m ethyl}pyridine-3-carbonitrile instead of 4-{[(4-chloro-6,7-dihydro-5H-cyclopenta[b]pyridin-2 -yl)oxy]methyl}benzonitrile
[MS (ESI) m/z 373.5 (M+H)⁺]

### Example 277

### 2-[(3,5-Difluoropyridin-2-yl)methoxy]-4-(pyrimidin-5-yl)-5,6,7,8-tetrahydroquinoline

The title compound was prepared as a pale yellow powder according to the procedure described in Example 150 using (3,5-difluoropyridin-2-yl)methanol instead of 6-(hydroxymethyl)pyridine-3-carbonitrile.
[MS (ESI) m/z 355.4 (M+H)⁺]

### Example 278

### 4-({[4-(Pyrimidin-5-yl)-5,6,7,8-tetrahydroquinolin-2-yl]oxy}methyl)benzonitrile

To 2-chloro-4-(pyrimidin-5-yl)-5,6,7,8-tetrahydroquino line (50 mg), 4-(hydroxymethyl)benzonitrile (54 mL), Pd₂(dba)₃ (19 mg), t-Bu-X-Phos (21 mg) and NaO-t-Bu (39 mg) was added toluene (2 mL). The mixture was degassed, then stirred under Ar atmosphere at 100°C for 3 hours. After the reaction mixture was allowed to return to room temperature, diluted with ethyl acetate, filtered off, and the filtrate was evaporated under reduced pressure. The resulting residue was purified by silica gel column chromatography and by recycling preparative gel permeation chromatography (Japan Analytical Industry, Co. Ltd., LC-9201) to give the title compound (16 mg) as a cream powder.
[MS (ESI) m/z 343.3 (M+H)⁺]

### Example 279

### 3-({[4-(Pyrimidin-5-yl)-5,6,7,8-tetrahydroquinolin-2-yl]oxy}methyl)benzonitrile

The title compound was prepared as a white powder according to the procedure described in Example 278 using 3-(hydroxymethyl)benzonitrile instead of 4-(hydroxymethyl)benzonitrile.
[MS (ESI) m/z 343.3 (M+H)⁺]

### Example 280

### 2-[(4-Fluorobenzyl)oxy]-4-(pyrimidin-5-yl)-5,6,7,8-tetrahydroquinoline

The title compound was prepared as a cream solid according to the procedure described in Example 278 using (4-fluorophenyl)methanol instead of 4-(hydroxymethyl)benzonitrile.
[MS (ESI) m/z 336.3 (M+H)⁺]

### Example 281

### 2-[(3-Fluorobenzyl)oxy]-4-(pyrimidin-5-yl)-5,6,7,8-tetrahydroquinoline

The title compound was prepared as a white solid according to the procedure described in Example 278 using (3-fluorophenyl)methanol instead of 4-(hydroxymethyl)benzonitrile.
[MS (ESI) m/z 336.3 (M+H)⁺]

### Example 282

### 4-({[4-(Pyrimidin-5-yl)-5,6,7,8-tetrahydroquinolin-2-yl]oxy}methyl)pyridine-2-carbonitrile

### [Step 1]

### Production of 4-{[(4-chloro-5,6,7,8-tetrahydroquinolin-2-yl)oxy]m ethyl}pyridine-2-carbonitrile

The title compound was prepared as a pale purple solid according to the procedure described in Example 276 Step 1 using 4-(bromomethyl)pyridine-2-carbonitrile instead of 6-(bromomethyl)pyridine-3-carbonitrile.

### [Step 2]

### Production of 4-({[4-(pyrimidin-5-yl)-5,6,7,8-tetrahydroquinolin-2-yl]oxy}methyl)pyridine-2-carbonitrile

The title compound was prepared as a yellow powder according to the procedure described in Example 258 using 4-{[(4-chloro-5,6,7,8-tetrahydroquinolin-2-yl)oxy]m ethyl}pyridine-2-carbonitrile instead of 4-{[(4-chloro-6,7-dihydro-5H-cyclopenta[b]pyridin-2 -yl)oxy]methyl}benzonitrile.
[MS (ESI) m/z 344.4 (M+H)⁺]

### Example 283

### 5-{2-[(6-Fluoropyridin-2-yl)methoxy]-5,6,7,8-tetrah ydroquinolin-4-yl}pyrimidin-2-amine hydrochloride

### [Step 1]

### Production of 2-[(6-fluoropyridin-2-yl)methoxy]-4-[(4-methoxybenz yl)oxy]-5,6,7,8-tetrahydroquinoline

The title compound was prepared as a yellow solid according to the procedure described in Example 278 using 2-chloro-4-[(4-methoxybenzyl)oxy]-5,6,7,8-tetrahydr oquinoline instead of 2-chloro-4-(pyrimidin-5-yl)-5,6,7,8-tetrahydroquino line and using (6-fluoropyridin-2-yl)methanol instead of 4-(hydroxymethyl)benzonitrile.

### [Step 2]

### Production of 2-[(6-fluoropyridin-2-yl)methoxy]-5,6,7,8-tetrahydr oquinolin-4-ol

The title compound was prepared as a white powder according to the procedure described in Example 191 Step 3 using 2-[(6-fluoropyridin-2-yl)methoxy]-4-[(4-methoxybenz yl)oxy]-5,6,7,8-tetrahydroquinoline instead of 2-[(3-fluoropyridin-2-yl)methoxy]-4-[(4-methoxybenz yl)oxy]-5,6,7,8-tetrahydroquinoline.

### [Step 3]

### Production of 2-[(6-fluoropyridin-2-yl)methoxy]-5,6,7,8-tetrahydr oquinolin-4-yl trifluoromethanesulfonate

The title compound was prepared as colorless oil according to the procedure described in Example 191 Step 4 using 2-[(6-fluoropyridin-2-yl)methoxy]-5,6,7,8-tetrahydr oquinolin-4-ol instead of 2-[(3-fluoropyridin-2-yl)methoxy]-5,6,7,8-tetrahydr oquinolin-4-ol.

### [Step 4]

### Production of 5-{2-[(6-fluoropyridin-2-yl)methoxy]-5,6,7,8-tetrah ydroquinolin-4-yl}pyrimidin-2-amine hydrochloride

The title compound was prepared as a white powder according to the procedure described in Example 191 Step 5 using 2-[(6-fluoropyridin-2-yl)methoxy]-5,6,7,8-tetrahydr oquinolin-4-yl trifluoromethanesulfonate instead of 2-[(3-fluoropyridin-2-yl)methoxy]-5,6,7,8-tetrahydr oquinolin-4-yl trifluoromethanesulfonate.
[MS (ESI) m/z 352.3 (M+H)⁺]
Elementary analysis as C₁₉H₁₈FN₅O·HCl+H₂O
Calcd. (%) C: 56.23.; H: 5.22.; N: 17.23
Found. (%) C: 55.90.; H: 4.76.; N: 17.11

### Example 284

### 3-({[4-(2-Methylpyrimidin-5-yl)-5,6,7,8-tetrahydroq uinolin-2-yl]oxy}methyl)benzonitrile

To 2-chloro-4-(2-methylpyrimidin-5-yl)-5,6,7,8-tetrahy droquinoline (50 mg), 3-(hydroxymethyl)benzonitrile (51 mL), Pd₂(dba)₃ (18 mg), t-Bu-X-Phos (20 mg), and NaO-t-Bu (37 mg) was added toluene (2 mL), and the mixture was degassed, then stirred under Ar atmosphere at 100°C for 3 hours. After the solvent of the reaction mixture was evaporated under reduced pressure, the resulting residue was purified by silica gel column chromatography to give the title compound (57 mg) as yellow oil.
[MS (ESI) m/z 357.3 (M+H)⁺]

### Example 285

### 4-({[4-(2-Methylpyrimidin-5-yl)-5,6,7,8-tetrahydroq uinolin-2-yl]oxy}methyl)benzonitrile

The title compound was prepared as a pale yellow solid according to the procedure described in Example 284 using 4-(hydroxymethyl)benzonitrile instead of 3-(hydroxymethyl)benzonitrile.
[MS (ESI) m/z 357.3 (M+H)⁺]

### Example 286

### 2-[(2-Methylpyridin-4-yl)methoxy]-4-(2-methylpyrimi din-5-yl)-5,6,7,8-tetrahydroquinoline

The title compound was prepared as yellow oil according to the procedure described in Example 284 using (2-methylpyridin-4-yl)methanol instead of 3-(hydroxymethyl)benzonitrile.
[MS (ESI) m/z 347.6 (M+H)⁺]

### Example 287

### 4-({[4-(2-Methylpyrimidin-5-yl)-5,6,7,8-tetrahydroq uinolin-2-yl]oxy}methyl)pyridine-2-carbonitrile

The title compound was prepared as a pale brown powder according to the procedure described in Example 282 Step 2 using 2-methyl-5-(4,4,5,5-tetramethyl-1,3,2-dioxaborolan-2-yl)pyrimidine instead of pyrimidin-5-ylboronic acid.
[MS (ESI) m/z 358.4 (M+H)⁺]

### Example 288

### 6-[({4-[6-(Hydroxymethyl)pyrazin-2-yl]-5,6,7,8-tetr ahydroquinolin-2-yl}oxy)methyl]pyridine-3-carbonitr ile

### [Step 1]

### Production of 6-({[4-(4,4,5,5-tetramethyl-1,3,2-dioxaborolan-2-yl )-5,6,7,8-tetrahydroquinolin-2-yl]oxy}methyl)pyridi ne-3-carbonitrile

The title compound was prepared according to the procedure described in Example 5 Step 1 using 6-{[(4-chloro-5,6,7,8-tetrahydroquinolin-2-yl)oxy]m ethyl}pyridine-3-carbonitrile instead of 2-(pyridin-2-ylmethoxy)-6,7-dihydro-5H-cyclopenta[b ]pyridin-4-yl trifluoromethanesulfonate, and using PdCl₂(PCy₃)₂ instead of Pd(dppf)Cl₂·CH₂Cl₂.

### [Step 2]

### Production of 6-[({4-[6-(hydroxymethyl)pyrazin-2-yl]-5,6,7,8-tetr ahydroquinolin-2-yl}oxy)methyl]pyridine-3-carbonitr ile

The title compound was prepared as a white powder according to the procedure described in Example 269 Step 5 using 6-({[4-(4,4,5,5-tetramethyl-1,3,2-dioxaborolan-2-yl )-5,6,7,8-tetrahydroquinolin-2-yl]oxy}methyl)pyridi ne-3-carbonitrile instead of 2-[(5-fluoropyridin-2-yl)methoxy]-4-(4,4,5,5-tetram ethyl-1,3,2-dioxaborolan-2-yl)-6,7-dihydro-5H-cyclo penta[b]pyridine and using Pd(dtbpf)Cl₂ instead of Pd(dppf)Cl₂·CH₂Cl₂.
[MS (ESI) m/z 374.4 (M+H)⁺]

### Example 289

### 4-(Pyridazin-3-yl)-2-(pyridin-2-ylmethoxy)-5,6,7,8-tetrahydroquinoline

The title compound was prepared as colorless oil according to the procedure described in Example 108 Step 2 using pyridazin-3-yl trifluoromethanesulfonate (see, for example, Tetrahedron, 2009, vol. 65, p. 8969-8980) instead of 3-bromo-2-fluoro-5-methylpyridine and using THF/water (1/1) instead of 1,4-dioxane/water (3/1).
[MS (ESI) m/z 319.3 (M+H)⁺]

### Test Example 1: Experiment for receptor function of mGluR5

Stable expression cells in which the human mGluR5 gene had been introduced into a Chinese hamster ovary cell (CHO-K1) were used for evaluation. The cells were suspended in a growth medium (MEM-α (D8042, available from Sigma) containing 10% inactivated FBS (Cat. No. 171012, available from CCB) and 2 mM glutamax (Cat. No. 35050, available from GIBCO)) and seeded on a black clear-bottom 96-well plate at 100,000 cells per well. One day after seeding, the medium was replaced by a medium (Hanks' balanced salt solution (HBSS buffer solution) (20 mM HEPES, 2.5 mM probencide (165-15472, available from Wako Pure Chemical Industries, Ltd.), and 10 x HBSS (14065-056, available from Gibco) diluted 10 times, pH 7.4) containing a calcium-sensitive dye Fluo-4 AM at a final concentration of 1 µM (F312, available from Dojindo Laboratories) and Pluronic F-127 at a final concentration of 0.0145% (P-2443, available from Sigma), followed by incubation at 37°C for one hour, thereby loading Fluo-4 AM into the cells.

Subsequently, the cells were washed twice with an HBSS buffer solution, to which was subsequently added the same buffer solution. Twenty minutes thereafter, the plate was transferred into a fluorescence screening system (FLIPR TETRA, available from Molecular Devices, LLC) and measured for a Ca²⁺ - dependent fluorescence change in the cells.

First of all, a fluorescence baseline was measured, and thereafter, fluorescence measurement was performed for 2 minutes while adding a test substance, an excessive amount of 6-methyl-2-(phenylethynyl)pyridine (hereinafter referred to as "MPEP") (final concentration: 10 µM), or the medium. Thereafter, fluorescence measurement was carried out for one minute while adding L-glutamic acid (final concentration: 10 µM).

While taking a fluorescence intensity caused by L-glutamic acid as 100% and a fluorescence intensity caused by L-glutamic acid in the presence of MPEP as 0%, respectively, function inhibitory activity against human mGluR5 was evaluated from the fluorescence intensity caused by L-glutamic acid in the presence of the test substance at each concentration, and an IC50 value was estimated according to a logistic equation (SAS System). The obtained results are shown in Tables 1 and 2.

**[Table 1]**

| Test substance (Example No.) | Experiment for receptor function of human mGluR5 IC50 (nM) | Test substance (Example No.) | Experiment for receptor function of human mGluR5 IC50 (nM) | Test substance (Example No.) | Experiment for receptor function of human mGluR5 IC50 (nM) |
|---|---|---|---|---|---|
| 1 | 144 | 86 | 15.1 | 165 | 37.9 |
| 2 | 5.26 | 89 | 41.4 | 167 | 20.4 |
| 3 | 12.0 | 90 | 152 | 168 | 153 |
| 4 | 147 | 91 | 16.9 | 169 | 13.9 |
| 18 | 105 | 92 | 40.1 | 173 | 161 |
| 23 | 39 | 94 | 75.0 | 176 | 70.8 |
| 24 | 36 | 95 | 42 | 182 | 123 |
| 25 | 60.6 | 96 | 56.3 | 183 | 148 |
| 26 | 73.6 | 101 | 23.4 | 185 | 26 |
| 28 | 104 | 102 | 51.2 | 186 | 26 |
| 32 | 31 | 104 | 51.7 | 187 | 36 |
| 34 | 15 | 107 | 81.2 | 191 | 36 |
| 35 | 58 | 113 | 68.8 | 194 | 11.4 |
| 36 | 71 | 115 | 98.7 | 195 | 24.3 |
| 37 | 79 | 116 | 133 | 196 | 170 |
| 38 | 45 | 120 | 206 | 197 | 93.7 |
| 39 | 122 | 122 | 72.7 | 198 | 56.3 |
| 40 | 78 | 123 | 58.8 | 199 | 24.5 |
| 41 | 30 | 124 | 24.8 | 200 | 144 |
| 44 | 166 | 125 | 45.6 | 201 | 36.2 |
| 46 | 207 | 126 | 99.8 | 202 | 43 |
| 48 | 72 | 138 | 95 | 204 | 230 |
| 49 | 55 | 143 | 38.0 | 205 | 93 |
| 50 | 176 | 144 | 19.4 | 206 | 36.5 |
| 53 | 11.4 | 145 | 74 | 207 | 346 |
| 54 | 20 | 146 | 176 | 208 | 125 |
| 55 | 348 | 147 | 19.7 | 214 | 131 |
| 56 | 495 | 148 | 22 | 223 | 47.3 |
| 58 | 55.6 | 149 | 19.3 | 224 | 25.6 |
| 63 | 59.9 | 150 | 63 | 225 | 102 |
| 64 | 297 | 151 | 439 | 226 | 52.1 |
| 66 | 108 | 152 | 44.8 | 227 | 36.2 |
| 68 | 92 | 153 | 316 | 231 | 304 |
| 69 | 94 | 154 | 280 | 232 | 28.9 |
| 73 | 16.2 | 157 | 333 | 233 | 10.9 |
| 82 | 153 | 158 | 253 | 234 | 41.4 |
| 83 | 16.2 | 160 | 152 | 237 | 34.5 |
| 84 | 112 | 161 | 359 | 238 | 23 |
| 85 | 178 | 164 | 27.9 | 240 | 24 |

**[Table 2]**

| Test substance (Example No.) | Experiment for receptor function of human mGluR5 IC50 (nM) | Test substance (Example No.) | Experiment for receptor function of human mGluR5 IC50 (nM) |
|---|---|---|---|
| 245 | 39 | 266 | 71 |
| 246 | 42 | 267 | 55 |
| 247 | 52 | 269 | 67 |
| 248 | 95 | 270 | 186 |
| 249 | 53 | 271 | 92.0 |
| 250 | 10.6 | 272 | 117 |
| 251 | 94.3 | 273 | 62 |
| 252 | 13.5 | 274 | 61 |
| 253 | 80 | 275 | 82.0 |
| 256 | 62.8 | 276 | 28.0 |
| 257 | 63.5 | 278 | 87 |
| 258 | 10.1 | 279 | 108 |
| 259 | 27.3 | 280 | 92 |
| 260 | 79 | 281 | 94 |
| 261 | 64 | 282 | 66 |
| 262 | 82 | 286 | 95 |
| 263 | 111 | 287 | 146 |
| 264 | 126 | 288 | 119 |
| 265 | 46.8 | | |

As described in Test Example 1, the compound of the present invention or a pharmaceutically acceptable salt thereof exhibits high mGluR5 inhibitory activity.

### Test Example 2: Action against formalin-induced pain behavior in mice

4 to 6-week-old male Slc:ICR mice (available from Japan SLC, Inc.) were used. A medium or a test substance (1 mg/kg or 3 mg/kg) was intravenously administered to each mouse, and immediately thereafter, 20 µL of 1% or 5% formalin was subcutaneously administered to a right hind paw of the mouse. Thereafter, the mice were quickly put into an observation cage and measured for the total time (sec) of pain-related behavior (licking or biting behavior against the right hindlimb) during 6 minutes, and an average value of the pain-related behavior time of each group was calculated.

The intensity of an analgesic action of the test substance was evaluated in terms of percent inhibition (%) of pain-related behavior time which is calculated as {(A - B)/A x 100}, wherein A is an average value of the pain-related behavior time of a medium-administered group, and B is an average value of the pain-related behavior time of a test substance-administered group. The obtained results are shown in Table 3.

**[Table 3]**

| Test substance (Example No.) | Concentration of formalin | Percent inhibition of pain-related behavior time (%) |
|---|---|---|
| 2 | 1% | 47.0*¹ |
| 3 | 1% | 25.1*¹ |
| 9 | 5% | 39.1*¹ |
| 12 | 1% | 66.8*¹ |
| 15 | 5% | 64.0*¹ |
| 23 | 5% | 47.9*¹ |
| 26 | 1% | 71.9*¹ |
| 82 | 1% | 59.8*² |
| 90 | 5% | 54.3*¹ |
| 163 | 5% | 37.2*² |
| 164 | 1% | 42.2*¹ |
| 165 | 1% | 52.3*¹ |
| 173 | 5% | 65.4*¹ |
| 187 | 5% | 59.4*¹ |
| 198 | 1% | 24.7*¹ |
| 214 | 5% | 55.3*¹ |
| 216 | 5% | 41.2*¹ |
| 226 | 1% | 26.8*¹ |
| 227 | 1% | 25.6*¹ |
| 231 | 5% | 66.1*¹ |
| 233 | 1% | 59.5*¹ |
| 238 | 5% | 77.9*¹ |
| 246 | 5% | 22.8*¹ |
| 257 | 5% | 51.1*¹ |
| 260 | 5% | 42.9*¹ |
| 268 | 5% | 53.9*¹ |
| 272 | 5% | 40.5*¹ |
| 277 | 5% | 60.9*¹ |
| 283 | 5% | 38.8*¹ |

| | | |
|---|---|---|
| *1 : A dosage of a test substance is 1mg / kg. *2 : A dosage of a test substance is 3 mg / kg. | | |

### Test Example 3: Action against pain-related behavior and micturition cycle in mice with cyclophosphamide (CP) - induced acute cystitis

An action of a test substance against visceral pain and frequent urination associated with cystitis, which was induced by intraperitoneal administration (20 mL/kg) of CP (300 mg/kg) to 4 to 5-week-old male Slc:ICR mice (available from Japan SLC, Inc.), was investigated. This experiment was performed according to a method described in European Journal of Pharmacology, 676 (2012), pp.75-80.

Three hours after the administration of CP, a test substance (1 mg/kg or 3 mg/kg) was intravenously administered, and observation by visual inspection and video recording were performed for 15 minutes immediately thereafter. In the observation by visual inspection, four pain-related behaviors (eye opening, rounded back, leg dragging, and behavior during urination) were scored, each rated on a scale of 0-10, according to the following criteria.
- Eye opening (normal: 0 point, sometimes closed or half-open eyes: 5 points, completely closed: 10 points)
- Rounded back (normal: 0 point, sometimes crouched: 5 points, crouched at almost all times: 10 points)
- Leg dragging (normal: 0 point, leg dragging: 10 points)
- Behavior during urination (normal: 0 point,shaking hip in micturition: 10 points)

In addition, the video was played back, and the frequency of licking behavior around the abdomen as a pain-related behavior was scored, rated on a scale of 0-10, according to the following criteria. - Licking behavior around the abdomen (0 to 3 times: 0 point, 4 times to 10 times: 5 points, 11 times or more: 10 points)

Furthermore, the video was played back, and the frequency of urination (urination marks) on a filter paper was counted and taken as an index for micturition cycle.

An average value of the pain-related behavior score of each group (total score of the five items: 50 points at maximum) and an average value of the frequency of urination (urination marks) on the filter paper were determined.

The strength of an analgesic action of the test substance against the CP-induced visceral pain was evaluated in terms of percent inhibition (%) of pain-related behavior score which is calculated as {(A - B)/A x 100}, wherein A is an average value of the pain-related behavior score of a medium-administered group, and B is an average value of the pain-related behavior score of a test substance-administered group.

In addition, the strength of a frequent urination inhibitory action of the test substance against the CP-induced frequent urination was evaluated in terms of percent inhibition (%) of the frequency of urination which is calculated as {(C - D)/C x 100}, wherein C is an average value of the frequency of urination of a medium-administered group, and D is an average value of the frequency of urination of a test substance-administered group. The obtained results are shown in Table 4.

**[Table 4]**

| Test substance (Example No.) | Percent inhibition of pain-related behavior score (%) | Percent inhibition of the frequency of the frequency urination (%) |
|---|---|---|
| 9 | 23.9*¹ | 36.3*¹ |
| 26 | 60.5*² | 57.3*² |
| 36 | 40.4*² | 31.3*² |
| 43 | 31.0*² | 41.9*² |
| 45 | 34.1*² | 33.8*² |
| 46 | 22.0*¹ | 31.5*¹ |
| 143 | 52.2*² | 47.7*² |
| 147 | 38.8*² | 21.1*² |
| 148 | 45.7*² | 31.5*² |
| 173 | 63.3*² | 52.6*² |
| 182 | 26.0*² | 43.2*² |
| 183 | 37.0*¹ | 22.7*¹ |
| 187 | 22.9*¹ | 51.6*¹ |
| 191 | 20.5*² | 36.5*² |
| 198 | 40.4*² | 37.6*² |
| 214 | 44.2*² | 25.3*² |
| 216 | 28.2*² | 30.1*² |
| 217 | 38.3*² | 40.4*² |
| 255 | 31.0*² | 26.4*² |
| 277 | 40.4*² | 24.8*² |

| | | |
|---|---|---|
| *1 : A dosage of a test substance is 1mg / kg. *2 : A dosage of a test substance is 3 mg / kg. | | |

### Formulation Example 1

### Tablet (oral tablet)

In an 80 mg tablet of the formulation:
Compound of the invention of Example 1: 5.0 mg
Corn starch: 46.6 mg
Crystalline cellulose: 24.0 mg
Methyl cellulose: 4.0 mg
Magnesium stearate: 0.4 mg

A mixed powder of this proportion is press-molded to form an oral tablet by an ordinary method.

### [Industrial applicability]

In view of the fact that the compound of the present invention or a pharmaceutically acceptable salt thereof exhibits mGluR5 inhibitory activity, in particular, it is useful as a preventive agent or therapeutic agent for pain (for example, acute pain, chronic pain, inflammatory pain, neuropathic pain, hyperalgesia, thermal hyperalgesia, allodynia, pain by noxious thermal stimulation, pain by noxious mechanical stimulation, pain in the lower urinary tract or reproductive organs or migraine), pruritus, a lower urinary tract symptom or lower urinary tract dysfunction, a lower urinary tract symptom or lower urinary tract dysfunction, gastroesophageal reflux disease (GERD), gastroesophageal reflux disease associated with transient lower esophageal sphincter relaxation (TLESR), or central nervous system disease.

## Claims

1. A pyridine derivative represented by the following general formula [1] or a pharmaceutically acceptable salt thereof: 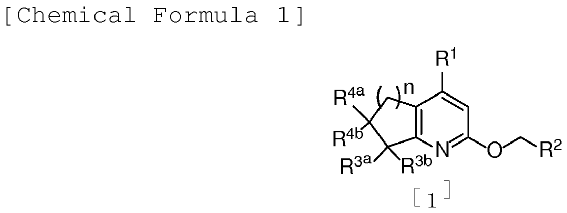 [wherein
R¹ represents phenyl, benzo[d][1,3]dioxolyl or heteroaryl;
the heteroaryl relative to R¹ is bound through a carbon atom on the heteroaryl ring;
the phenyl, benzo[d][1,3]dioxolyl or heteroaryl relative to R¹ may be substituted at a substitutable arbitrary position (s) with one to three same or different gruops selected from the group consisting of
(i) halogen,
(ii) cyano,
(iii) hydroxy,
(iv) nitro,
(v) alkoxy,
(vi) cycloalkyl,
(vii) alkylsulfonate,
(viii) alkyl,
(ix) hydroxyalkyl,
(x) alkoxyalkyl,
(xi) monohalogenoalkyl,
(xii) dihalogenoalkyl,
(xiii) trihalogenoalkyl,
(xiv) amino which may be substituted at a substitutable arbitrary position (s) with one or two same or different groups selected from the group consisting of alkyl, aminoalkyl, alkoxycarbonylaminoalkyl, monoalkylaminoalkyl, dialkylaminoalkyl, and alkylsulfonyl,
(xv) saturated cyclic amino which may be substituted at a substitutable arbitrary position(s) with one or two oxos,
(xvi) alkylcarbonyl,
(xvii) alkoxycarbonyl,
(xviii) hydroxycarbonyl,
(xix) carbamoyl which may be substituted at a substitutable arbitrary position(s) with one or two same or different groups selected from the group consisting of alkyl, cycloalkyl, and (cycloalkyl)alkyl and
(xx) a group represented by the following general formula [2] : 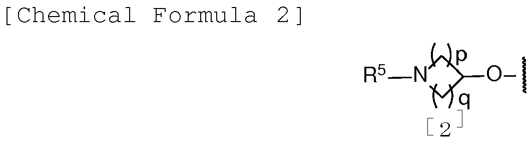 (wherein R⁵ represents hydrogen, alkyl or alkylcarbonyl; p and q may be the same as or different from each other and each represents 1 or 2); and
when it contains a nitrogen atom as the ring constituting atom in the heteroaryl relative to R¹, an oxygen atom may coordinate to the nitrogen atom;
R² represents phenyl or heteroaryl and is bound through a carbon atom on the ring;
the phenyl or heteroaryl relative to R² may be substituted at a substitutable arbitrary position(s) with one or three same or different groups selected from the group consisting of cyano, halogen, cycloalkyl, alkoxy, carbamoyl, alkenyl, alkyl, hydroxyalkyl, alkoxyalkyl, monohalogenoalkyl, dihalogenoalkyl, trihalogenoalkyl, amino, monoalkylamino and dialkylamino; and
when it contains a nitrogen atom as the ring constituting atom in the heteroaryl relative to R², an oxygen atom may coordinate to the nitrogen atom;
R^{3a} represents hydrogen, and R^{3b} represents hydrogen, alkyl, hydroxy, halogen, alkoxy or alkylcarbonyloxy, or R^{3a} and R^{3b} taken together with the adjacent carbon atom represent a group represented by the following general formula [3] or [4]: 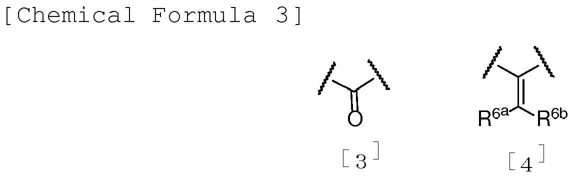 (wherein R^{6a} and R^{6b} may be the same as or different from each other and each represents hydrogen or alkyl);
R^{4a} and R^{4b} may be the same as or different from each other and each represents hydrogen or alkyl; and
n represents an integer of 1 to 3.]

2. The pyridine derivative or a pharmaceutically acceptable salt thereof according to claim 1, wherein R¹ is phenyl or heteroaryl.

3. The pyridine derivative or a pharmaceutically acceptable salt thereof according to claim 1, wherein R¹ is phenyl, pyridyl, pyrimidyl, pyrazinyl or pyridazinyl.

4. The pyridine derivative or a pharmaceutically acceptable salt thereof according to claim 1, wherein R¹ is phenyl, pyridyl, pyrimidyl, pyrazinyl or pyridazinyl, and R¹ may be substituted with one or two gruoups selected from the group consisting of alkyl, halogen, cyano, amino, monoalkylamino, dialkylamino, hydroxyalkyl and alkylsulfonate.

5. The pyridine derivative or a pharmaceutically acceptable salt thereof according to claim 1, wherein R² is phenyl, pyridyl, pyrazinyl, pyrimidyl, oxazolyl, imidazolyl or thiazolyl.

6. The pyridine derivative or a pharmaceutically acceptable salt thereof according to claim 1, wherein R² is phenyl, pyridyl, pyrazinyl, pyrimidyl, oxazolyl, imidazolyl or thiazolyl, and R² may be substituted with one or two groups selected from the group consisting of alkyl, hydroxyalkyl, alkoxyalkyl, halogen and cyano.

7. The pyridine derivative or a pharmaceutically acceptable salt thereof according to claim 1, wherein R^{3a} is hydrogen, and R^{3b} is hydrogen, alkyl, halogen, hydroxy or alkoxy.

8. The pyridine derivative or a pharmaceutically acceptable salt thereof according to claim 1, wherein R^{3a}, R^{3b}, R^{4a} and R^{4b} are each hydrogen.

9. The pyridine derivative or a pharmaceutically acceptable salt thereof according to claim 1, wherein R¹ is phenyl, pyridyl, pyrimidyl, pyrazinyl or pyridazinyl; R² is phenyl, pyridyl, pyrazinyl, pyrimidyl, oxazolyl, imidazolyl or thiazolyl; and R^{3a}, R^{3b}, R^{4a} and R^{4b} are each hydrogen.

10. The pyridine derivative or a pharmaceutically acceptable salt thereof according to claim 1, wherein R¹ is phenyl, pyridyl, pyrimidyl, pyrazinyl or pyridazinyl, and R¹ may be substituted with one or two gruops selected from the group consisting of alkyl, halogen, cyano, amino, monoalkylamino, dialkylamino, hydroxyalkyl and alkylsulfonate; R² is phenyl, pyridyl, pyrazinyl, pyrimidyl, oxazolyl, imidazolyl or thiazolyl, and R² may be substituted with one or two groups selected from the group consisting of alkyl, hydroxyalkyl, alkoxyalkyl, halogen and cyano; and R^{3a}, R^{3b}, R^{4a} and R^{4b} are each hydrogen.

11. A pyridine derivative according to any one of the following (1) to (95):
(1)
4-(pyridin-3-yl)-2-(pyridin-2-ylmethoxy)-6,7-dihydr o-5H-cyclopenta[b]pyridine,
(2)
3-[2-(pyridin-2-ylmethoxy)-6,7-dihydro-5H-cyclopent a[b]pyridin-4-yl]pyridine-2-carbonitrile,
(3)
5-[2-(pyridin-2-ylmethoxy)-6,7-dihydro-5H-cyclopent a[b]pyridin-4-yl]pyridin-3-yl methanesulfonate,
(4)
5-{2-[(5-fluoropyridin-2-yl)methoxy]-6,7-dihydro-5H -cyclopenta[b]pyridin-4-yl}pyridine-2-carbonitrile,
(5)
2-(benzyloxy)-4-(pyrimidin-5-yl)-6,7-dihydro-5H-cyc lopenta[b]pyridine,
(6)
3-({[4-(pyrimidin-5-yl)-6,7-dihydro-5H-cyclopenta[b ]pyridin-2-yl]oxy}methyl)benzonitrile,
(7)
2-[(3-fluoropyridin-2-yl)methoxy]-4-(pyrimidin-5-yl )-6,7-dihydro-5H-cyclopenta[b]pyridine,
(8)
2-[(3,5-difluoropyridin-2-yl)methoxy]-4-(pyrimidin-5-yl)-6,7-dihydro-5H-cyclopenta[b]pyridine,
(9)
2-[(6-methylpyridin-3-yl)methoxy]-4-(pyrimidin-5-yl )-6,7-dihydro-5H-cyclopenta[b]pyridine,
(10)
4-(2-methylpyrimidin-5-yl)-2-(pyridin-2-ylmethoxy)-6,7-dihydro-5H-cyclopenta[b]pyridine,
(11)
5-[2-(pyridin-2-ylmethoxy)-6,7-dihydro-5H-cyclopent a[b]pyridin-4-yl]pyrimidine-2-carbonitrile,
(12)
2-[(3-fluoropyridin-2-yl)methoxy]-4-(2-methylpyrimi din-5-yl)-6,7-dihydro-5H-cyclopenta[b]pyridine,
(13)
2-[(3,5-difluoropyridin-2-yl)methoxy]-4-(2-methylpy rimidin-5-yl)-6,7-dihydro-5H-cyclopenta[b]pyridine,
(14)
2-[(3,6-difluoropyridin-2-yl)methoxy]-4-(2-methylpy rimidin-5-yl)-6,7-dihydro-5H-cyclopenta[b]pyridine,
(15)
5-{2-[(3,5-difluoropyridin-2-yl)methoxy]-6,7-dihydr o-5H-cyclopenta[b]pyridin-4-yl}pyrimidine-2-carboni trile,
(16)
2-[(6-methylpyridin-2-yl)methoxy]-4-(2-methylpyrimi din-5-yl)-6,7-dihydro-5H-cyclopenta[b]pyridine,
(17)
6-({[4-(2-methylpyrimidin-5-yl)-6,7-dihydro-5H-cycl openta[b]pyridin-2-yl]oxy}methyl)pyridine-2-carboni trile,
(18)
6-({[4-(2-methylpyrimidin-5-yl)-6,7-dihydro-5H-cycl openta[b]pyridin-2-yl]oxy}methyl)pyridine-3-carboni trile,
(19)
5-{2-[(3-fluoropyridin-2-yl)methoxy]-6,7-dihydro-5H -cyclopenta[b]pyridin-4-yl}pyrimidine-2-carbonitril e,
(20)
2-fluoro-4-[2-(pyridin-2-ylmethoxy)-6,7-dihydro-5H-cyclopenta[b]pyridin-4-yl]benzonitrile,
(21)
3-fluoro-5-[2-(pyridin-2-ylmethoxy)-6,7-dihydro-5H-cyclopenta[b]pyridin-4-yl]phenyl methanesulfonate,
(22)
4-(pyridin-3-yl)-2-(pyrimidin-2-ylmethoxy)-5,6,7,8-tetrahydroquinoline,
(23)
2-[(4-methyl-1,3-oxazol-2-yl)methoxy]-4-(pyridin-3-yl)-5,6,7,8-tetrahydroquinoline,
(24)
4-(pyridin-3-yl)-2-(1,3-thiazol-2-ylmethoxy)-5,6,7, 8-tetrahydroquinoline,
(25)
2-[(1-methyl-1H-imidazol-2-yl)methoxy]-4-(pyridin-3 -yl)-5,6,7,8-tetrahydroquinoline,
(26)
4-(4-methylpyridin-3-yl)-2-(pyridin-2-ylmethoxy)-5, 6,7,8-tetrahydroquinoline,
(27)
5-[2-(pyridin-2-ylmethoxy)-5,6,7,8-tetrahydroquinol in-4-yl]pyridine-2-carbonitrile,
(28)
{5-[2-(pyridin-2-ylmethoxy)-5,6,7,8-tetrahydroquino lin-4-yl]pyridin-3-yl}methanol,
(29)
N-methyl-5-[2-(pyridin-2-ylmethoxy)-5,6,7,8-tetrahy droquinolin-4-yl]pyridin-3-amine,
(30)
N,N-dimethyl-5-[2-(pyridin-2-ylmethoxy)-5,6,7,8-tet rahydroquinolin-4-yl]pyridin-3-amine,
(31)
5-{2-[(3-fluoropyridin-2-yl)methoxy]-5,6,7,8-tetrah ydroquinolin-4-yl}pyridine-2-carbonitrile,
(32)
2-(pyridin-2-ylmethoxy)-4-(pyrimidin-5-yl)-5,6,7,8-tetrahydroquinoline,
(33)
2-[(3-fluoropyridin-2-yl)methoxy]-4-(pyrimidin-5-yl )-5,6,7,8-tetrahydroquinoline,
(34)
2-[(3,6-difluoropyridin-2-yl)methoxy]-4-(pyrimidin-5-yl)-5,6,7,8-tetrahydroquinoline,
(35)
6-({[4-(pyrimidin-5-yl)-5,6,7,8-tetrahydroquinolin-2-yl]oxy}methyl)pyridine-2-carbontrile,
(36)
2-[(6-methylpyridin-2-yl)methoxy]-4-(pyrimidin-5-yl )-5,6,7,8-tetrahydroquinoline,
(37)
[6-({[4-(pyrimidin-5-yl)-5,6,7,8-tetrahydroquinolin -2-yl]oxy}methyl)pyridin-2-yl]methanol,
(38)
5-[2-(pyridin-2-ylmethoxy)-5,6,7,8-tetrahydroquinol in-4-yl]pyrimidin-2-amine,
(39)
5-[2-(pyridin-2-ylmethoxy)-5,6,7,8-tetrahydroquinol in-4-yl]pyrimidine-2-carbonitrile,
(40)
2-[(6-methylpyridin-2-yl)methoxy]-4-(2-methylpyrimi din-5-yl)-5,6,7,8-tetrahydroquinoline,
(41)
6-({[4-(2-methylpyrimidin-5-yl)-5,6,7,8-tetrahydroq uinolin-2-yl]oxy}methyl)pyridine-2-carbonitrile,
(42)
6-({[4-(2-methylpyrimidin-5-yl)-5,6,7,8-tetrahydroq uinolin-2-yl]oxy}methyl)pyridine-3-carbonitrile,
(43)
2-[(5-fluoropyridin-2-yl)methoxy]-4-(2-methylpyrimi din-5-yl)-5,6,7,8-tetrahydroquinoline,
(44)
2-[(3-fluoropyridin-2-yl)methoxy]-4-(2-methylpyrimi din-5-yl)-5,6,7,8-tetrahydroquinoline,
(45)
2-[(3,5-difluoropyridin-2-yl)methoxy]-4-(2-methylpy rimidin-5-yl)-5,6,7,8-tetrahydroquinoline,
(46)
5-{2-[(3-fluoropyridin-2-yl)methoxy]-5,6,7,8-tetrah ydroquinolin-4-yl}pyrimidin-2-amine,
(47)
3-[2-(pyridin-2-ylmethoxy)-5,6,7,8-tetrahydroquinol in-4-yl]pyrazine-2-carbonitrile,
(48)
(6-{2-[(5-fluoropyridin-2-yl)methoxy]-5,6,7,8-tetra hydroquinolin-4-yl}pyrazin-2-yl)methanol,
(49)
(6-{2-[(3-fluoropyridin-2-yl)methoxy]-5,6,7,8-tetra hydroquinolin-4-yl}pyrazin-2-yl)methanol,
(50)
7-ethyl-2-(pyridin-2-ylmethoxy)-4-(pyrimidin-5-yl)-6,7-dihydro-5H-cyclopenta[b]pyridine,
(51)
3-({[4-(pyridin-3-yl)-6,7-dihydro-5H-cyclopenta[b]p yridin-2-yl]oxy}methyl)benzonitrile,
(52)
5-{2-[(3,5-difluoropyridin-2-yl)methoxy]-6,7-dihydr o-5H-cyclopenta[b]pyridin-4-yl}pyridine-2-carbonitr ile,
(53)
4-[({4-[5-(hydroxymethyl)pyridin-3-yl]-6,7-dihydro-5H-cyclopenta[b]pyridin-2-yl}oxy)methyl]benzonitril e,
(54)
(5-{2-[(4-fluorobenzyl)oxy]-6,7-dihydro-5H-cyclopen ta[b]pyridin-4-yl}pyridin-3-yl)methanol,
(55)
(5-{2-[(3-fluorobenzyl)oxy]-6,7-dihydro-5H-cyclopen ta[b]pyridin-4-yl}pyridin-3-yl)methanol,
(56)
6-({[4-(5-fluoropyridin-3-yl)-6,7-dihydro-5H-cyclop enta[b]pyridin-2-yl]oxy}methyl)pyridine-3-carbonitr ile,
(57)
3-({[4-(2-fluoropyridin-4-yl)-6,7-dihydro-5H-cyclop enta[b]pyridin-2-yl]oxy}methyl)benzonitrile,
(58)
6-({[4-(2-fluoropyridin-4-yl)-6,7-dihydro-5H-cyclop enta[b]pyridin-2-yl]oxy}methyl)pyridine-3-carbonitr ile,
(59)
3-[({4-[5-(hydroxymethyl)pyridin-3-yl]-6,7-dihydro-5H-cyclopenta[b]pyridin-2-yl}oxy)methyl]benzonitril e,
(60)
6-({[4-(pyrimidin-5-yl)-6,7-dihydro-5H-cyclopenta[b ]pyridin-2-yl]oxy}methyl)pyridine-3-carbonitrile,
(61)
2-[(3,6-difluoropyridin-2-yl)methoxy]-4-(pyrimidin-5-yl)-6,7-dihydro-5H-cyclopenta[b]pyridine,
(62)
2-[(2-methylpyridin-4-yl)methoxy]-4-(pyrimidin-5-yl )-6,7-dihydro-5H-cyclopenta[b]pyridine,
(63)
2-[(4-fluorobenzyl)oxy]-4-(pyrimidin-5-yl)-6,7-dihy dro-5H-cyclopenta[b]pyridine,
(64)
4-({[4-(pyrimidin-5-yl)-6,7-dihydro-5H-cyclopenta[b ]pyridin-2-yl]oxy}methyl)benzonitrile,
(65)
5-[2-(pyridin-2-ylmethoxy)-6,7-dihydro-5H-cyclopent a[b]pyridin-4-yl]pyrimidin-2-amine,
(66)
2-[(4-fluorobenzyl)oxy]-4-(2-methylpyrimidin-5-yl)-6,7-dihydro-5H-cyclopenta[b]pyridine,
(67)
4-({[4-(2-methylpyrimidin-5-yl)-6,7-dihydro-5H-cycl openta[b]pyridin-2-yl]oxy}methyl)benzonitrile,
(68)
2-[(2-methylpyridin-4-yl)methoxy]-4-(2-methylpyrimi din-5-yl)-6,7-dihydro-5H-cyclopenta[b]pyridine,
(69)
5-{2-[(3-fluorobenzyl)oxy]-6,7-dihydro-5H-cyclopent a[b]pyridin-4-yl}pyrimidine-2-amine,
(70)
4-({[4-(2-aminopyrimidin-5-yl)-6,7-dihydro-5H-cyclo penta[b]pyridin-2-yl]oxy}methyl)benzonitrile,
(71)
3-({[4-(2-aminopyrimidin-5-yl)-6,7-dihydro-5H-cyclo penta[b]pyridin-2-yl]oxy}methyl)benzonitrile,
(72)
2-[(3-fluorobenzyl)oxy]-4-(2-methylpyrimidin-5-yl)-6,7-dihydro-5H-cyclopenta[b]pyridine,
(73)
2-({[4-(2-methylpyrimidin-5-yl)-6,7-dihydro-5H-cycl openta[b]pyridin-2-yl]oxy}methyl)-1,3-oxazole-4-car bonitrile,
(74)
{6-[2-(pyridin-2-ylmethoxy)-6,7-dihydro-5H-cyclopen ta[b]pyridin-4-yl]pyrazin-2-yl}methanol,
(75)
(6-{2-[(5-fluoropyridin-2-yl)methoxy]-6,7-dihydro-5 H-cyclopenta[b]pyridin-4-yl}pyrazin-2-yl)methanol,
(76)
3-[({4-[6-(hydroxymethyl)pyrazin-2-yl]-6,7-dihydro-5H-cyclopenta[b]pyridin-2-yl}oxy)methyl]benzonitril e,
(77)
4-(pyridazin-3-yl)-2-(pyridin-2-ylmethoxy)-6,7-dihy dro-5H-cyclopenta[b]pyridine,
(78)
2-[(3-fluoropyridin-2-yl)methoxy]-4-(pyridazin-3-yl )-6,7-dihydro-5H-cyclopenta[b]pyridine,
(79)
4-({[4-(pyridazin-3-yl)-6,7-dihydro-5H-cyclopenta[b ]pyridin-2-yl]oxy}methyl)benzonitrile,
(80)
6-({[4-(1-methyl-1H-pyrazol-5-yl)-6,7-dihydro-5H-cy clopenta[b]pyridin-2-yl]oxy}methyl)pyridine-3-carbo nitrile,
(81)
6-({[4-(pyridazin-3-yl)-6,7-dihydro-5H-cyclopenta[b ]pyridin-2-yl]oxy}methyl)pyridine-3-carbonitrile,
(82)
6-[({4-[5-(hydroxymethyl)pyridin-3-yl]-5,6,7,8-tetr ahydroquinolin-2-yl}oxy)methyl]pyridine-3-carbonitr ile,
(83)
2-[(3,5-difluoropyridin-2-yl)methoxy]-4-(pyrimidin-5-yl)-5,6,7,8-tetrahydroquinoline,
(84)
4-({[4-(pyrimidin-5-yl)-5,6,7,8-tetrahydroquinolin-2-yl]oxy}methyl)benzonitrile,
(85)
3-({[4-(pyrimidin-5-yl)-5,6,7,8-tetrahydroquinolin-2-yl]oxy}methyl)benzonitrile,
(86)
2-[(4-fluorobenzyl)oxy]-4-(pyrimidin-5-yl)-5,6,7,8 - tetrahydroquinoline,
(87)
2-[(3-fluorobenzyl)oxy]-4-(pyrimidin-5-yl)-5,6,7,8 - tetrahydroquinoline,
(88)
4-({[4-(pyrimidin-5-yl)-5,6,7,8-tetrahydroquinolin-2-yl]oxy}methyl)pyridine-2-carbonitrile,
(89)
5-{2-[(6-fluoropyridin-2-yl)methoxy]-5,6,7,8-tetrah ydroquinolin-4-yl}pyrimidin-2-amine,
(90)
3-({[4-(2-methylpyrimidin-5-yl)-5,6,7,8-tetrahydroq uinolin-2-yl]oxy}methyl)benzonitrile,
(91)
4-({[4-(2-methylpyrimidin-5-yl)-5,6,7,8-tetrahydroq uinolin-2-yl]oxy}methyl)benzonitrile,
(92)
2-[(2-methylpyridin-4-yl)methoxy]-4-(2-methylpyrimi din-5-yl)-5,6,7,8-tetrahydroquinoline,
(93)
4-({[4-(2-methylpyrimidin-5-yl)-5,6,7,8-tetrahydroq uinolin-2-yl]oxy}methyl)pyridine-2-carbonitrile,
(94)
6-[({4-[6-(hydroxymethyl)pyrazin-2-yl]-5,6,7,8-tetr ahydroquinolin-2-yl}oxy)methyl]pyridine-3-carbonitr ile and
(95)
4-(pyridazin-3-yl)-2-(pyridin-2-ylmethoxy)-5,6,7,8-tetrahydroquinoline;
or a pharmaceutically acceptable salt thereof.

12. A pharmaceutical composition comprising the pyridine derivative or a pharmaceutically acceptable salt thereof according to any one of claims 1 to 11 as an active ingredient.

13. An mGluR5 inhibitor comprising the pyridine derivative or a pharmaceutically acceptable salt thereof according to any one of claims 1 to 11 as an active ingredient.

14. A preventive agent or therapeutic agent for pain, comprising the pyridine derivative or a pharmaceutically acceptable salt thereof according to any one of claims 1 to 11 as an active ingredient.

15. The preventive agent or therapeutic agent according to claim 14, wherein the pain is acute pain, chronic pain, inflammatory pain, neuropathic pain, hyperalgesia, thermal hyperalgesia, allodynia, pain by noxious thermal stimulation, pain by noxious mechanical stimulation, pain in the lower urinary tract or reproductive organs or migraine.

16. The preventive agent or therapeutic agent according to claim 15, wherein the pain is acute pain.

17. The preventive agent or therapeutic agent according to claim 16, wherein the acute pain is herpetic pain, pain after tooth extraction or postoperative pain.

18. The preventive agent or therapeutic agent according to claim 15, wherein the pain is chronic pain.

19. The preventive agent or therapeutic agent according to claim 18, wherein the chronic pain is cancer pain, pain associated with fibromyalgia, pain in complex regional pain syndrome or pain in postoperative scar pain syndrome.

20. The preventive agent or therapeutic agent according to claim 15, wherein the pain is inflammatory pain.

21. The preventive agent or therapeutic agent according to claim 20, wherein the inflammatory pain is pain associated with rheumatoid arthritis or pain associated with osteoarthritis.

22. The preventive agent or therapeutic agent according to claim 15, wherein the pain is neuropathic pain.

23. The preventive agent or therapeutic agent according to claim 22, wherein the neuropathic pain is trigeminal neuralgia, postherpetic neuralgia, pain associated with painful diabetic neuropathy or pain associated with carcinomatous neuropathy.

24. The preventive agent or therapeutic agent according to claim 15, wherein the pain is pain in the lower urinary tract or reproductive organs.

25. The preventive agent or therapeutic agent according to claim 24, wherein the pain in the lower urinary tract or reproductive organs is pain associated with bacterial cystitis, pain associated with interstitial cystitis or pain associated with pelvic pain syndrome.

26. A preventive agent or therapeutic agent for pruritus, comprising the pyridine derivative or a pharmaceutically acceptable salt thereof according to any one of claims 1 to 11 as an active ingredient.

27. A preventive agent or therapeutic agent for a lower urinary tract symptom or lower urinary tract dysfunction, comprising the pyridine derivative or a pharmaceutically acceptable salt thereof according to any one of claims 1 to 11 as an active ingredient.

28. The preventive agent or therapeutic agent according to claim 27, wherein the pain in the lower urinary tract symptom or lower urinary tract dysfunction is frequent urination.

29. The preventive agent or therapeutic agent according to any one of claims 27 or 28, wherein a disease accompanied by lower urinary tract symptom or lower urinary tract dysfunction is overactive bladder, bacterial cystitis, interstitial cystitis, or pelvic pain syndrome.

30. A preventive agent or therapeutic agent for gastroesophageal reflux disease (GERD) or gastroesophageal reflux disease associated with transient lower esophageal sphincter relaxation (TLESR), comprising the pyridine derivative or a pharmaceutically acceptable salt thereof according to any one of claims 1 to 11 as an active ingredient.

31. A preventive agent or therapeutic agent for central nervous system disease, comprising the pyridine derivative or a pharmaceutically acceptable salt thereof according to any one of claims 1 to 11 as an active ingredient.

32. The preventive agent or therapeutic agent according to claim 31, wherein the central nervous system disease is L-dopa-induced dyskinesia, psychosis, anxiety, depression, Alzheimer's disease, fragile X syndrome, Parkinson's disease, Huntington's disease, morphine tolerance, alcohol dependence or food addiction.
